# EUROPEAN PATENT APPLICATION

(11) **EP 4 317 418 A1**
(43) Date of publication of application: **07.02.2024**
(21) Application number: 22781264.1
(22) Date of filing: 31.03.2022
(51) Int. Cl.: C12N 5/0735

(54) **METHOD FOR PRODUCING OVARIAN SOMATIC CELL-LIKE CELLS, AND METHOD FOR INDUCING DIFFERENTIATION OF PRIMATE PLURIPOTENT STEM CELLS INTO OVARIAN SOMATIC CELL-LIKE CELLS**

(30) Priority: 31.03.2021 US 202163168263 P
(71) Applicant: Kyoto University, Kyoto-shi, Kyoto 606-8501 (JP); Kyushu University, National University Corporation, Nishi-ku Fukuoka-shi Fukuoka 819-0395 (JP)
(72) Inventor: SAITOU, Mitinori, Kyoto-shi, Kyoto 606-8501 (JP); KOJIMA, Yoji, Kyoto-shi, Kyoto 606-8501 (JP); KAWASAKI, Masanori, Kyoto-shi, Kyoto 606-8501 (JP); HAYASHI, Katsuhiko, Fukuoka-shi, Fukuoka 819-0395 (JP); MATSUOKA, Hideki, Kyoto-shi, Kyoto 606-8501 (JP)
(74) Representative: Cabinet Becker et Associés
(86) International application number: PCT/JP2022/016658
(87) International publication number: WO 2022/211054

(57) **Abstract**

A method for producing ovarian somatic cell-like cells includes a step 1 of culturing primate pluripotent stem cells in a medium including a GSK3 inhibitor and a ROCK inhibitor; a step 2 of culturing the cells from the step 1 in a medium including BMP4, retinoic acid, and an MEK inhibitor; and a step 3 of culturing the cells from the step 2 in a base medium to obtain ovarian somatic cell-like cells. A method for inducing the differentiation of primate pluripotent stem cells into ovarian somatic cell-like cells includes inducing the differentiation of primate pluripotent stem cells into ovarian somatic cell-like cells using the method for producing ovarian somatic cell-like cells.

## Description

### [Technical Field]

The present invention relates to a method for producing ovarian somatic cell-like cells and a method for inducing differentiation of primate pluripotent stem cells into ovarian somatic cell-like cells.

Priority is claimed on United States Patent No. 63/168,263, filed March 31, 2021, the content of which is incorporated herein by reference.

### [Background Art]

A germ cell is a cell of which the origin is a primordial germ cell, which differentiates into a sperm or an egg, and forms a new individual by fusion thereof. On the other hand, the ovary, which is a reproductive organ that performs generation, maturation, and ovulation of eggs, is derived from an intermediate mesoderm formed after gastrulation and differentiated into one layer of coelomic epithelium, and then a part thereof is proliferated and thickened to form a genital ridge. Then, in a case where a predetermined period elapses after fertilization, the cells receive more sex-specific stimuli, and differentiation thereof into granulosa cells or stromal cells that constitute the ovary proceeds.

It has been reported that PGC-like cells (PGCLCs) derived from mouse primordial germ cells (PGCs) and pluripotent stem cells can be aggregate-cultured in vitro with mouse fetal gonadal somatic cells to be differentiated into functional sperm or eggs that can contribute to offspring (see, for example, Patent Document 1). However, in this method, while the eggs are matured by in vitro culture, it is necessary to collect ovarian somatic cells (follicles) that grow the eggs from the living body. Accordingly, it is difficult to apply the transplant to various animals including humans from which ovarian tissue is difficult to collect.

On the other hand, the present inventors have reported a method for inducing differentiation of mouse pluripotent stem cells into ovarian somatic cell-like cells (see, for example, Non-Patent Document 1).

In a case where the method described in Patent Document 1 is used, human or cynomolgus monkey PGCLCs do not start meiosis as in germ cells of a living body even in a case where aggregation culture with ovarian somatic cells of a mouse fetus is continued for a long period of time of several months. A main cause thereof is considered to be because the differentiation and maturation of the germ cells, which take several months, are not synchronized with maturation of mouse gonadal somatic cells, which proceeds for several days, and the secretion factors differ depending on the species.

In addition, even in a case where an attempt is made to obtain ovarian somatic cell-like cells using pluripotent stem cells of primates such as humans by utilizing the method described in Non-Patent Document 1, the time and the factors required for maturation of the gonadal somatic cells are different in primates such as mice and humans. Therefore, the differentiation into ovarian somatic cell-like cells cannot be induced.

### [Citation List]

### [Patent Document]

[Patent Document 1]
PCT International Publication No. WO 2017/047799

### [Non-Patent Document]

[Non-Patent Document 1]
Yoshino T et al., "Generation of ovarian follicles from mouse pluripotent stem cells.", Science, Vol. 373, No. 6552, p. eabe0237, 2021.

### [Summary of Invention]

### [Technical Problem]

The present invention has been made in consideration of the circumstances, and thus, provides a method for producing ovarian somatic cell-like cells by which ovarian somatic cell-like cells at any developmental stage can be efficiently obtained from primate pluripotent stem cells, and a method for inducing differentiation of primate pluripotent stem cells into ovarian somatic cell-like cells.

### [Solution to Problem]

The present inventors have conducted repeated intensive studies to achieve the object, and as a result, they have found that primate pluripotent stem cells can be induced to be differentiated into FOXL2-positive ovarian somatic cell-like cells by culturing the primate pluripotent stem cells in the presence of a GSK3 inhibitor and a ROCK inhibitor to induce the cells into the mesoderm, then culturing the cells in the presence of BMP4, retinoic acid, and an MEK inhibitor to induce the cells into the intermediate mesoderm that expresses OSR1 or WT1, and further continuing to culture the cells in a base medium, thereby leading to completion of the present invention.

That is, the present invention includes the following aspects.
(1) A method for producing ovarian somatic cell-like cells, the production method including:
   a step 1 of culturing primate pluripotent stem cells in a medium including a GSK3 inhibitor and a ROCK inhibitor;
   a step 2 of culturing the cells from the step 1 in a medium including BMP4, retinoic acid, and an MEK inhibitor; and
   a step 3 of culturing the cells from the step 2 in a base medium to obtain ovarian somatic cell-like cells.
(2) The production method according to (1),
   in which the culture of the step 1 is performed for 3 days or longer.
(3) The production method according to (1) of (2),
   in which the culture of the step 2 is performed for 3 days or longer.
(4) The production method according to any one of (1) to (3),
   in which the culture of the step 3 is performed for 1 week or more.
(5) The production method according to any one of (1) to (4),
   in which the medium in the step 1 further includes BMP4.
(6) The production method according to any one of (1) to (5),
   in which the step 1 includes
   a step 1-1 of culturing primate pluripotent stem cells in a medium including a GSK3 inhibitor and a ROCK inhibitor, and
   a step 1-2 of culturing the cells from the step 1-1 in a medium including a GSK3 inhibitor and Activin A.
(7) The production method according to any one of (1) to (6),
   in which the culture of the step 1 is plane culture.
(8) The production method according to (7),
   in which a container coated with a cell scaffold material is used in the plane culture.
(9) The production method according to (7) or (8),
   in which the medium in the step 2 further includes a ROCK inhibitor.
(10) The production method according to any one of (1) to (9),
   in which the medium in the step 1 further includes bFGF.
(11) The production method according to any one of (1) to (10),
   in which the medium in the step 2 further includes a hedgehog signaling activator.
(12) The production method according to any one of (1) to (11),
   in which the step 3 includes
   a step 3-1 of culturing the cells from the step 2 in a medium including an MEK inhibitor, and
   a step 3-2 of culturing the cells from the step 3-1 in a base medium to obtain ovarian somatic cell-like cells.
(13) The production method according to any one of (1) to (12),
   in which the ovarian somatic cell-like cells are embryonic ovarian somatic cell-like cells.
(14) The production method according to any one of (1) to (13),
   in which the primate pluripotent stem cells are derived from cynomolgus monkey or human.
(15) A method for inducing differentiation of primate pluripotent stem cells into ovarian somatic cell-like cells, the method including:
   inducing differentiation of primate pluripotent stem cells into ovarian somatic cell-like cells using the production method according to any one of (1) to (14).

### [Advantageous Effects of Invention]

According to the production method and the method for inducing differentiation of the present embodiment, ovarian somatic cell-like cells at any developmental stage can be efficiently obtained from primate pluripotent stem cells.

### [Brief Description of Drawings]

FIG. 1 is a view showing a configuration of a construct introduced into each gene locus of a human iPS cell strain in Example 1.
FIG. 2 is a view showing a protocol in a case of inducing differentiation from mouse ES cells into ovarian somatic cell-like cells.
FIG. 3A is a view showing a protocol for inducing differentiation into ovarian somatic cell-like cells using human iPS cells in "1." of Example 1.
FIG. 3B is a view showing bright field images (left side) and fluorescent images (center and right side) of embryoid bodies in "1." of Example 1.
FIG. 3C is a view showing FACS plots of OSR1-tdTomato and NR5A1-EGFP in "1." of Example 1.
FIG. 4A is a view showing a protocol for inducing differentiation into ovarian somatic cell-like cells using human iPS cells in "2." of Example 1.
FIG. 4B is a view showing FACS plots of OSR1-tdTomato and NR5A1-EGFP in "2." of Example 1.
FIG. 5A is a view showing a protocol for inducing differentiation into ovarian somatic cell-like cells using human iPS cells in "3." of Example 1.
FIG. 5B is a view showing bright field images (left side) and fluorescent images (a center and a right side) of embryoid bodies in "3." of Example 1.
FIG. 5C is a view showing FACS plots of OSR1-tdTomato and NR5A1-EGFP in "3." of Example 1.
FIG. 5D is a view showing the results of examining a time course of the expression of OSR1 and NR5A1 in a case where a period of step 1 in "3." of Example 1 was set to 4 days. The upper part shows the number of induction days, the middle part shows the bright field images, and the lower part shows the FACS plots of OSR1-tdTomato and NR5A1-EGFP.
FIG. 6A is a view showing a protocol for inducing differentiation into ovarian somatic cell-like cells using human iPS cells in "4." of Example 1.
FIG. 6B is a view showing bright field images (upper image) and fluorescent images (mold and lower images) of embryoid bodies in "4." of Example 1.
FIG. 6C is a view showing FACS plots of OSR1-tdTomato and NR5A1-EGFP in "4." of Example 1.
FIG. 7A is a view showing a protocol for inducing differentiation into ovarian somatic cell-like cells using human iPS cells in "5." of Example 1.
FIG. 7B is a view showing bright field images (left side) and fluorescent images (a center and a right side) of embryoid bodies in "5." of Example 1.
FIG. 7C is a view showing FACS plots of OSR1-tdTomato and NR5A1-EGFP in "5." of Example 1.
FIG. 8A is a view showing a protocol for inducing differentiation into ovarian somatic cell-like cells using human iPS cells in "6." of Example 1.
FIG. 8B is a view showing bright field images (left side) and fluorescent images (a center and a right side) of embryoid bodies in "6." of Example 1.
FIG. 8C is a view showing FACS plots of OSR 1-tdTomato and NR5A1 -EGFP in "6." of Example 1.
FIG. 9A is a view showing bright field images (left side) and fluorescent images (central and right sides) of embryoid bodies under the condition in which PD0325901 (1.0 µM) was added in "7." of Example 1.
FIG. 9B is a view showing FACS plots of OSR1-tdTomato and NR5A1-EGFP under the condition in which PD0325901 (1.0 µM) was added in "7." of Example 1.
FIG. 9C is a view showing bright field images (left side) and fluorescent images (central and right sides) of embryoid bodies under the condition in which PD0325901 was not added in "7." of Example 1.
FIG. 9D is a view showing FACS plots of OSR1-tdTomato and NR5A1-EGFP under the condition in which PD0325901 was not added in "7." of Example 1.
FIG. 10A is a view showing a protocol for inducing differentiation into ovarian somatic cell-like cells using human iPS cells in "8." of Example 1.
FIG. 10B is a view showing bright field images (left rows of each condition) and fluorescent images (central and right rows of each condition) of embryoid bodies in "8." of Example 1.
FIG. 10C is a view showing FACS plots of OSR1-tdTomato and NR5A1-EGFP in "8." of Example 1.
FIG. 11A is a view showing bright field images (left side) and fluorescent images (central and right sides) of embryoid bodies under the condition of a concentration of KSR of 5% in "9." of Example 1.
FIG. 11B is a view showing FACS plots of OSR1-tdTomato and NR5A1-EGFP under the condition of a concentration of KSR of 5% in "9." of Example 1.
FIG. 11C is a view showing bright field images (left side) and fluorescent images (central and right sides) of embryoid bodies under the condition of a concentration of KSR of 7.5% in "9." of Example 1.
FIG. 11D is a view showing FACS plots of OSR1-tdTomato and NR5A1-EGFP under the condition of a concentration of KSR of 7.5% in "9." of Example 1.
FIG. 12A is a view showing bright field images (left side) and fluorescent images (central and right sides) of embryoid bodies on day 15 of induction in "10." of Example 1.
FIG. 12B is a view showing FACS plots of OSR1-tdTomato and NR5A1-EGFP on day 15 of induction in "10." of Example 1.
FIG. 12C is a view showing bright field images (left side) and fluorescent images (center and right side) of embryoid bodies on day 23 of induction in "10." of Example 1.
FIG. 12D is a view showing FACS plots of OSR1-tdTomato and NR5A1-EGFP on day 23 of induction in "10." of Example 1.
FIG. 13A is a view showing bright field images (left side) and fluorescent images (central and right sides) of embryoid bodies on day 15 of induction in "11." of Example 1.
FIG. 13B is a view showing FACS plots of OSR1-tdTomato and NR5A1-EGFP on day 15 of induction in "11." of Example 1.
FIG. 13C is a view showing bright field images (left side) and fluorescent images (center and right side) of embryoid bodies on day 23 of induction in "11." of Example 1.
FIG. 13D is a view showing FACS plots of OSR1 -tdTomato and NR5A1-EGFP on day 23 of induction in "11." of Example 1.
FIG. 14A is a view showing a protocol for inducing differentiation into ovarian somatic cell-like cells using human iPS cells in "12." of Example 1.
FIG. 14B is a view showing bright field images (left side) and fluorescent images (central and right sides) of embryoid bodies under the condition in which a sonic hedgehog (SHH) was not added in "12." of Example 1.
FIG. 14C is a view showing FACS plots of OSR1-tdTomato and NR5A1-EGFP under the condition in which SHH was not added in "12." of Example 1.
FIG. 14D is a view showing bright field images (left side) and fluorescent images (a center and a right side) of the embryoid bodies under the condition in which SHH was added in "12." of Example 1.
FIG. 14E is a view showing FACS plots of OSR1-tdTomato and NR5A1-EGFP under the condition in which SHH was added in "12." of Example 1.
FIG. 15A is a view showing a protocol for inducing differentiation into ovarian somatic cell-like cells using human iPS cells in "13." of Example 1.
FIG. 15B is a graph showing the measurement results of quantitative PCR of each marker gene in "13." of Example 1.
FIG. 16A is a view showing FACS plots of OSR1-tdTomato and NR5A1(SF1)-EGFP in "14." of Example 1.
FIG. 16B is a graph showing the measurement results of each marker gene by quantitative PCR in "14." of Example 1.
FIG. 17 is a graph showing the measurement results of quantitative PCR for each marker gene in "15." of Example 1.
FIG. 18A is a view showing a protocol for inducing differentiation into ovarian somatic cell-like cells using human iPS cells in "16." of Example 1.
FIG. 18B is an image obtained by observing the temporal change of cells in "16." of Example 1.
FIG. 18C is a view showing FACS plots of OSR1 -tdTomato and NR5A1(SF1)-EGFP in "16." of Example 1.
FIG. 18D is a graph showing the measurement results of each marker gene by quantitative PCR in "16." of Example 1.
FIG. 18E is a view showing fluorescent immunostaining images of embryoid bodies in "16." of Example 1.
FIG. 18F is a view showing fluorescent immunostaining images of embryoid bodies in "16." of Example 1.
FIG. 18G is a view showing FACS plots of OSR1-tdTomato and NR5A1(SF1)-EGFP in "16." of Example 1.
FIG. 18H is a graph showing the measurement results of quantitative PCR of each marker gene in "16." of Example 1.
FIG. 19A is a graph showing the measurement results of quantitative PCR for each marker gene in "17." of Example 1.
FIG. 19B is a view showing FACS plots of OSR1-tdTomato and NR5A1(SF1)-EGFP in "17." of Example 1.
FIG. 20A is a view showing a protocol for inducing differentiation into ovarian somatic cell-like cells using human iPS cells in "18." of Example 1.
FIG. 20B is a view showing bright field images of embryoid bodies in "18." of Example 1.
FIG. 20C is a view showing bright field images of embryoid bodies in "18." of Example 1.
FIG. 20D is a view showing FACS plots of OSR1 -tdTomato and NR5A1(SF1)-EGFP in "18." of Example 1.
FIG. 20E is a view showing fluorescent immunostaining images of embryoid bodies in "18." of Example 1.
FIG. 20F is a graph showing the measurement results of each marker gene by quantitative PCR in "18." of Example 1.
FIG. 20G is a view showing a protocol for inducing differentiation into ovarian somatic cell-like cells using human iPS cells established based on the results up to "18." in Example 1.
FIG. 21 is a view showing FACS plots of PDGFRα and SF1 in "19." of Example 1.
FIG. 22A is a view showing bright field images (left side) and FACS plots of OSR1-tdTomato and NR5A1(SF1)-EGFP of embryoid bodies in "20." of Example 1.
FIG. 22B is a view showing fluorescent immunostaining images of embryoid bodies on day 37 of induction in "20." of Example 1.
FIG. 22C is a view showing fluorescent immunostaining images of embryoid bodies on day 57 of induction in "20." of Example 1.
FIG. 22D is a view showing fluorescent immunostaining images of embryoid bodies on day 86 of induction in "20." of Example 1.
FIG. 22E is a view showing fluorescent immunostaining images of embryoid bodies on day 86 of induction in "20." of Example 1.
FIG. 22F is a view showing fluorescent immunostaining images of embryoid bodies on day 86 of induction in "20." of Example 1.
FIG. 23A is a view showing FACS plots on days 7 to 10 of induction in "21." of Example 1.
FIG. 23B is a view showing FACS plots on days 11 to 15 of induction in "21." of Example 1.
FIG. 24A is a view showing the configuration of a construct introduced into a TBXT gene locus of cynomolgus monkey ES cells and a change in configuration of the construct after introduction in Example 2.
FIG. 24B is a view showing the configuration of a construct introduced into a NR5A1 gene locus of cynomolgus monkey ES cells and a change in configuration of the construct after introduction in Example 2.
FIG. 24C is a view showing the configuration of a construct introduced into an OSR1 gene locus of the cynomolgus monkey ES cells and a change in configuration of the construct after the introduction in Example 2.
FIG. 24D is a view showing the configuration of a construct introduced into the FOXL2 gene locus of the cynomolgus monkey ES cells and a change in configuration of the construct after the introduction in Example 2.
FIG. 24E is a view showing the configuration of a construct introduced into the WT1 gene locus of the cynomolgus monkey ES cells and a change in configuration of the construct after the introduction in Example 2.
FIG. 24F is a view showing the configuration of a construct introduced into a GATA4 gene locus of cynomolgus monkey ES cells and a change in configuration of the construct after introduction in Example 2.
FIG. 25A is a view showing a protocol for inducing differentiation into ovarian somatic cell-like cells using cynomolgus monkey ES cells in "1." of Example 2.
FIG. 25B is a view showing bright field images (left upper part, upper left part of the center, and right side) and fluorescent images (upper right part, lower left part, and lower right part of the right side) of embryoid bodies in "1." of Example 2.
FIG. 25C is a view showing FACS plots of OSR1 -tdTomato and TBXT-EGFP in "1." of Example 2.
FIG. 26A is a view showing a protocol for inducing differentiation into ovarian somatic cell-like cells using cynomolgus monkey ES cells in "2." of Example 2.
FIG. 26B is a view showing FACS plots of OSR1-tdTomato and TBXT-EGFP in "2." of Example 2.
FIG. 27A is a view showing a protocol for inducing differentiation into ovarian somatic cell-like cells using cynomolgus monkey ES cells in "3." of Example 2.
FIG. 27B is a view showing FACS plots of OSR1-tdTomato and TBXT-EGFP on day 4 of induction in "3." of Example 2.
FIG. 27C is a view showing FACS plots of OSR1-tdTomato and TBXT-EGFP on day 6 of induction in "3." of Example 2.
FIG. 28A is a view showing a protocol for inducing differentiation into ovarian somatic cell-like cells using cynomolgus monkey ES cells in "4." of Example 2.
FIG. 28B is a view showing FACS plots of OSR1-tdTomato and TBXT-EGFP in "4." of Example 2.
FIG. 29A is a view showing a protocol for inducing differentiation into ovarian somatic cell-like cells using cynomolgus monkey ES cells in "5." of Example 2.
FIG. 29B is a view showing FACS plots of OSR1-tdTomato and TBXT-EGFP in "5." of Example 2.
FIG. 30A is a view showing a protocol for inducing differentiation into ovarian somatic cell-like cells using cynomolgus monkey ES cells in "6." of Example 2.
FIG. 30B is a view showing FACS plots of OSR1-tdTomato and TBXT-EGFP under the condition in which Y-27632 in "6." of Example 2 was added for 1 day.
FIG. 30C is a view showing FACS plots of OSR1-tdTomato and TBXT-EGFP under the condition in which Y-27632 in "6." of Example 2 was added for 2 days.
FIG. 31A is a view showing a protocol for inducing differentiation into ovarian somatic cell-like cells using cynomolgus monkey ES cells in "7." of Example 2. In the figure, "MC" means medium change. Hereinafter, the term is used with the same meaning.
FIG. 31B is a view showing FACS plots of OSR1-tdTomato and TBXT-EGFP in "7." of Example 2.
FIG. 31C is a graph showing the measurement results of each marker gene by quantitative PCR in "7." of Example 2.
FIG. 32A is a view showing a protocol for inducing differentiation into ovarian somatic cell-like cells using cynomolgus monkey ES cells in "8." of Example 2.
FIG. 32B is a view showing FACS plots on days 2 to 14 days of induction in "8." of Example 2.
FIG. 33A is a view showing a protocol for inducing differentiation into ovarian somatic cell-like cells using cynomolgus monkey ES cells in "9." of Example 2.
FIG. 33B is a view showing FACS plots of FOXL2-tdTomato and NR5A1-EGFP in "9." of Example 2.
FIG. 34A is a view showing a protocol for inducing differentiation into ovarian somatic cell-like cells using cynomolgus monkey ES cells in "10." of Example 2.
FIG. 34B is a view showing FACS plots of FOXL2-tdTomato and NR5A1-EGFP in "10." of Example 2.
FIG. 34C is a view showing bright field images of embryoid bodies in "10." of Example 2.
FIG. 35A is a view showing a protocol for inducing differentiation into ovarian somatic cell-like cells using cynomolgus monkey ES cells in "11." of Example 2.
FIG. 35B is a view showing FACS plots of FOXL2-tdTomato and NR5A1-EGFP in "11." of Example 2.
FIG. 36A is a view showing a protocol for inducing differentiation into ovarian somatic cell-like cells using cynomolgus monkey ES cells in "12." of Example 2.
FIG. 36B is a view showing FACS plots of FOXL2-tdTomato and NR5A1-EGFP in "12." of Example 2.
FIG. 37A is a view showing a protocol for inducing differentiation into ovarian somatic cell-like cells using cynomolgus monkey ES cells in "13." of Example 2.
FIG. 37B is a view showing FACS plots of FOXL2-tdTomato and NR5A1-EGFP in "13." of Example 2.
FIG. 37C is a graph showing the measurement results of each marker gene by quantitative PCR in "13." of Example 2.
FIG. 38A is a view showing a protocol for inducing differentiation into ovarian somatic cell-like cells using cynomolgus monkey ES cells in "14." of Example 2.
FIG. 38B is a view showing FACS plots of FOXL2-tdTomato and NR5A1-EGFP in "14." of Example 2.
FIG. 39A is a view showing a protocol for inducing differentiation into ovarian somatic cell-like cells using cynomolgus monkey ES cells in "15." of Example 2.
FIG. 39B is a view showing FACS plots of FOXL2-tdTomato and NR5A1-EGFP in "15." of Example 2.
FIG. 39C is a view showing bright field images of embryoid bodies in "15." of Example 2.
FIG. 40A is a view showing a protocol for inducing differentiation into ovarian somatic cell-like cells using cynomolgus monkey ES cells in "16." of Example 2.
FIG. 40B is a view showing FACS plots of FOXL2-tdTomato and NR5A1-EGFP under the condition in which the periods of step 1, step 2, and step 3 are 2-2-12 (days) in "16." of Example 2.
FIG. 40C is a view showing FACS plots of FOXL2-tdTomato and NR5A1-EGFP under the condition in which the periods of step 1, step 2, and step 3 are 3-2-11 (days) in "16." of Example 2.
FIG. 40D is a view showing FACS plots of FOXL2-tdTomato and NR5A1-EGFP under the condition in which the periods of step 1, step 2, and step 3 are 4-2-10 (days) in "16." of Example 2.
FIG. 40E is a view showing FACS plots of FOXL2-tdTomato and NR5A1-EGFP under the condition in which the periods of step 1, step 2, and step 3 are 2-3-11 (days) in "16." of Example 2.
FIG. 40F is a view showing FACS plots of FOXL2-tdTomato and NR5A1-EGFP under the condition in which the periods of step 1, step 2, and step 3 are 3-3-10 (days) in "16." of Example 2.
FIG. 40G is a view showing FACS plots of FOXL2-tdTomato and NR5A1-EGFP under the condition in which the periods of step 1, step 2, and step 3 are 4-3-9 (days) in "16." of Example 2.
FIG. 40H is a view showing FACS plots of FOXL2-tdTomato and NR5A1-EGFP on day 17 of induction, in which a culture was added for one day under the condition in which the periods of step 1, step 2, and step 3 are 3-3-10 (days) in "16." of Example 2.
FIG. 40I is a graph showing the measurement results of quantitative PCR for each marker gene in "16." of Example 2.
FIG. 40J is a graph showing the measurement results of each marker gene by quantitative PCR in "16." of Example 2.
FIG. 40K is a graph showing the measurement results of each marker gene by quantitative PCR in "16." of Example 2.

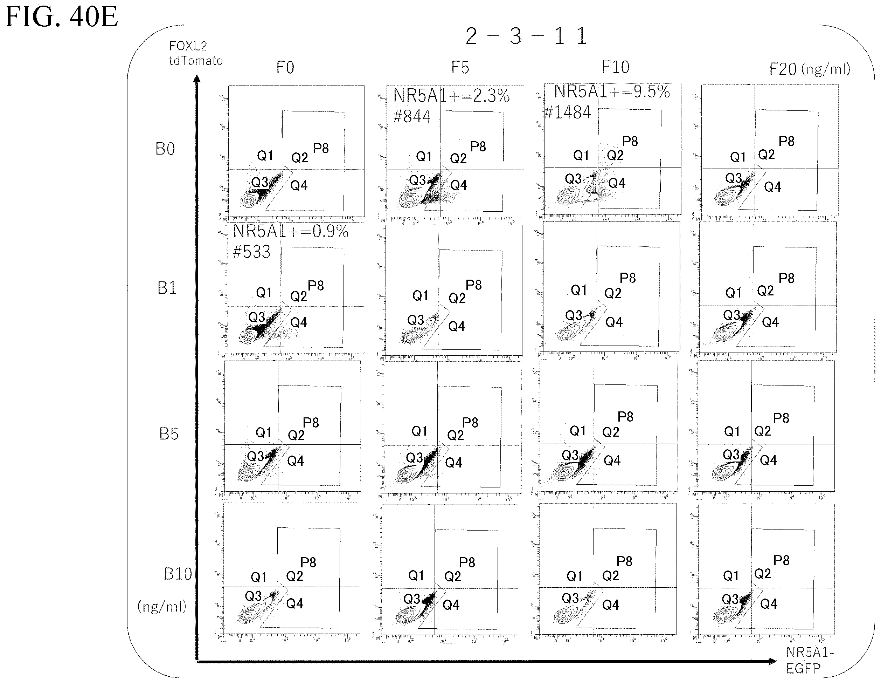

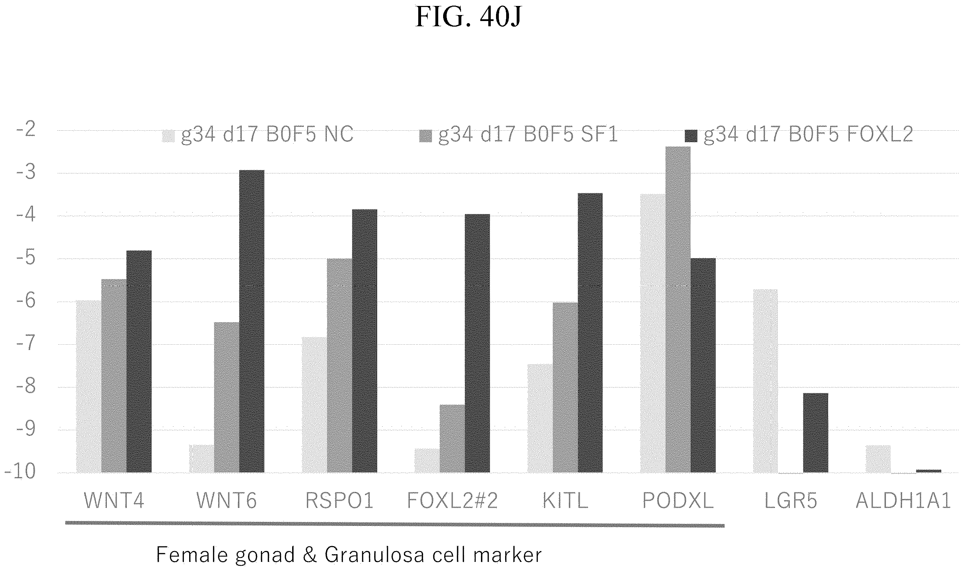

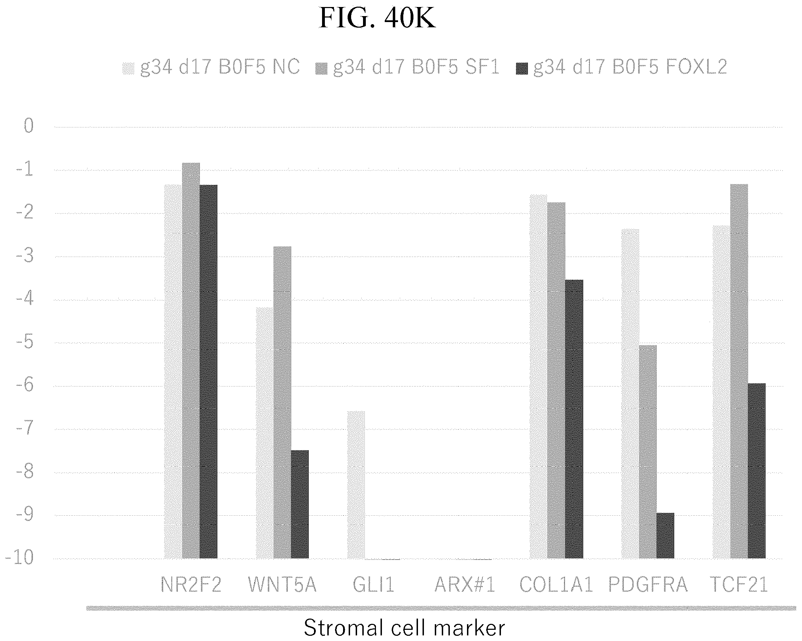

FIG. 40E is a view showing fluorescent immunostaining images of embryoid bodies on day 17 of induction in "16." of Example 2.

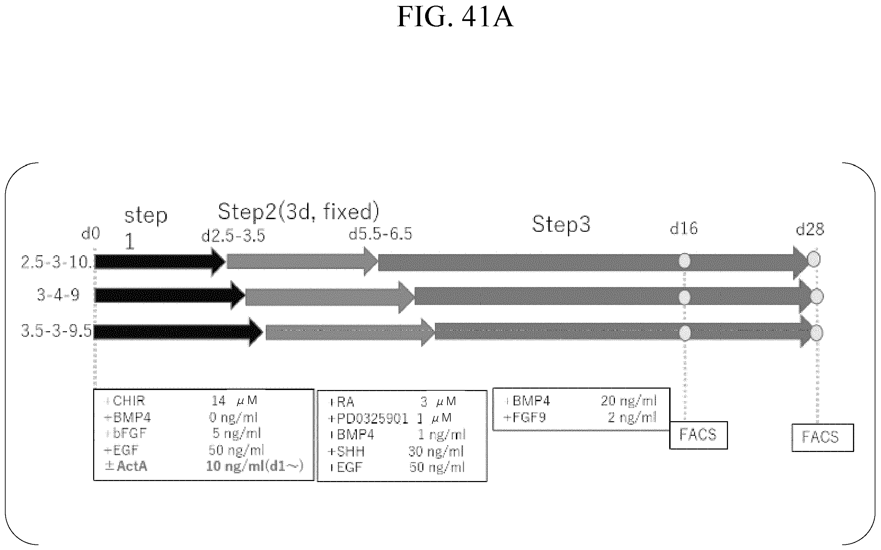

FIG. 41A is a view showing a protocol for inducing differentiation into ovarian somatic cell-like cells using cynomolgus monkey ES cells in "17." of Example 2.

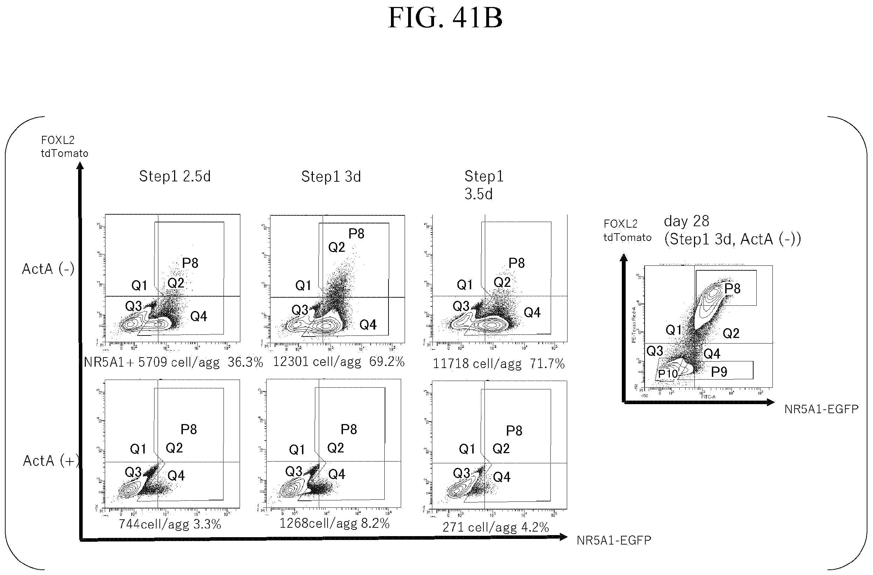

FIG. 41B is a view showing FACS plots of FOXL2-tdTomato and NR5A1-EGFP on days 16 and 28 of induction in "17." of Example 2.

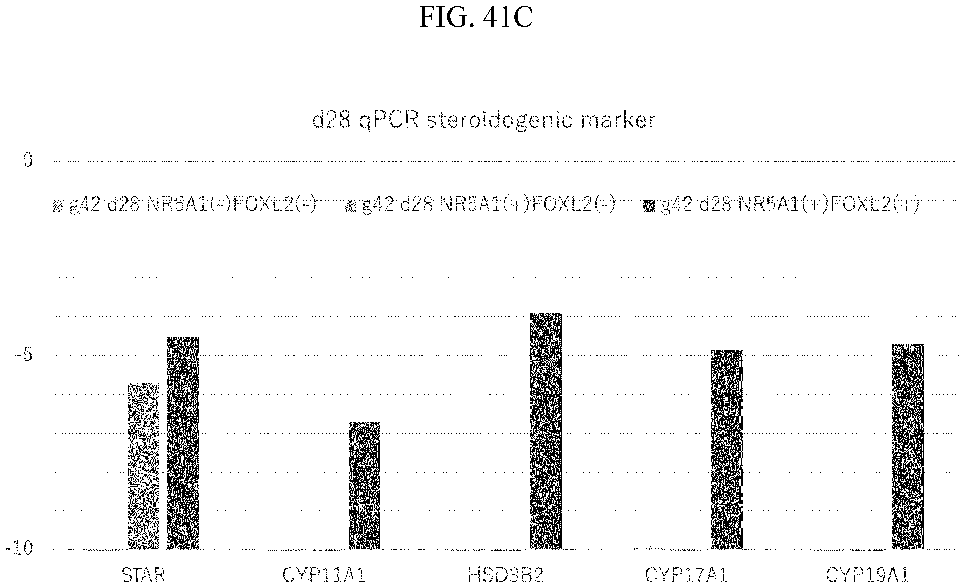

FIG. 41C is a graph showing the measurement results of each marker gene by quantitative PCR in "17." of Example 2.

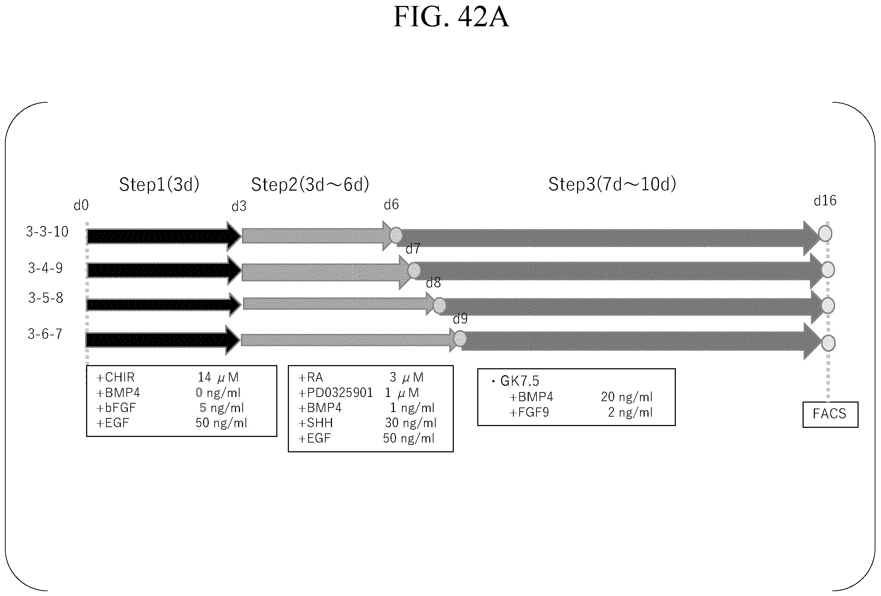

FIG. 42A is a view showing a protocol for inducing differentiation into ovarian somatic cell-like cells using cynomolgus monkey ES cells in "18." of Example 2.

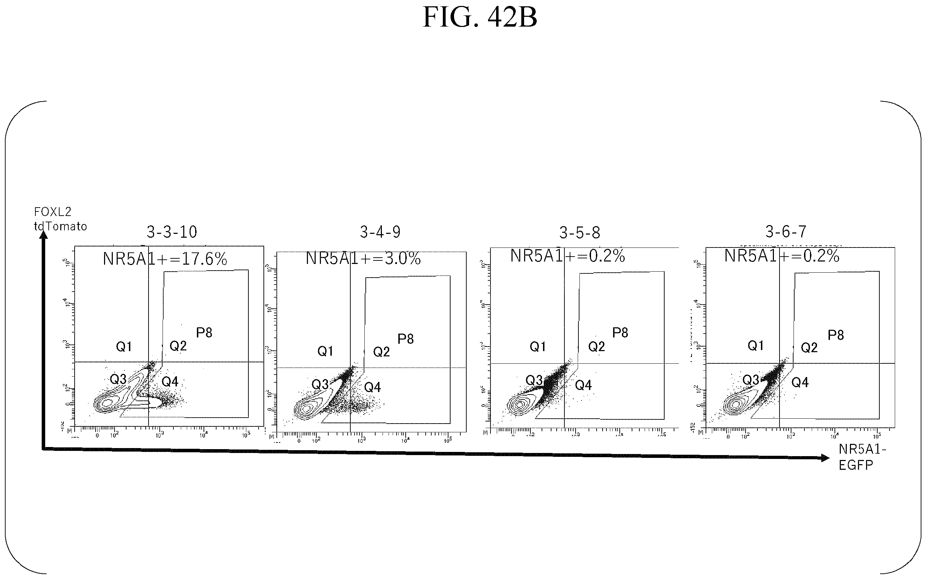

FIG. 42B is a view showing FACS plots of FOXL2-tdTomato and NR5A1-EGFP in "18." of Example 2.

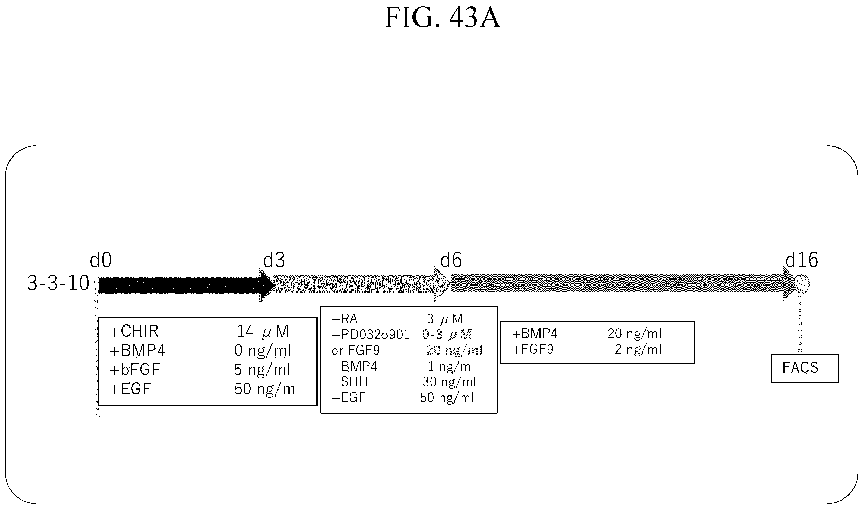

FIG. 43A is a view showing a protocol for inducing differentiation into ovarian somatic cell-like cells using cynomolgus monkey ES cells in "19." of Example 2.

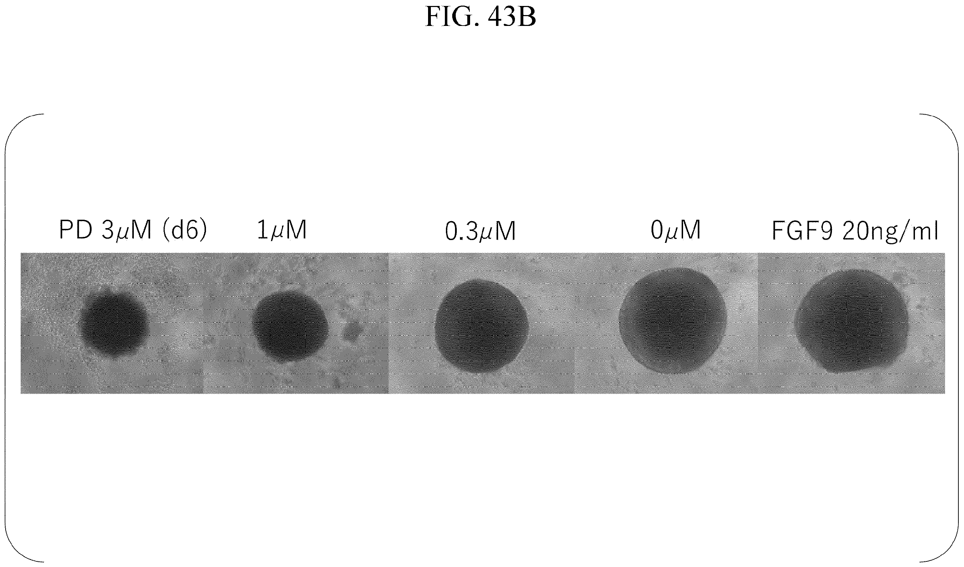

FIG. 43B is a view showing bright field images of embryoid bodies in "19." of Example 2.

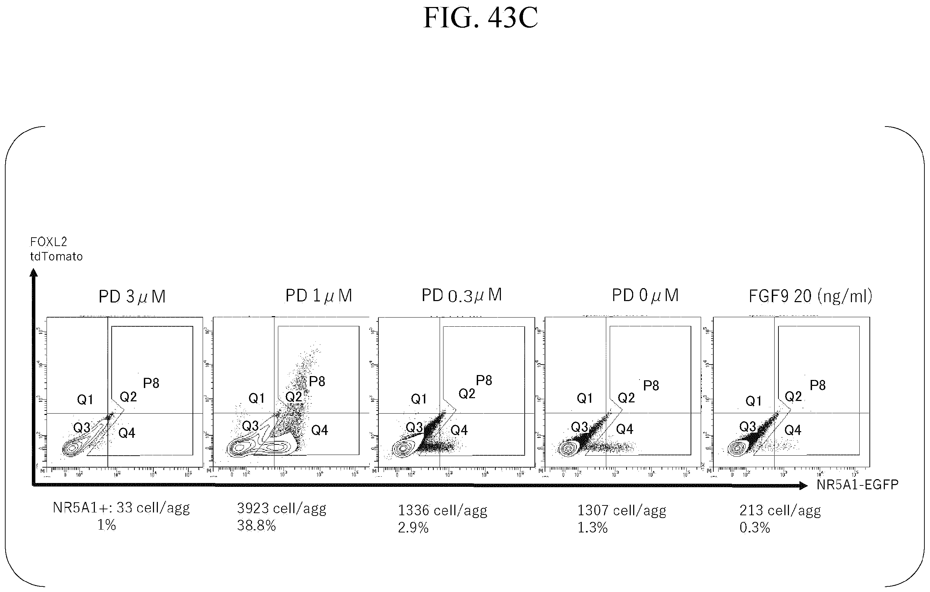

FIG. 43C is a view showing FACS plots of FOXL2-tdTomato and NR5A1-EGFP in "19." of Example 2.

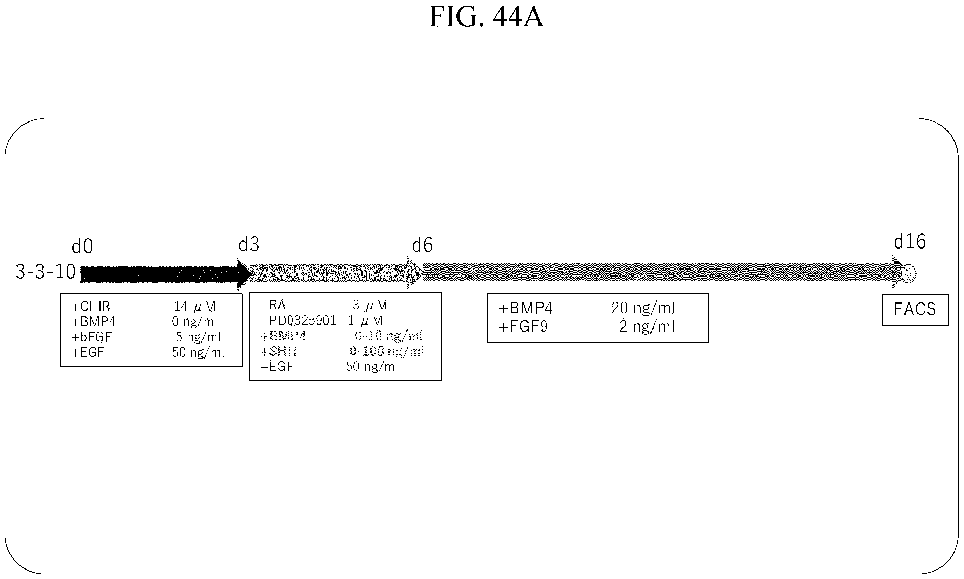

FIG. 44A is a view showing a protocol for inducing differentiation into ovarian somatic cell-like cells using cynomolgus monkey ES cells in "20." of Example 2.

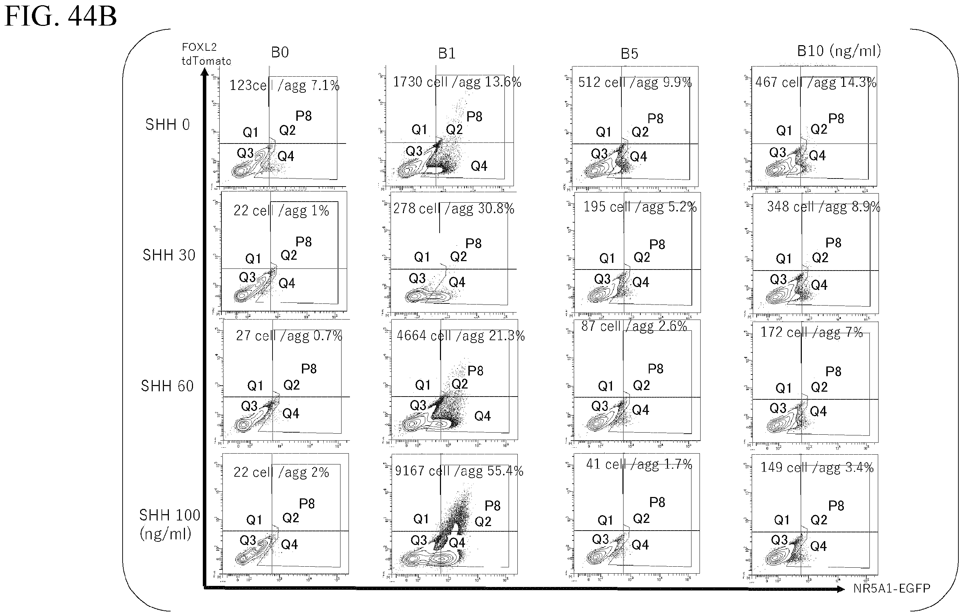

FIG. 44B is a view showing FACS plots of FOXL2-tdTomato and NR5A1-EGFP in "20." of Example 2.

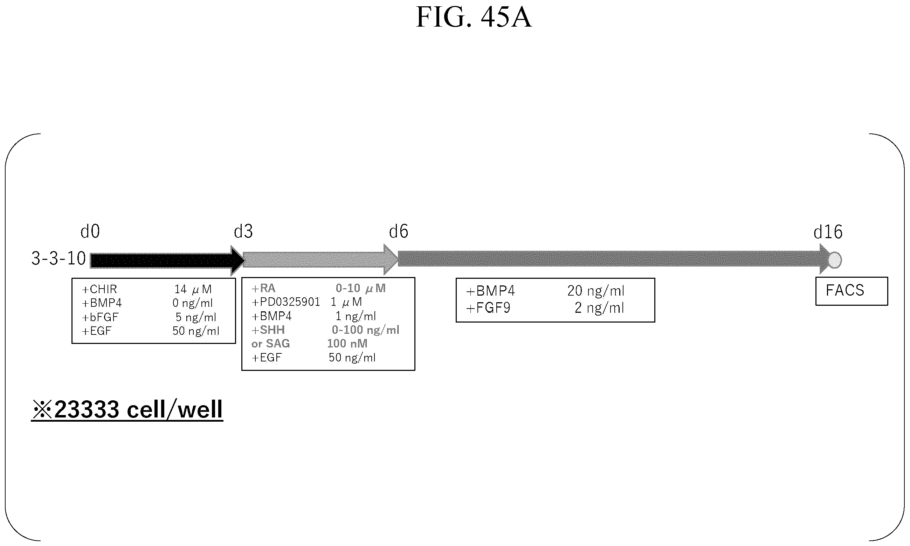

FIG. 45A is a view showing a protocol for inducing differentiation into ovarian somatic cell-like cells using cynomolgus monkey ES cells in "21." of Example 2.

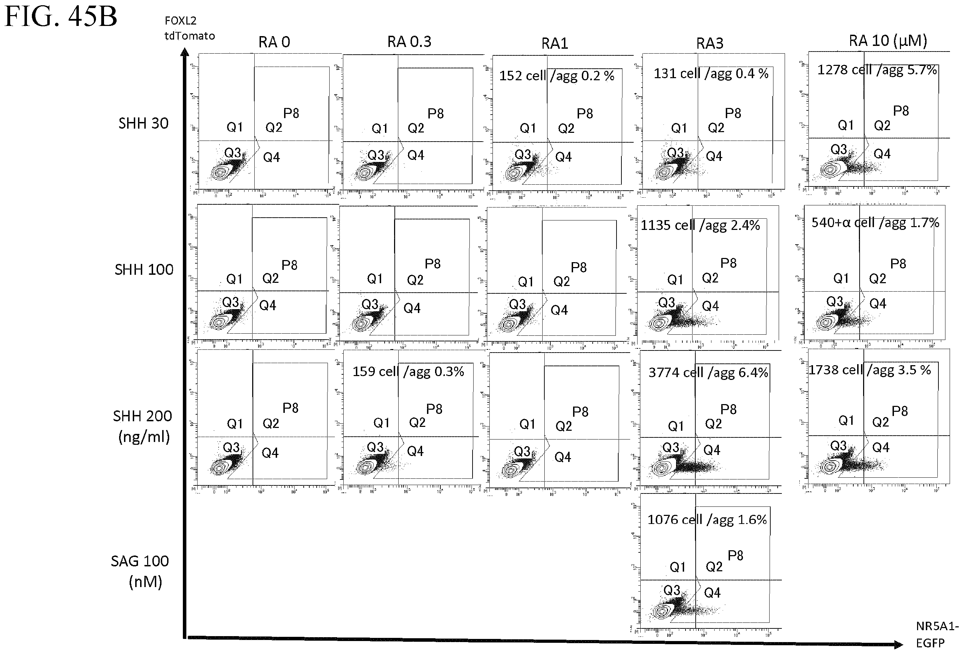

FIG. 45B is a view showing FACS plots of FOXL2-tdTomato and NR5A1-EGFP in "21." of Example 2.

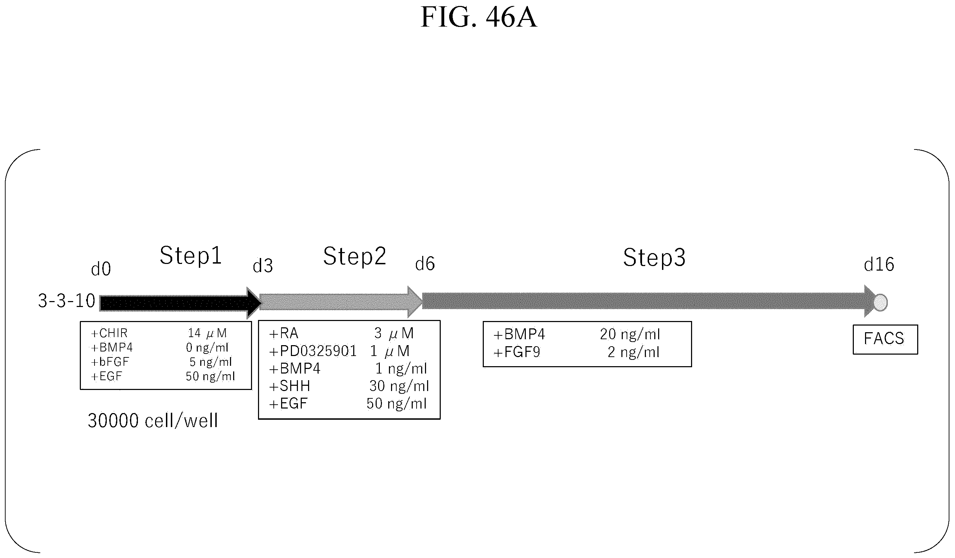

FIG. 46A is a view showing a protocol for inducing differentiation into ovarian somatic cell-like cells using cynomolgus monkey ES cells in "22." of Example 2.

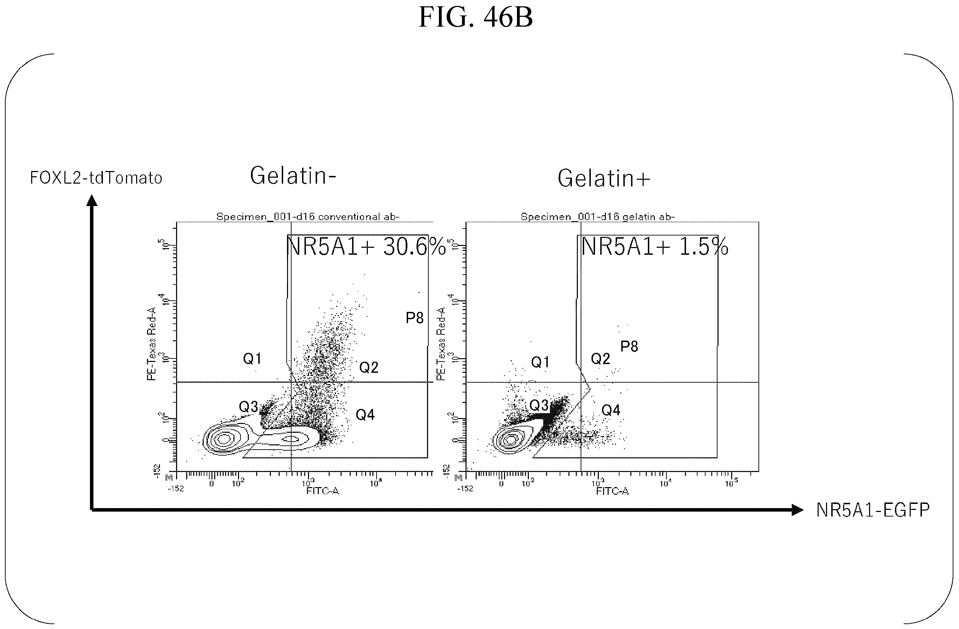

FIG. 46B is a view showing FACS plots of FOXL2-tdTomato and NR5A1-EGFP in "22." of Example 2.

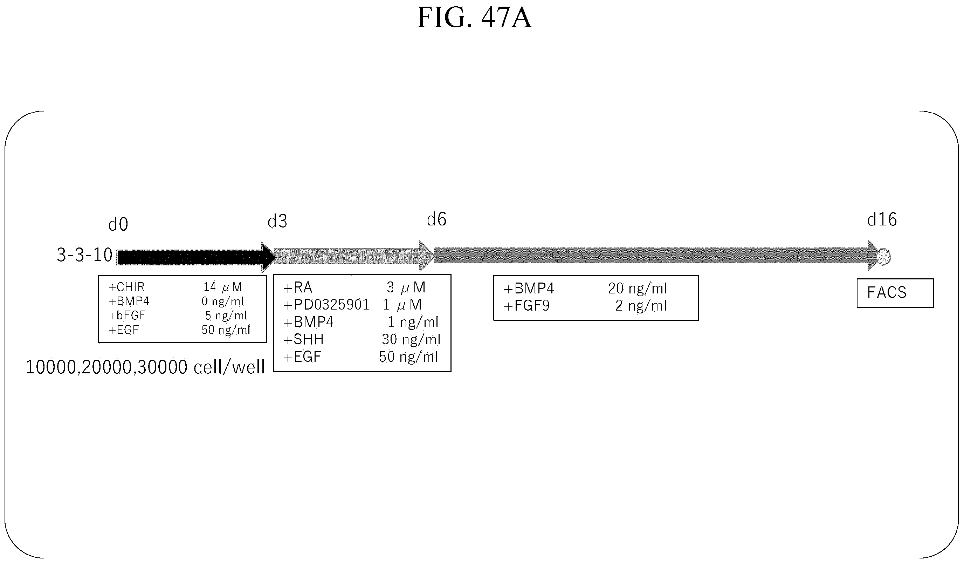

FIG. 47A is a view showing a protocol for inducing differentiation into ovarian somatic cell-like cells using cynomolgus monkey ES cells in "23." of Example 2.

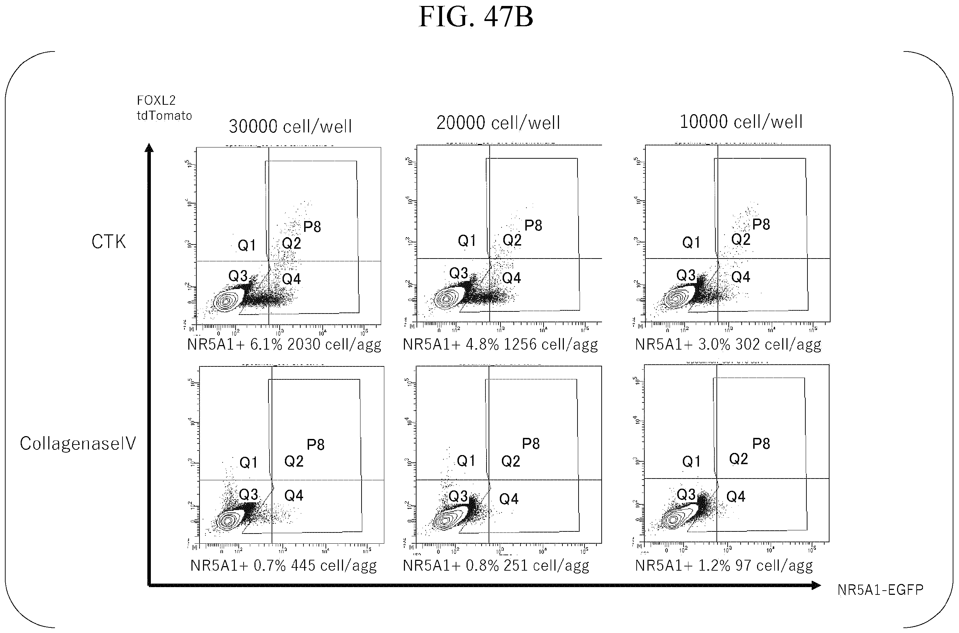

FIG. 47B is a view showing FACS plots of FOXL2-tdTomato and NR5A1-EGFP in "23." of Example 2.

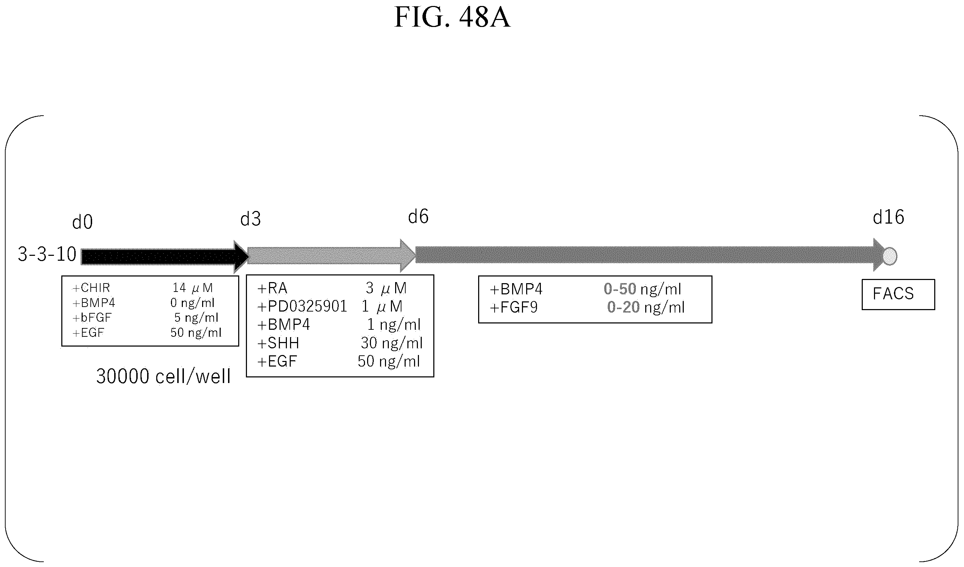

FIG. 48A is a view showing a protocol for inducing differentiation into ovarian somatic cell-like cells using cynomolgus monkey ES cells in "24." of Example 2.

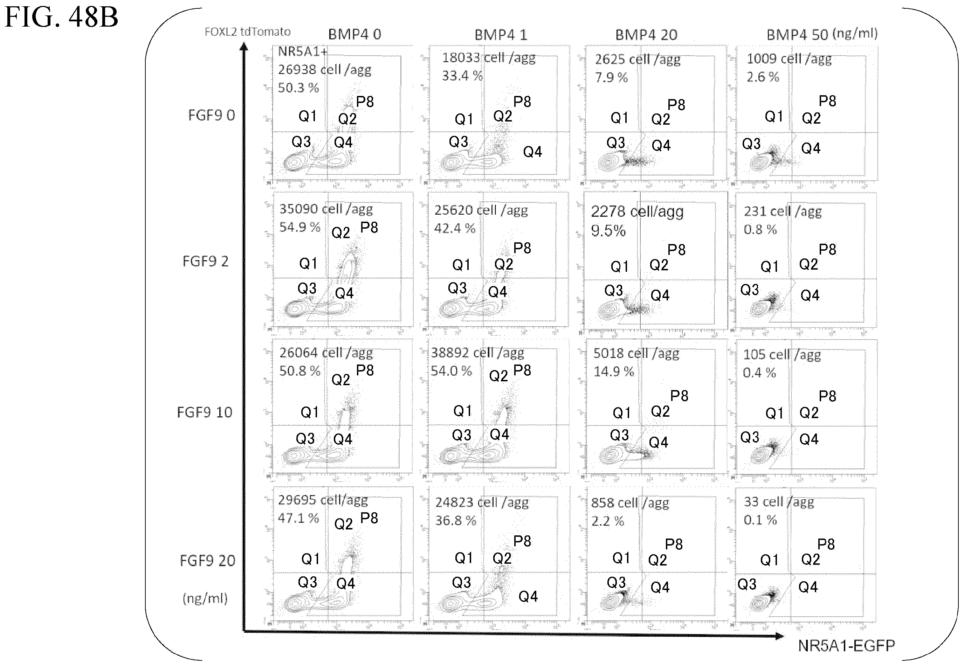

FIG. 48B is a view showing FACS plots of FOXL2-tdTomato and NR5A1-EGFP in "24." of Example 2.

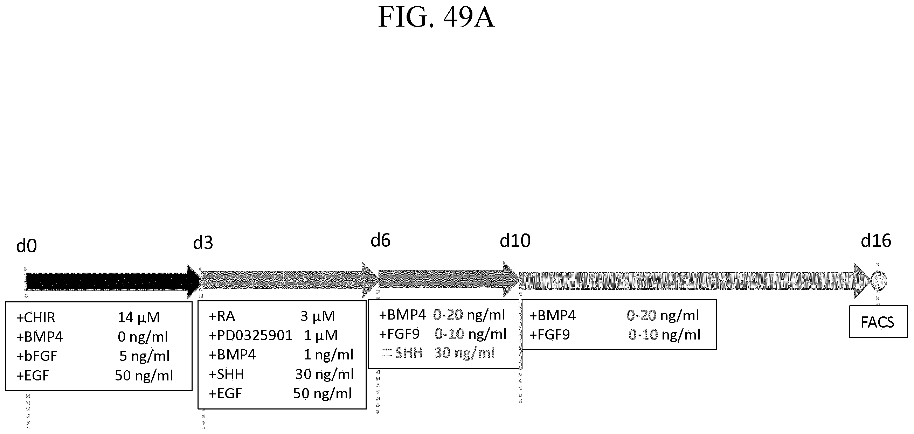

FIG. 49A is a view showing a protocol for inducing differentiation into ovarian somatic cell-like cells using cynomolgus monkey ES cells in "25." of Example 2.

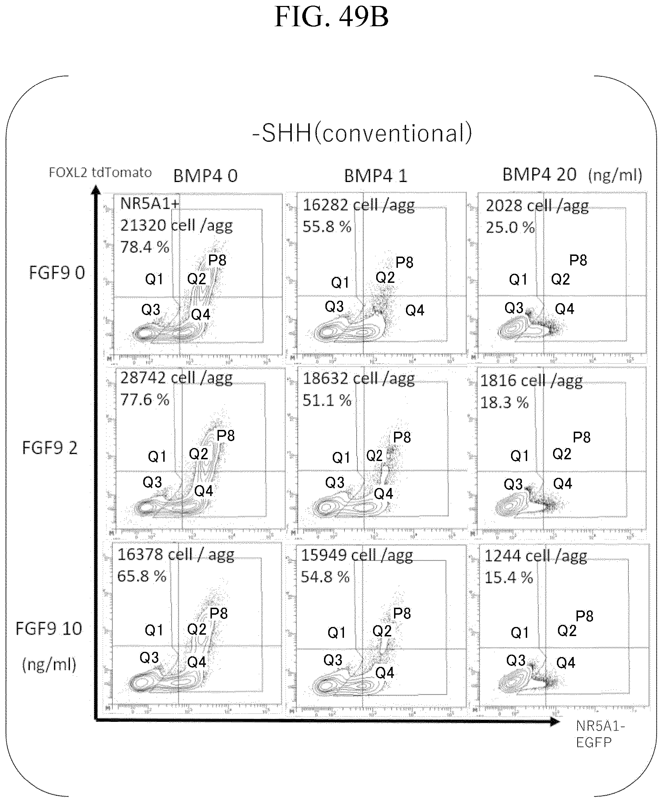

FIG. 49B is a view showing FACS plots of FOXL2-tdTomato and NR5A1-EGFP under the condition in which SHH was not added in "25." of Example 2.

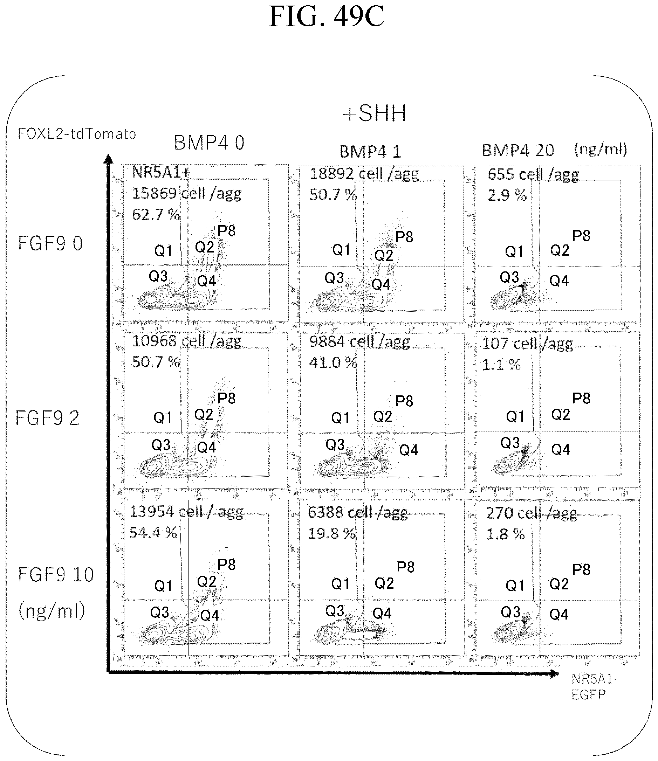

FIG. 49C is a view showing FACS plots of FOXL2-tdTomato and NR5A1-EGFP under the condition in which SHH was added in "25." of Example 2.

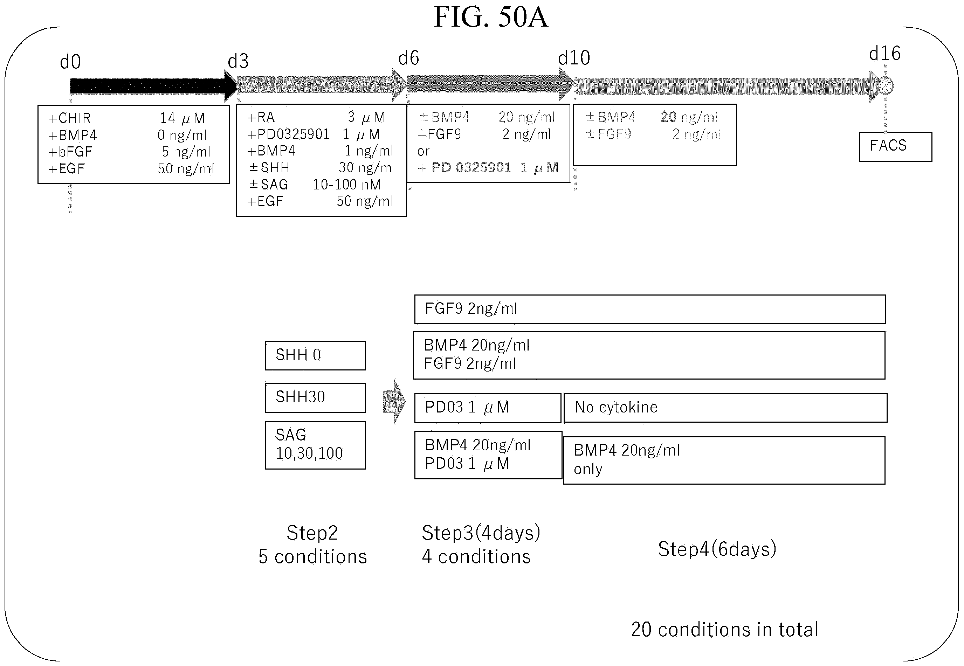

FIG. 50A is a view showing a protocol for inducing differentiation into ovarian somatic cell-like cells using cynomolgus monkey ES cells in "26." of Example 2.

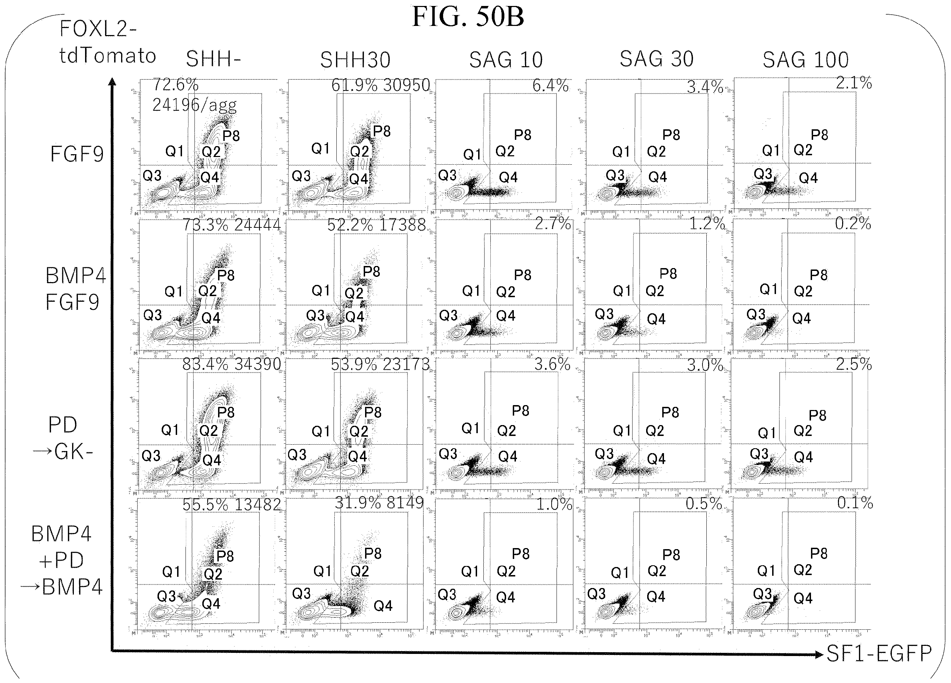

FIG. 50B is a view showing FACS plots of FOXL2-tdTomato and NR5A1-EGFP in "26." of Example 2.

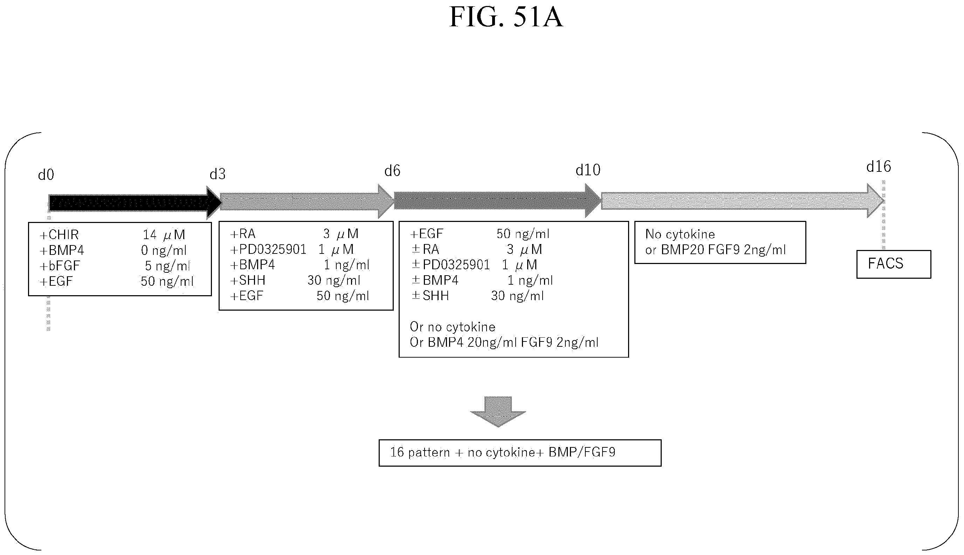

FIG. 51A is a view showing a protocol for inducing differentiation into ovarian somatic cell-like cells using cynomolgus monkey ES cells in "27." of Example 2.

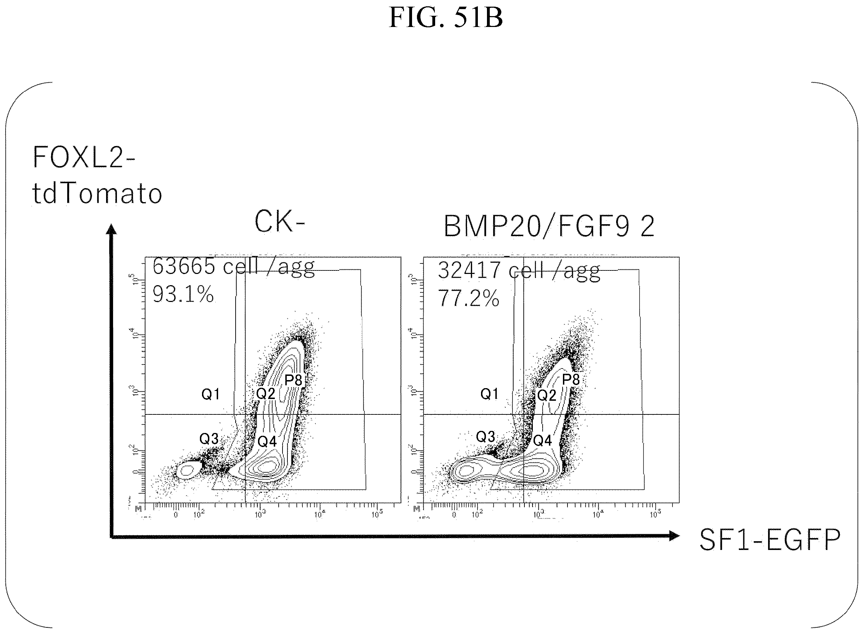

FIG. 51B is a view showing FACS plots of FOXL2-tdTomato and NR5A1-EGFP of a control in "27." of Example 2.

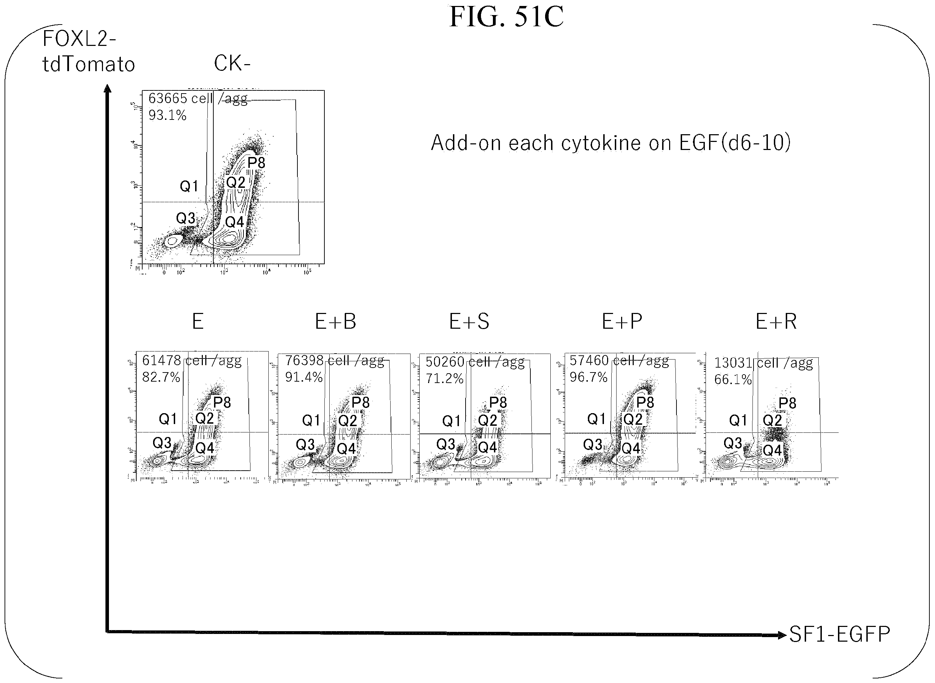

FIG. 51C is a view showing FACS plots of FOXL2-tdTomato and NR5A1-EGFP under the condition in which a control (CK-) and each cytokine were added in "27." of Example 2.

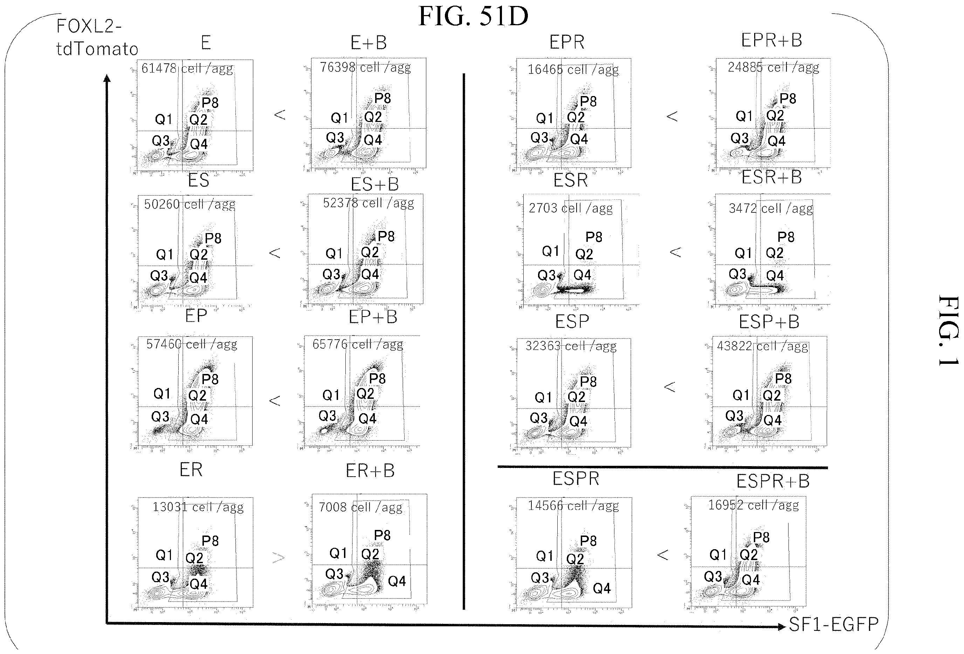

FIG. 51D is a view showing FACS plots of FOXL2-tdTomato and NR5A1-EGFP under the condition in which BMP4 was not added and the condition in which BMP4 was added in "27." of Example 2.

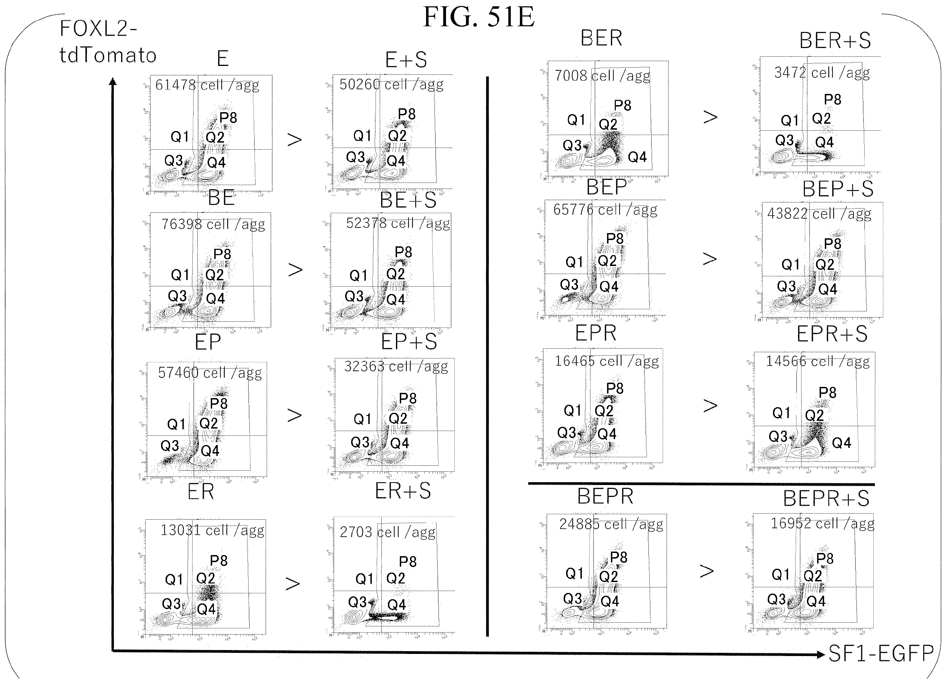

FIG. 51E is a view showing FACS plots of FOXL2-tdTomato and NR5A1-EGFP under the condition in which SHH was not added and the condition in which SHH was added in "27." of Example 2.

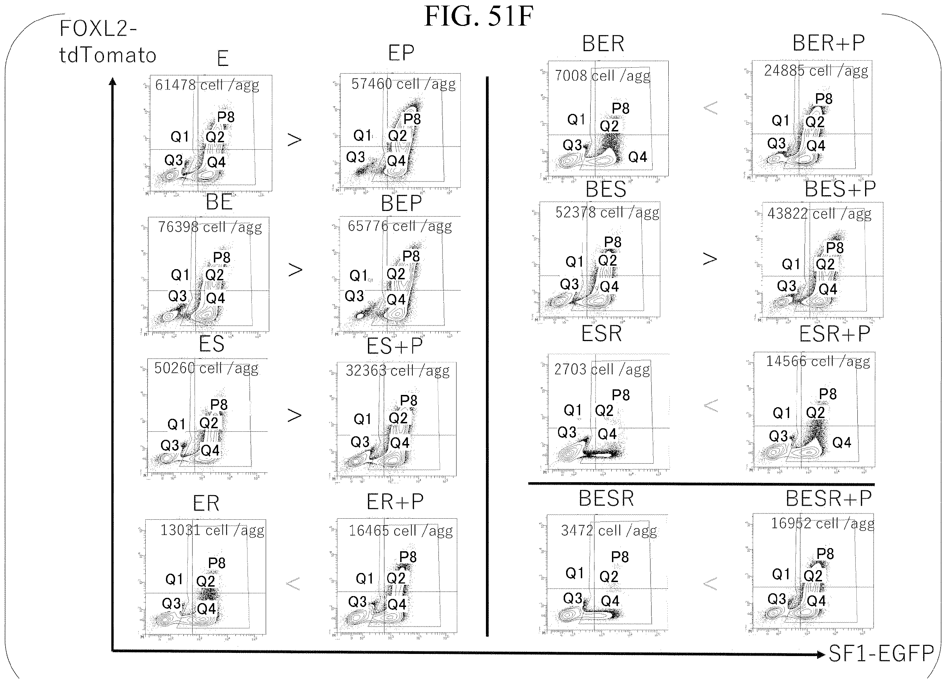

FIG. 51F is a view showing FACS plots of FOXL2-tdTomato and NR5A1-EGFP under the condition in which PD0325901 was not added and the condition in which PD0325901 was added in "27." of Example 2.

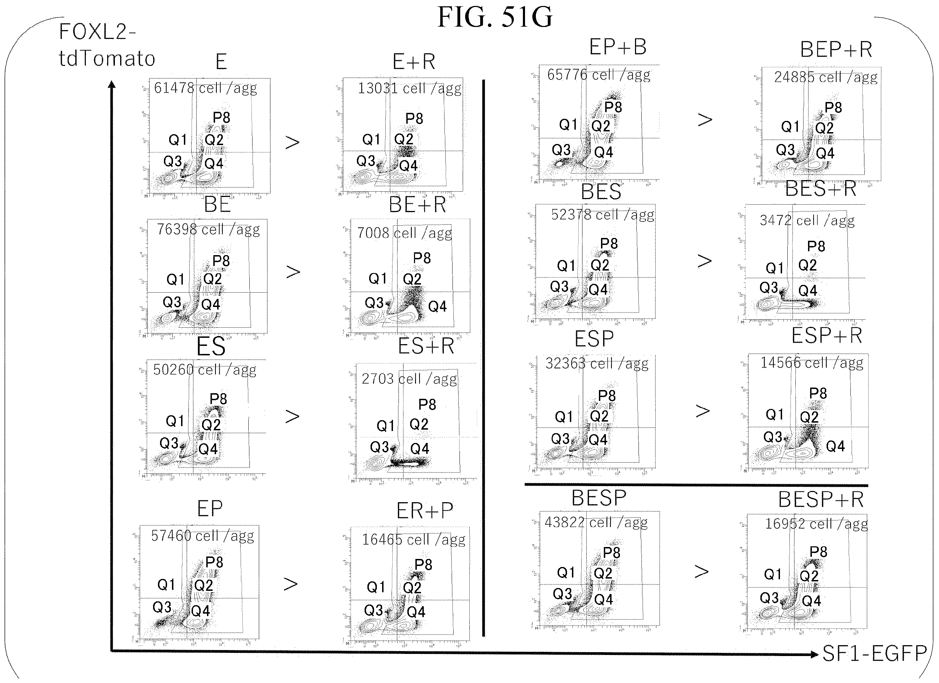

FIG. 51G is a view showing FACS plots of FOXL2-tdTomato and NR5A1-EGFP under the condition in which retinoic acid (RA) was not added and the condition in which RA was added in "27." of Example 2.

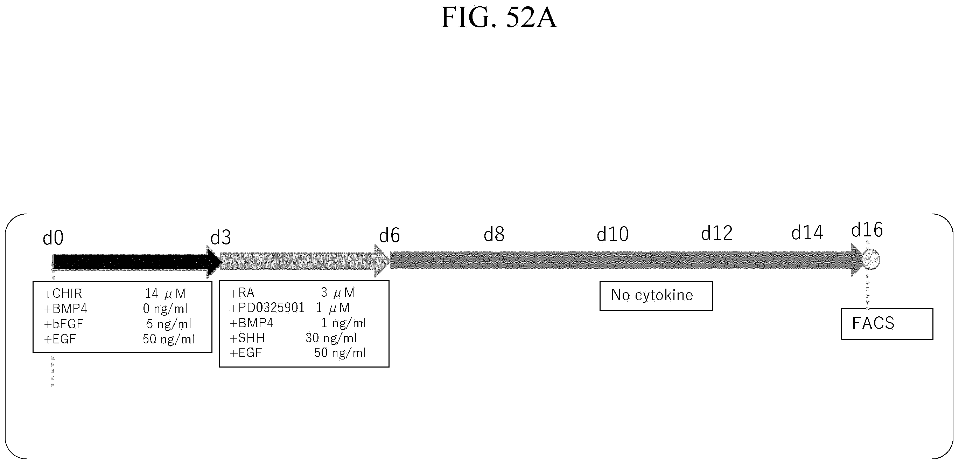

FIG. 52A is a view showing a protocol for inducing differentiation into ovarian somatic cell-like cells using cynomolgus monkey ES cells in "28." of Example 2.

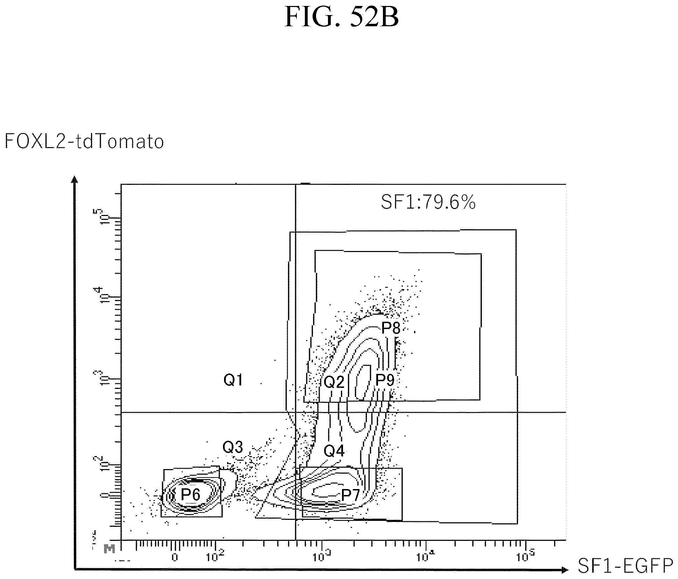

FIG. 52B is a view showing FACS plots of FOXL2-tdTomato and NR5A1 (SF1)-EGFP in "28." of Example 2.

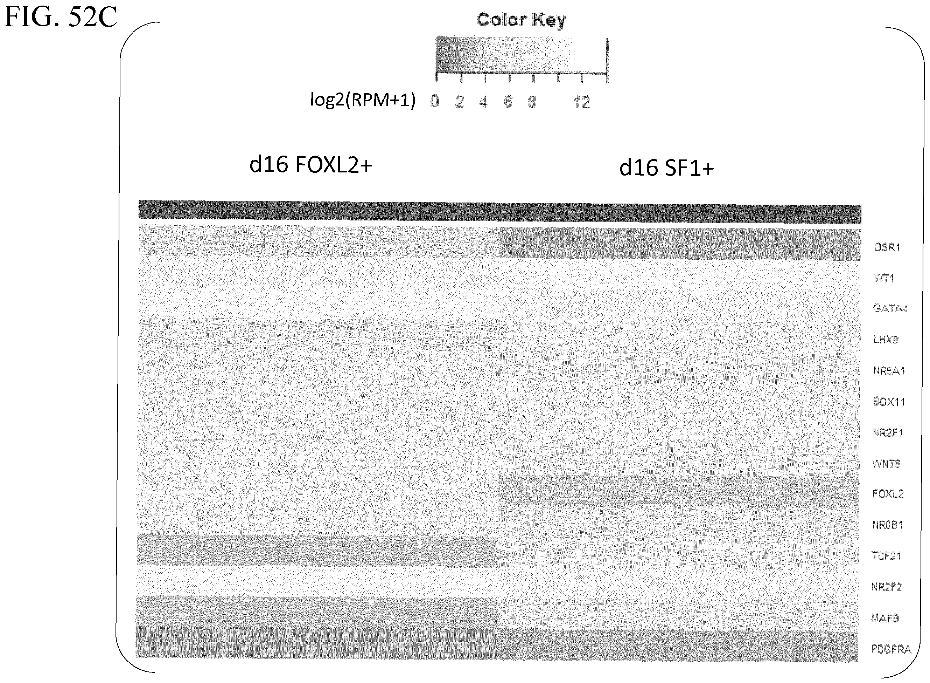

FIG. 52C is a view showing the results of RNAseq in "28." of Example 2.

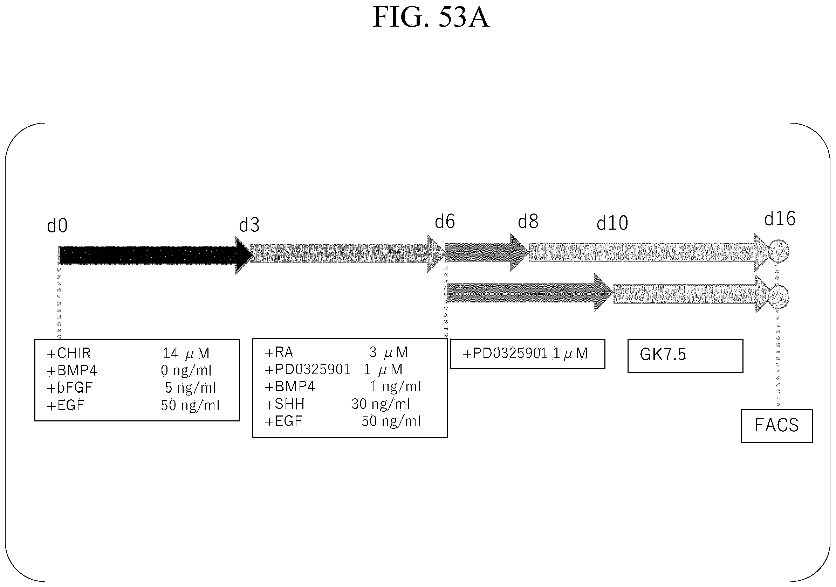

FIG. 53A is a view showing a protocol for inducing differentiation into ovarian somatic cell-like cells using cynomolgus monkey ES cells in "29." of Example 2.

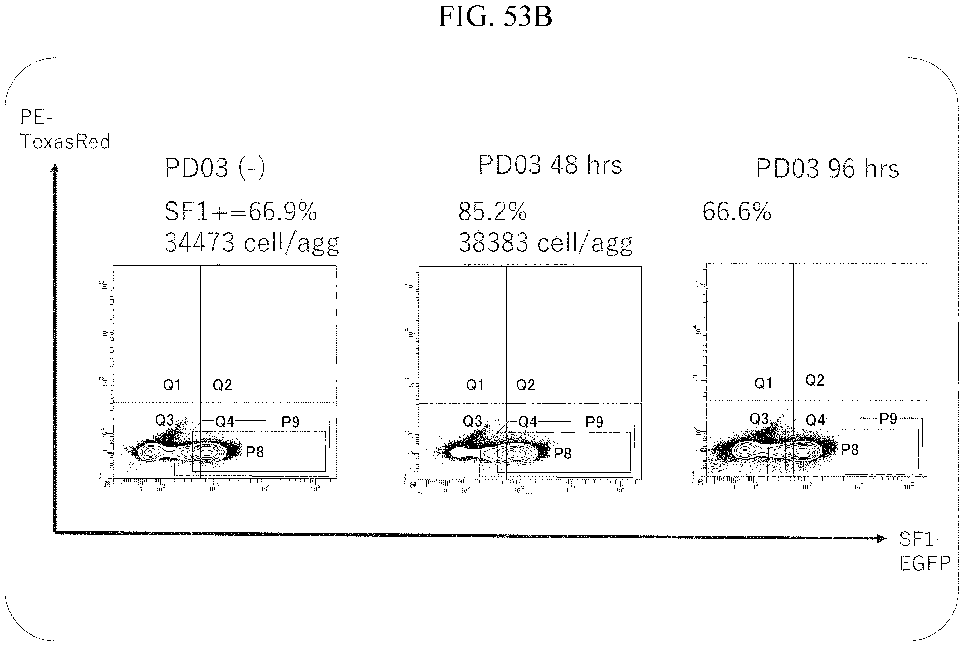

FIG. 53B is a view showing FACS plots of PE-Texas Red and NR5A1 (SF1)-EGFP in "29." of Example 2.

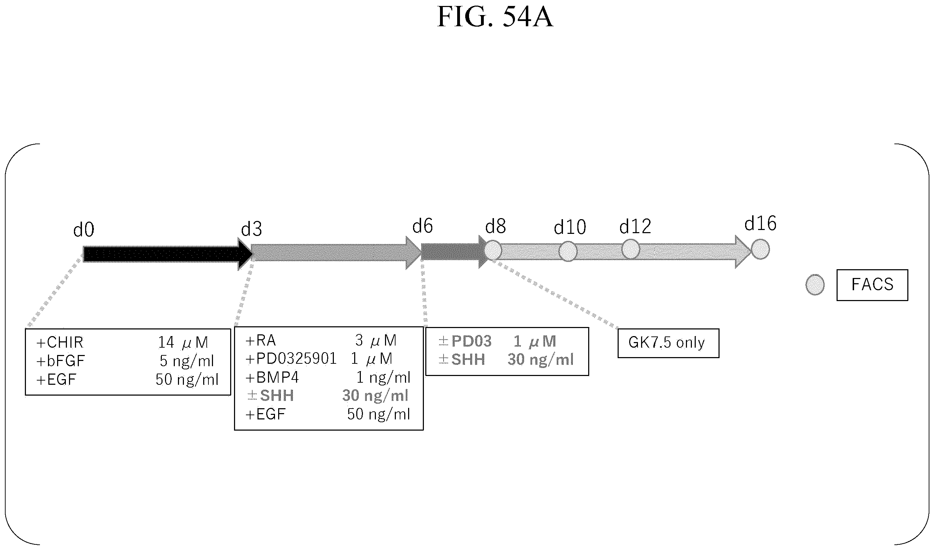

FIG. 54A is a view showing a protocol for inducing differentiation into ovarian somatic cell-like cells using cynomolgus monkey ES cells in "30." of Example 2.

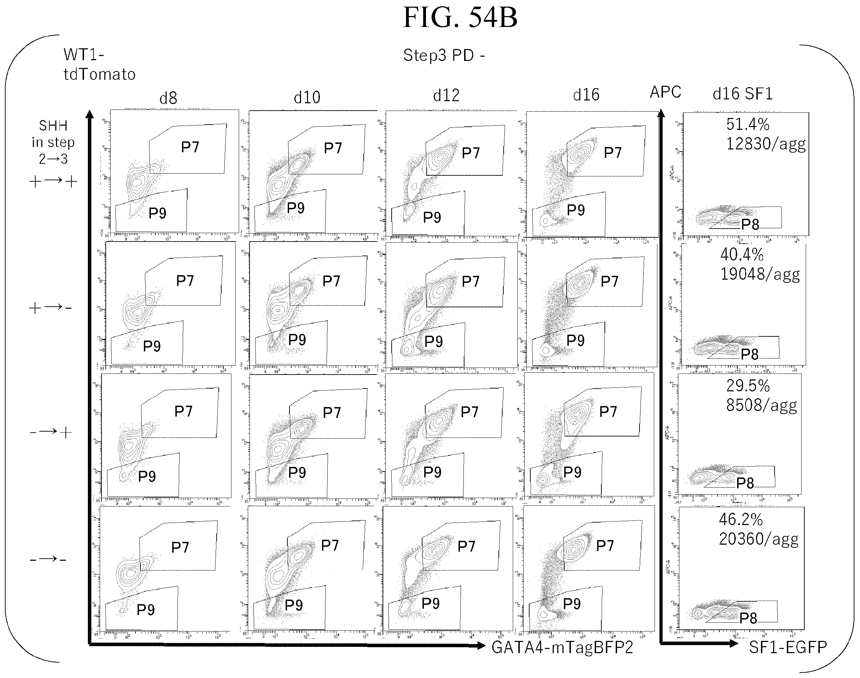

FIG. 54B is a view showing FACS plots under the condition in which PD0325901 was not added in "30." of Example 2.

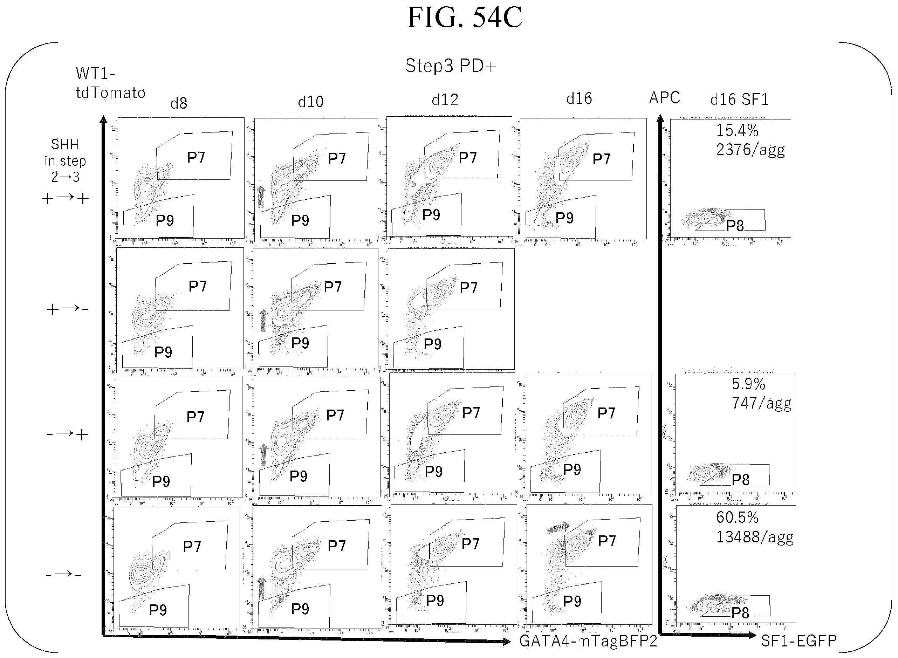

FIG. 54C is a view showing FACS plots under the condition in which PD0325901 in "30." of Example 2 was added.

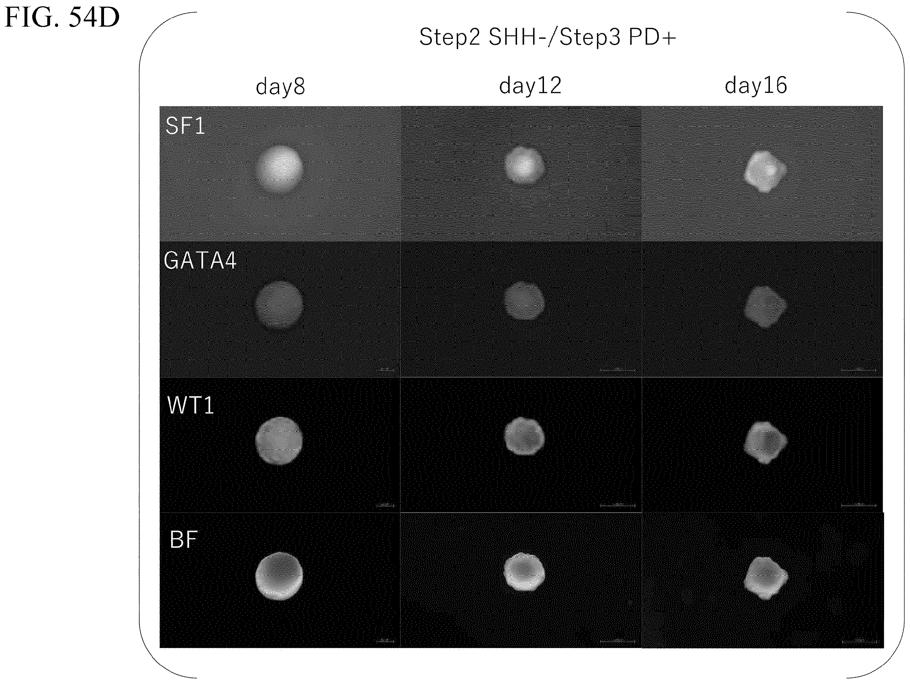

FIG. 54D is a view showing fluorescent images of the cell mass in "30." of Example 2.

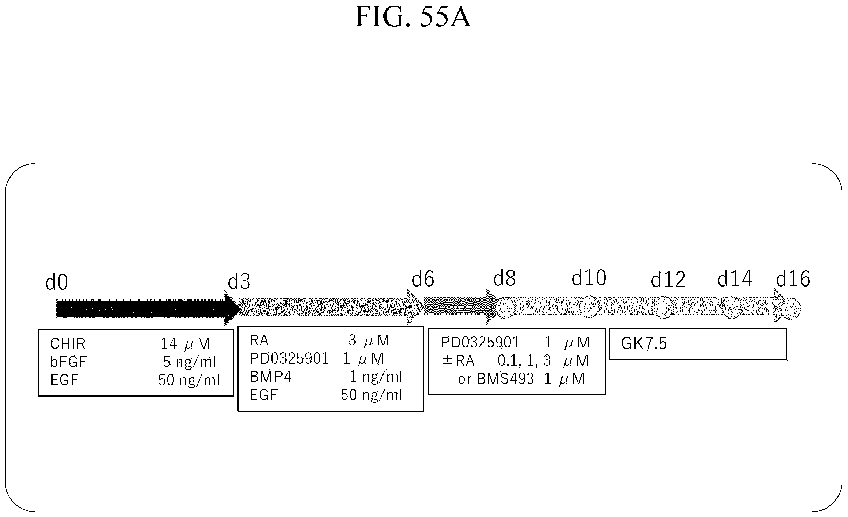

FIG. 55A is a view showing a protocol for inducing differentiation into ovarian somatic cell-like cells using cynomolgus monkey ES cells in "31." of Example 2.

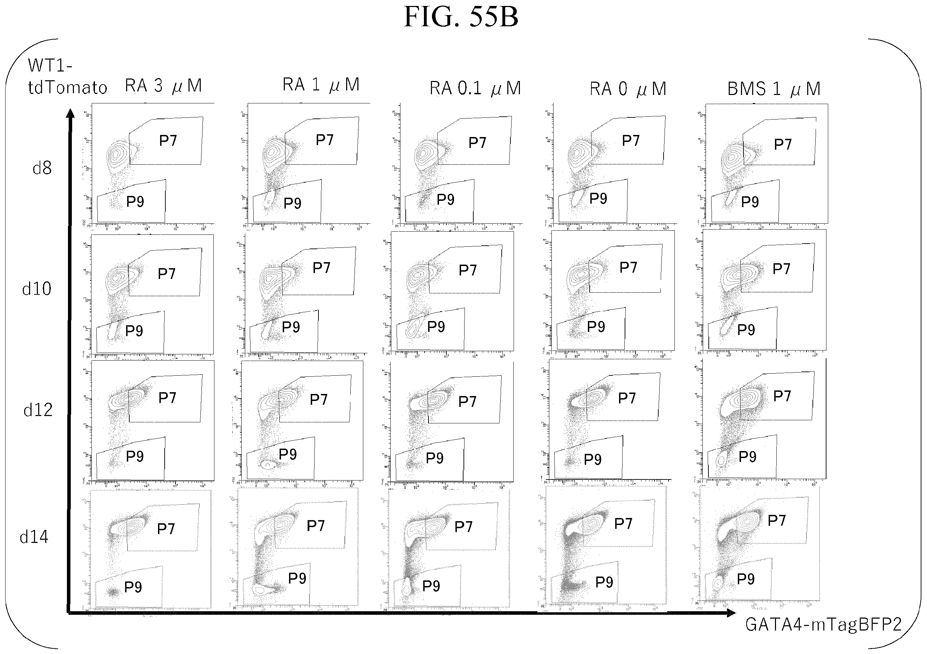

FIG. 55B is a view showing FACS plots of WT1-tdTomato and GATA4-mTagBFP2 in "31." of Example 2.

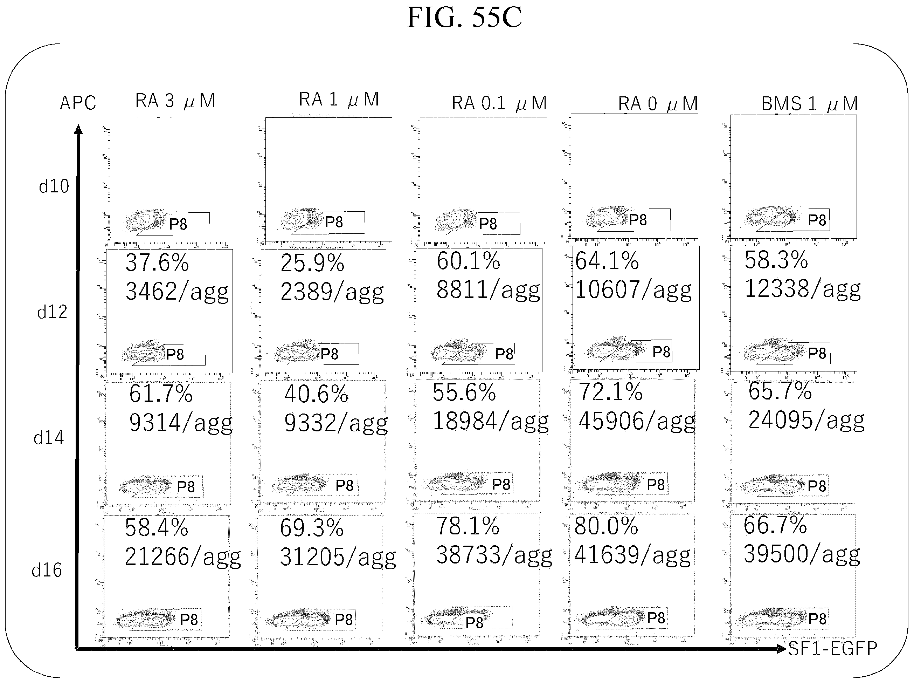

FIG. 55C is a view showing FACS plots of APC and NR5A1 (SF1)-EGFP in "31." of Example 2.

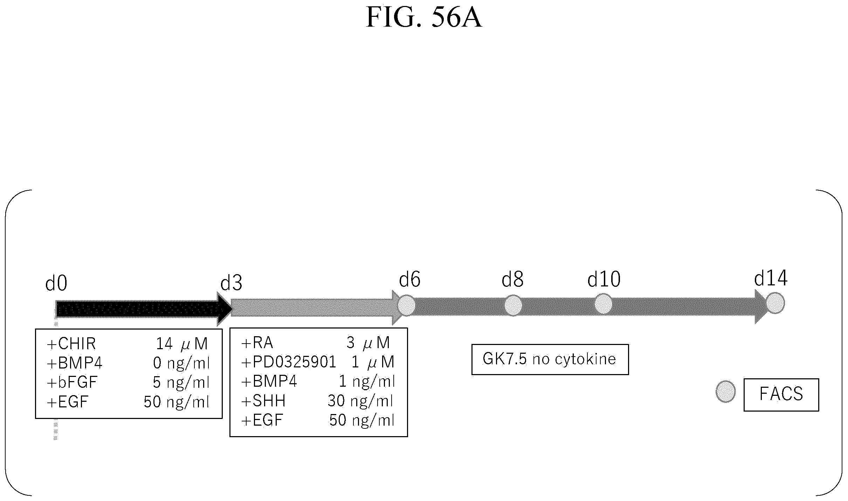

FIG. 56A is a view showing a protocol for inducing differentiation into ovarian somatic cell-like cells using cynomolgus monkey ES cells in "32." of Example 2.

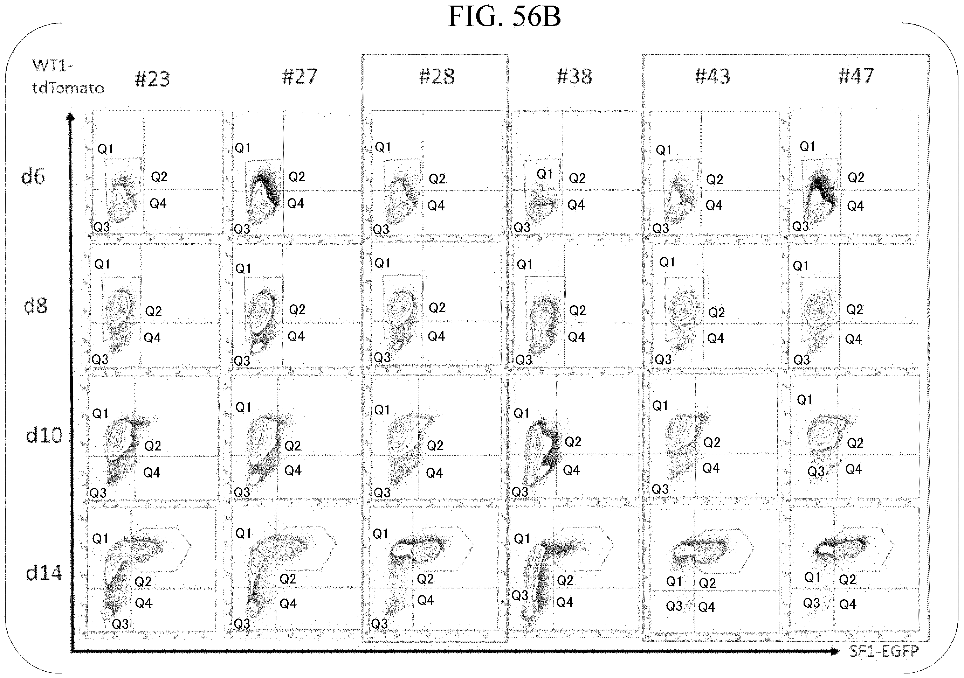

FIG. 56B is a view showing FACS plots of WT1-tdTomato and NR5A1(SF1)-EGFP in "32." of Example 2.

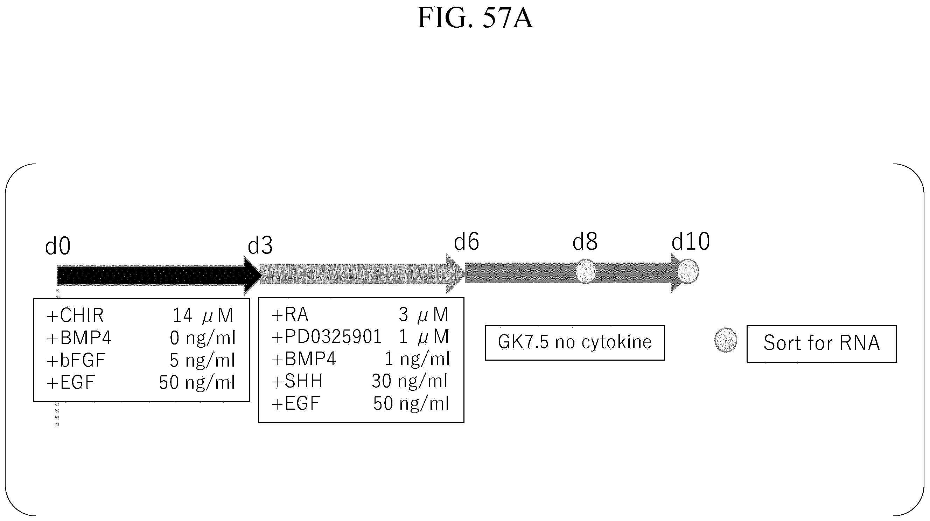

FIG. 57A is a view showing a protocol for inducing differentiation into ovarian somatic cell-like cells using cynomolgus monkey ES cells in "33." of Example 2.

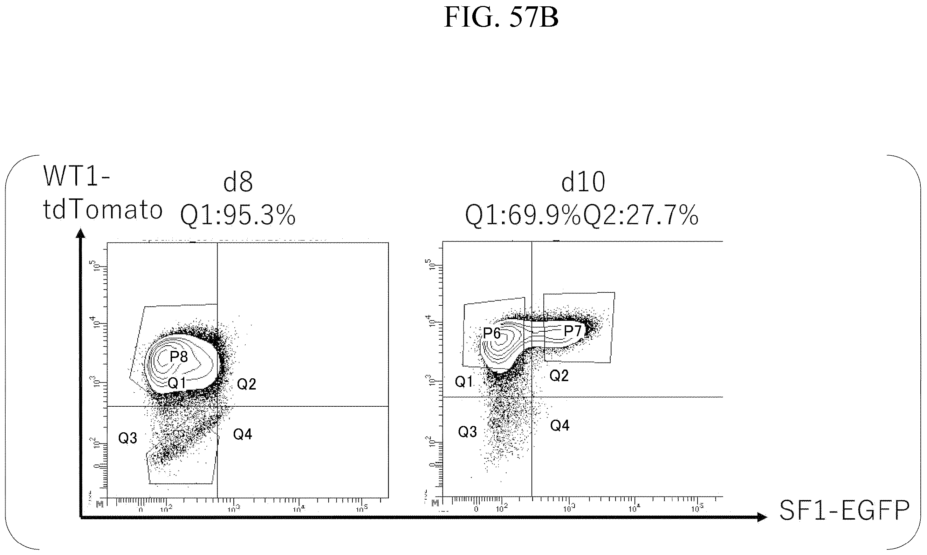

FIG. 57B is a view showing FACS plots of WT1-tdTomato and NR5A1(SF1)-EGFP in "33." of Example 2.

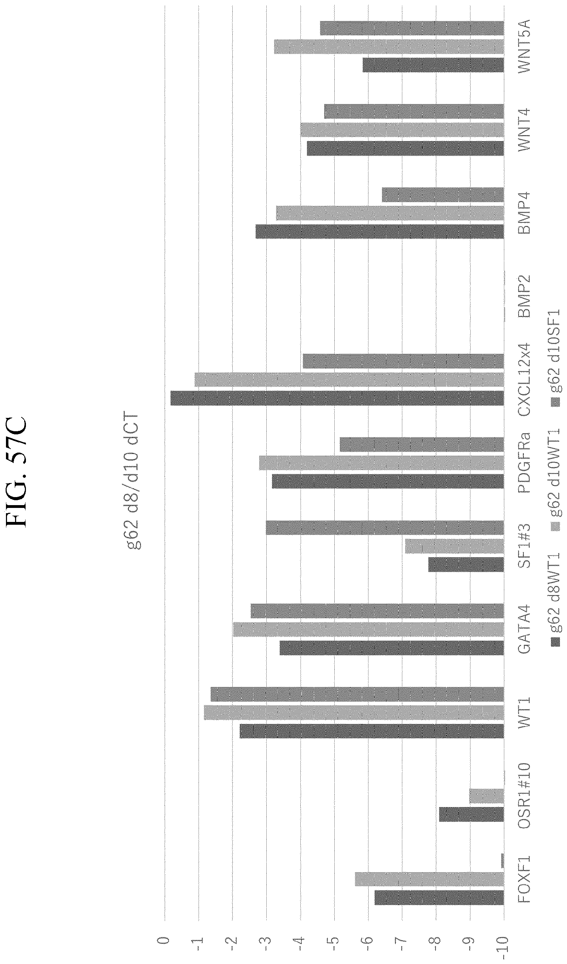

FIG. 57C is a graph showing the measurement results of quantitative PCR of each marker gene in "33." of Example 2.

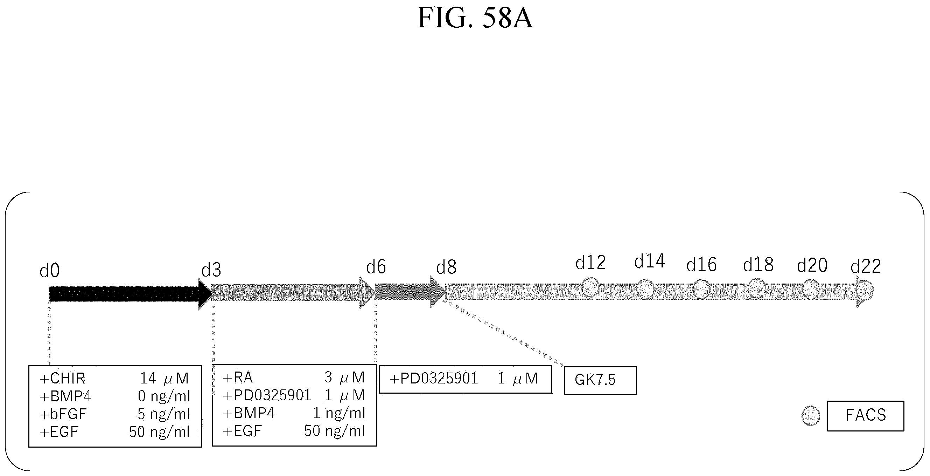

FIG. 58A is a view showing a protocol for inducing differentiation into ovarian somatic cell-like cells using cynomolgus monkey ES cells in "34." of Example 2.

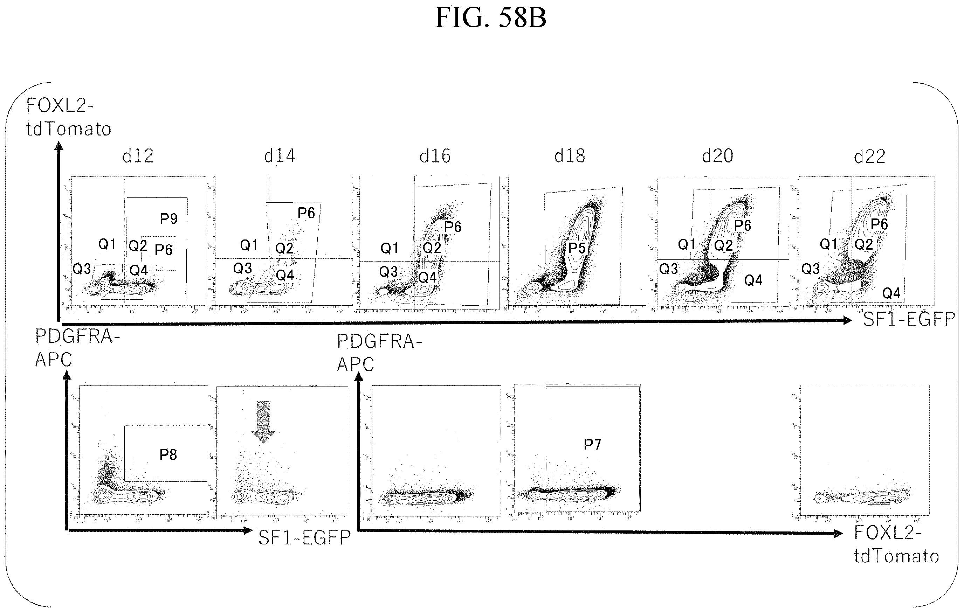

FIG. 58B is a view showing FACS plots on days 12 to 22 of induction in "34." of Example 2.

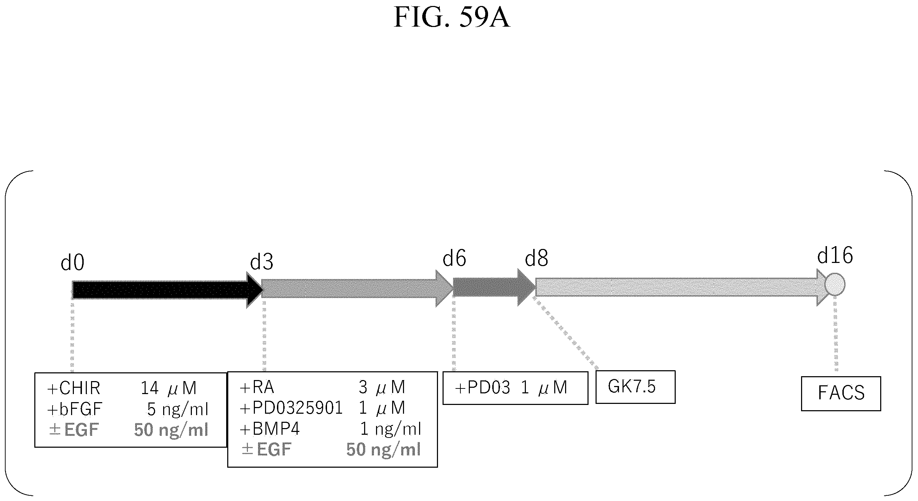

FIG. 59A is a view showing a protocol for inducing differentiation into ovarian somatic cell-like cells using cynomolgus monkey ES cells in "35." of Example 2.

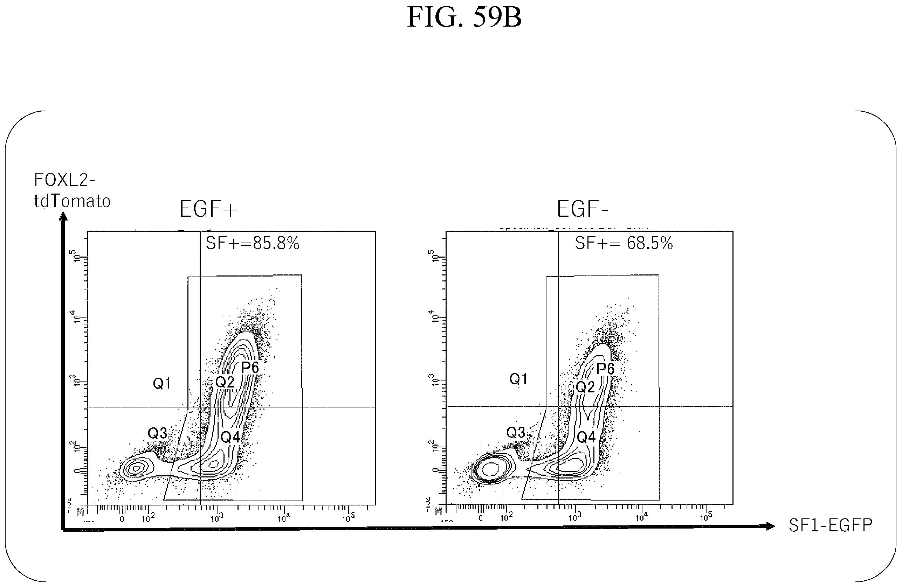

FIG. 59B is a view showing FACS plots of FOXL2-tdTomato and NR5A1(SF1)-EGFP in "35." of Example 2.

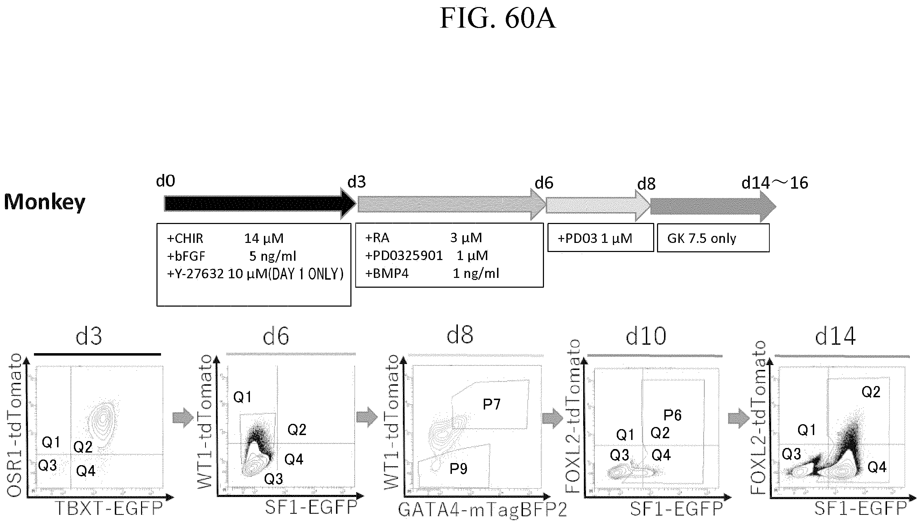

FIG. 60A is a view (upper part) showing a protocol for inducing differentiation into ovarian somatic cell-like cells using cynomolgus monkey ES cells established based on results up to "35." of Example 2, and FACS plots (lower part) on days 3 to 14 of induction.

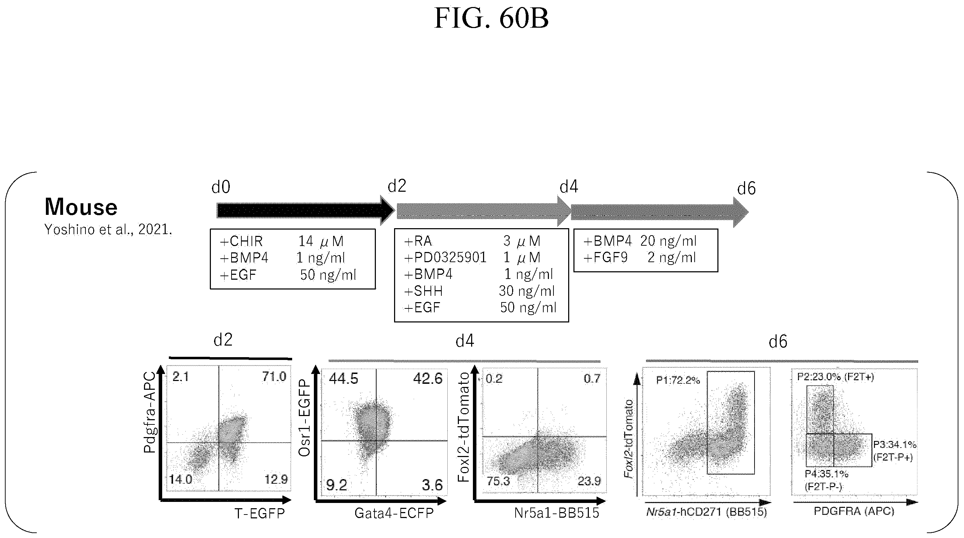

FIG. 60B is a view (upper side) showing a protocol in a case of inducing differentiation from mouse ES cells into ovarian somatic cell-like cells, and FACS plots (lower side) on days 2 to 6 of induction.

### [Description of Embodiments]

### <Method for Producing Ovarian Somatic Cell-Like Cells>

The method for producing ovarian somatic cell-like cells of the present embodiment (which may be hereinafter simply referred to as "the production method of the present embodiment") includes the following steps:
a step 1 of culturing primate pluripotent stem cells in a medium including a GSK3 inhibitor and a ROCK inhibitor;
a step 2 of culturing the cells from step 1 in a medium including BMP4, retinoic acid, and an MEK inhibitor; and
a step 3 of culturing the cells from step 2 in a base medium to obtain ovarian somatic cell-like cells.

According to the production method of the present embodiment, ovarian somatic cell-like cells at any developmental stage can be efficiently obtained from primate pluripotent stem cells. That is, the production method of the present embodiment can also be said to be a method for inducing differentiation of primate pluripotent stem cells into ovarian somatic cell-like cells.

Hereinafter, each of steps constituting the production method of the present embodiment will be described in detail.

### [Step 1]

In step 1, primate pluripotent stem cells are cultured in a medium including a GSK3 inhibitor and a ROCK inhibitor.

In the present specification, the term "pluripotent stem cell" refers to an undifferentiated cell having "self-renewal ability" capable of proliferating while maintaining an undifferentiated state and "differentiation pluripotency" capable of being differentiated into all three germ layer lines. The pluripotent stem cell is not limited to the following, and examples thereof include induced pluripotent stem cells (iPS cells), embryonic stem cells (ES cells), embryonic germ cells derived from primordial germ cells (EG cells), and testis tissue. Examples thereof include multipotent germline STEM cells (mGS cells) isolated in the process of establishing and culturing GS cells from, and Muse cells isolated from bone marrow mesenchymal cells. The pluripotent stem cells listed above can be obtained by known methods.

The pluripotent stem cells used in step 1 are derived from primates. The term "primates" refers to mammals belonging to the order Primates, and examples of the primates include the suborder Prosimii such as fox monkeys or Loris, and treeshrews, and Anthropoidea such as monkeys (including cynomolgus monkeys), apes, and humans. Among these, cynomolgus monkeys or humans are preferable.

Furthermore, the term "iPS cells" in the present specification refers to cells that can be reprogrammed into cells of various tissues and organs by introducing some genes into differentiated somatic cells. In the method of the present embodiment, the iPS cells used for inducing differentiation of primordial germ cells may be derived from primary cultured cells of somatic cells collected from an appropriate donor, or may be derived from an established cell strain. Since the iPS cells can induce differentiation into any germ layer cells, the somatic cells used for preparing the iPS cells may be, in principle, derived from either ectoderm or endoderm cells. Cells in skin, hair, gingiva, blood, or the like, which are less invasive and easy to collect, are suitable as somatic cells used for the preparation of the iPS cells. As for the method for preparing iPS cells, a method known in the art may be followed. Specifically, for example, the preparation methods described in known references such as "Okita K. et al.," Generation of germline-competent induced pluripotent stem cells.", Nature, Vol. 448, p313-317, 2007." (Reference 1), and "Hamanaka S. et al., "Generation of germline-competent rat induced pluripotent stem cells.", PLoS One, Vol. 6, Issue 7, e22008, 2011." (Reference 2) are available.

Moreover, the ES cells can be obtained by a known method. For example, they can be established by collecting an inner cell mass from the blastocyst of a fertilized egg of a target animal and culturing the inner cell mass on a feeder cell derived from fibroblasts. In addition, ES cells established by culturing an early embryo produced by nuclear transplantation of a nucleus of a somatic cell can also be used.

The medium used in step 1 includes a GSK3 inhibitor and a ROCK inhibitor as essential additives.

The GSK3 inhibitor is a compound having inhibitory activity against a glycogen synthase 3 (GSK3). Examples of such a compound include CHIR99021 (CAS Number: 252917-06-9), SB216763 (CAS Number: 280744-09-4), SB415286 (CAS Number: 264218-23-7), CHIR98014 (CAS Number: 252935-94-7), AZD1080 (CAS number: 612487-72-6), and LY2090314 (CAS number: 603288-22-8), and among these, CHIR99021 is preferable.

The concentration of the GSK3 inhibitor in the medium is preferably 5 µM or more, more preferably 10 µM or more, still more preferably 12 µM or more, and particularly preferably 14 µM or more. In a case where the concentration of the GSK3 inhibitor is equal to or more than the lower limit value, a WNT signal can be activated, and thus, the cells can be more efficiently differentiated into the mesoderm. The upper limit of the concentration of the GSK3 inhibitor is not particularly limited, but may be, for example, 20 µM. Furthermore, the unit "µM" indicates a concentration that is 1/1,000,000 of the molecular weight (mol/L) in 1 liter of a growth medium, and hereinafter means the same concentration.

The ROCK inhibitor is a compound having inhibitory activity against a Rho-associated kinase (ROCK). Examples of such a compound include Y-27632 (CAS number: 146986-50-7), Fasudil (CAS number: 105628-07-7), Y39983 (CAS number: 203911-26-6), Wf-536 (CAS Number: 539857-64-2), SLx-2119 (CAS Number: 911417-87-3), Azabenzimidazole-aminofurazans (CAS Number: 850664-21-0), DE-104, H-1152P (CAS number: 872543-07-6), a Rho kinase α inhibitor (ROKα inhibitor), XD-4000, HMN-1152, 4-(1-aminoalkyl)-N-(4-pyridyl)cyclohexane-carboxamides (4-(1-aminoalkyl)-N-(4-pyridyl)cyclohexane-carboxamides), Rhostatin, BA-210, BA-207, Ki-23095, and VAS-012. Among these, Y-27632 is preferable.

The concentration of the ROCK inhibitor in the medium can be usually set to 1 µM or more and 20 µM or less, and is preferably 5 µM or more and 15 µM or less, and more preferably 8 µM or more and 12 µM or less. In a case where the concentration of the ROCK inhibitor is within the range, cell death in step 1 can be more effectively suppressed.

In a case where human pluripotent stem cells are used, it is preferable that the medium used in step 1 further include a bone morphogenetic protein 4 (BMP4). As a result, the expression of GATA4 is promoted and the differentiation into mesoderm can be more efficiently achieved.

On the other hand, in a case where cynomolgus monkey pluripotent stem cells are used, it is preferable that the medium used in step 1 not include BMP4. As a result, the induction efficiency for NR5A1 -positive cells can be further improved.

In a case where the medium used in step 1 includes BMP4, the concentration of BMP4 in the medium can be 0.1 ng/mL or more, and is preferably 0.3 ng/mL or more, more preferably 0.5 ng/mL or more, and still more preferably 0.7 ng/mL or more. On the other hand, the concentration of BMP4 in the medium can be 10 ng/mL or less, and is preferably 5 ng/mL or less, more preferably 3 ng/mL or less, and still more preferably 1.3 ng/mL or less. In a case where the concentration of BMP4 is within the range, the expression of GATA4 is promoted, and thus, the differentiation into the mesoderm can be more efficiently achieved.

In a case where cynomolgus monkey pluripotent stem cells are used, it is preferable that the medium used in step 1 further include a basic fibroblast growth factor (bFGF; also referred to as FGF2). As a result, the differentiation into the mesoderm can be more efficiently acheved.

On the other hand, in a case where human pluripotent stem cells are used, it is preferable that the medium used in step 1 not include bFGF. As a result, the induction efficiency for NR5A1-positive cells can be further improved.

In a case where the medium used in step 1 includes bFGF, the concentration of bFGF in the medium can be 0.1 ng/mL or more, and is preferably 0.5 ng/mL or more, more preferably 1 ng/mL or more, and still more preferably 3 ng/mL or more. On the other hand, the concentration of bFGF in the medium can be 10 ng/mL or less, and is preferably 9 ng/mL or less, more preferably 8 ng/mL or less, and still more preferably 7 ng/mL or less. In a case where the concentration of bFGF is within the range, the differentiation into the mesoderm can be more efficiently achieved.

The medium used in step 1 may or may not include an epidermal growth factor (EGF).

Examples of the base medium to be used by the addition of the additive in step 1 include, but are not limited to, an αMEM medium, a Neurobasal medium, a Neural Progenitor Basal medium, an NS-A medium, a BME medium, a BGJb medium, a CMRL 1066 medium, a minimum essential medium (MEM), an Eagle MEM, a Dulbecco's modified Eagle's medium (DMEM), a Glasgow MEM (GMEM), an Improved MEM Zinc Option, IMDM, a Medium 199 medium, a DMEM/F12 medium, a StemPro-34SFM medium, a Ham's medium, an RPMI 1640 medium, an HTF medium, a Fischer's medium, an Advanced DMEM, an Advanced DMEM/F12, an Advanced MEM, an Advanced RPMI medium, and a mixed medium thereof. Among those, in a case where cynomolgus monkey-derived pluripotent stem cells are used, the GMEM is preferable, and in a case where human-derived pluripotent stem cells are used, the Advanced DMEM or the Advanced DMEM/F12 is preferable, and the Advanced DMEM/F12 is more preferable.

The medium may be either a serum-containing medium or a serum-free medium. The serum-free medium is preferably used. The serum-free medium (SFM) means a medium that does not include any of unprocessed or unpurified serum, and examples thereof include a medium including purified blood-derived components or animal tissue-derived components (proliferation factors and the like). The concentration of the serum (for example, a fetal bovine serum (FBS) and a human serum) can be 0 v/v% or more and 20 v/v% or less, and is preferably 0 v/v% or more and 5 v/v% or less, more preferably 0 v/v% or more and 2 v/v% or less, and still more preferably 0 v/v% (that is, serum-free). The SFM may include any serum substitute. Examples of the serum substitute include albumin (for example, lipid-rich albumin and albumin substitutes such as recombinant albumin; plant starch, dextrans, and protein hydrolysates), transferrin (or other iron transporters), fatty acids, insulin, collagen precursors, trace elements, 2-mercaptoethanol, 3'-thioglycerol, and equivalents thereto. In addition, other examples include KnockOut (registered trademark) Serum Replacement (KSR), and GlutaMax (registered trademark). These can be used singly or in combination of two or more kinds thereof.

The medium may include other known additives. The additive is not particularly limited, but examples thereof include growth factors other than the above-described ones, polyamines, minerals, sugars (for example, glucose), organic acids (for example, pyruvic acid or lactic acid), salts thereof (a sodium salt, a potassium salt, and the like), amino acids (for example, non-essential amino acids (NEAA) and L-glutamine), reducing agents (for example, 2-mercaptoethanol), vitamins (for example, ascorbic acid and d-biotin), steroids, antibiotics (for example, streptomycin and penicillin), buffers (for example, HEPES), nutritional additives (for example, a B27 supplement, an N2 supplement, and a StemPro-Nutrient Supplement). These can be used singly or in combination of two or more kinds thereof. Each additive can be included in a known concentration range.

In human pluripotent stem cells, examples of a base medium to be preferably used include Advanced DMEM or Advanced DMEM/F12 which includes KSR (a preferred concentration: 6.0 v/v% or more and 9.0 v/v% or less), 2-mercaptoethanol (a preferred concentration: 0.01 mM or more), and 0.5 mM or less), penicillin and streptomycin (a preferred concentration: 50 U/mL or more and 150 U/mL or less), and GlutaMax (registered trademark) (a preferred concentration: 0.5 mM or more and 5.0 mM or less).

In human pluripotent stem cells, examples of a base medium to be more preferably used include Advanced DMEM/F12 which includes KSR (a preferred concentration: 7.0 v/v% or more and 8.0 v/v% or less), 2-mercaptoethanol (a preferred concentration: 0.05 mM or more), and 0.15 mM or less), penicillin and streptomycin (a preferred concentration: 80 U/mL or more and 120 U/mL or less), and GlutaMax (registered trademark) (a preferred concentration: 1.5 mM or more and 2.5 mM or less).

In cynomolgus monkey pluripotent stem cells, examples of the base medium to be preferably used include GMEM which includes KSR (a preferred concentration: 7.0 v/v% or more and 8.0 v/v% or less), 2-mercaptoethanol (a preferred concentration: 0.05 mM or more, and 0.15 mM or less), NEAA (a preferred concentration: 0.05 mM or more and 0.15 mM or less), pyruvate (a preferred concentration: 0.5 mM or more and 1.5 mM or less), penicillin and streptomycin (a preferred concentration: 80 U/mL or more and 120 U /mL or less), and L-glutamine (a preferred concentration: 1.5 mM or more and 2.5 mM or less).

The culture of step 1 can be performed, for example, by seeding primate pluripotent stem cells in a known cell non-adhesive or low-adhesive culture container, and culturing the primate pluripotent stem cells.

The culture conditions are not limited to the following ranges, but the culture can be performed, for example, in an atmosphere of 1 v/v% or more and 10 v/v% or less of carbon dioxide and 90 v/v% or more and 99 v/v% or less of air atmosphere.

The culture temperature is about 30°C or higher and 40°C or lower, and preferably about 37°C.

The culture period is preferably 2 days or longer, and more preferably 3 days or longer and 5 days or less.

In a case where human pluripotent stem cells are used, in order to promote more appropriate induction of differentiation into ovarian somatic cells, the culture of step 1 is performed by seeding the primate pluripotent stem cells in a known cell adhesive culture container, and preferably by carrying out plane culture.

For the plane culture in step 1, it is preferable to use a cell adhesive culture container coated with a cell scaffold material. As the cell scaffold material, a known extracellular matrix (ECM) can be used, and specific examples thereof include, but are not limited to, Matrigel (registered trademark) (manufactured by BD Biosciences) including fibronectin, vitronectin, laminin, entactin, and collagen IV.

In a case where human pluripotent stem cells are used, step 1 preferably includes the following step 1-1 and step 1-2:
a step 1-1 of culturing primate pluripotent stem cells in a medium including a GSK3 inhibitor and a ROCK inhibitor; and
a step 1-2 of culturing the cells from step 1-1 in a medium including a GSK3 inhibitor and Activin A.

As the GSK3 inhibitor and the ROCK inhibitor used in step 1-1 and step 1-2, those exemplified above can be used. In addition, in step 1-1 and step 1-2, the additive can be added to the base medium exemplified above and used.

Activin A used in step 1-2 is a TGF-β family member produced by multiple cell species during a process of development. In a case where Activin A is added, differentiation into ovarian somatic cells can be performed by posteriorization.

The concentration of Activin A in the medium can be set to 1 ng/mL or more and 30 ng/mL or less, and is preferably 3 ng/mL or more and 20 ng/mL or less, more preferably 5 ng/mL or more and 15 ng/mL or less, and still more preferably 8 ng/mL or more and 12 ng/mL or less. In a case where the concentration of Activin A is within the range differentiation into ovarian somatic cells can be performed by the posteriorization.

The culture conditions in step 1-1 and step 1-2 are not limited to the following ranges, but the culture is performed, for example, in an atmosphere of 1 v/v% or more and 10 v/v% or less of carbon dioxide and 90 v/v% or more and 99 v/v% or less of air atmosphere.

The culture temperature is about 30°C or higher and 40°C or lower, and preferably about 37°C.

The culture period may be any number of days so that the total culture period in steps 1-1 and 1-2 is the culture period in step 1, and the culture period of each of step 1-1 and step 1-2 is preferably 1 day or longer, and more preferably 2 days or longer and 3 days or less.

The differentiation of the cells obtained in step 1 into the mesoderm can be confirmed by analyzing the expression levels of marker genes such as OSR1 and TBXT, using a known method such as immunostaining, FACS analysis, quantitative PCR, and RNAseq.

### [Step 2]

In step 2, the cells from step 1 are cultured in a medium including BMP4, retinoic acid, and an MEK inhibitor.

As the BMP4 used in step 2, the same BMP4 as that described in step 1 can be used.

In step 2, the concentration of BMP4 in the medium can be set to 0.1 ng/mL or more, and is preferably 0.3 ng/mL or more, more preferably 0.5 ng/mL or more, and still more preferably 0.7 ng/mL or more. On the other hand, the concentration of BMP4 in the medium can be 10 ng/mL or less, and is preferably 5 ng/mL or less, more preferably 3 ng/mL or less, and still more preferably 1.3 ng/mL or less. In a case where the concentration of BMP4 is within the range, the expression of GATA4 is promoted, and thus, the differentiation into the intermediate mesoderm can be more efficiently achieved.

In step 2, the concentration of retinoic acid in the medium can be set to 0.1 µM or more and 10 µM or less, and is preferably 0.3 µM or more and 7 µM or less, more preferably 0.5 µM or more and 5 µM or less, and still more preferably 1 µM or more and 3 µM or less. In a case where the concentration of retinoic acid is within the range, differentiation into the intermediate mesoderm can be more efficiently achieved.

The MEK inhibitor used in step 2 is a compound having inhibitory activity against the mitogen-activated extracellular signal-related kinase. Examples of such a compound include, but are not limited to, PD0325901 (CAS number: 391210-10-9), trametinib (trade name "Mekinist", CAS number: 871700-17-3), selumetinib (CAS number: 606143-52-6), MEK162 (CAS number: 606143-89-9), and CH4987655 (CAS number: 874101-00-5). Among these, PD0325901 is preferable.

The concentration of the MEK inhibitor in the medium is preferably 0.1 µM or more and 5 µM or less, more preferably 0.5 µM or more and 3 µM or less, and still more preferably 0.8 µM or more and 1.2 µM or less. In a case where the concentration of the MEK inhibitor is within the range, NR5A1-positive cells can be more efficiently induced.

In a case where human pluripotent stem cells are used, and further in a case where the plane culture is performed in step 1, the medium used in step 2 preferably further includes a ROCK inhibitor. As a result, cell death can be more effectively suppressed in a case where the cell obtained in step 1 is re-seeded as a single cell and suspension-cultured in step 2. As the ROCK inhibitor, those exemplified in step 1 can be used.

In a case where the medium used in step 2 includes the ROCK inhibitor, the concentration can be usually set to 1 µM or more and 20 µM or less, and is preferably 5 µM or more and 15 µM or less, and more preferably 8 µM or more and 12 µM or less. In a case where the concentration of the ROCK inhibitor is within the range, cell death in step 2 can be more effectively suppressed.

In a case where human pluripotent stem cells are used, the medium used in step 2 preferably further includes a hedgehog signaling activator. As a result, NR5A1-positive cells can be more efficiently induced.

On the other hand, in a case where cynomolgus monkey pluripotent stem cells are used, it is preferable that the medium used in step 2 not include the hedgehog signaling activator. As a result, differentiation into NR5A1-positive cells can be more efficiently achieved.

Examples of the hedgehog signaling activator used in step 2 include, but are not limited to, a sonic hedgehog (SHH) and a smooth agonist (SAG; CAS number: 364590-63-6). Among these, the SHH is preferable.

In a case where the medium used in step 2 includes the hedgehog signaling activator, the concentration of the hedgehog signaling activator in the medium can be 1 ng/mL or more, and is preferably 5 ng/mL or more, more preferably 15 ng/mL or more, and still more preferably 25 ng/mL or more. On the other hand, the concentration of the hedgehog signaling activator in the medium can be 50 ng/mL or less, and is preferably 45 ng/mL or less, more preferably 40 ng/mL or less, and still more preferably 35 ng/mL or less. In a case where the concentration of the hedgehog signaling activator is within the range, NR5A1-positive cells can be more efficiently induced.

The medium used in step 2 may or may not include an epidermal growth factor (EGF).

In step 2, the above-described additive can be added to the base medium and used. As the base medium, the same base media as those exemplified in step 1 can be used. In addition, as the other additives to be added to the medium, the same additives as those exemplified in step 1 can be used.

The culture of step 1 can be performed, for example, by seeding primate pluripotent stem cells in a known cell non-adhesive or low-adhesive culture container, and culturing the primate pluripotent stem cells.

The culture conditions are not limited to the following ranges, but the culture can be performed, for example, in an atmosphere of 1 v/v% or more and 10 v/v% or less of carbon dioxide and 90 v/v% or more and 99 v/v% or less of air atmosphere.

The culture temperature is about 30°C or higher and 40°C or lower, and preferably about 37°C.

The culture period is preferably 2 days or longer, and more preferably 3 days or longer.

The differentiation of the cells obtained in step 2 into the intermediate mesoderm can be confirmed by analyzing the expression levels of marker genes such as WT1 and NR5A1, using a known method such as immunostaining, FACS analysis, quantitative PCR, and RNAseq.

### [Step 3]

In step 3, the cells from step 2 are cultured in a base medium to obtain ovarian somatic cell-like cells.

As the base medium used in step 3, the same base media as those exemplified in step 1 can be used.

In a case where human pluripotent stem cells are used, it is preferable that the base medium not include additives such as BMP4, an FGF signaling activator, and an MEK inhibitor. Furthermore, examples of the FGF signaling activator mentioned herein include an FGF family protein. Examples thereof include, but are not limited to, FGF9, bFGF, and FGF4.

That is, in a case where human pluripotent stem cells are used, examples of the base medium preferably used in step 3 include Advanced DMEM or Advanced DMEM/F12 which includes KSR (a preferred concentration: 6.0 v/v% or more and 9.0 v/v% or less), 2-mercaptoethanol (a preferred concentration: 0.01 mM or more), and 0.5 mM or less), penicillin and streptomycin (a preferred concentration: 50 U/mL or more and 150 U/mL or less), and GlutaMax (registered trademark) (a preferred concentration: 0.5 mM or more and 5.0 mM or less).

In a case where human pluripotent stem cells are used, examples of the base medium more preferably used in step 3 include Advanced DMEM/F12 which includes KSR (a preferred concentration: 7.0 v/v% or more and 8.0 v/v% or less), 2-mercaptoethanol (a preferred concentration: 0.05 mM or more), and 0.15 mM or less), penicillin and streptomycin (a preferred concentration: 80 U/mL or more and 120 U/mL or less), and GlutaMax (registered trademark) (a preferred concentration: 1.5 mM or more and 2.5 mM or less).

The culture of step 3 can be performed, for example, by seeding primate pluripotent stem cells in a known cell non-adhesive or low-adhesive culture container, and culturing the primate pluripotent stem cells.

The culture conditions are not limited to the following ranges, but the culture can be performed, for example, in an atmosphere of 1 v/v% or more and 10 v/v% or less of carbon dioxide and 90 v/v% or more and 99 v/v% or less of air atmosphere.

The culture temperature is about 30°C or higher and 40°C or lower, and preferably about 37°C.

The culture period is preferably 1 week or more.

On the other hand, in a case where cynomolgus monkey pluripotent stem cells are used, it is preferable to use a medium further including an MEK inhibitor for one day or longer, more preferably about 2 days (for example, 48 hours ± 12 hours, and preferably 48 hours ± 6 hours) from the start of induction in step 3 in order to further improve the induction efficiency of NR5A1-positive cells.

That is, in a case where cynomolgus monkey pluripotent stem cells are used, step 3 preferably includes the following step 3-1 and step 3-2:
a step 3-1 of culturing the cells from step 2 in a medium including an MEK inhibitor; and
a step 3-2 of culturing the cells from step 3-1 in a base medium to obtain ovarian somatic cell-like cells.

Examples of the MEK inhibitor include the same ones as those exemplified in step 2 described above. The MEK inhibitor can be used by adding the MEK inhibitor to the base medium exemplified in step 1.

In step 3-1, the concentration of the MEK inhibitor in the medium is preferably 0.1 µM or more and 5 µM or less, more preferably 0.5 µM or more and 3 µM or less, and still more preferably 0.8 µM or more and 1.2 µM or less. In a case where the concentration of the MEK inhibitor is within the range, the induction efficiency of NR5A1-positive cells can be further improved.

The medium used in step 3-1 is preferably a medium in which only the MEK inhibitor has been added to a base medium and no other additives are included. That is, in a case where cynomolgus monkey pluripotent stem cells are used, examples of the medium preferably used in step 3-1 include GMEM which includes an MEK inhibitor (a preferred concentration: 0.8 µM or more and 1.2 µM or less), KSR (a preferred concentration: 7.0 v/v% or more and 8.0 v/v% or less), 2-mercaptoethanol (a preferred concentration: 0.05 mM or more, and 0.15 mM or less), NEAA (a preferred concentration: 0.05 mM or more and 0.15 mM or less), pyruvate (a preferred concentration: 0.5 mM or more and 1.5 mM or less), penicillin and streptomycin (a preferred concentration: 80 U/mL or more and 120 U /mL or less), and L-glutamine (a preferred concentration: 1.5 mM or more and 2.5 mM or less).

In addition, the medium used in step 3-2 is preferably a base medium including no other additives. That is, in a case where cynomolgus monkey pluripotent stem cells are used, examples of the base medium preferably used in step 3-2 include GMEM which includes KSR (a preferred concentration: 7.0 v/v% or more and 8.0 v/v% or less), 2-mercaptoethanol (a preferred concentration: 0.05 mM or more, and 0.15 mM or less), NEAA (a preferred concentration: 0.05 mM or more and 0.15 mM or less), pyruvate (a preferred concentration: 0.5 mM or more and 1.5 mM or less), penicillin and streptomycin (a preferred concentration: 80 U/mL or more and 120 U /mL or less), and L-glutamine (a preferred concentration: 1.5 mM or more and 2.5 mM or less).

The culture conditions in step 3-1 and step 3-2 are not limited to the following ranges, but the culture is performed, for example, in an atmosphere of 1 v/v% or more and 10 v/v% or less of carbon dioxide and 90 v/v% or more and 99 v/v% or less of air atmosphere.

The culture temperature is about 30°C or higher and 40°C or lower, and preferably about 37°C.

The culture period may be any number of days so that the total culture period in the steps 3-1 and 3-2 is the culture period in step 3, and the culture period in step 3-1 is preferably 1 day or longer, and preferably about 2 days (for example, 48 hours ± 12 hours, and preferably 48 hours ± 6 hours). The culture period in step 3-2 can be defined as the remaining period obtained by subtracting the culture period in step 3-1 from the culture period in step 3.

The differentiation of the cells obtained in step 3 into ovarian somatic cell-like cells can be confirmed by analyzing the expression levels of marker genes such as FOXL2 and FDGFRα, using a known method such as immunostaining, FACS analysis, quantitative PCR, and RNAseq.

The ovarian somatic cell-like cells obtained by the production method of the present embodiment are preferably cells having the same characteristics as the ovarian somatic cells of the fetus, that is, embryonic ovarian somatic cell-like cells, and have characteristics of being differentiated into granulosa cells that form follicles in the future, stromal cells, and the like.

In addition, as shown in Examples described later, the ovarian somatic cell-like cells obtained by the production method of the present embodiment using cynomolgus monkey pluripotent stem cells have a high percentage of cells having the characteristics of granulosa cells. Therefore, this method can also be referred to as a method for inducing differentiation of cynomolgus monkey pluripotent stem cells into granulosa cells.

The ovarian somatic cell-like cells obtained by the production method of the present embodiment can be aggregated and cultured with PGCs of primates or PGCLCs induced from pluripotent stem cells to construct spheroids that reproduce the developing ovary. By culturing or transplanting the cells into living bodies, there is a possibility that maturation of germ cells, referred to as progression of attenuating division, which could not be reproduced in vitro so far, and formation of follicles can be reproduced. That is, there is a possibility that a method for regenerating an ovary, a method for producing an egg, or a method for treating infertility, including aggregation and co-culture of the ovarian somatic cell-like cells obtained by the above-described production method, and PGCs or PGCLCs of primates, can be provided.

### [Examples]

Hereinafter, the present invention will be described in more detail with reference to Examples and Comparative Examples, but the present invention is not limited to these Examples.

### <Test for Examining Induction of Differentiation from Human iPS Cells into Ovarian Somatic Cell-Like Cells>

### [Materials]

### 1. Human iPS Cell Strain

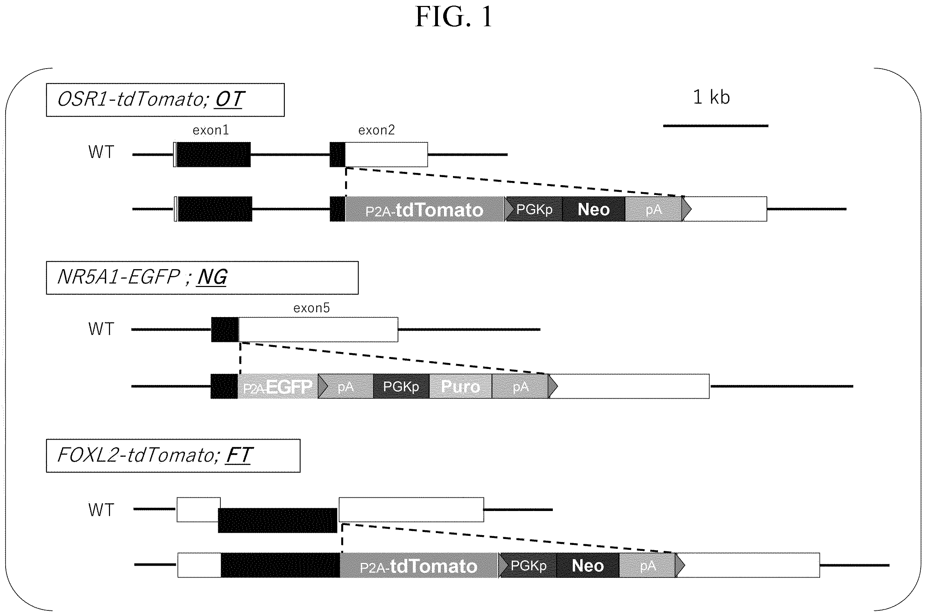

In order to conveniently observe the expression of genes typical at each developmental stage, a human iPS cell line 1390G3 was genetically modified, and a DNA sequence that emits red fluorescence (tdTomato) with the expression of the OSR1 gene, green fluorescence (EGFP) with the expression of the NR5A1 gene, and red fluorescence (tdTomato) with the expression of the FOXL2 gene was manufactured (see FIG. 1, and after introduction into the chromosome, a portion of "PGKp-Neo (or Puro)-pA" portion was deleted with Cre). An iPS cell line into which two DNA sequences of OSR1 and NR5A1 had been introduced (which may be hereinafter abbreviated as "OTNG"), and an iPS cell line into which two DNA sequences of NR5A1 and FOXL2 had been introduced (which may be hereinafter abbreviated as "NGFT") were manufactured. In the present test, the OTNG strain was used in most of the experiments. Furthermore, NR5A1 and SF1 are the same gene.

### 2. Medium and Drug

The supply sources and the model numbers of the media, the drugs, and the like are shown in the following table.

**[Table 1]**

| | **Supplier** | **REF** |
|---|---|---|
| Collagenase type IV | MPbio | 195110 |
| TrypLEselect | Gibco | 12563029 |
| Accumax | Innovative cell technologies, Inc. or Nacalai tesque | AM105 17087-54 |
| IWR-1 | Sigma | 10161-25MG |
| bFGF | Invitrogen | 13256-029 |
| PBS (-) | Nacalai tesque | 11480-35 |
| Y-27632 | TOCRIS | 1254-10 |
| CHIR99021 | TOCRIS | 4423 |
| BMP4 | R&D | 314-BP-01M |
| EGF | peprotech | AF-100-15 |
| BMS493 | Sigma | B6688-5MG |
| Retinoic acid | Sigma | R2625-50MG |
| Sonic Hedgehog | R&D | 1845-SH-100 |
| PD0325901 | Fujifilm Wako | 162-25291 |
| PD173074 | stemgent | 04-0008 |
| LDN193189 | stemgent | 04-0074 |
| SAG | R&D | 4366/1 |
| FGF9 | Peprotech R&D | 100-23-50 273-F9 |

**[Table 2]**

| | **Supplier** | **REF** |
|---|---|---|
| Advanced DMEM/F12 | Gibco | 12634010 |
| Advanced RPMI | Gibco | 12633020 |
| Neurobasal | Gibco | 12348017 |
| Albumax I | Gibco | 11020062 |
| ITS-G | Gibco | 41400045 |
| NEAA | Gibco | 11140-050 |
| Penicillin streptomycin | Gibco | 15140122 |
| L-glutamine | Gibco | 25030081 |
| GMEM | Gibco | 11710-035 |
| Sodium Pyruvate | Gibco | 11360070 |
| 2-ME | Gibco | 21985-023 |
| Knockout^{®} Serum Replacement | Gibco | 10828028 |
| 0.25% Trypsin-EDTA, phenol red | Gibco | 25200072 |
| Trypsin-EDTA (0.5%), no phenol red | Gibco | 15400054 |
| FBS | Nichirei | 174012 |
| 10x PBS with Calcium chloride and magnesium chloride | Sigma | D1283-500ML |
| Bovine Albumin Fraction V solution 7.5% | Gibco | 15260-037 |
| Nunclon Sphera 96-well Sphera-Treated, U-Shaped-Bottom Microplate | Thermo Fisher Scientific | 174925 |
| Greiner CELLSTAR, Cell-Repellent, 96 well micro plates, V-bottom | Greiner | 651970 |
| Water | Nacalai tesque | 06442-95 |
| PBS | Nacalai tesque | 14249-95 |

### [Method]

### 1. Fluorescent Immunostaining and Observation by Fluorescence Microscope

The antibodies used for immunostaining are as follows.
Rat anti-EGFP antibody (04404-84 manufactured by Nacalai Tesque, Inc., dilution rate: 250 times)
Rabbit anti-FOXL2 antibody (ab246511 manufactured by Abeam, dilution rate: 200 times)
Rabbit anti-laminin antibody (ab111575 manufactured by Abeam, dilution rate: 200 times)

In addition, the cells at each stage were observed using a fluorescence microscope (ZEISS confocal laser scanning, LSM780).

### 2. Fluorescence Activated Cell Sorting Analysis (FACS)

The cells at each stage were analyzed using a flow cytometer (BD FACSAria III).

### 3. Quantitative PCR

The relative expression level of each marker gene was confirmed using a PCR apparatus (BIO-RAD CFX384 Real-Time System) with the following primers and conditions. In the tables, the PPIA gene and the RPLP0 gene were used as housekeeping genes. The difference in qPCR is a difference from the average value taken as "(PPIA + RPLP0)/2". In addition, the ERCC gene was used as an absolute control between samples.

**[Table 3]**

| Name of | | Base sequence (5' → 3') | SEQ. NO. |
|---|---|---|---|
| OSR1 | Forward | CACTCACTCTCCGCAGTCAG | 1 |
| | Reverse | GCGCCTCTCCCAGAAGTATT | 2 |
| WT1 v2 | Forward | ATTTATAGGCCAGGGCATGTGT | 3 |
| | Reverse | TGTTAGACAAGATTCACCCCCG | 4 |
| GATA4 | Forward | CCTCTTTCTCAGCAGAGCTGTA | 5 |
| | Reverse | CTCTGCTACAGCCAGTAGGATT | 6 |
| NR5A1 | Forward | TAACATGAAAGCGGGACGGA | 7 |
| | Reverse | AGGTCATGAAGGACACTCTGC | 8 |
| FOXL2 v1 | Forward | AAATGTCCCCGGATCTTTCGAT | 9 |
| | Reverse | AAAACAAAAAGCAGGGAGGACG | 10 |
| FOXL2 v2 | Forward | TCCCCTCAGTTTATGTCCTCCT | 11 |
| | Reverse | GAAAGAGACGAGCCCAGTAGAA | 12 |
| FOXL2 v3 | Forward | GAGCAAACACACGTATTGGTCC | 13 |
| | Reverse | GTGCAGTCCATGGCTAGACG | 14 |
| CYP17A1 | Forward | TGGCCCCACAACACAGATTT | 15 |
| | Reverse | GGCATTGCCACAAGCTGAAA | 16 |
| GATA3 | Forward | GGATGCCAAGAAGTTTAAGGAA | 17 |
| | Reverse | GCCAGTGAAAGGAAACAAAACT | 18 |
| GATA6 | Forward | TTTTTCCTTTTGCAACGTGCCT | 19 |
| | Reverse | GGCCACTGGAATCATTTCTACC | 20 |
| MC2R | Forward | AACAGAAGCTTCAGGGCTCA | 21 |
| | Reverse | GTGCAGTTTGGTGGTGCTTT | 22 |
| SOX9 v1 | Forward | CATCTTCTCTTGGAGTGAGGGAG | 23 |
| | Reverse | AGCAATCCTCAAACTCTCTAGCC | 24 |
| WNT4 v2 | Forward | CAGTTCCCATCATACCAGCATCC | 25 |
| | Reverse | AGACCCAGAAAGAGGGAACAATG | 26 |

**[Table 4]**

| Name of gene | | Base sequence (5' → 3') | SEQ. NO. |
|---|---|---|---|
| PPIA | Forward | TTGATCATTTGGTGTGTTGGGC | 27 |
| | Reverse | AAGACTGAGATGCACAAGTGGT | 28 |
| RPLP0 | Forward | GAAACTCTGCATTCTCGCTTCC | 29 |
| | Reverse | ACTCGTTTGTACCCGTTGATGA | 30 |
| PDGFRA | Forward | TCCCCACAGGCACATTAACT | 31 |
| | Reverse | TCTCACACATTCACCACACCA | 32 |
| HOXA7 | Forward | GCAGGGTACAGGACAACACA | 33 |
| | Reverse | AGGATGTGTTAATGGGGTGACT | 34 |
| HOXB9 | Forward | TCTACTGGAATGTCCCCTTGC | 35 |
| | HOXC9 Reverse | TACTCTCTGCTTCCGTTCCTC | 36 |
| HOXC9 | Forward | CGGCAGGTCAAAATCTGGTTTC | 37 |
| | Reverse | GGGTTTAGGACTGCTCCTTGTC | 38 |
| HOXD10 | Forward | AGGACTGGGAAAGCGGAAAC | 39 |
| | Reverse | GCAGCACGAACATAATGACCC | 40 |
| HOXD11 | Forward | CGATTCCTTCCCACGGTCAA | 41 |
| | Reverse | TATTAGGATCCGCTCGGCCA | 42 |
| ERCC-00096 | Forward | GATCCCGGAAGATACGCTCTAAG | 43 |
| | Reverse | CGCAGGTTGATGCTTCCAATAAA | 44 |

**[Table 5]**

| Composition | | |
|---|---|---|
| Syber Green | 5.0 µL | |
| Primer mix (1 µM) | 2.5 µL | |
| 1/25 cDNA | 2.5 µL | |
| Total | 10.0 µL | |

| PCR conditions | | |
|---|---|---|
| 95°C | 10 min | |
| 95°C | 15 sec | 40 cycles |
| 60°C | 1 min | |
| 66°C | 10 sec | |

### [Example 1]

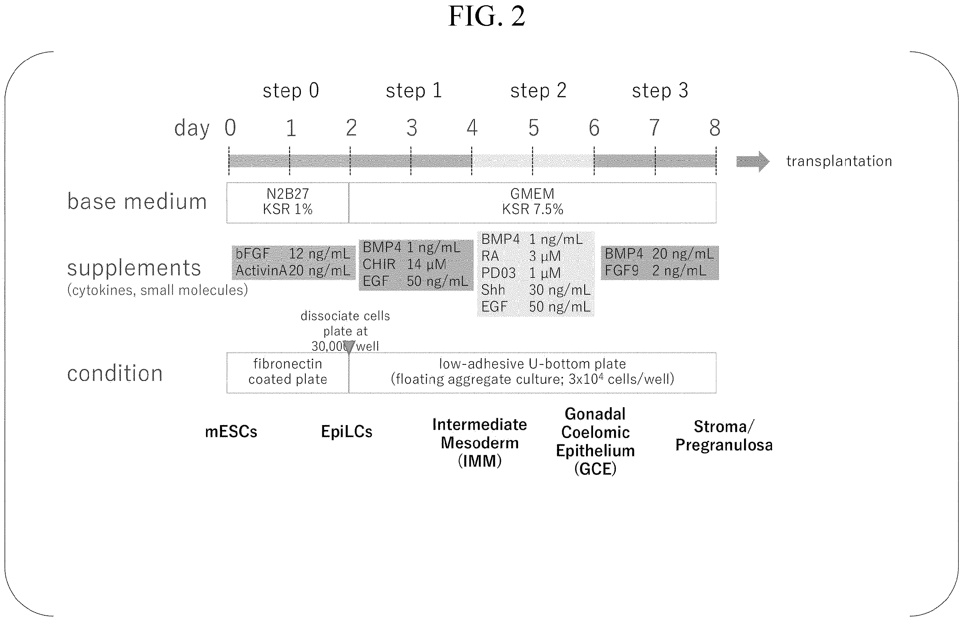

FIG. 2 is a view showing a protocol in a case of inducing differentiation from mouse ES cells into ovarian somatic cell-like cells. In FIG. 2, bFGF represents a basic fibroblast growth factor (FGF2), BMP4 represents a bone morphogenetic protein 4, CHIR represents CHIR99021 which is a GSK3 inhibitor having an activating action on the WNT pathway, EGF represents an epidermal growth factor, RA represents retinoic acid, PD03 represents PD0325901, which is an MEK inhibitor having an inhibitory effect on the FGF pathway, SHH represents a sonic hedgehog, and FGF9 represents a fibroblast proliferation 9. Hereinafter, the abbreviations above will be used.

In mice, first, ES cells (ESCs) are differentiated into EpiLCs by culturing the ESCs on a culture dish coated with fibronectin for 42 hours (step 0).

Thereafter, the cells are peeled off once, the colonies are dissociated, and the number of cells is counted. In a case where these are cultured on a low-adhesive plate for 30,000 cells, the cells adhere to each other in a suspended state and form a cell aggregate. At this time, differentiation into the mesoderm to the intermediate mesoderm by using BMP4, a small molecule CHIR99021 that activates a WNT signal, and EGF is performed (step 1).

Subsequently, the medium was replaced with a medium including BMP4, RA, a small molecule PD0325901 that suppresses FGF signaling, SHH, and EGF, and the induction from the gonadal coelomic epithelium to the genital ridge was performed over 2 days (step 2).

Then, the cells were replaced with a medium including BMP4 and FGF9, and the cells were observed to be divided into lineages of the stroma and the pregranulosa for 1 to 2 days (step 3).

The ES cells of monkeys and human iPS cells are considered to be similar to EpiLC in a slightly advanced state in terms of differentiation, as compared with mouse ES cells. Therefore, in the examination using monkey or human pluripotent stem cells in the present test, step 0 was omitted and the process was started from step 1. In addition, unlike mouse EpiLC, human and monkey pluripotent stem cells undergo cell death in a case where colonies are dissociated to the level of one cell, but the small molecule Y27632, which is a ROCK inhibitor, is known to be effective for preventing cell death. For this reason, Y27632 was added to step 1 to perform examination. In addition, a 96-well V-bottom plate was used as a culture plate and the cells were seeded so as to have 10,000 cells per well.

### 1. Trial for Same Period as in Mice

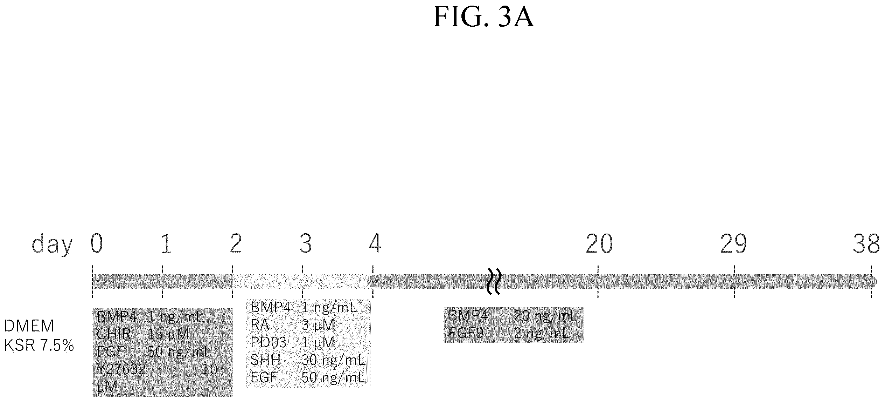

FIG. 3A is a view showing a protocol for inducing differentiation of ovarian somatic cell-like cells using human iPS cells.

In a test using human cells, the base medium was changed from GMEM (used in mice) to DMEM including 7.5 v/v% KnockOut (registered trademark) Serum Replacement (KSR). In addition, the base medium includes 0.1 mM 2-mercaptoethanol, 100 U/mL penicillin/streptomycin, and 2 mM GlutaMax (registered trademark), but the additives and concentrations thereof are fixed, and the description following "2." is omitted. The periods of step 1 and step 2 were set to be the same as those in the mouse protocol, and culture was continued under the condition of step 3. The entire medium was basically changed every two or three days. At the time of subculturing iPS cells, 30,000 cells were seeded on a low-adhesive plate to start the culture as aggregates.

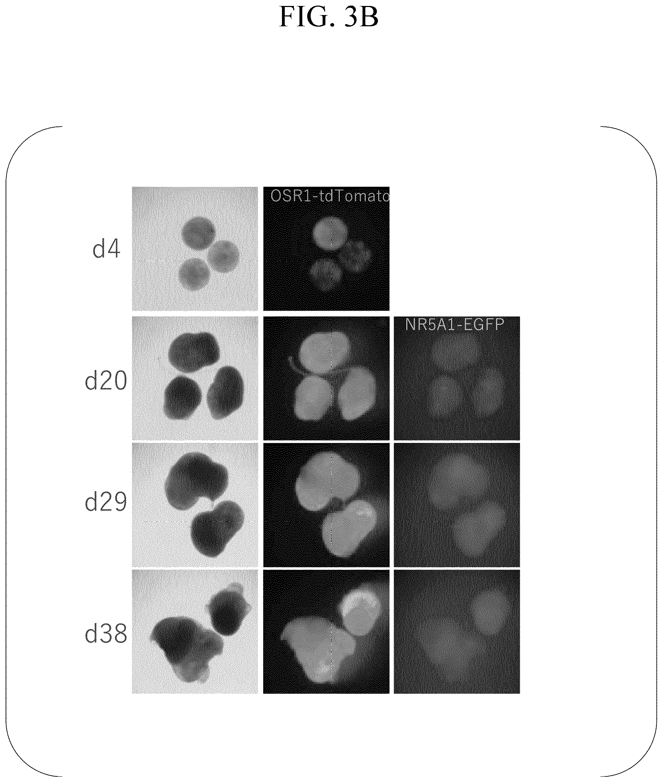

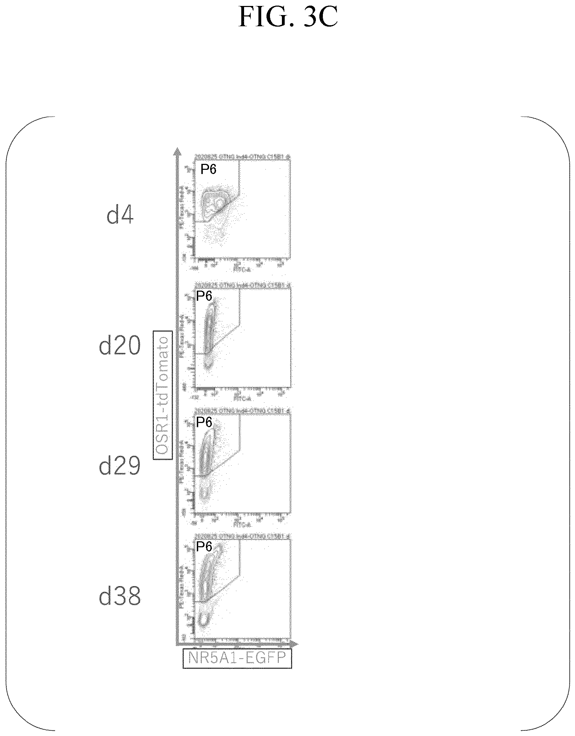

The expression of the fluorescent reporter of OSR1 was recognized on day 4 (d4) at the time of the completion of step 2 (see FIG. 3B). However, thereafter OSR1 continued to be expressed even though 34 days had passed after the transition to step 3, but NR5A1 were not seen (see FIGS. 3B and 3C).

### 2. Optimization of Number of Cells in Step 1

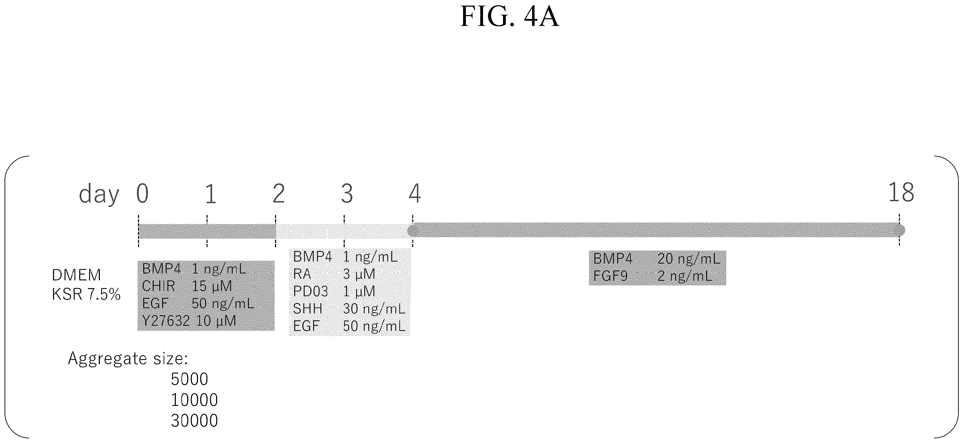

Since in a case where the initial number of cells is too large, there is a possibility that the signal does not permeate into the inside, a reduction in the number of cells per cell aggregate was examined. The detailed protocol is shown in FIG. 4A.

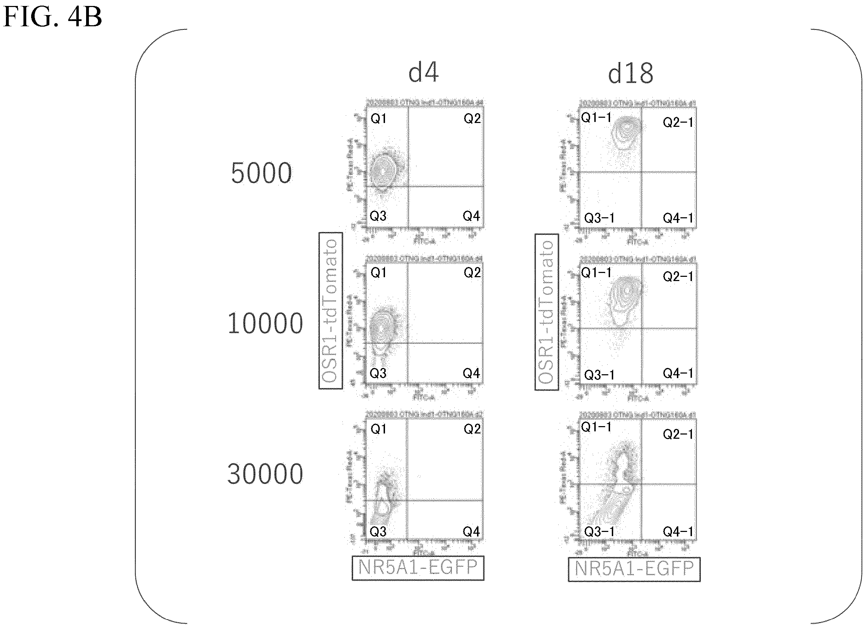

As a result, the expression of NR5A1 was not observed by this differentiation method, but it was revealed that the expression of OSR1 uniformly increased in a case where the number of cells was 10,000 or less, and in a case where the number of cells was 30,000, the number of cells expressing neither OSR1 nor NR5A1 increased (see FIG. 4B). Hereinafter, in the case of using human iPS cells, the cells were started from 10,000 cells.

### 3. Optimization 1 of Period of Step 1

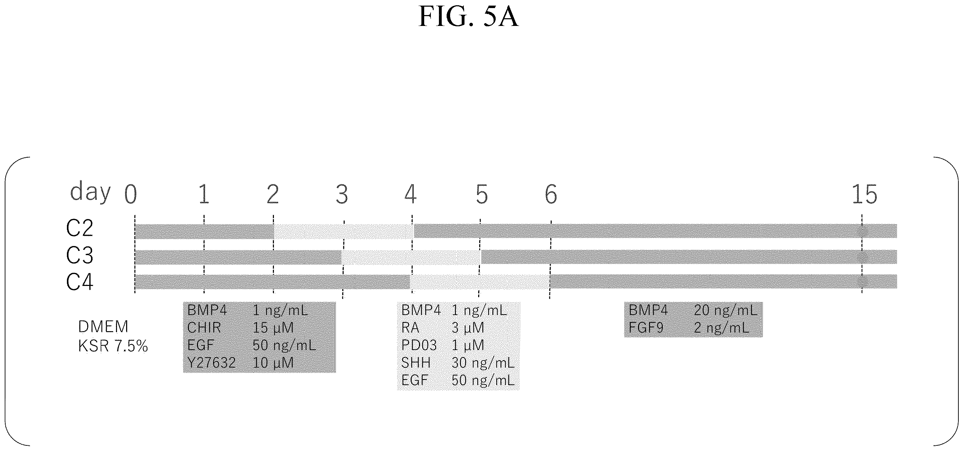

Since in a case where the period of step 1 was short, differentiation into the tissues near the head was expected whereas in a case where the period of step 1 was long, differentiation into tail-side tissues was expected, the period of step 1 was changed to 2 days, 3 days, and 4 days (the total period of step 1 and step 2 was 4 days, 5 days, and 6 days) to perform the examination. The detailed protocol is shown in FIG. 5A.

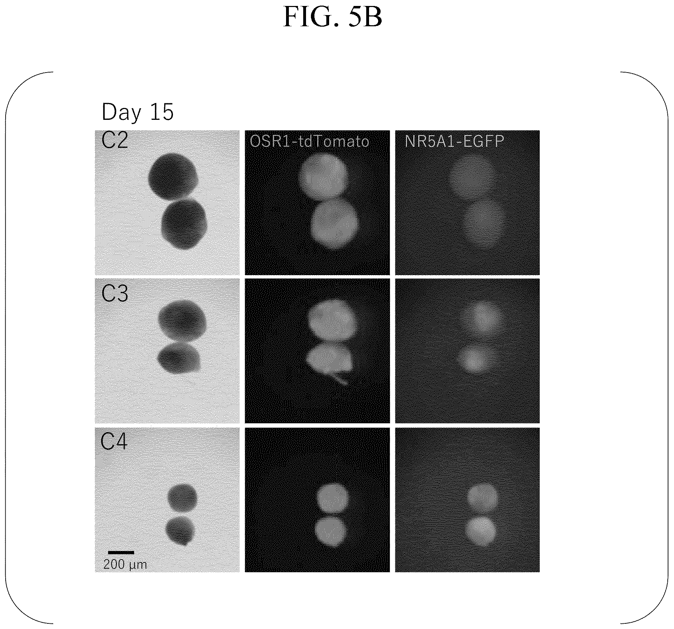

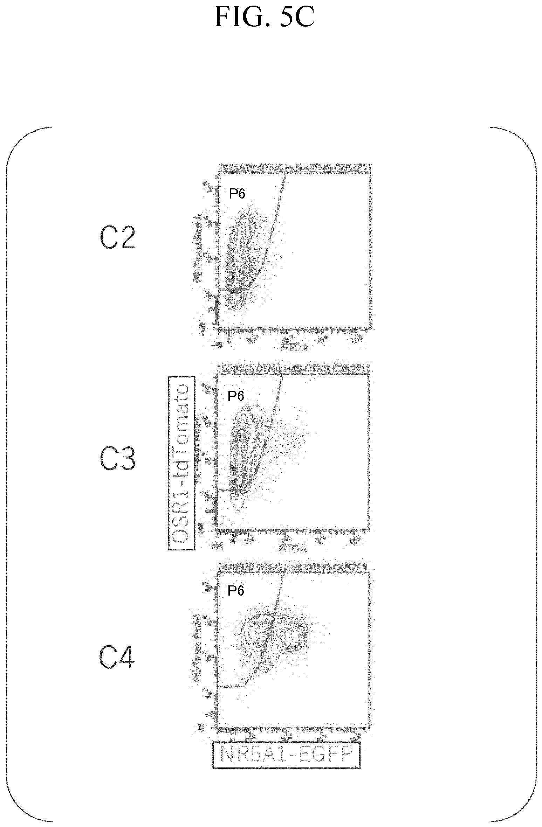

As a result, in a case where the period of step 1 was set to 3 days, a few NR5A1-positive cells were observed on day 17, and in a case where the period of step 1 was set to 4 days, a larger number of cells became NR5A1-positive cells (see FIGS. 5B and 5C).

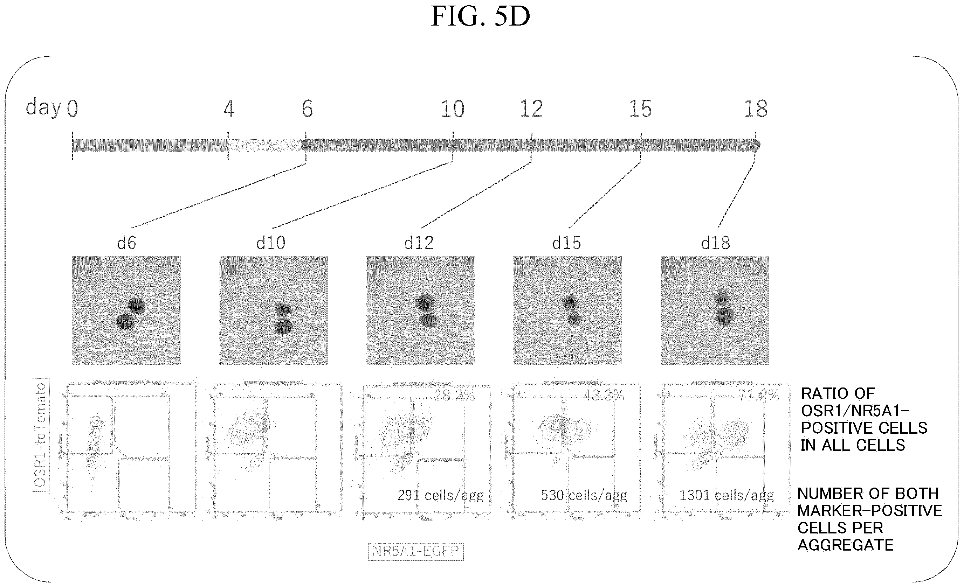

In addition, FIG. 5D is a view showing the results of examining a time course of the expression of OSR1 and NR5A1 in a case where a period of step 1 was set to 4 days.

As shown in FIG. 5D, NR5A1 started to be expressed after day 4 of step 3 and gradually transitioned to NR5A1-positive cells, and eventually, the number of OSR1-positive NR5A1-negative cells was reduced. In addition, also in cells positive for both OSR1 and NR5A1, the expression of OSR1 was slightly reduced.

### 4. Optimization 2 of Period of Step 1 and Effect of Combination Use with Other Agents in Step 1

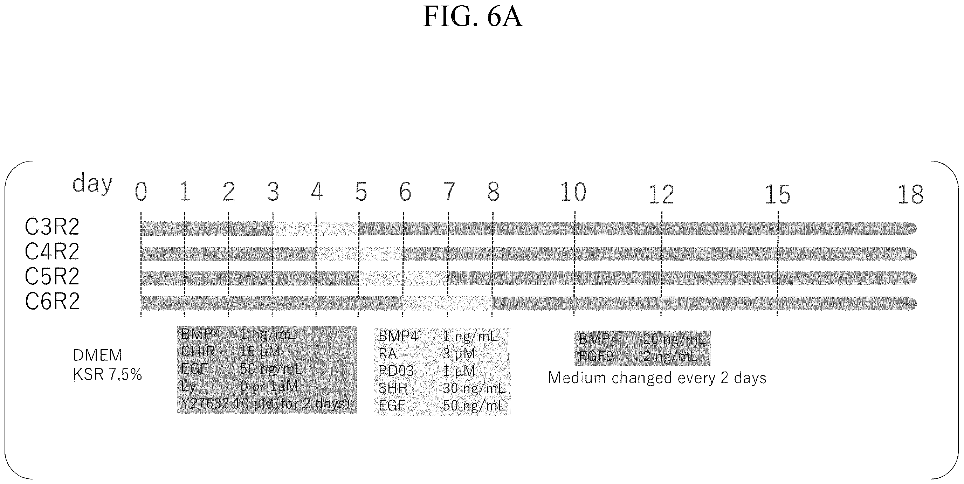

Subsequently, it was examined what would occur in a case where the period in step 1 was further extended. In this experiment, the period of step 1 was adjusted to 3 days, 4 days, 5 days, and 6 days (the total period of step 1 and step 2 was 5 days, 6 days, 7 days, and 8 days) to perform the examination. Furthermore, the effect of a small molecule Ly294002 (phosphatidylinositol-3 kinase; PI3K inhibitor, abbreviated as "Ly" in the drawing), which may suppress the differentiation into the tissues near the head, was also evaluated. The detailed protocol is shown in FIG. 6A.

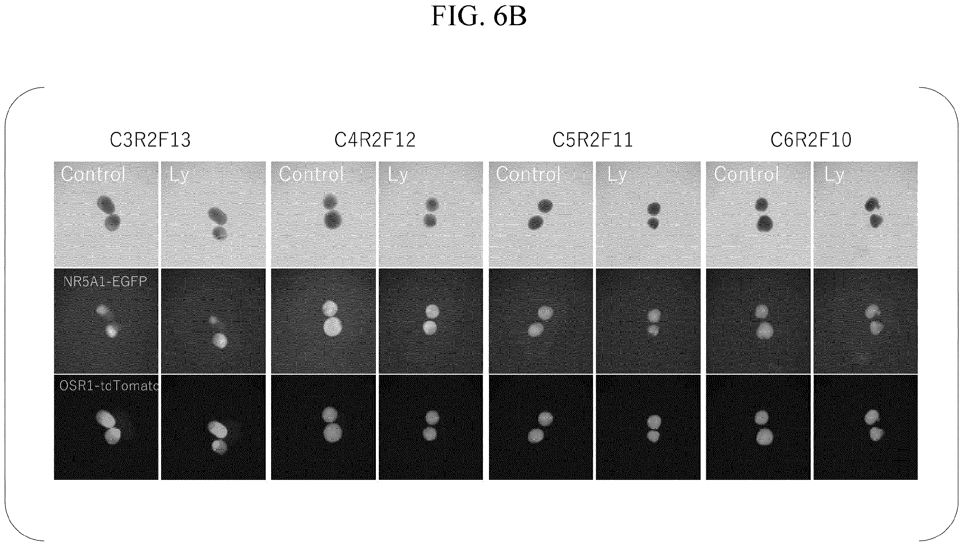

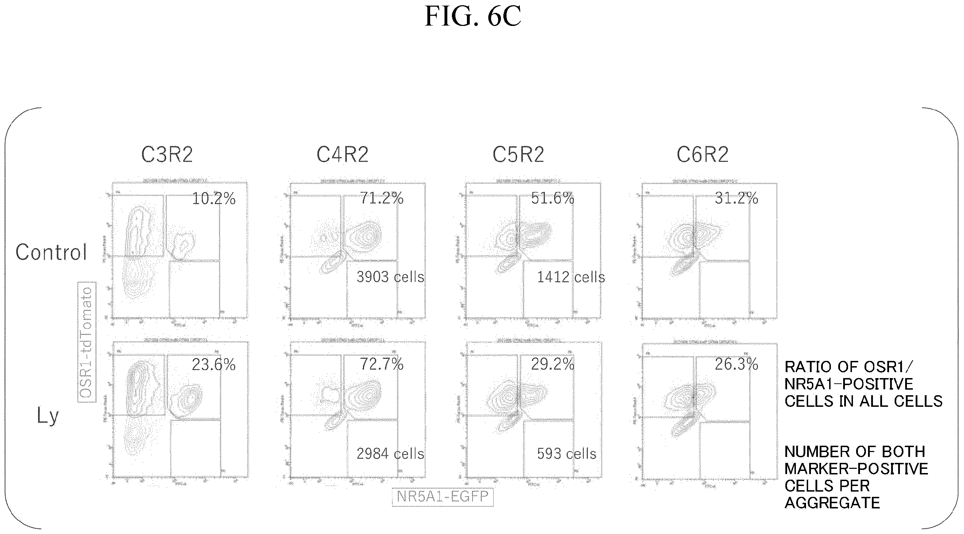

In a case of being evaluated on day 18, it was found that cells expressing both OSR1 and NR5A1 were obtained regardless of whether the period of step 1 was 3 days or 6 days (see FIG. 6B). However, in a case where FACS analysis was performed, it was found that the condition in which the period of step 1 was 4 days (C4R2F12) was the best for the number of cells positive for both OSR1 and NR5A1 (see FIG. 6C). In addition, it was found that the addition of Ly294002 did not cause an increase in induction efficiency, but the number of cells collectable was rather reduced (see FIG. 6C).

Based on these results, a protocol was adopted in which the period of step 1 was 4 days and Ly294002 was not added.

### 5. Optimization of Period of Step 2

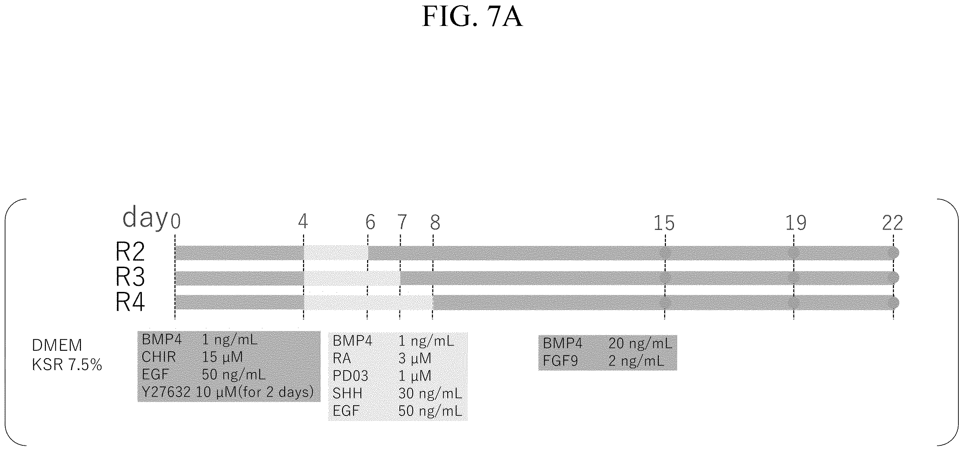

Next, the period of step 1 was fixed at 4 days, and the period of step 2 was adjusted to 2 days, 3 days, and 4 days (the total period of step 1 and step 2 was 6 days, 7 days, and 8 days) to perform the examination. The detailed protocol is shown in FIG. 7A.

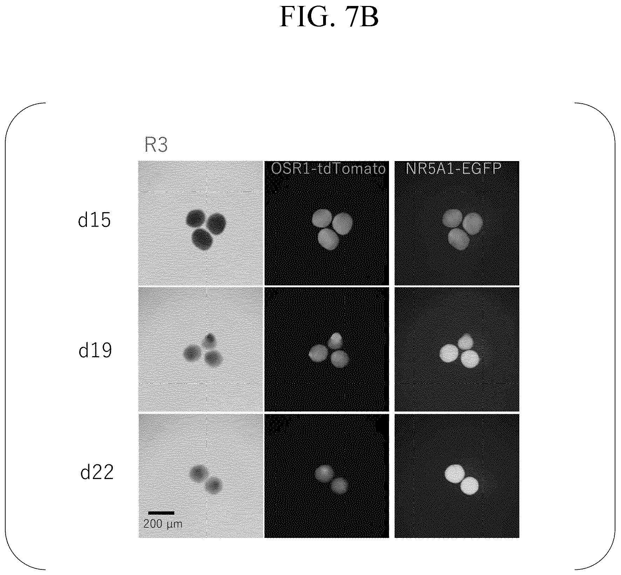

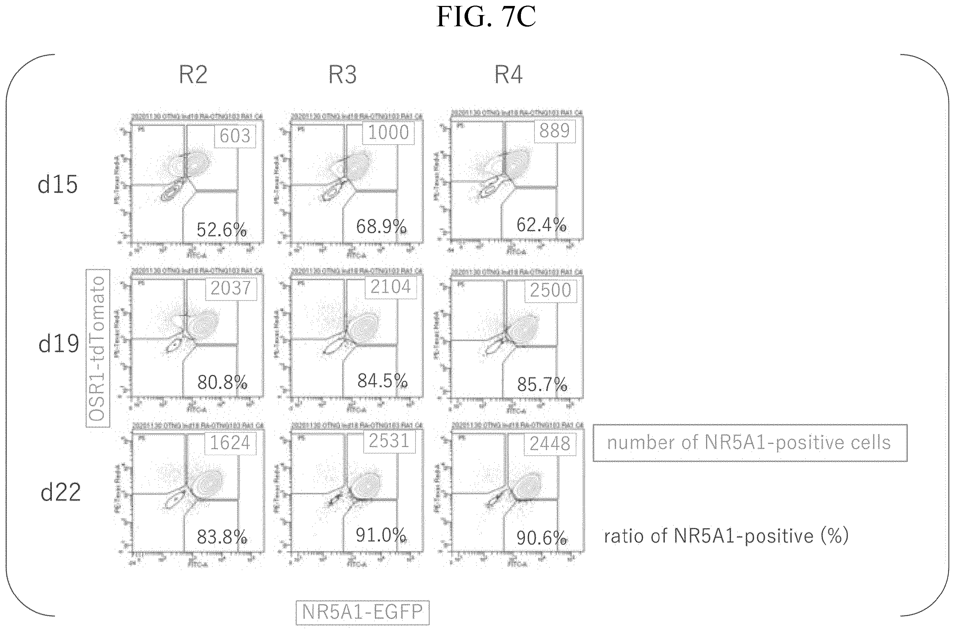

As a result, the proportion and the actual number of the NR5A1-positive cells obtained were higher in a case where the period of step 2 was 3 days (R3) than in a case where the period of step 2 was 2 days (R2) (see FIGS. 7B and 7C). On the other hand, under the condition in which the period of step 2 was 4 days (R4), substantially no change was observed in a case where the period of step 2 was 3 days (see FIG. 7C).

Based on these results, the period of step 2 was set to 3 days thereafter.

### 6. Effect of Combination Use with Other Agents in Step 1

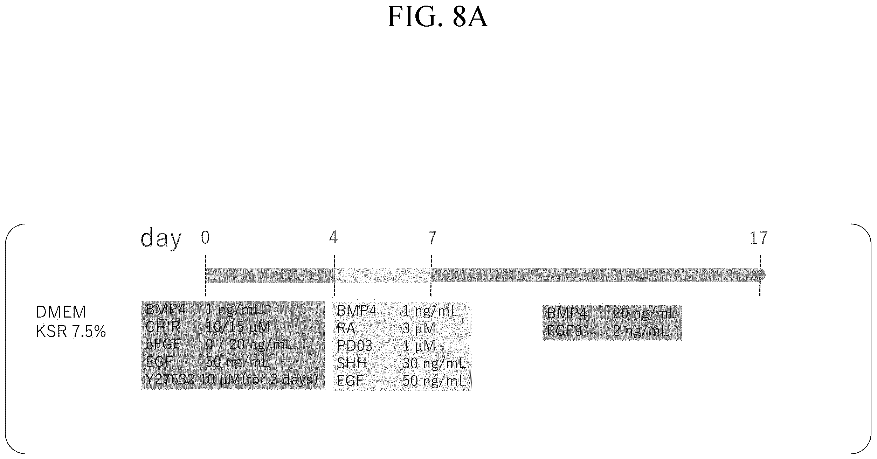

After the period of step 2 was set to 3 days, it was evaluated whether the efficiency could be increased by changing the concentration of CHIR99021 in step 1 again and adding bFGF. The detailed protocol is shown in FIG. 8A.

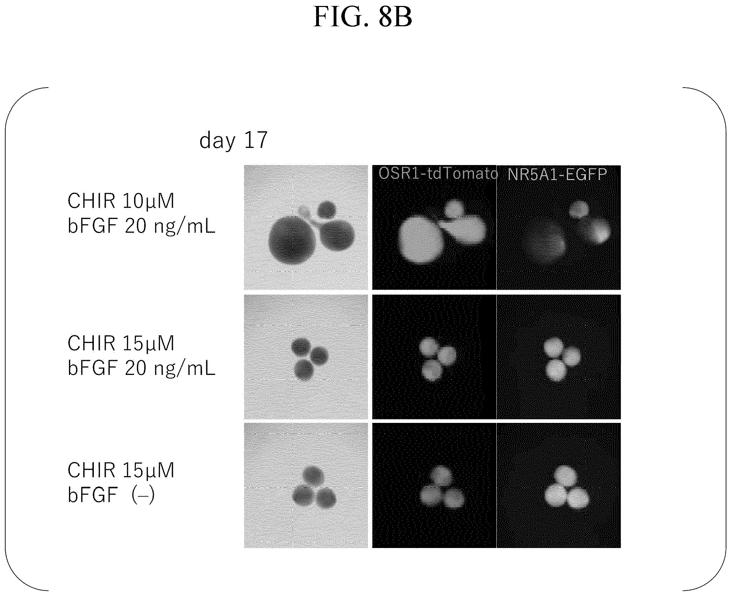

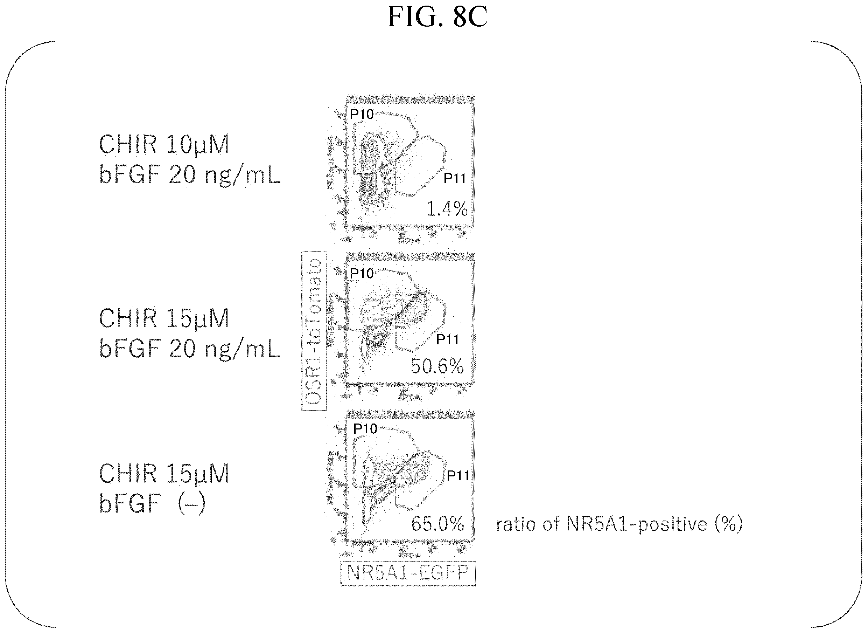

As a result, it was found that in a case where the concentration of CHIR99021 was 15 µM as compared to 10 µM, the proportion of NR5A1-positive cells was excellent, and in a case where bFGF was not present, the differentiation induction efficiency was higher (see FIGS. 8B and 8C).

### 7. Optimization of RA Concentration in Step 2

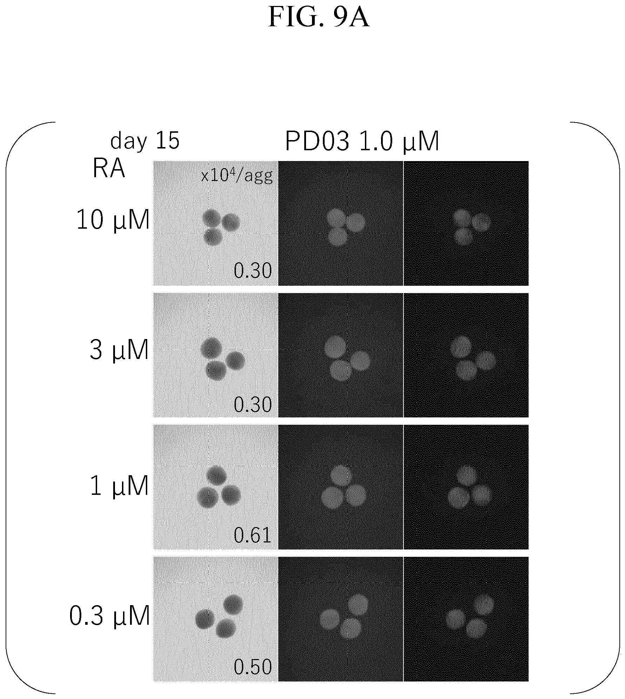

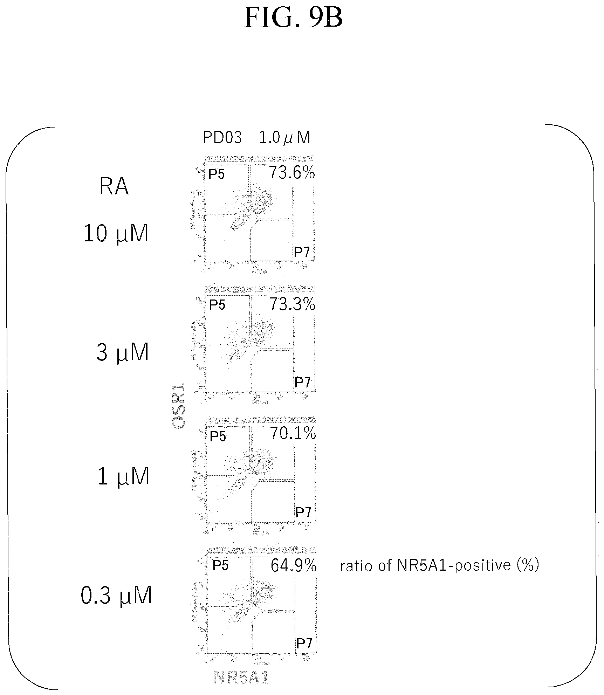

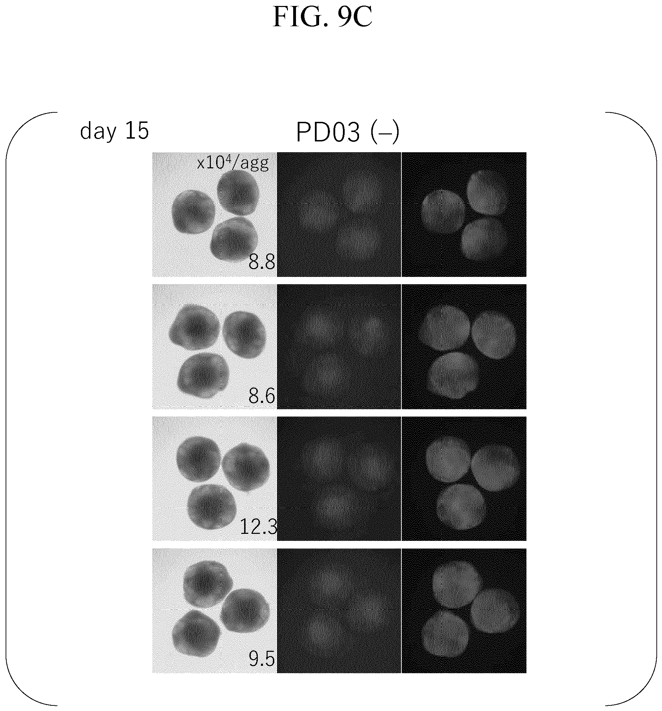

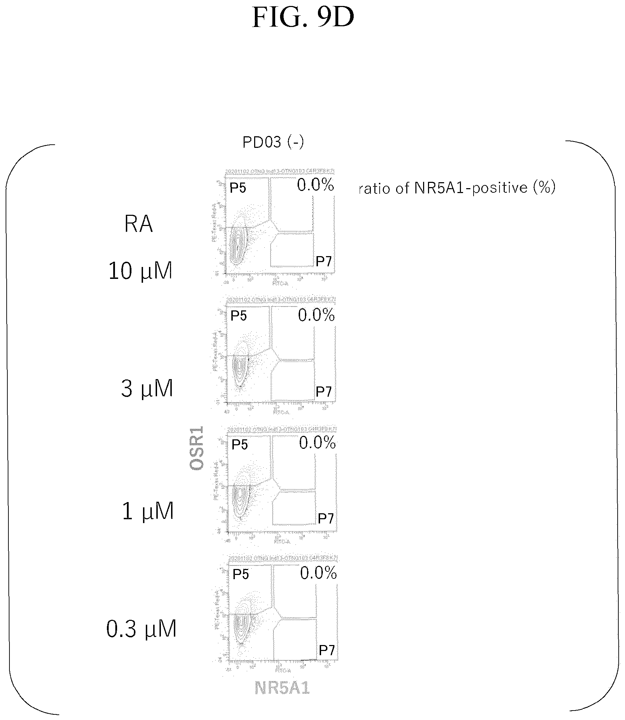

The test was carried out under the same conditions as before, except that the period of step 1 was 4 days, the period of step 2 was 3 days, the concentration of RA in step 2 was adjusted to 0.3, 1, 3, and 10 µM, and the PD0325901 was adjusted to 1 and 0 µM (see FIG. 9A).

In a case of performing the evaluation on day 15 of induction, it was found that in a case where PD0325901 was not added, NR5A1-positive cells were not observed at all (see FIGS. 9A to 9D). On the other hand, it can be seen that with regard to the concentration of RA, the induction was performed with a high percentage of the concentration even at 0.3 µM or 10 µM, but under the same conditions of both the proportion of NR5A1-positive cells and the number of NR5A1-positive cells as the mouse condition, 1 µM or more and 3 µM was particularly good (see FIGS. 9A to 9D).

### 8. Optimization of KSR and PD0325901 Concentrations in Step 2

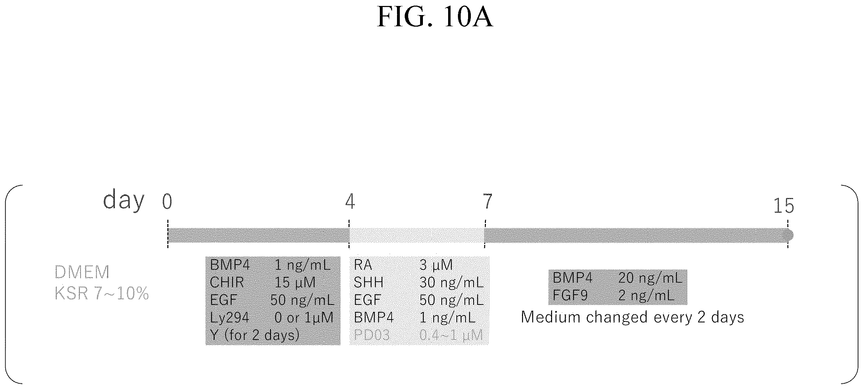

Subsequently, the concentrations of KSR and PD0325901 were finely set and compared. The cells were cultured at 7, 8, 9, and 10 v/v% for KSR and at 0.4, 0.6, 0.8, and 1.0 µM for PD0325901, and the evaluation was performed on day 15. The detailed protocol is shown in FIG. 10A.

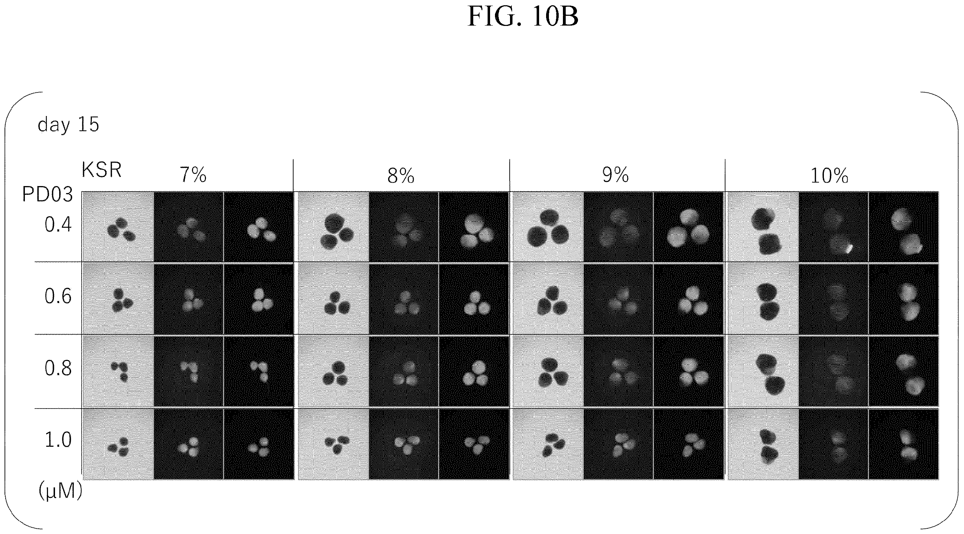

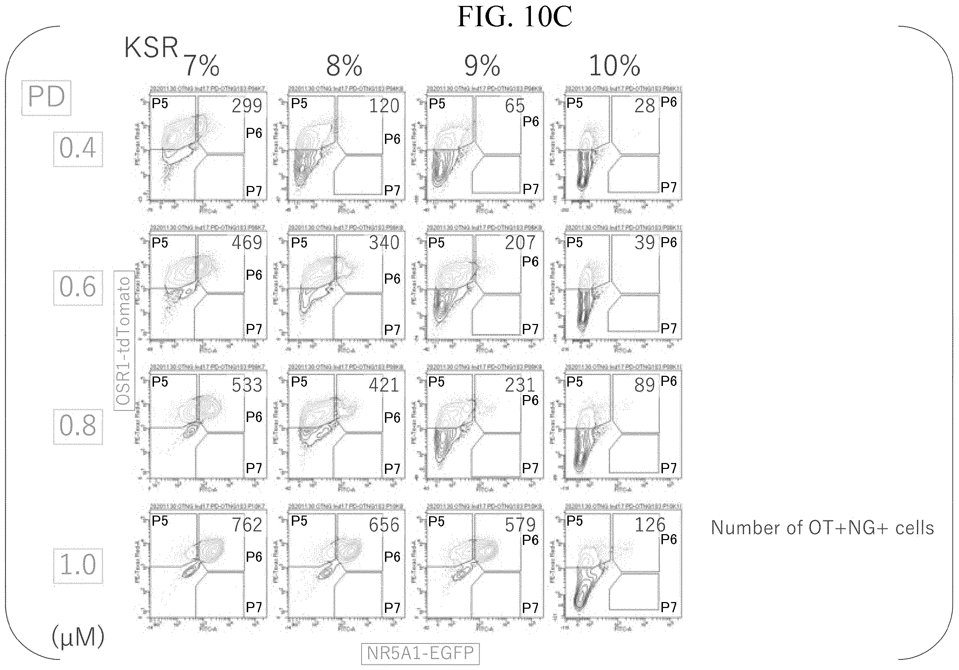

It was recognized that in a case where KSR was dark or in a case where PD0325901 was weak, NR5A1-positive cells did not appear (see FIG. 10B). Similar results were obtained from FACS analysis (see FIG. 10C).

In a case where the concentration of KSR was 7 v/v% and the PD0325901 concentration was 1.0 µM, the differentiation induction efficiency was the highest. However, with an object of obtaining 1,000 or more cells starting from 10,000 cells, the next attempt was made to change the base medium.

### 9. Examination of Base Medium 1

Five kinds of media, that is, DMEM, Advanced DMEM, Advanced DMEM/F12, Advanced MEM, and Advanced RPMI were used with the concentration of KSR adjusted to 5.0 and 7.5 v/v%. For the culture period, the period of step 1 was set to 4 days and the period of step 2 was set to 3 days to perform the evaluation on day 23 of induction. In the present test, an iPS cell line different from the cell strains so far was used. Specifically, fluorescent reporter cell lines of FOXL2-tdTomato and NR5A1-EGFP (NGFT strain) were used instead of the fluorescent reporter cell strains of OSR1-tdTomato and NR5A1-EGFP (OTNG strain).

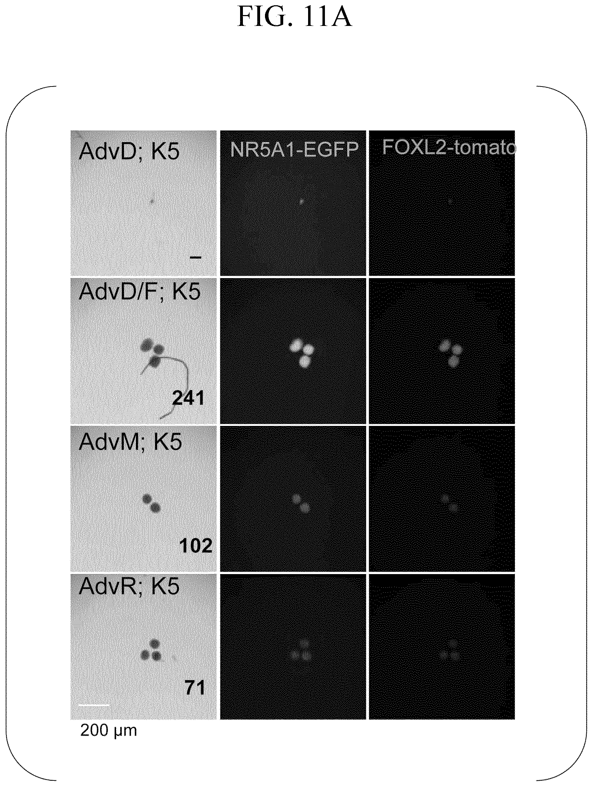

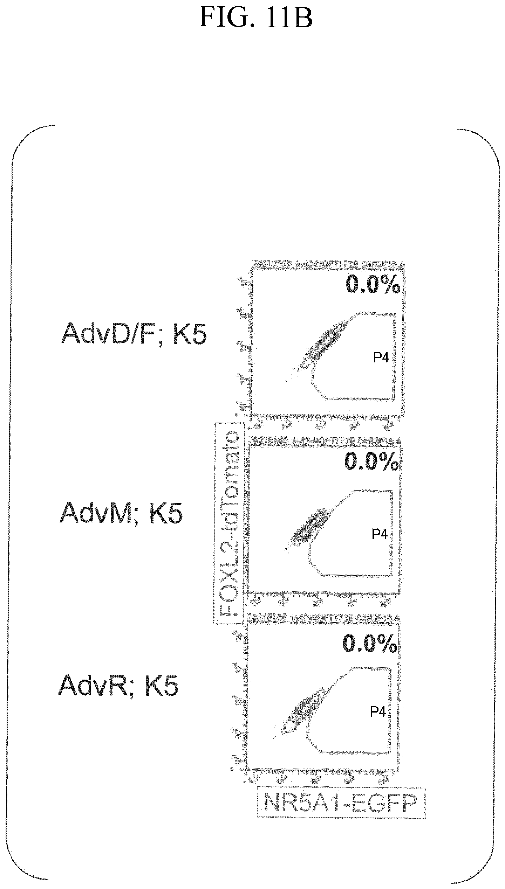

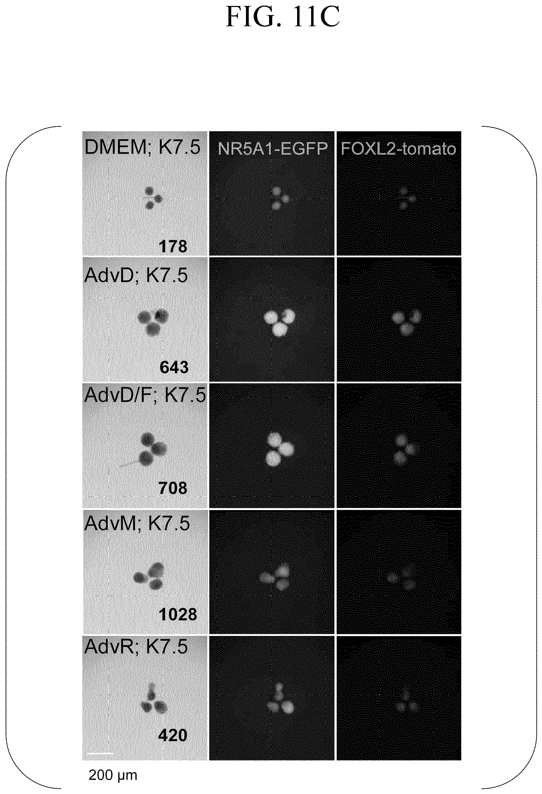

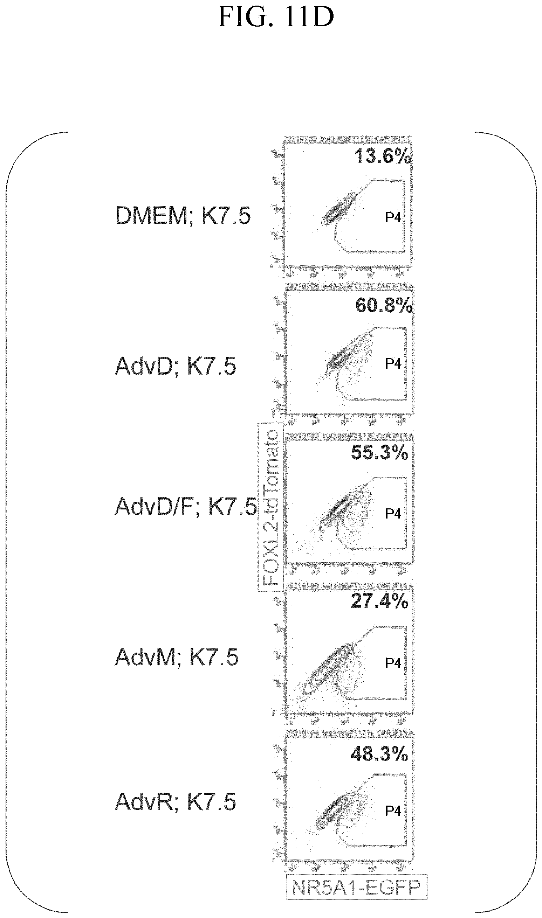

As a result, it was found that with a concentration of KSR of 7.5 v/v%, the differentiation induction efficiency was excellent, and Advanced DMEM and Advanced DMEM/F12 were particularly excellent in differentiation induction efficiency (see FIG. 11A to FIG. 11D). In FIG. 11A, there is no photograph of DMEM having a concentration of KSR of 5 v/v% since the cells were dead.

### 10. Examination of Base Medium 2

Five kinds of media, that is, DMEM, Advanced DMEM, Advanced DMEM/F12, Advanced MEM, and Advanced RPMI were examined under the same conditions as in "9.", except that the concentration of KSR was fixed at 7.5 v/v% and the OTNG strain was used. Evaluation was performed on days 15 and 23 of induction.

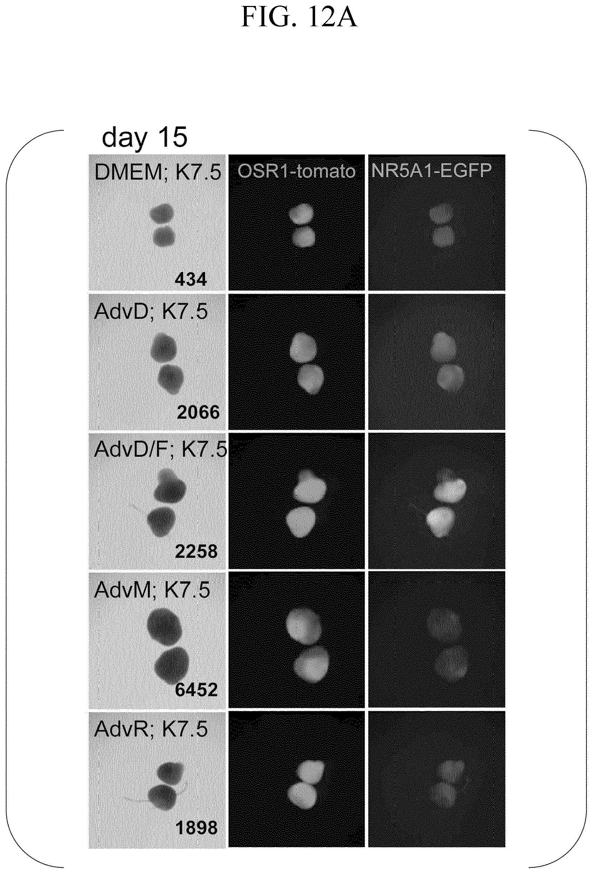

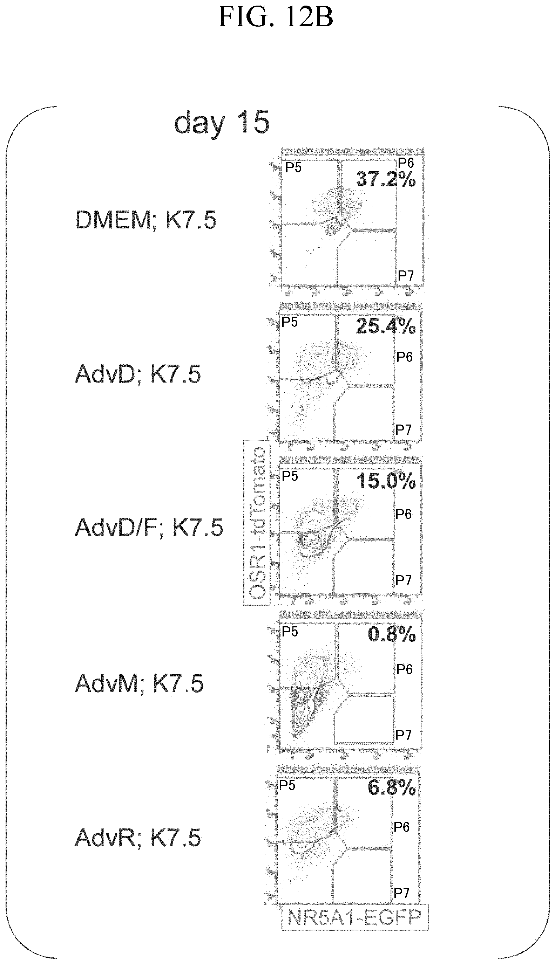

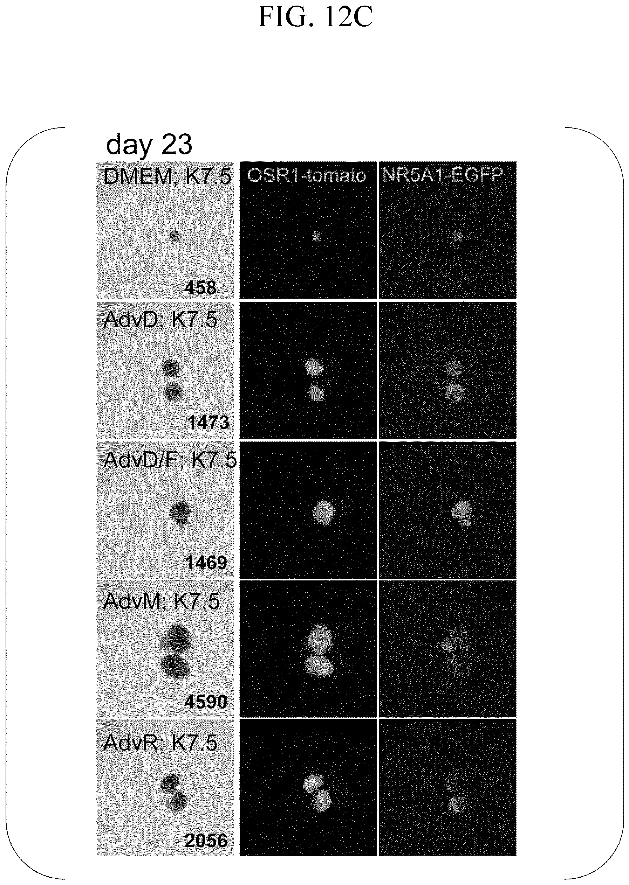

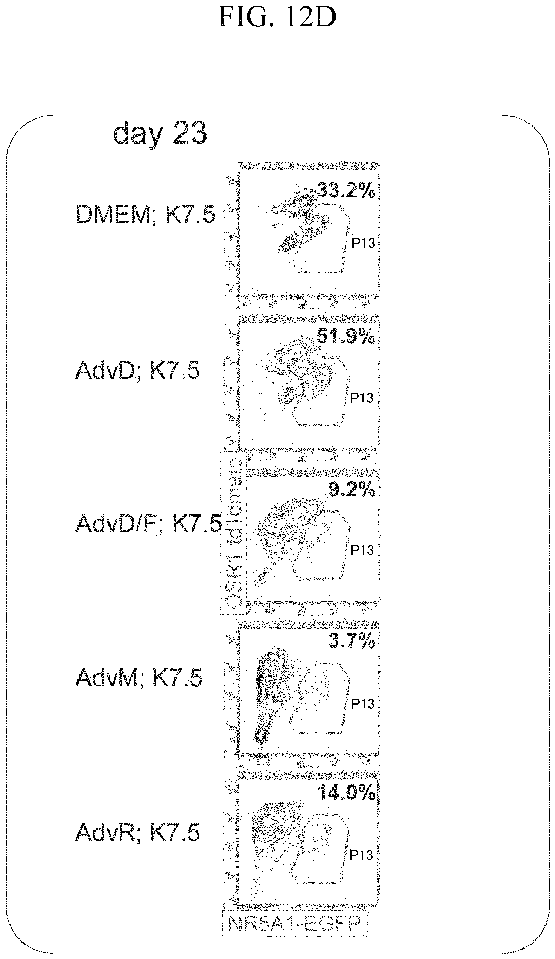

As a result, Advanced DMEM, Advanced DMEM/F12, and Advanced RPMI had a higher differentiation induction efficiency than DMEM (see FIG. 12A to FIG. 12D). On the other hand, it was found that in Advanced MEM, the number of cells increased, but the cells did not easily become NR5A1-positive (see FIGS. 12A to 12D). In the present test, the differentiation induction efficiency was higher in Advanced DMEM than in Advanced DMEM/F12, but subsequent examination revealed that Advanced DMEM/F12 had the same differentiation induction efficiency (not shown).

### 11. Examination of Base Medium 3

Five kinds of media, that is, DMEM, Advanced DMEM, Advanced DMEM/F12, Advanced MEM, and Advanced RPMI were examined under the same culture conditions as in "10.", except that SHH was added during the first 6 days in step 3 (that is, from day 7 to day 13 of induction). Evaluation was performed on days 15 and 23 of induction.

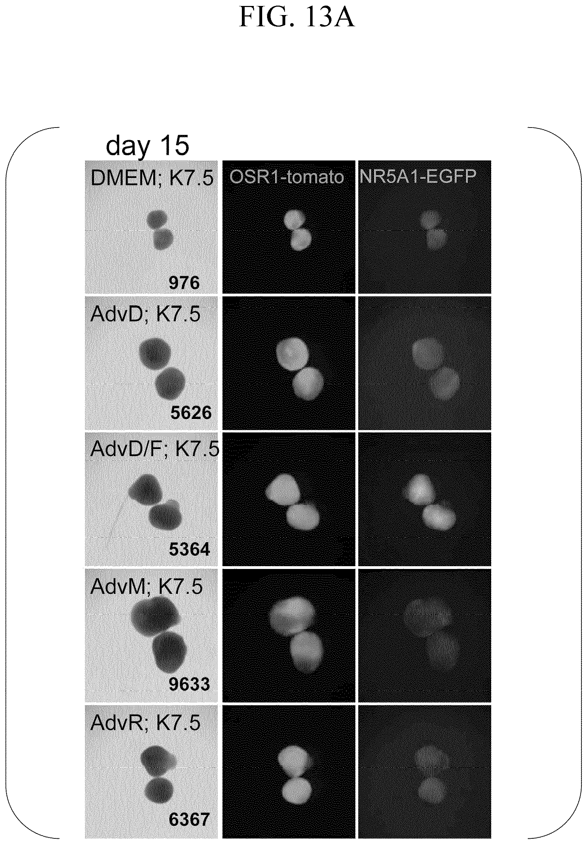

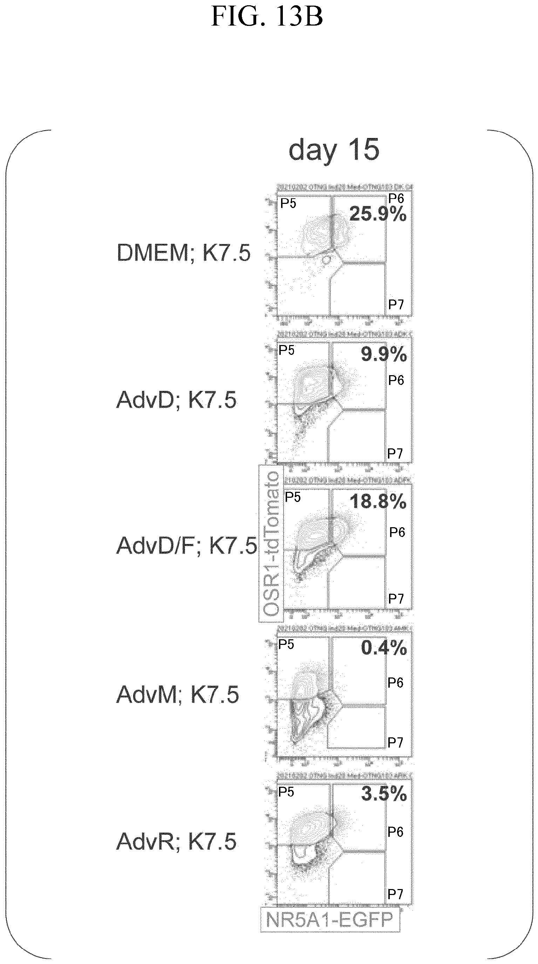

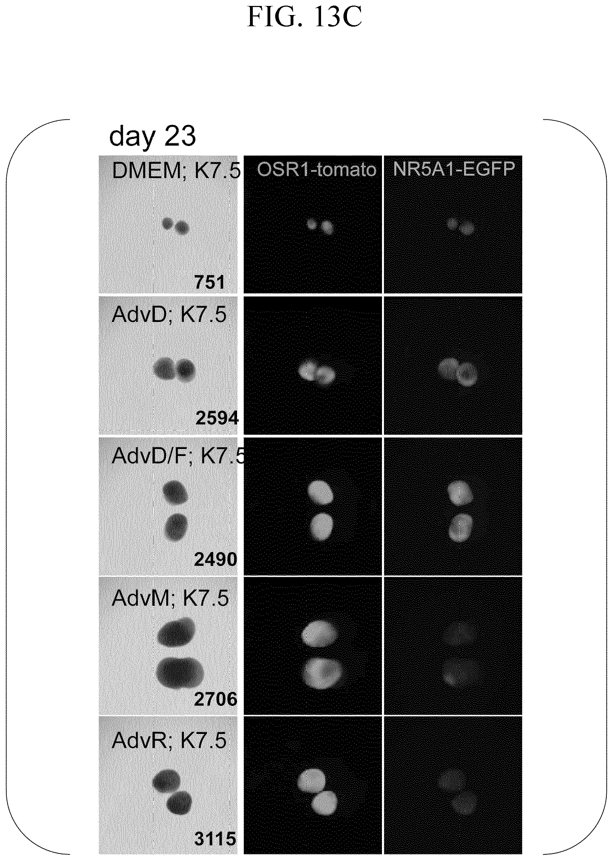

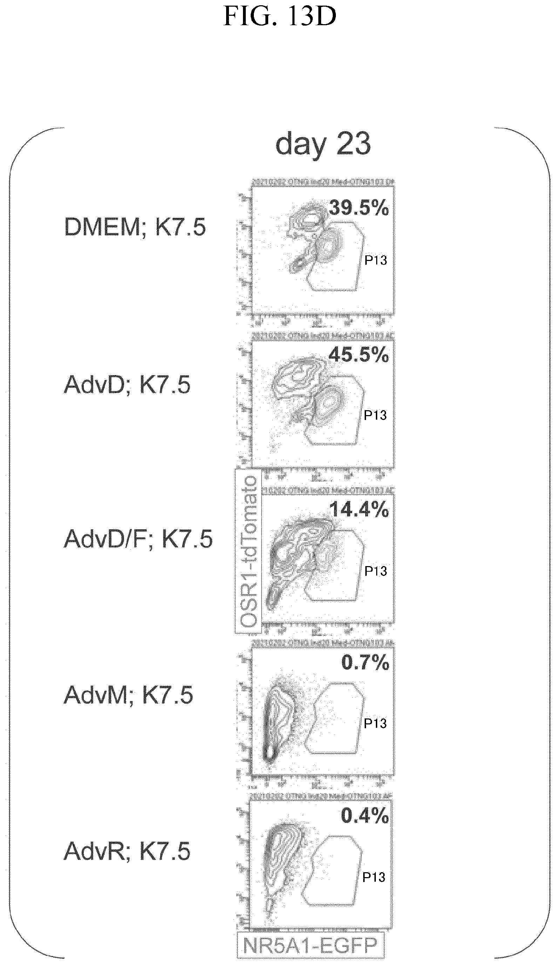

As a result, almost no NR5A1-positive cells were found in Advanced RPMI (see FIGS. 13A to 13D). On the other hand, in Advanced DMEM and Advanced DMEMIF12, the number of the obtained NR5A1-positive cells was larger than that in DMEM.

Based on these results, it was seen that the differentiation induction efficiency tended to be higher in a case where SHH was continuously added from the culture period of step 2 to the culture period of step 3.

### 12. Optimization of SHH Concentration in Step 2

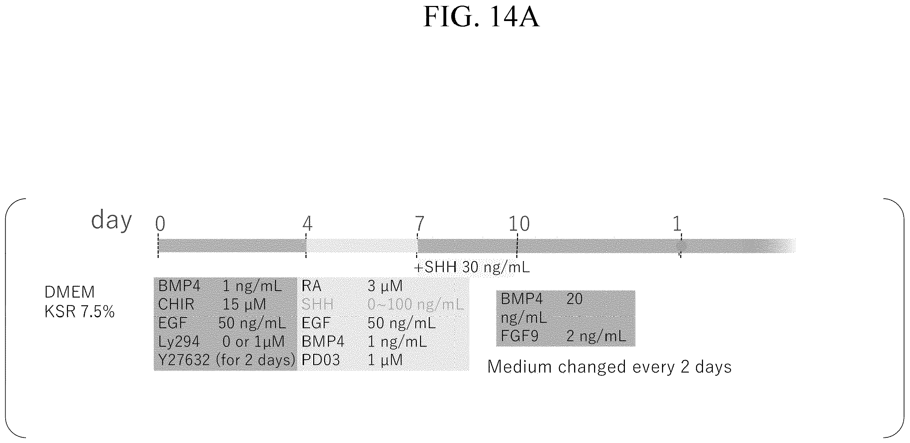

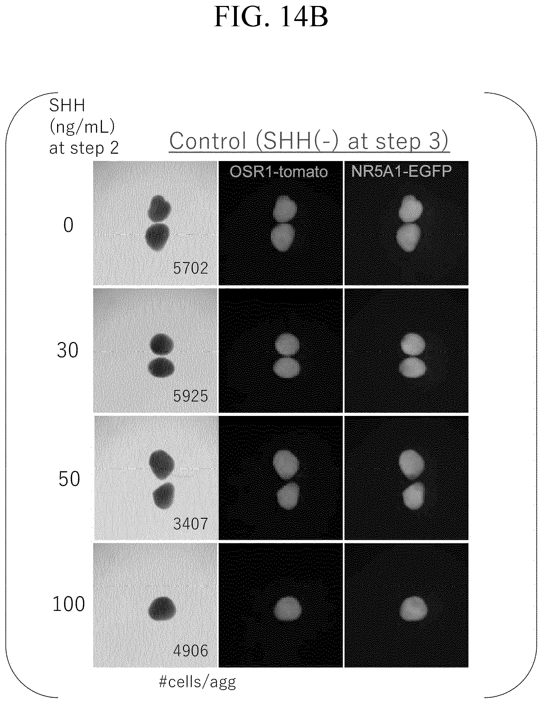

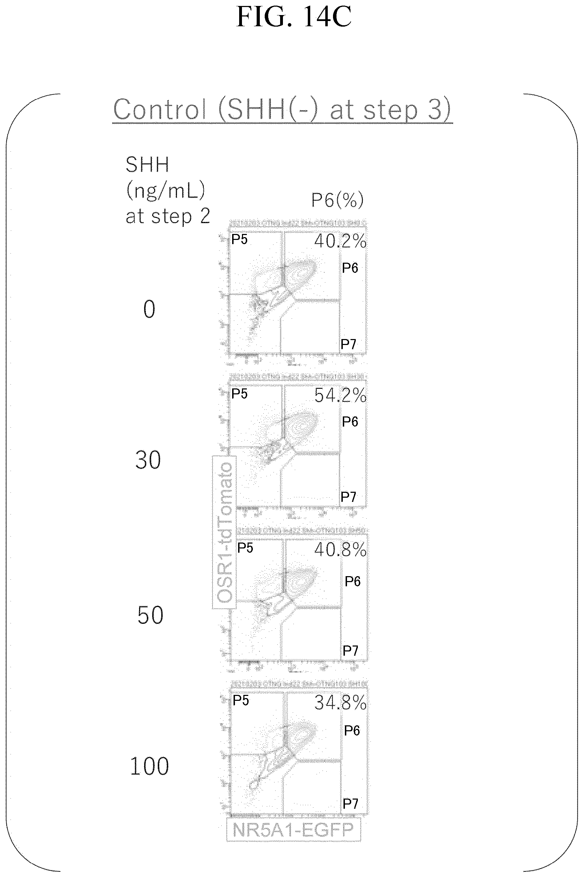

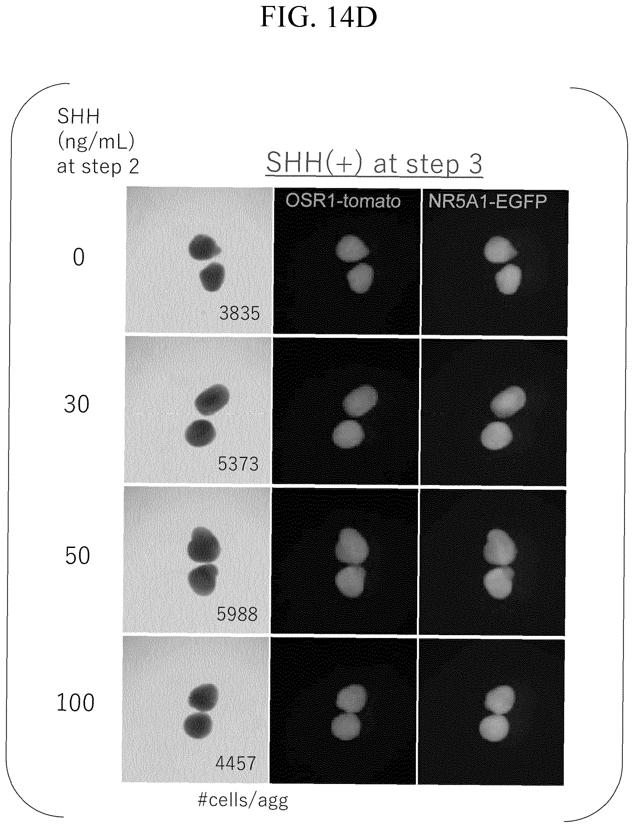

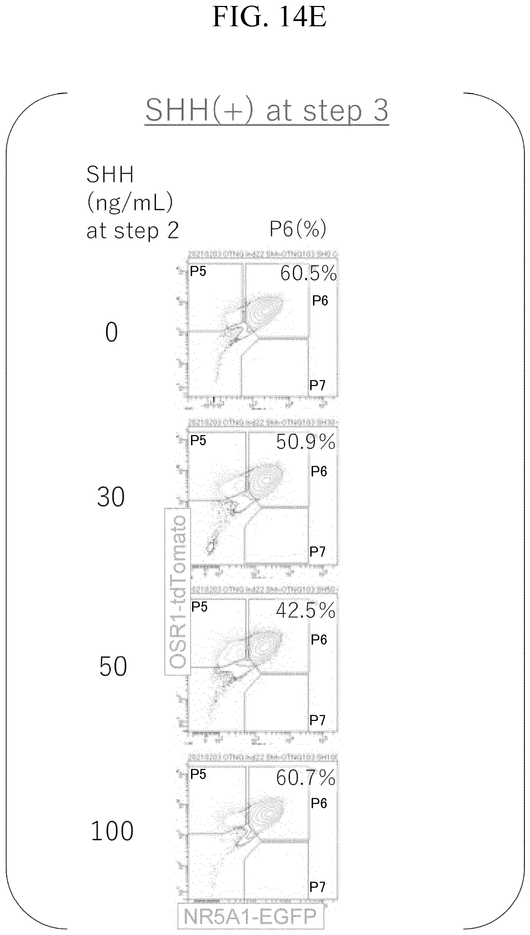

Subsequently, the optimization of the concentration of SHH in step 2 was examined and the effect of the Shh administration in the early stage of step 3 was re-examined. The concentration of SHH in step 2 was adjusted to four concentrations of 0, 30, 50, and 100 ng/mL, the concentration of SHH in step 3 was adjusted to 0 or 30 ng/mL to fix the addition period of SHH in step 3 for 3 days, and the concentrations of other drugs were not changed for the examination. The base medium used was DMEM containing 7.5 v/v% KSR. The detailed protocol is shown in FIG. 14A.

As a result, the appearance of NR5A1-positive cells was recognized even without adding SHH in step 2, and even in a case where the concentration of SHH increased, no significant change was recognized in the number of cells and the differentiation induction efficiency. On the other hand, the induction efficiency tended to be higher in a case where SHH was added in step 3, but the number of cells did not significantly increase.

From these results, although SHH was not essential for the induction of gonadal somatic cells, the number of the obtained NR5A1-positive cells tended to increase in a case where SHH was added in the early stage of step 3 rather than step 2.

### 13. Examination of Marker Genes

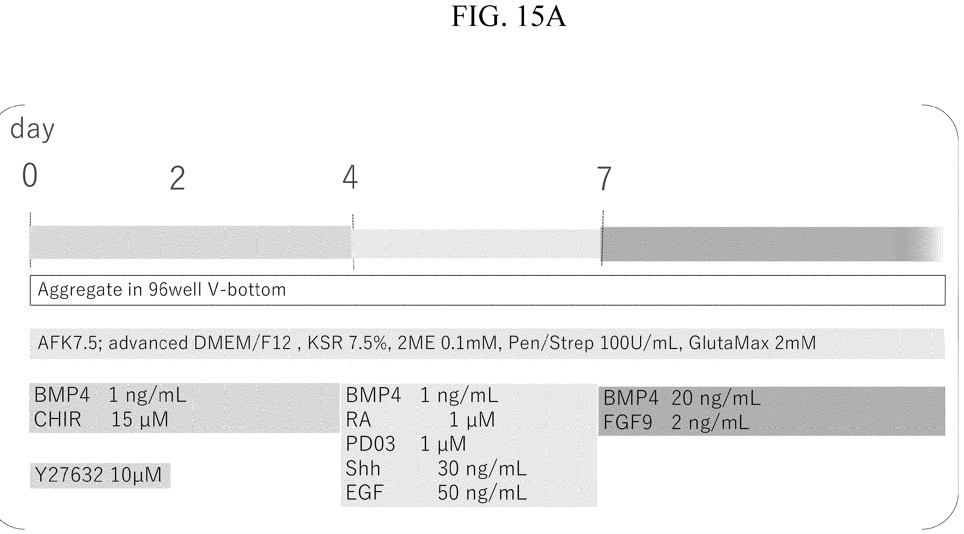

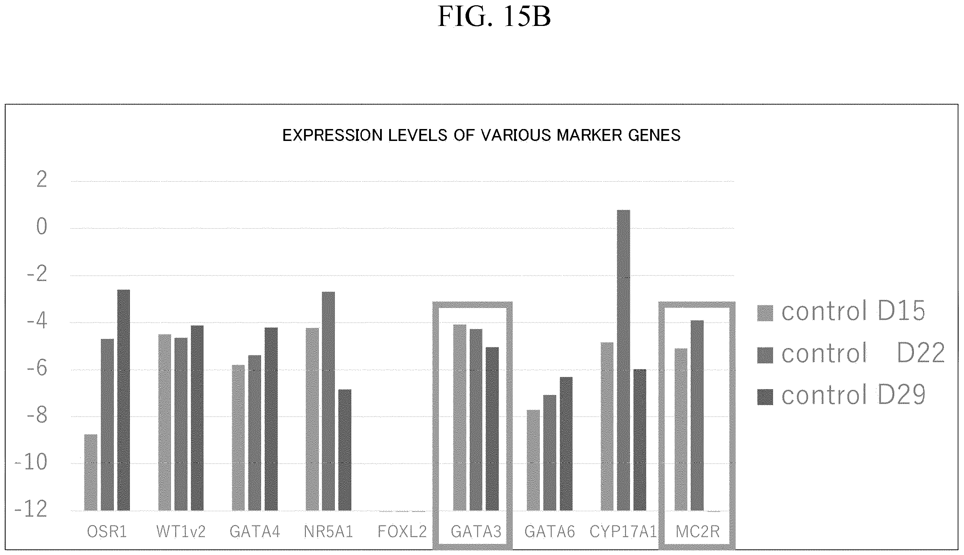

As a result of the examinations up to "12." above, the differentiation induction was performed according to the protocol shown in FIG. 15A. However, during the culture of step 3, the size of the embryoid bodies decreased with the passage of time, and many dead cells were observed. In addition, as a result of performing qPCR of NR5A1 (SF1)-positive cells on days 15, 22, and 29 of induction, expression of MC2R, which is an ACTH receptor as a marker gene of adrenal cells which are an anterior organ, was recognized, and the expression of GATA3, which is a proximal (forward) marker gene, was high (see FIG. 15B). Therefore, it was shown that there was a high possibility that cells have adrenal properties. On the other hand, no expression of FOXL2, which is a marker gene of granulosa cells, was observed (refer to FIG. 15B).

From the results above, it was revealed that as marker genes for the evaluation of the tail (posterior), HOXD9 and HOXD10 were selected as marker genes for ovarian somatic cells and HOXA7 and HOXB9 were selected as marker genes for adrenal cells, based on the previous report (Reference 3: Dolle P et al., "Two gene members of the murine HOX-5 complex show regional and cell-type specific expression in developing limbs and gonads.", The EMBO Journal, Vol. 8, No. 5, pp. 1507-1515, 1989.; Reference Document 4: Zubair M et al., "Two-step regulation of Ad4BP/SF-1 gene transcription during fetal adrenal development: initiation by a Hox-Pbx1-Prep1 complex and maintenance via autoregulation by Ad4BP/SF-1", Mol Cell Biol., Vol. 26, No. 11, pp. 4111-4121, 2006.), and then evaluated.

### 14. Examination of Presence or Absence of bFGF in Step 1 and Optimization of RA Concentration in Step 2

The evaluation was performed on day 15 of induction under the same conditions as in the protocol shown in FIG. 15A, except that the concentration of bFGF in step 1 was adjusted to 0 and 5 ng/mL and the concentration of RA in step 2 was adjusted to 0.1 (100 nM), 0.5 (500 nM), and 1 µM.

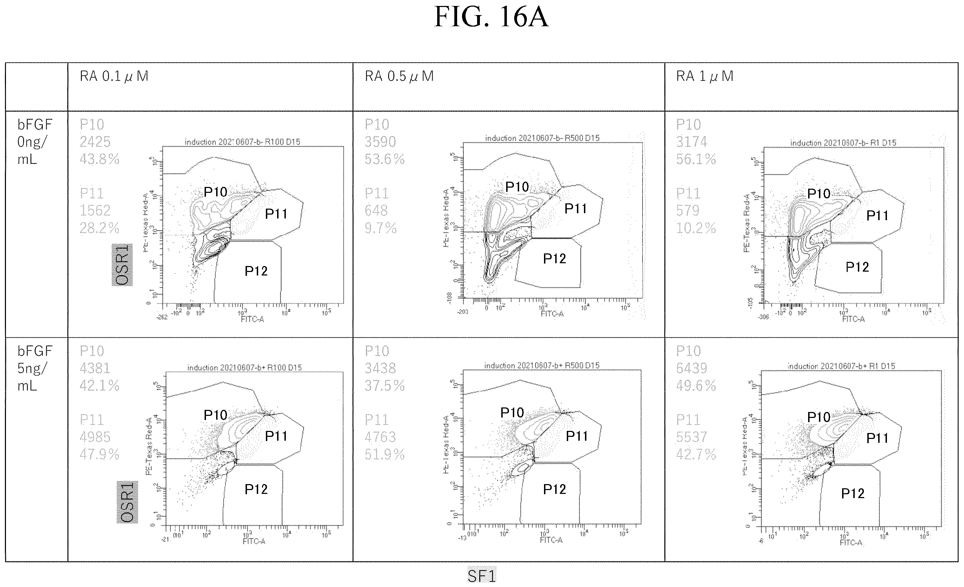

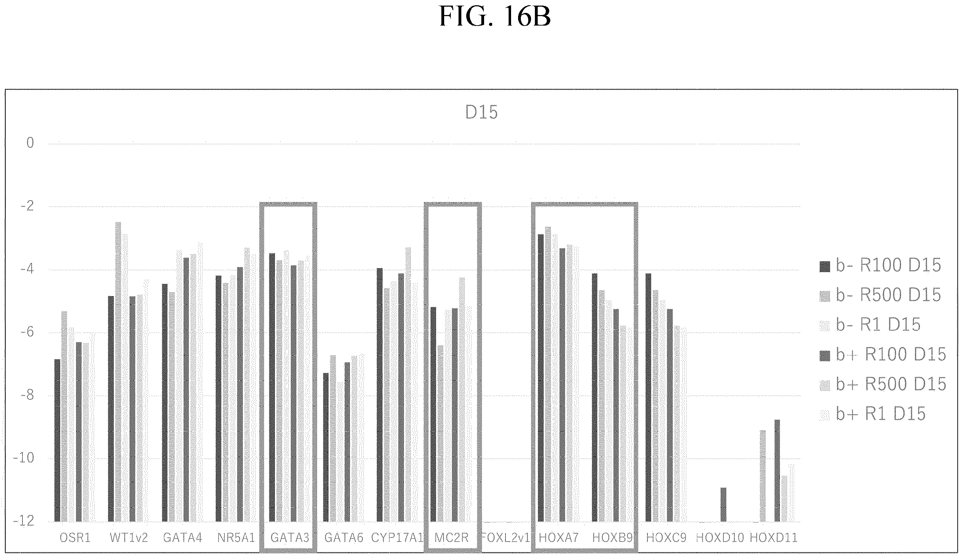

As a result, even in a case where bFGF was added in step 1 of even in a case where the concentration of RA was decreased in step 2, the expression of the marker gene of posteriorization was not recognized (see FIGS. 16A and 16B). In addition, it was shown that since the size of the embryoid bodies also decreased over time, there was a high possibility that the culture conditions were not appropriate as culture conditions for promoting posteriorization.

### 15. Optimization 3 of Period of Step 1

In a case where the period of step 1 was short, the differentiation into the head-side tissues was expected, whereas in a case where the period of step 1 was long, the differentiation into tail-side tissues was expected. Thus, the test was carried out under the same conditions as in the protocol shown in FIG. 15A, except that the period of step 1 was changed to 4 days and 7 days (the total period of step 1 and step 2 was 7 days and 10 days). An OTNG strain was used as a cell strain.

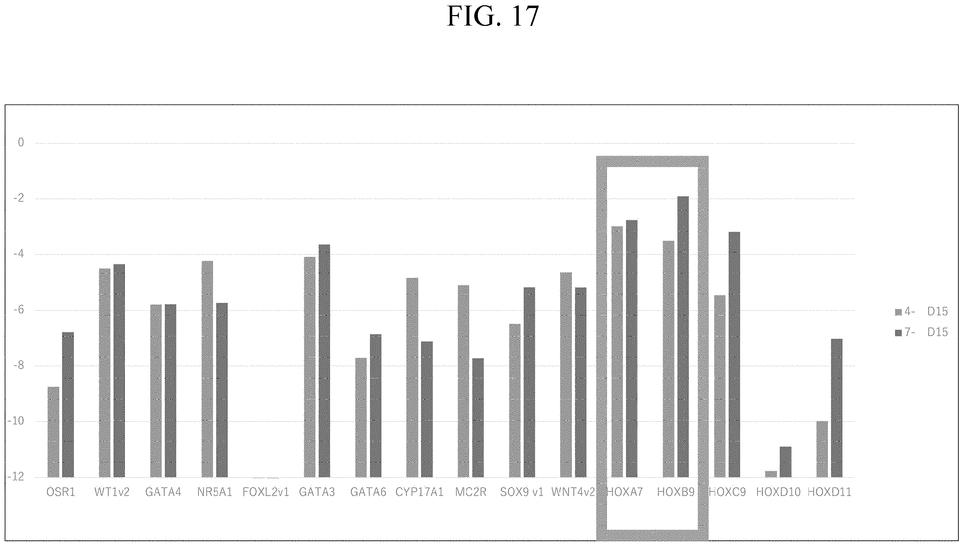

As a result, the expression of HOXB9, which was expressed in the adrenal gland (Adrenal) but not expressed in the ovarian body (Gonad), was recognized (see FIG. 17).

In addition, even in a case where the period of step 1 was extended or a combination use with other agents was examined as in "14.", the expression of HOXB9 did not become negative.

### 16. Optimization 1 of Culture Method of Step 1

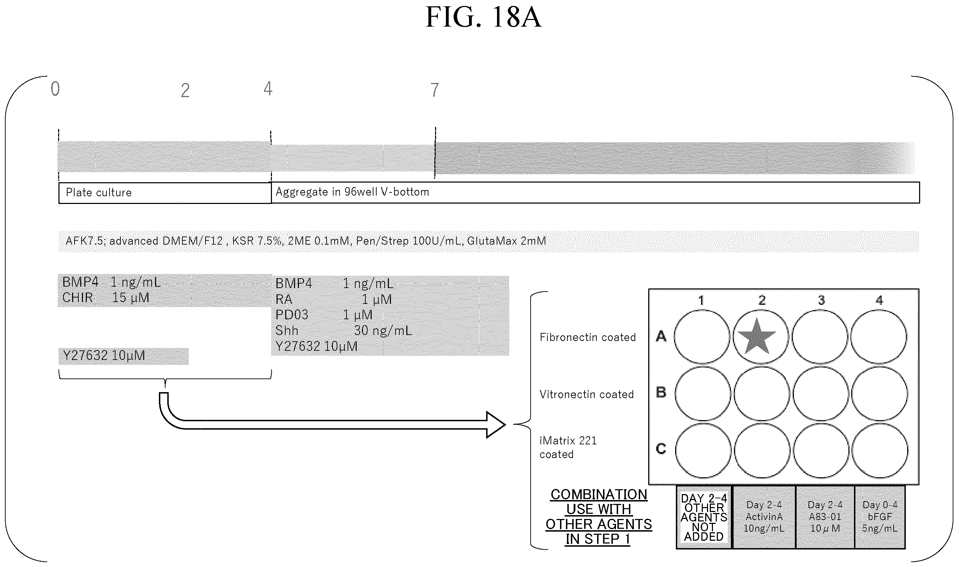

In the culture of step 1, it was assumed that there was heterogeneous stimulation of cytokine signaling due to the manufacture of embryoid bodies at an early stage. In order to solve this problem, a plane culture was examined instead of a suspension culture on a V-bottom plate in order to more efficiently and uniformly provide cell populations with cytokines for posteriorization by the plane culture. In addition, a combination use with a coating agent for a plate and other agents was also examined. The detailed protocol is shown in FIG. 18A. An OTNG strain was used as a cell strain.

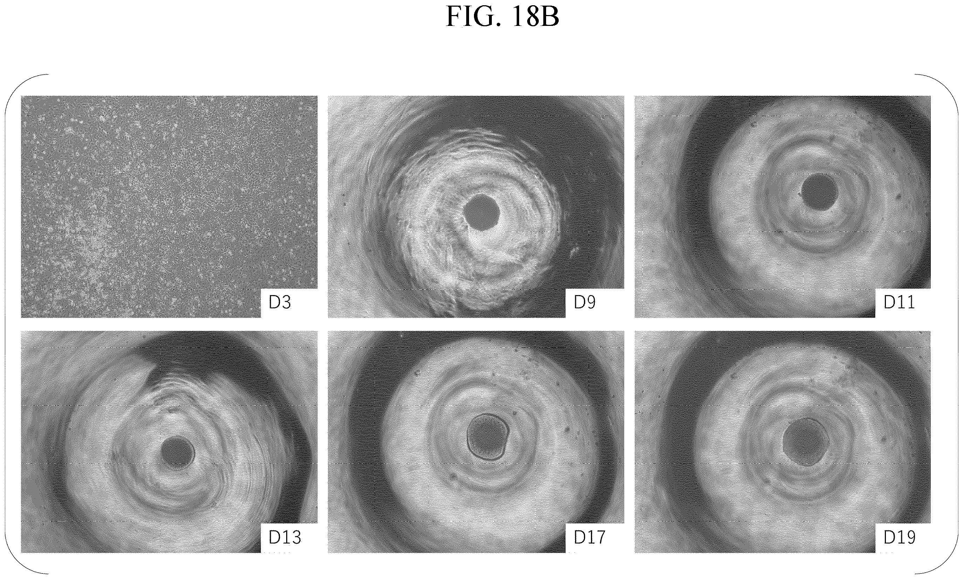

In a representative example, the culture of step 1 was performed by a plane culture using a plate coated with fibronectin, and the temporal change (observation images on days 3, 9, 11, 13, 17, and 19 of induction) of embryoid bodies under culture conditions (asterisked conditions in FIG. 18A), in which Activin A (10 ng/mL) was added between day 2 and day 4 of induction, is shown in FIG. 18B.

As shown in FIG. 18B, in the samples culture conditions in which the culture of step 1 was performed by a plane culture using a plate coated with fibronectin, and Activin A (10 ng/mL) was added between day 2 and day 4 of induction, the size of the embryoid bodies increased over time. In addition, in the samples under the culture conditions in which the plane culture was performed using a plate coated with vitronectin, and Activin A (10 ng/mL) was added between day 2 and day 4 of induction, the size of the embryoid bodies increased over time (not shown) as in the samples using fibronectin. On the other hand, in the samples under the culture conditions in which no other agents were used in combination, bFGF was added, and A830-01 was added between day 2 and day 4 of induction, the states of the embryoid bodies were poor (dead).

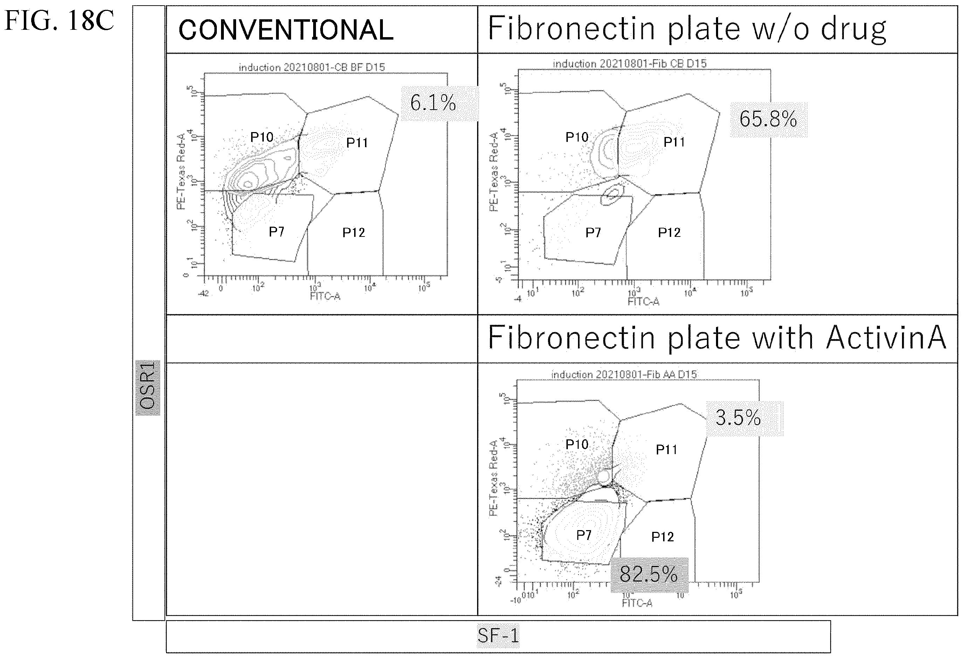

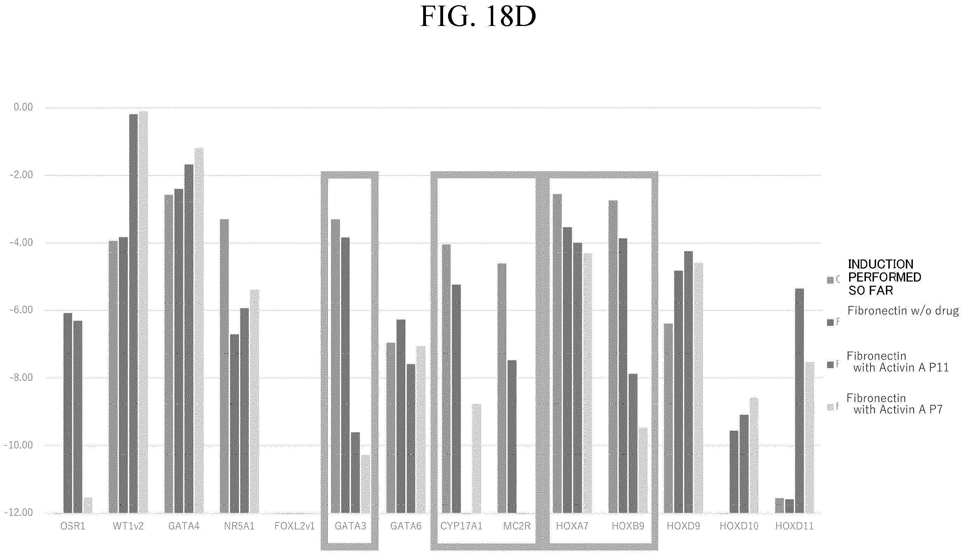

In addition, on day 15 of induction, the expression levels of the marker genes were measured by FACS analysis and quantitative PCR. The results are shown in FIG. 18C (FACS analysis) and FIG. 18D (quantitative PCR). In FIG. 18C and FIG. 18D, "Conventional" is the culture condition in the protocol of FIG. 15A. "Fibronectin plate w/o drug" refers to culture conditions in which the culture of step 1 was performed by a plane culture using a plate coated with fibronectin, and no other drug was added from day 2 to day 4 of induction. "Fibronectin plate with Activin A" refers to culture conditions in which the culture of step 1 was performed by a plane culture using a plate coated with fibronectin, and Activin A (10 ng/mL) was added from day 2 to day 4 of induction. In FIG. 18D, "P7" is an OSR1- (negative) and SF1+ (positive) cell, and "P11" is an OSR1+ (positive) and SF1+ (positive) cell.

As shown in FIG. 18C, the culture of step 1 was performed by a plane culture, and under the culture condition in which no other drug was added between day 2 and day 4 of induction, the proportion of the OSR1+ (positive) and SF1+ (positive) cells increased. In addition, under the culture condition in which Activin A (10 ng/mL) was added between day 2 and day 4 of induction, the proportion of the OSR1- (negative) and SF1+ (positive) cells further increased.

As shown in FIG. 18D, the culture of step 1 was performed by a plane culture using a plate coated with fibronectin, and under the culture conditions where Activin A (10 ng/mL) was added between day 2 and day 4 of induction, the expression of GATA3 and HOXB9 was decreased. In addition, in the samples under the culture conditions in which the plane culture was performed using a plate coated with vitronectin, and Activin A (10 ng/mL) was added between day 2 and day 4 of induction, the same quantitative PCR results as with the samples using fibronectin were obtained (not shown).

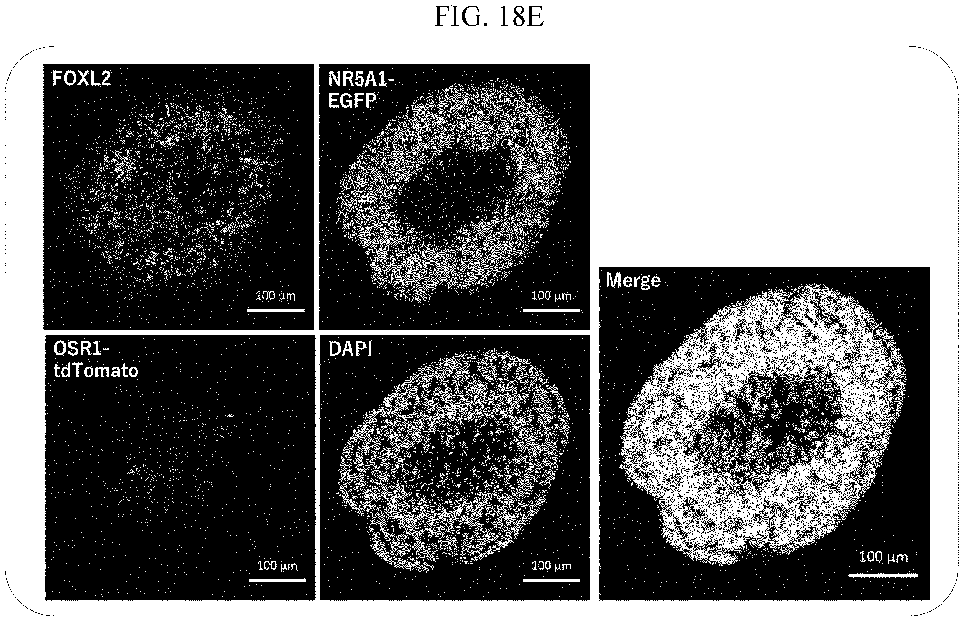

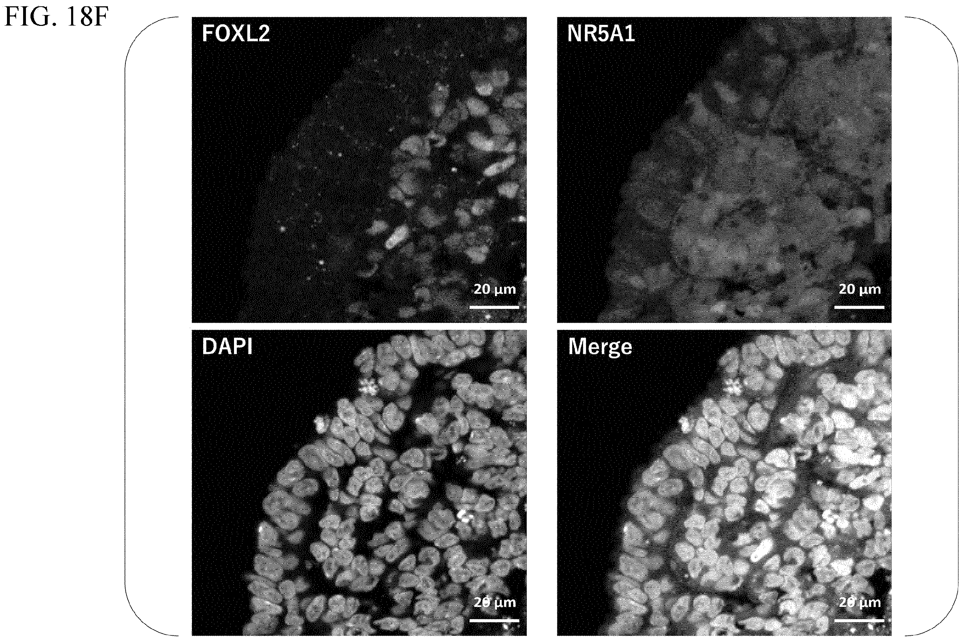

In the samples under the culture conditions in which the culture of step 1 was performed by a plane culture using a plate coated with fibronectin, and Activin A (10 ng/mL) was added between day 2 and day 4 of induction, immunostaining using an anti-FOXL2 antibody was performed on day 22 of induction. The immunostaining images are shown in FIG. 18E (magnification: 20 times) and FIG. 18F (magnification: 63 times).

As shown in FIG. 18E and FIG. 18F, dead cells gathered in the central part of the embryoid body since the central part of the embryoid body was not stained with DAPI. In the outermost layer, only SF1 (NR5A1) was positive and FOXL2 was expressed from the inside thereof.

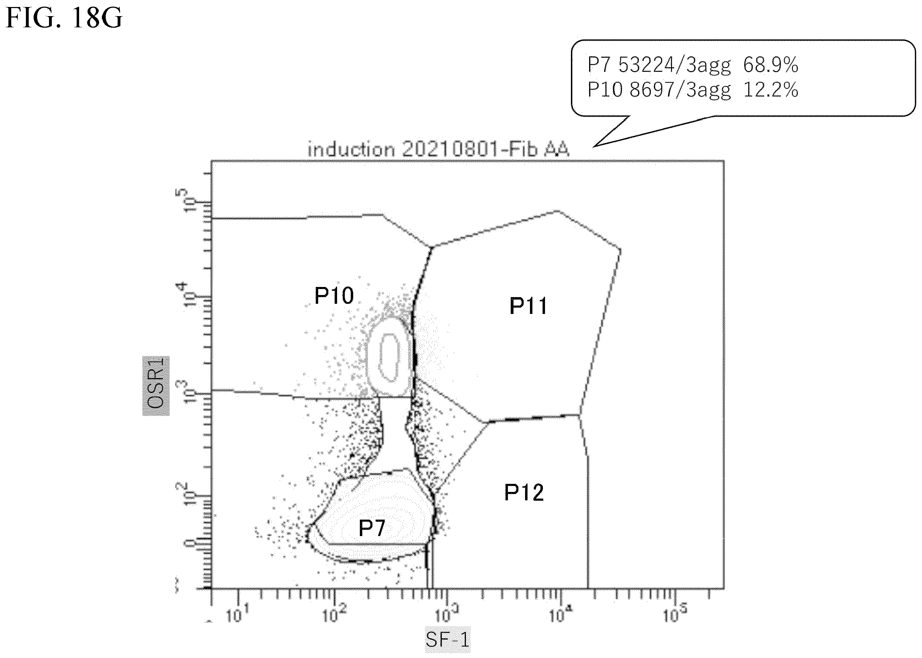

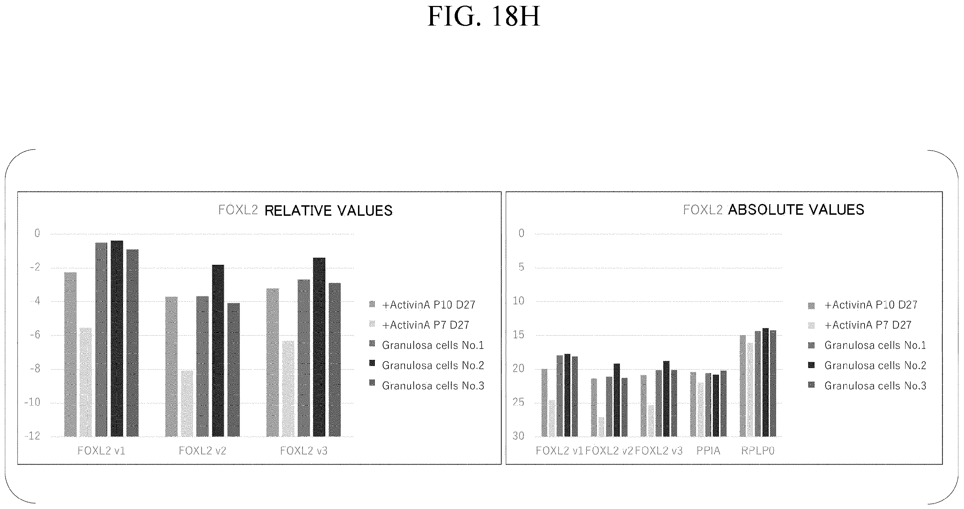

Furthermore, in the samples under the culture conditions in which the culture of step 1 was performed by a plane culture using a plate coated with fibronectin, and Activin A (10 ng/mL) was added between day 2 and day 4 of induction, the expression levels of the marker genes were measured by FACS analysis and quantitative PCR on day 27 of induction. The results are shown in FIG. 18G (FACS analysis) and FIG. 18H (quantitative PCR). In FIG. 18G and FIG. 18H, "P7" in FIG. 18G is an OSR1-(negative) and SF1+ (positive) cell and "P10" is an OSR1+ (positive) cell and an SF1+ (weakly positive) cell. "P7" and "P10" are used with the same meanings in the subsequent drawings. In FIG. 18H, as a control, the expression of a marker gene of ovarian somatic cells collected from a living body was also confirmed.

As shown in FIG. 18G and FIG. 18H, in the samples under the culture conditions in which the culture of step 1 was performed by a plane culture using a plate coated with fibronectin, and Activin A (10 ng/mL) was added between day 2 and day 4 of induction, the expression of FOXL2 almost equivalent to that in the ovarian somatic cells collected from the living body was recognized. In "P7" rather than in "P10", the expression of FOXL2 was low in OSR1- (negative) and SF1+ (positive) cells. Since these cells form the outermost layer in the immunostaining images of FIG. 18E and FIG. 18F, it was presumed that there were a number of FOXL2-negative cells.

### 17. Reproducibility Confirmation Test

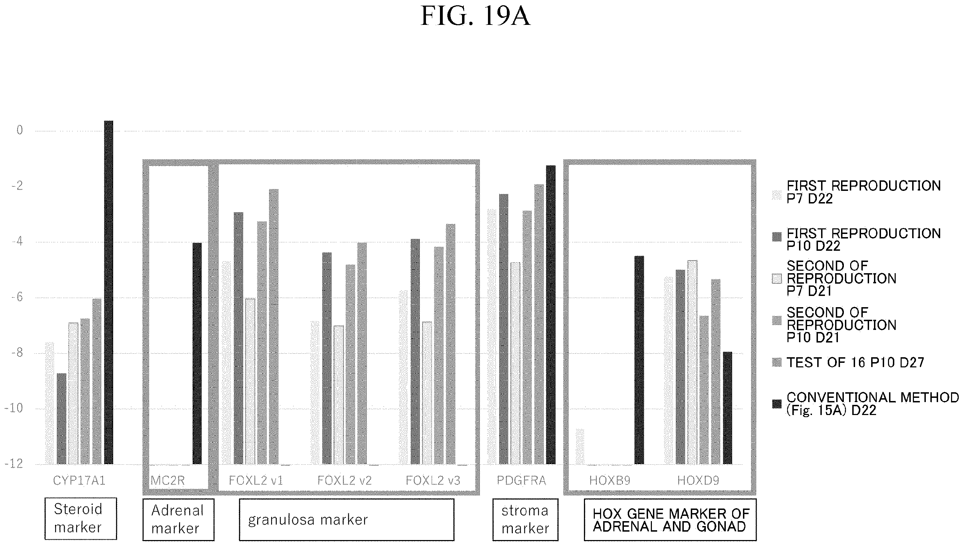

The reproducibility confirmation test under the culture conditions of "16." was conducted twice. An OTNG strain was used as a cell strain. On day 21 or 22 of induction, the expression levels of the marker genes were measured by quantitative PCR. The results are shown in FIG. 19A.

As shown in FIG. 19A, the expression of FOXL2 was confirmed in both of the two reproducibility confirmation tests. In addition, it was considered that a decrease in HOXB9 was an index of posteriorization.

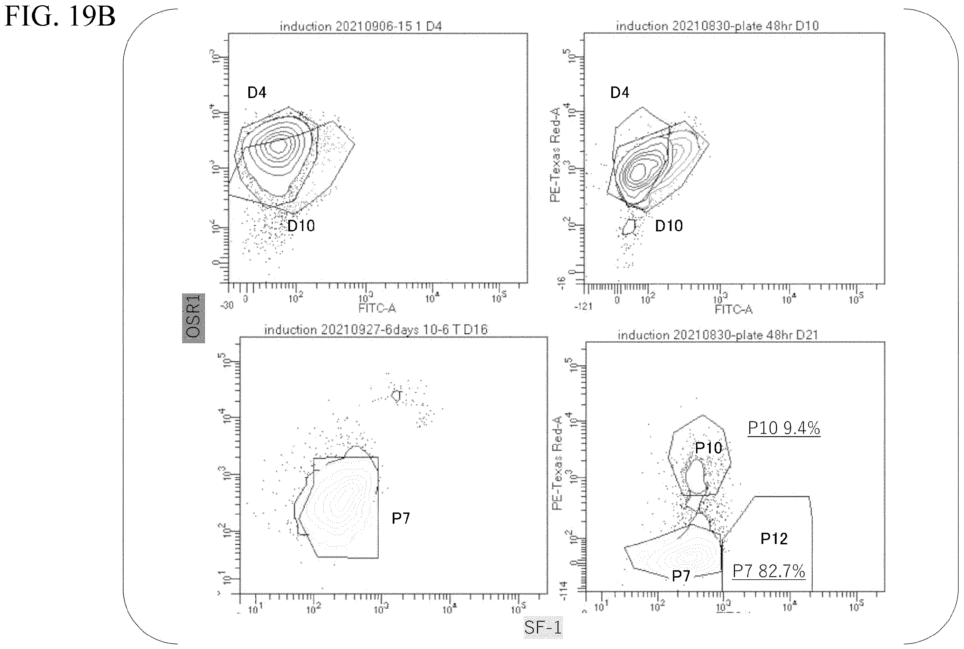

In addition, in the reproducibility test, FACS analysis was performed on days 4, 10, 16, and 21 of induction. The results are shown in FIG. 19B.

As shown in FIG. 19B, on day 4 of induction, the number of the OSR1+ (positive) and SF1(NR5A1)- (negative) cells increased, and on day 10 of induction, the number of the OSR1+ (positive) cells decreased and the number of the SF1 (NR5A1)+ (positive) cells started to increase. On day 16 of induction, the number of the OSR1-(negative) and SF1(NR5A1)+ (positive) cells increased, and on day 21 of induction, the number of the OSR1- (negative) or OSR1+ (positive) and SF1(NR5A1)+ (positive) cells increased.

### 18. Optimization 2 of Culture Method of Step 1 and Effect of Combination Use with Other Agents in Step 3

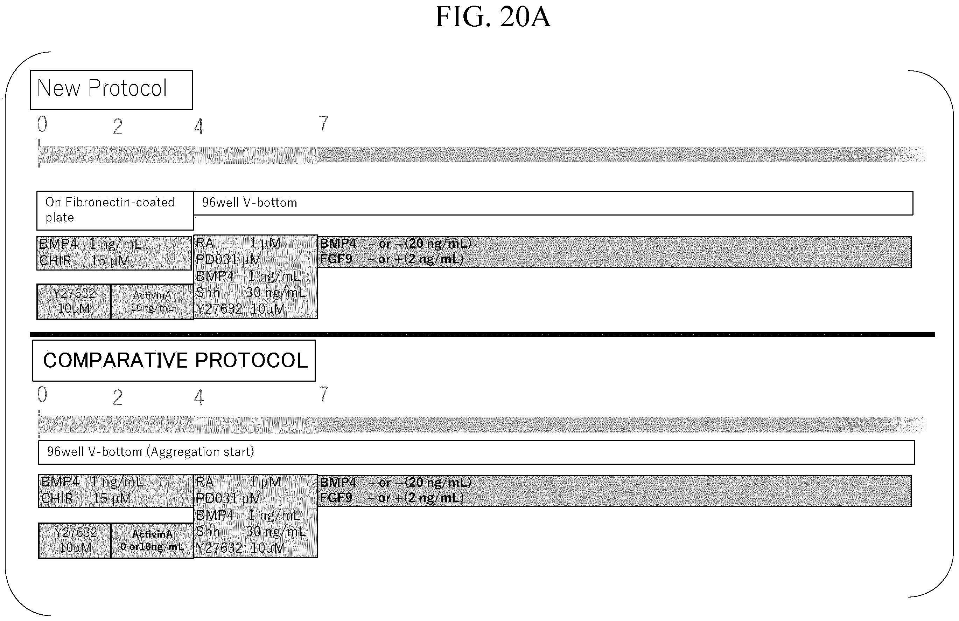

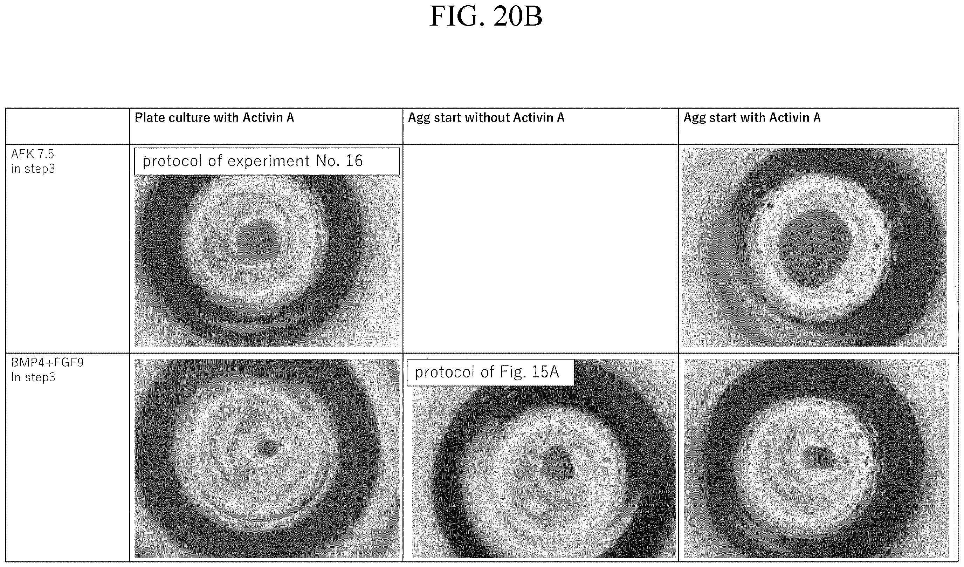

The presence or absence of the addition of Activin A in step 1, and the culture method were set for a plane culture using a plate coated with fibronectin or a suspension culture using a V-bottom plate, and furthermore, the presence of absence of addition of BMP4 and FGF9 in step 3 was examined. A specific protocol is shown in FIG. 20A. An OTNG strain was used as a cell strain. The observation image of the embryoid body under each culture condition on day 21 of induction is shown in FIG. 20B.

As shown in FIG. 20B, in the samples under the culture conditions in which the culture of step 1 was performed by a plane culture using a plate coated with fibronectin, Activin A (10 ng/mL) was added from day 2 to day 4 of induction, and BMP4 and FGF9 were not added in step 3, the embryoid bodies was growing significantly.

Large embryoid bodies were obtained even in the samples under the culture conditions in which the culture of step 1 was changed to a suspension culture using a V-bottom plate, Activin A (10 ng/mL) was added thereto from day 2 to day 4 of induction, and BMP4 and FGF9 were not added in step 3. Thus, immunostaining was performed on day 22 of induction. However, the expression of FOXL2, SF1 (NR5A1-EGFP), and OSR1 (OSR-tdTomato) was not recognized (not shown).

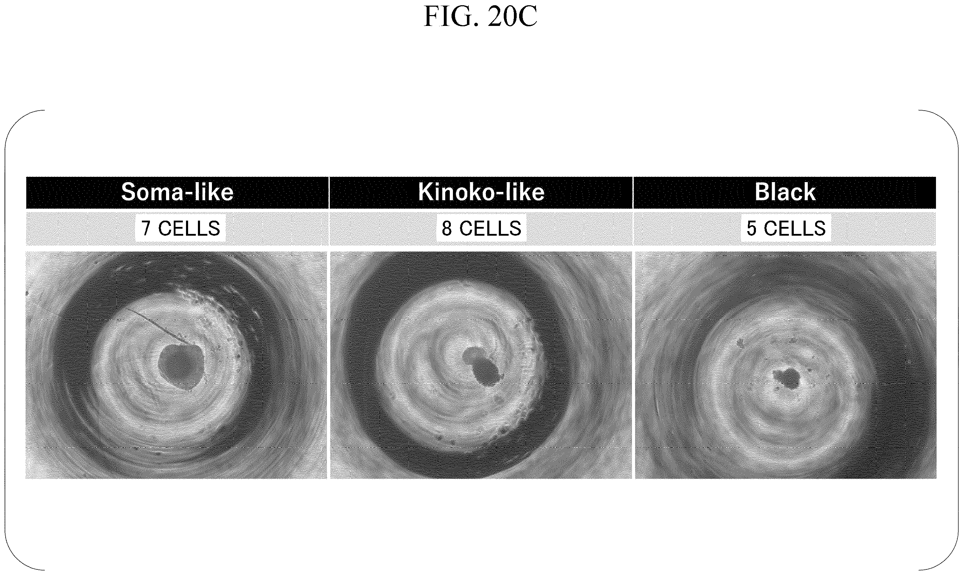

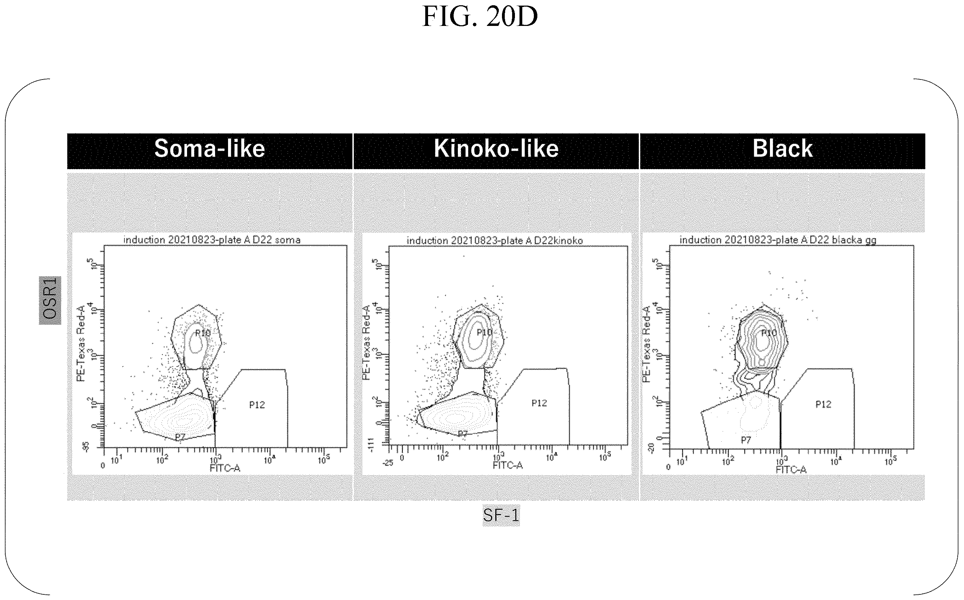

On the other hand, in the samples under the culture conditions in which the culture of step 1 was performed by a plane culture using a plate coated with fibronectin, Activin A (10 ng/mL) was added thereto between day 2 and day 4 of induction, and BMP4 and FGF9 were not added in step 3, embryoid bodies with an ovarian somatic cell-like structure (Soma-like), a mushroom-like structure (Kinoko-like), and a black dot structure (Black) were observed on day 17 of induction (see FIG. 20C). FACS analysis was performed on the embryoid body with each structure on day 22 of induction. The results are shown in FIG. 20D.

As shown in FIG. 20D, the same tendency of the FACS plots was observed for the Soma-like and Kinoko-like embryoid bodies.

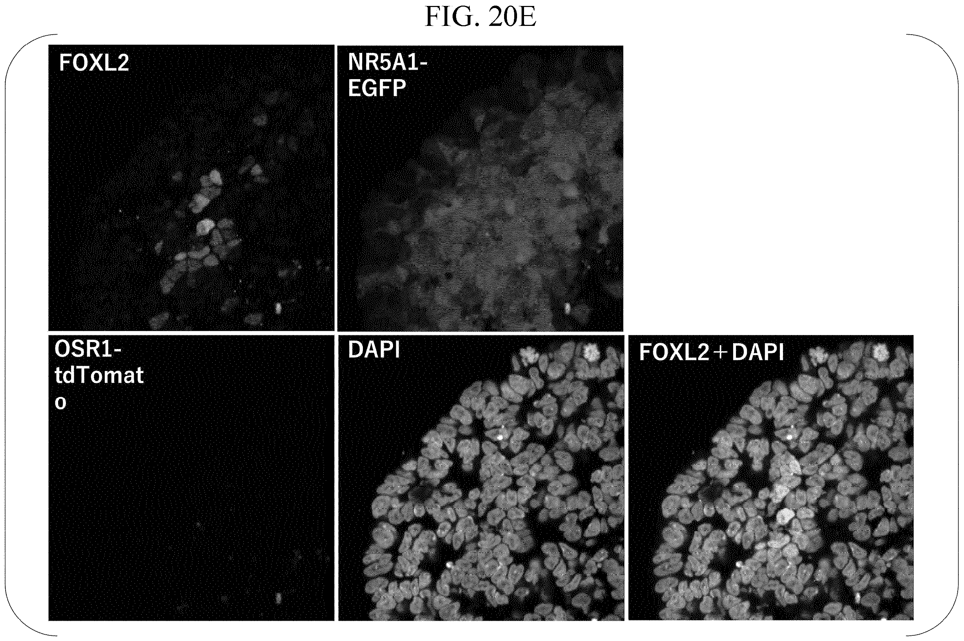

In addition, the results of immunostaining of the induction of the "Kinoko-like" embryoid bodies on day 17 are shown in FIG. 20E.

As shown in FIG. 20E, in the outermost layer, only SF1 (NR5A1) was positive and FOXL2 was expressed from the inside thereof. These were the same results as the immunostaining images of the embryoid bodies in "16." described above (see FIGS. 18E and 18F).

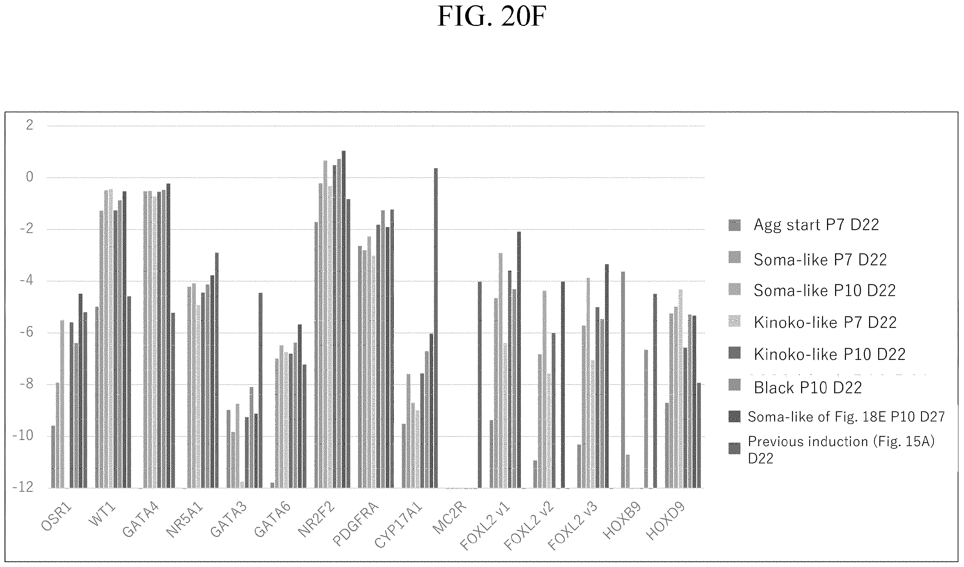

Furthermore, the expression levels of the marker genes were measured by quantitative PCR on day 22 of induction. The results are shown in FIG. 20F.

As shown in FIG. 20F, almost no expression of FOXL2 was observed in the samples in which the culture in step 1 was suspension-cultured on a V-bottom plate. In addition, the expression of GATA3 was low, but the expression of HOXB9 was high, and it cannot be said to have differentiation into ovarian somatic cells.

The expression modes of FOXL2 in P7 and P10 of the Kinoko-like embryoid bodies were each almost the same as the expression modes of FOXL2 in P7 and P10 of the Soma-like embryoid bodies. Therefore, it was speculated that the Soma-like and Kinoko-like embryoid bodies have the properties of ovarian somatic cells.

On the other hand, the expression of FOXL2 was also recognized in the embryoid bodies of Black, but the expression mode of HOXB9 was different from that in the Soma-like and Kinoko-like embryoid bodies.

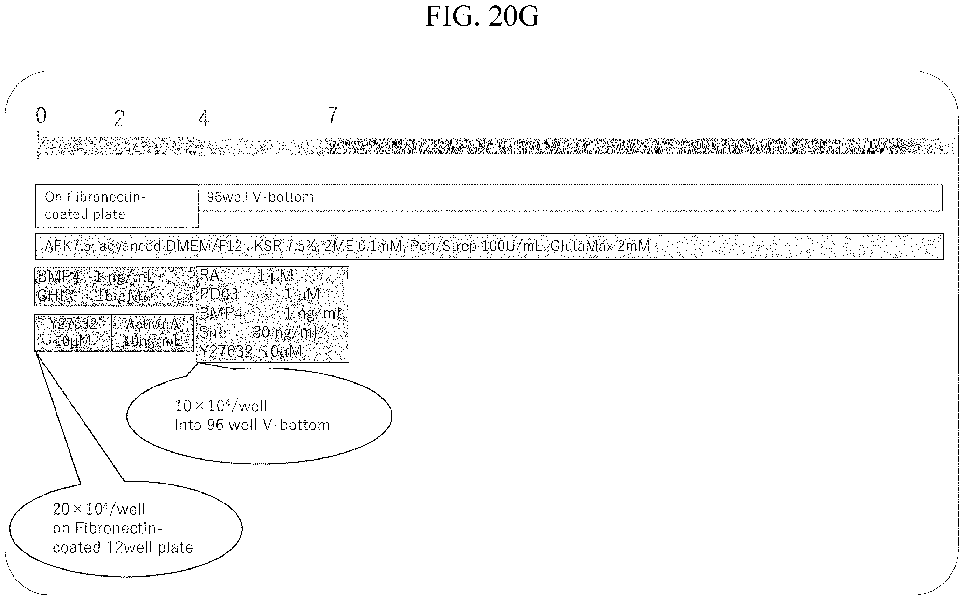

Based on the results above, the test was performed based on the protocol shown in FIG. 20G.

### 19. Confirmation of Expression of PDGFRα

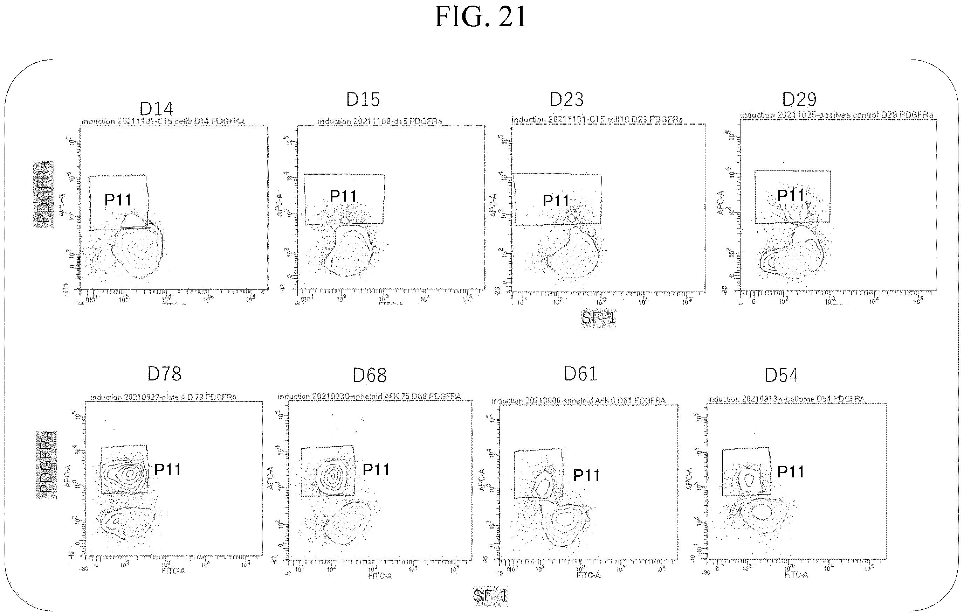

It is considered that granulosa cells and stromal cells are present in the ovarian somatic cells, and that FOXL2 is expressed in the granulosa cells and PDGFRα is expressed in the stromal cells. Therefore, in the cells obtained by the protocol shown in FIG. 20G (the OTNG strain was used as a cell strain), changes in the expression of PDGFRα were confirmed by FACS (FIG. 21 and Table 6). In FIG. 21 and Table 6, "P11" indicates a PDGFRα+ (positive) cell. In addition, the anti-PDGFRα antibody used in the FACS analysis was an APC anti-human CD140a (PDGFRα) antibody (No. 323512) manufactured by Biolgend, Inc. As a protocol, a single cell was used, then reacted with the anti-PDGFRα antibody (incubation on ice at 4°C for 30 minutes), and then washed twice.

**[Table 6]**

| **Induction date** | **FACS date** | **P11 cell/agg** |
|---|---|---|
| 0823 | D78 | 46.0% (6,699) |
| 0830 | D68 | 33.4% (30,295 + α) |
| 0906 | D61 | 14.7% (10,167) |
| 0913 | D54 | 15.5% (21,926) |
| 1025 | D29 | 18.5% (3,011) |
| 1101 | D23 | 6.5% (602) |
| 1108 | D15 | 7.9% (1,485) |
| 1101 | D14 | 8.6% (149) |

As shown in FIG. 21, an increase in the proportion of cells expressing PDGFRα was recognized before and after 30 days of induction.

### 20. Examination of Long-Term Culture in Step 3

Before examining whether mature eggs are obtained by co-aggregating the ovarian somatic cell-like cells obtained by the protocol shown in FIG. 20G and culturing the cells with PGCLCs, it was examined whether the ovarian somatic cell-like cells could maintain the properties as the ovarian somatic cells in a case where a long-term culture was performed based on the fact that the co-aggregate culture takes a long period of time. Specifically, in the protocol shown in FIG. 20G, the long-term culture was performed while maintaining the culture conditions of step 3. An OTNG strain was used as a cell strain.

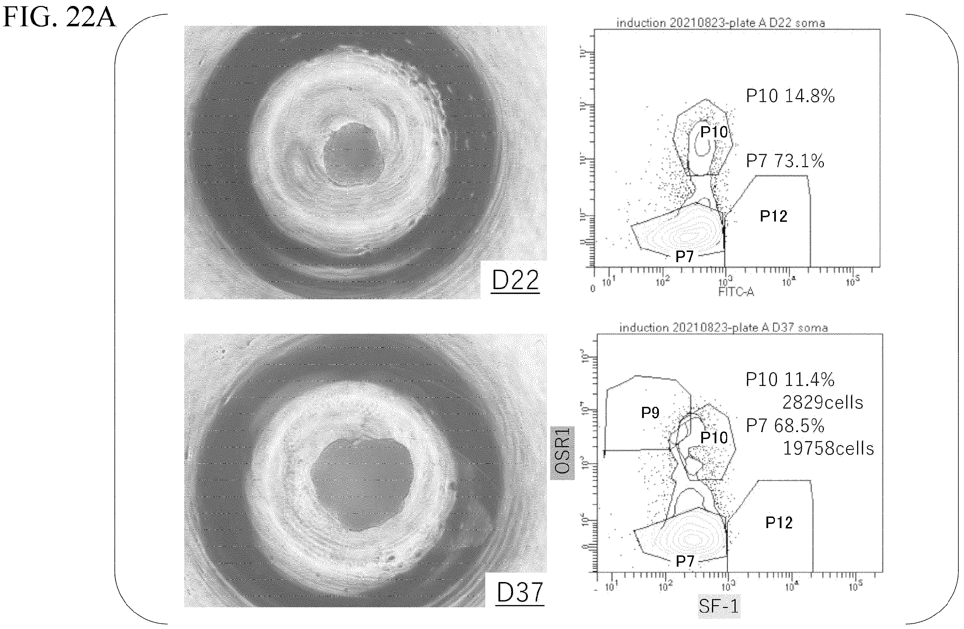

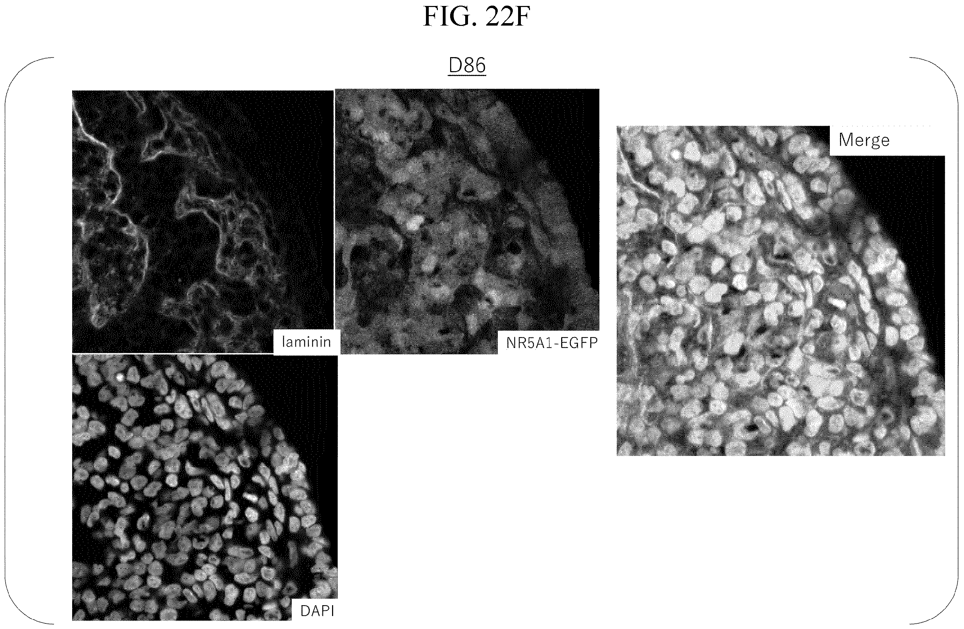

The observation images (left side) of embryoid bodies on day 22 and day 37 of induction and the results of FACS analysis (right side) are shown in FIG. 22A.

As shown in FIG. 22A, it was found that the embryoid bodies on day 37 of induction were larger in size than the embryoid bodies on day 22 of induction, and the number of SF1- (negative) cells tended to increase in the OSR1+ (positive) cells.

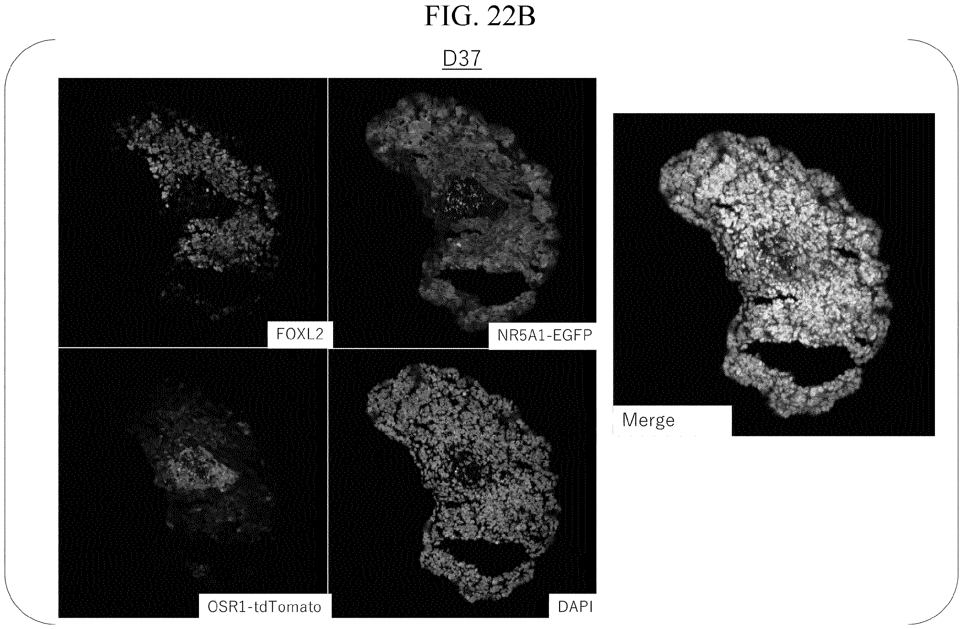

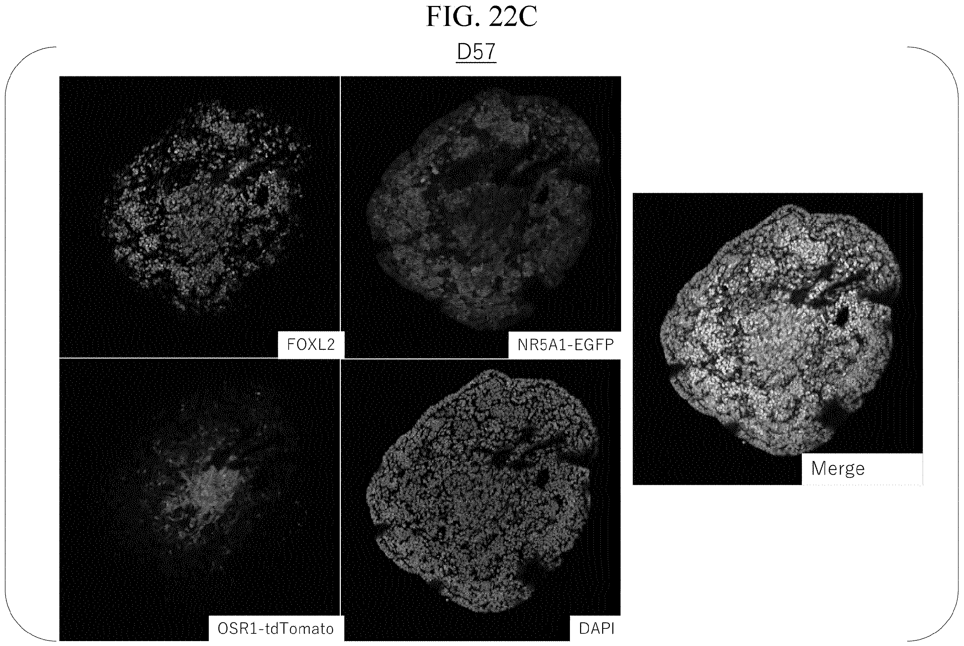

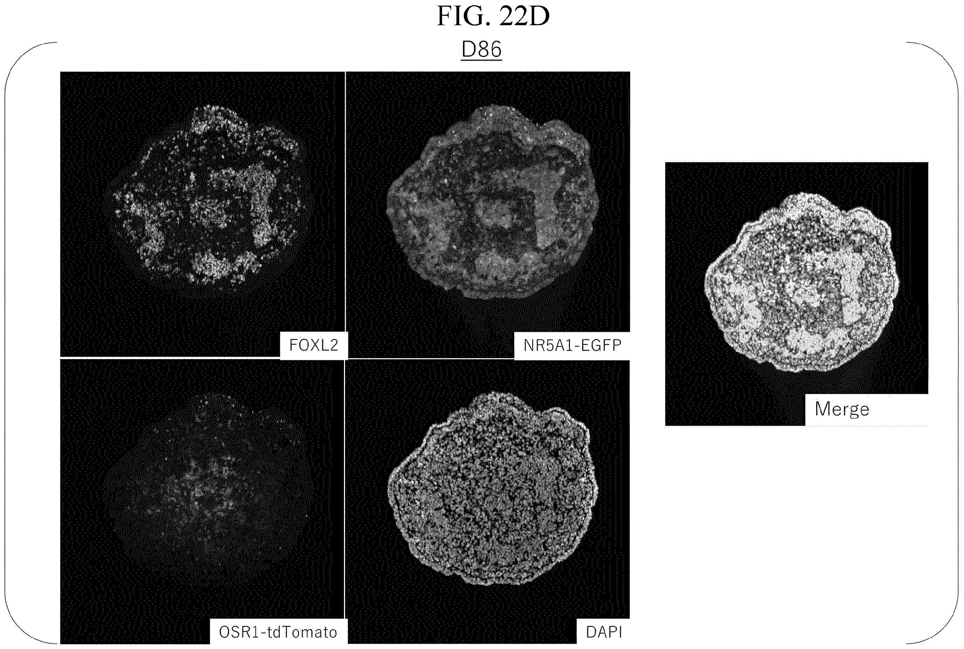

In addition, immunostaining was performed on days 37, 57, and 86 of induction. The results are shown in FIG. 22B (day 37), FIG. 22C (day 57), and FIG. 22D to FIG. 22F (day 86).

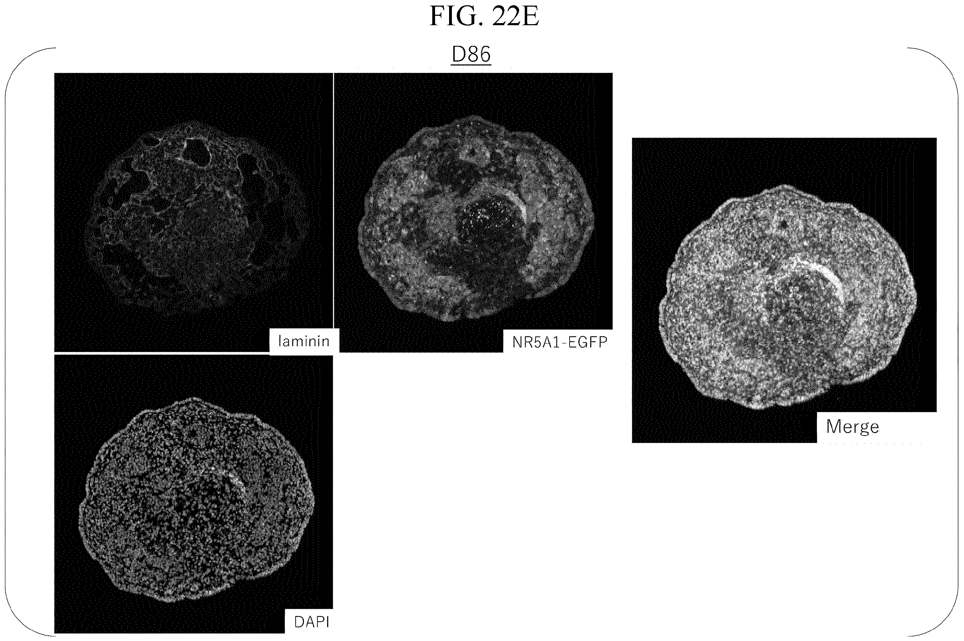

On day 37 of induction, the expression of FOXL2 could be sufficiently maintained (see FIG. 22B). On day 57 of induction, the OSR1+ (positive) and FOXL2+ (positive) cell population was formed in the central part, and the FOXL2+ (positive) cells were also enriched in the central part (see FIG. 22C). On day 86 of induction, the distributions of SF1+ (positive) and FOXL2+ (positive) were consistent (see FIG. 22D), and laminin was present such that it surrounded SF1+ (positive) (see FIG. 22E). Since granulosa cells are formed in the shape of islands as being surrounded by laminin, it was confirmed that the granulosa cells had a structure similar to the ovarian cord in vivo at day 86 of induction.

### 21. Confirmation of Expression of SF1 (NR5A1) and Cell State in Step 3

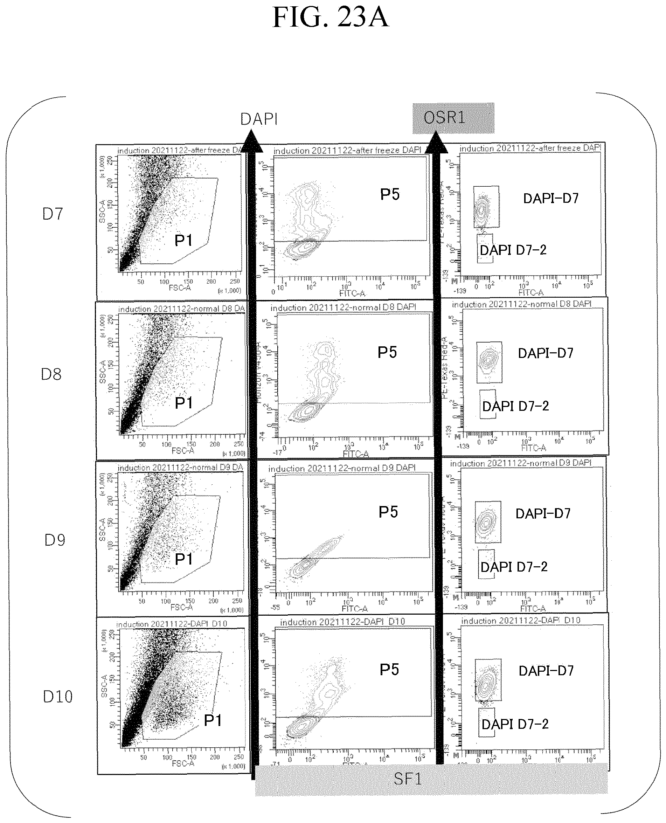

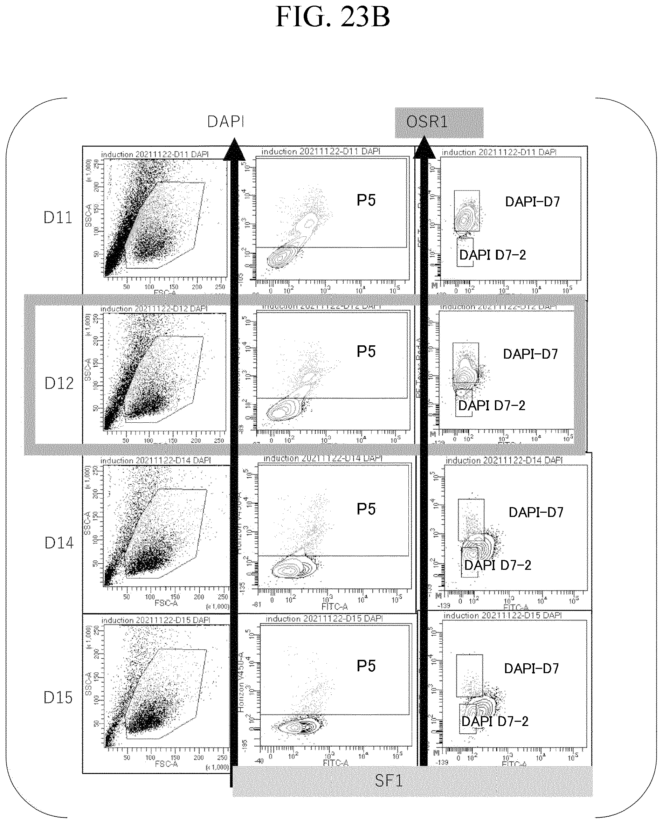

Since an embryoid body is not formed well with a small number of cells, there is a possibility that the number of living cells significantly decreased in step 2. Therefore, confirmation of the cell states was performed. Specifically, the culture was performed using an OTNG strain as a cell strain according to the protocol shown in FIG. 20G, and FACS analysis was performed on days 7, 8, 9, 10, 11, 12, 14, and 15 of induction. In the FACS analysis, OSR1+/- and SF1+/- were plotted after removing dead cells with DAPI (see FIG. 23A and FIG. 23B).

As shown in FIG. 23A and FIG. 23B, a population of living cells in P1 tended to increase from day 7 to day 15 of induction. In addition, the number of DAPI-positive cells tended to decrease gradually. The number of the SF1-positive cells increased from day 12 of induction.

### <Test for Examining Induction of Differentiation from Cynomolgus Monkey ES Cells into Ovarian Somatic Cell-Like Cells>

### [Materials]

### 1. Cynomolgus Monkey ES Cell Line

For the purpose of maintaining the quality of a cynomolgus monkey female ES15XRi cell line and a knock-in reporter line thereof, only colonies having good morphology were manually picked up and passaged. Alternatively, high-quality ES cells were concentrated and passaged using SSEA-4 and TRA1-60, which are pluripotency markers, by flow cytometry. As for other methods for passage culture, the passage culture was performed in accordance with a document previously reported (Reference Document 5: Sakai Y et al., "Induction of the germ cell fate from pluripotent stem cells in cynomolgus monkeys.", Biology of Reproduction, Vol. 102, Issue 3, pp. 620-638, 2020.). The number of seeded cells at the time of passage was appropriately adjusted.

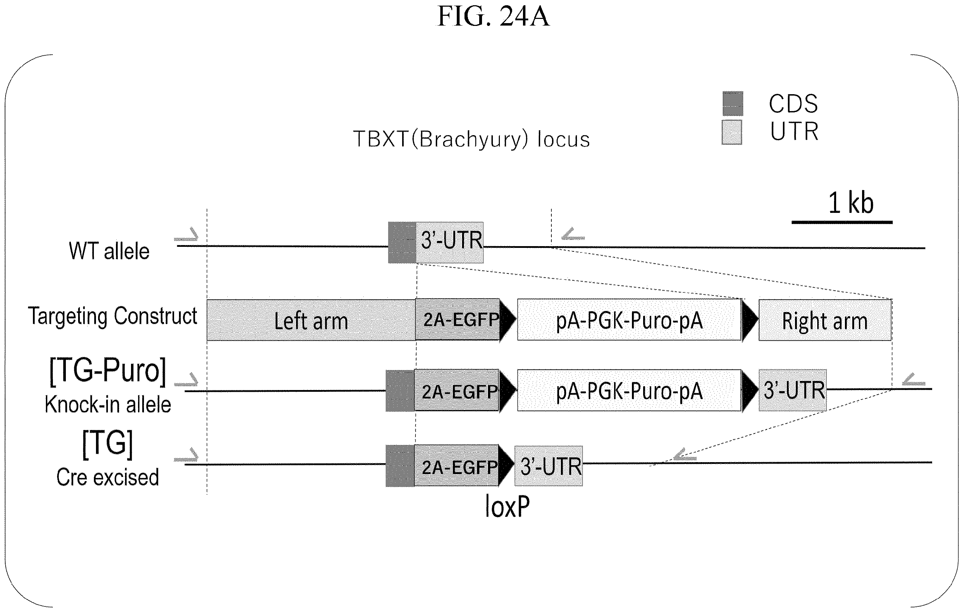

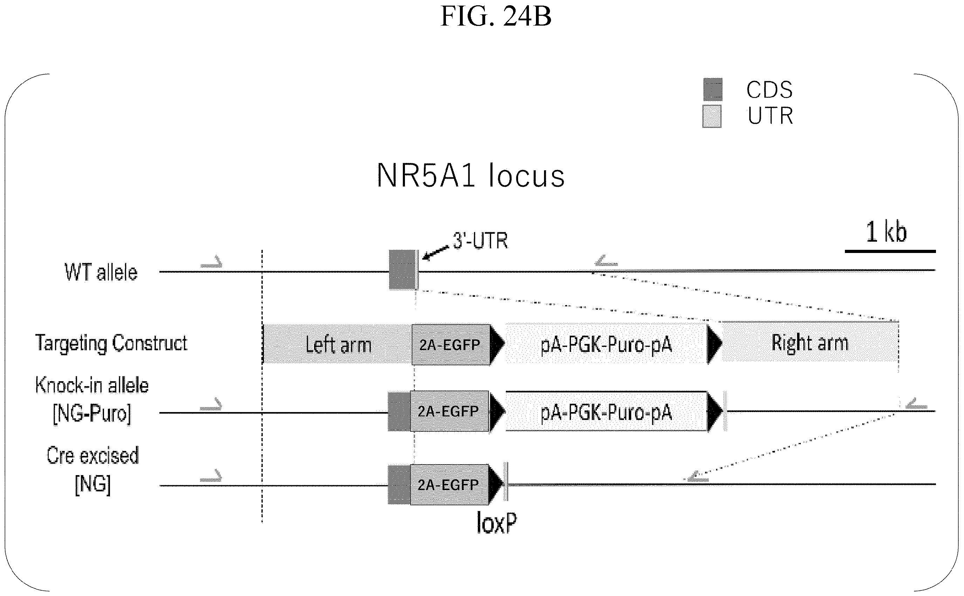

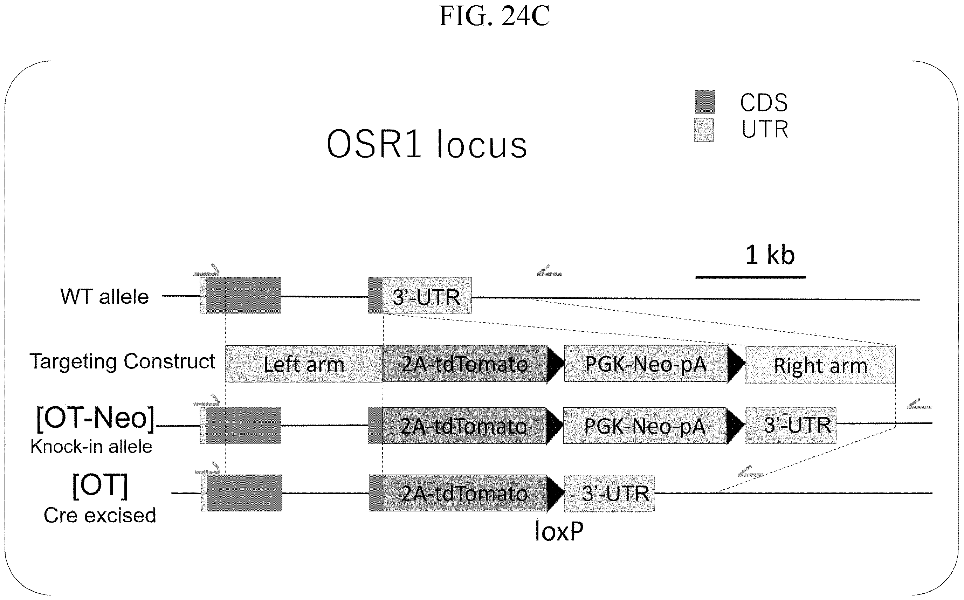

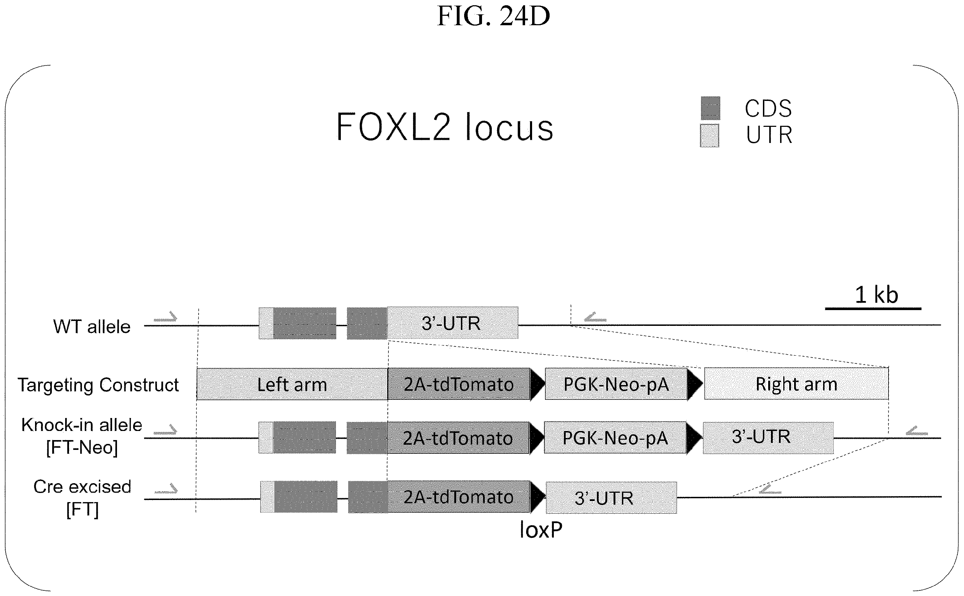

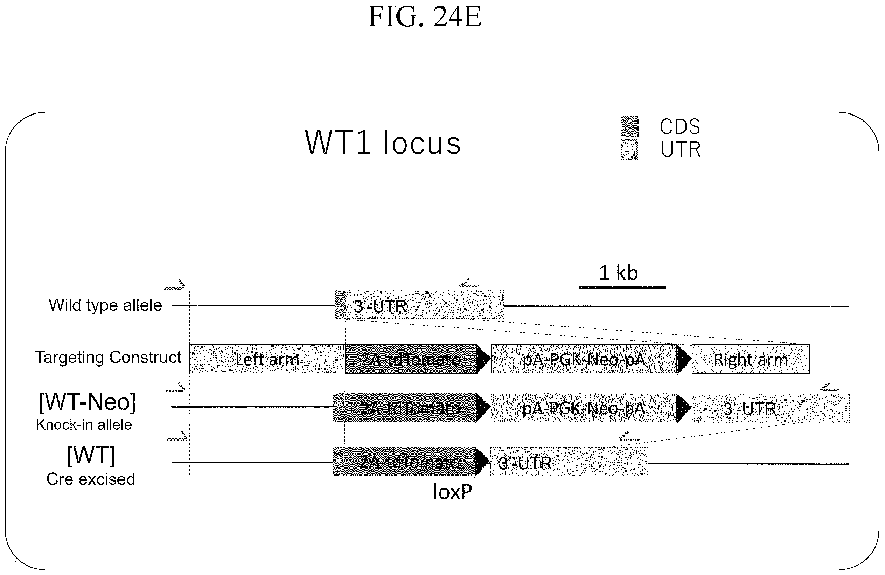

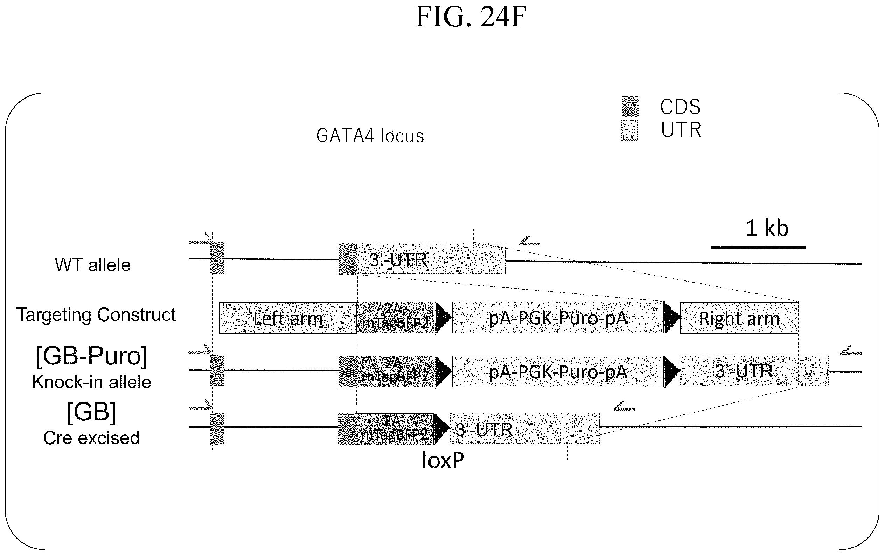

In order to conveniently observe the expression of genes represented by each developmental stage, the stop codons of TBXT, OSR1, NR5A1, FOXL2, WT1, and GATA4 were deleted from the cynomolgus monkey female ES15XRi cell strain using a Cas9 nickase and gene targeting, 2A and EGFP (TBXT, NR5A1), tdTomato (OSR1, FOXL2, and WT1), and 2A and mTagBFP2 (GATA4) were inserted, and then a drug-resistant cassette sandwiched between loxP's was knocked in (FIGS. 24A to 24F; and in FIG. 24A to FIG. 24F, "PGK" represents a PGK promoter). Thereafter, the drug-resistant cassette was removed using Cre to manufacture the following five double reporter cell strains.
(1) TBXT-EGFP/OSR1-tdTomato (which may be hereinafter abbreviated as a "TGOT strain");
(2) NR5A1-EGFP/FOXL2-tdTomato (which may be hereinafter abbreviated as a "NGFT strain");
(3) a single reporter strain of NR5A1-EGFP (which may be hereinafter abbreviated as an "NG-Puro strain". This strain has not been subjected to the removal of the drug-resistant cassette by Cre);
(4) a triple reporter strain of WT1-tdTomato/GATA4-mTagBFP2/NR5A1-EGFP (which may be hereinafter abbreviated to as a "WTGBNG strain"); and
(5) WT1-tdTomato/NR5A1-EGFP (which may be hereinafter abbreviated as a "WTNG strain").

### 2. Medium and Drug

With regard to a medium, a drug, and the like, those described in Tables 1 and 2 above were used.

### [Method]

### 1. Fluorescent Immunostaining and Observation by Fluorescence Microscope

The antibodies used for immunostaining are as follows.
(Primary antibody: a dilution rate is 250 times for any of antibodies)
   Rabbit anti-FOXL2 antibody (ab246511) manufactured by Abeam, Inc.
   Goat anti-tdTomato antibody (AB8181-200) manufactured by SICGEN
   Rat anti-GFP antibody (04404-84) manufactured by Nacalai Tesque, Inc.
(Secondary antibody: a dilution rate is 500 times for any of antibodies)
   Donkey anti rat Alexa Fluor 488 (A21208) manufactured by Thermo Fisher Scientific, Inc.
   Donkey anti goat Alexa Fluor 568 (A11057) manufactured by Thermo Fisher Scientific, Inc.
   Donkey anti rabbit Alexa Fluor 647 plus (A32795) manufactured by Thermo Fisher Scientific, Inc.

In addition, the cells at each stage were observed using a fluorescence microscope (ZEISS confocal laser scanning, LSM780).

### 2. Fluorescence Activated Cell Sorting Analysis (FACS)

The cells at each stage were analyzed using a flow cytometer (BD FACSAria 111).

### 3. Quantitative PCR

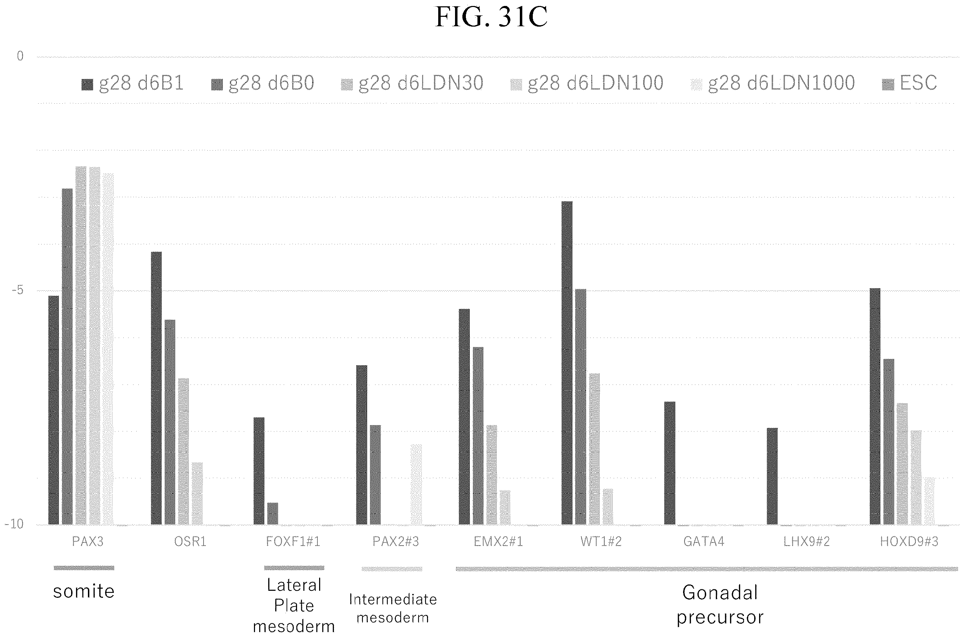

The relative expression level of each marker gene was confirmed using a PCR apparatus (BIO-RAD CFX384 Real-Time System) with the following primers and conditions. In the table, the primers of PAX3, OSR1, PAX2 #3, EMX2 #1, WT1 #2, and LHX9 #2 were used only in the experiment of FIG. 31C, and the primers of OSR1 #10, CXCL12 x4, BMP2, and BMP4 were used only for the experiment in FIG. 57C. The GAPDH gene and the PPIA gene were measured as housekeeping genes, and relative values with the average of Ct values of the two genes as a control value are shown in the graph. Although not shown in the graph, ERCC-00096, ERCC-00009, ERCC-00022, and ERCC-00025 were used in combination as a control, except for the experiment of FIG. 31C.

**[Table 7]**

| Name of | | Base sequence (5' → 3') | SEQ. |
|---|---|---|---|
| PAX3 | Forward | TGGCAATGGCCTCTCACCT | 45 |
| | Reverse | GAGAGCGCGTAATCAGTCTGG | 46 |
| OSR1 | Forward | GCTAAAGCCCCAGAGACGTG | 47 |
| | Reverse | CGCCGGTAAGGTTTTGCTG | 48 |
| FOXF1 #1 | Forward | GATTCAGGATTGCGGGGACG | 49 |
| | Reverse | GCGGTGTTTTGGTTATTAGGGAAAG | 50 |
| PAX2 #3 | Forward | TTGAGCGTCCTTCCTACCCT | 51 |
| | Reverse | GGAGTACTCGTTCCCCTGTT | 52 |
| EMX2 #1 | Forward | GCAGTTGGCACATAGCCTC | 53 |
| | Reverse | TTGTTGCGAATCTGAGCCTTC | 54 |
| EMX2 | Forward | TGGCCGAGTTCAACCATTCA | 55 |
| | Reverse | AGTGCTTGAAAACTTGCCGC | 56 |
| WT1 #2 | Forward | GCGCCCAGTACAGAATACAC | 57 |
| | Reverse | AAGAGTCGGGGCTACTCCA | 58 |
| WT1 | Forward | TAAAAGAAAATAGGGGCTGGTCC | 59 |
| | Reverse | CCTCACCCAGGAAGTCTCATT | 60 |
| GATA4 | Forward | TGGCTATAGCAGAGAATACCTTTGAACCA | 61 |
| | Reverse | ACAGGTTTGTGGGTTAGGGAGGGT | 62 |
| LHX9 #2 | Forward | CGCTGCAGTTGTTTCCCATTA | 63 |
| | Reverse | GTGGAGGAGAAAAGAGGCGT | 64 |
| HOXD4 | Forward | CACCCCCAGCTTCATACCAA | 65 |
| | Reverse | TGGGGCTATGTTTCAGAAGGAG | 66 |
| HOXD8 | Forward | TGAACCGCCCCTGTAATCG | 67 |
| | Reverse | GCGAACAAAACGAAGGCAATAC | 68 |
| HOXD9 #3 | Forward | TAGTTTAGTCCCCTCACCCCT | 69 |
| | Reverse | TCCGCACTGTTGTCGCTC | 70 |
| HOXD10 | Forward | ACTGTAGAGCATCCATCCATCC | 71 |
| | Reverse | TTCGTGGACTAACCACACACA | 72 |
| HOXD11 | Forward | TCACCCCTTGGTCTCTTTGC | 73 |
| | Reverse | ACTCGCGTTCCAGTTCGC | 74 |
| MEOX1 | Forward | ATGTAACCCCACCTCTCCAC | 75 |
| | Reverse | ATGCTCTAACCAGCCATCTCA | 76 |

**[Table 8]**

| Name of | | Base sequence (5' → 3') | SEQ. |
|---|---|---|---|
| NR5A1 | Forward | CTCTGCCCCCTGAAATGTGT | 77 |
| | Reverse | GGGGCTGGGGTGATTCTTAA | 78 |
| LHX1 | Forward | ACAGATTTGCAGGGCTTTTGG | 79 |
| | Reverse | GGCAACTGTCTGAATAGCATGTTT | 80 |
| HAND1 | Forward | AATGATCCGCCTTGACACCC | 81 |
| | Reverse | TCACCTTCCTGCATCTGGTTC | 82 |
| AMHR2 | Forward | GCCTGCCACTTCAATGTTCAG | 83 |
| | Reverse | TAGGAGAAAGGGTACAGGCAGG | 84 |
| LGR4 | Forward | TCATGTAGTTTGTATAAAATGTGGGAAG | 85 |
| | Reverse | AAAACTTGTAAATAGTTGGCCTTACAAAA | 86 |
| WNT4 | Forward | TCTGGATGCTGCTAACCCAC | 87 |
| | Reverse | GTCACAATGGCAAAGAGCCT | 88 |
| WNT6 | Forward | GGCCTCTAGGAGGAAACCAAC | 89 |
| | Reverse | ACTAACCTCACCCACCATCC | 90 |
| RSPO1 | Forward | GGCATGCATCGAGGAATCGT | 91 |
| | Reverse | TGCACAGATGCCAGTGGTC | 92 |
| FOXL2 #2 | Forward | CGACCTAGGTGGTCTTGTGG | 93 |
| | Reverse | TTGGTCCCCCAAACAAGTTACA | 94 |
| KITL | Forward | GATTATAGTGTCCACTTGCCACC | 95 |
| | Reverse | AAGGGTATATCTGTGCATCCATCT | 96 |
| PODXL | Forward | CTGATCCCTCAGGTCTGCTG | 97 |
| | Reverse | ACTTCCTGGGCAAACTGTTGA | 98 |
| LGR5 | Forward | GGTTCAGTAACATTAAGGACCATGA | 99 |
| | Reverse | AAAGAAAATGGACAGAGAAATGCAA | 100 |
| ALDHA1 | Forward | AGAGTAAACTGCTCTGTTCCCA | 101 |
| | Reverse | TTTGACAAGCAGACTGACATCC | 102 |
| NR2F2 | Forward | AGCCAAGGAATGTGTCCAAGAC | 103 |
| | Reverse | AATTCAAGAACTAAGCGGGACC | 104 |
| WNT5A | Forward | AGATGCAATTTTCCTCCATTCCT | 105 |
| | Reverse | TGTATCAGTTTGAACCTAGAAGATTGA | 106 |
| GLI1 | Forward | GAGATCCTTGCCTGGGGAAA | 107 |
| | Reverse | TGTGACGGATGAGATTCCCT | 108 |

**[Table 9]**

| Name of | | Base sequence (5' → 3') | SEQ. |
|---|---|---|---|
| ARX v1 | Forward | CCGAAACCTTCAAGATAAAGGCA | 109 |
| | Reverse | TTTGAGCGTGACACTTCTCC | 110 |
| COL1A1 | Forward | GGATGGGGACTTGTGAATTTTTC | 111 |
| | Reverse | GGTGGAAAGTGAGCAACGG | 112 |
| PDGFRA | Forward | GCTGGCCTGAGAAACACAATTT | 113 |
| | Reverse | ACGTCCCTCTTCACAAAAATAGGA | 114 |
| TCF21 | Forward | ACAACGACGAGGAGATTCGT | 115 |
| | Reverse | GCTTGCAGAGTCATTCACGG | 116 |
| STAR | Forward | GCTTTACGGCTCAAGAATGCC | 117 |
| | Reverse | TGCTGTGCAGTGAATGGGAG | 118 |
| CYP11A1 | Forward | ACCCAGGAGTGATCGGAGAC | 119 |
| | Reverse | GGACAGAGGACTGAAGCTGTA | 120 |
| HSD3B2 | Forward | TCATACGCCAGAGGACAGAC | 121 |
| | Reverse | CACAGTCATTCAGAATCAGCCAC | 122 |
| CYP17A1 | Forward | CAGGCTGAGGGTAGCACCTA | 123 |
| | Reverse | GTCGGGGGTTGCATCTCTAA | 124 |
| CYP19A1 | Forward | CGTGGTCAGAGTAGAACCCC | 125 |
| | Reverse | TCCACGACCACATACAATGATTC | 126 |
| OSR1 #10 | Forward | GGAGCCGGGCGTTATATTG | 127 |
| | Reverse | AACTCCAGTGATAAATGTCCGTTAC | 128 |
| CXCL12 x4 | Forward | GTGGAGCCATATGAATGTCAGTAG | 129 |
| | Reverse | TGTGGCTAACATTGAAAAGCTGC | 130 |
| BMP2 | Forward | CCCACTTTTCTAACTTTTCGCCC | 131 |
| | Reverse | AAAAGCAAGTTGATGGGTCAGC | 132 |
| BMP4 | Forward | ATTCCAGTATCCCCAAAGCCTG | 133 |
| | Reverse | GATCTCAGCGACACCCACATC | 134 |

**[Table 10]**

| Name of gene | | Base sequence (5' → 3') | SEQ. NO. |
|---|---|---|---|
| GAPDH | Forward | AACAGGGTGGTGGACCTCAT | 135 |
| | Reverse | TTCCTCTTGTGCTCTCGCTG | 136 |
| PPIA | Forward | ACAGGTCCTGGCATCTTGTC | 137 |
| | Reverse | CTCAGTCTTGGCAGTGCAGA | 138 |
| ERCC-00096 | Forward | GATCCCGGAAGATACGCTCTAAG | 139 |
| | Reverse | CGCAGGTTGATGCTTCCAATAAA | 140 |
| ERCC-00009 | Forward | AATTACAGTTAGGCCGCTGACA | 141 |
| | Reverse | ATAAGCGCTGTCACGGATGTAT | 142 |
| ERCC-00022 | Forward | ATCACTTCTGGCCGTCCTTATG | 143 |
| | Reverse | TTCCCCAATGACCTTAACTCCG | 144 |
| ERCC-00025 | Forward | CTACCCAGATAACATCGCTCCC | 145 |
| | Reverse | TATCAGTACTTCCAAGCAGCCC | 146 |

**[Table 11]**

| Composition | | |
|---|---|---|
| 2x powersybr | 5.0 µL | |
| Primer mix (5 µM) | 0.4 µL | |
| 1/20 or 1/40 cDNA | 4.0 µL | |
| water | 0.6 µL | |
| Total | 10.0 µL | |

| PCR conditions | | |
|---|---|---|
| 95°C | 10 min | |
| 95°C | 15 sec | 40 cycles |
| 60°C | 1 min | |

### 4. RNA-seq

After determining the base sequences of all transcripts by a next-generation sequencer (NGS) (NextSeq 550 manufactured by Illumina, Inc.), the expression level of a specific gene was calculated.

### [Example 2]

### 1. Examination of ROCK Inhibitor in Step 1

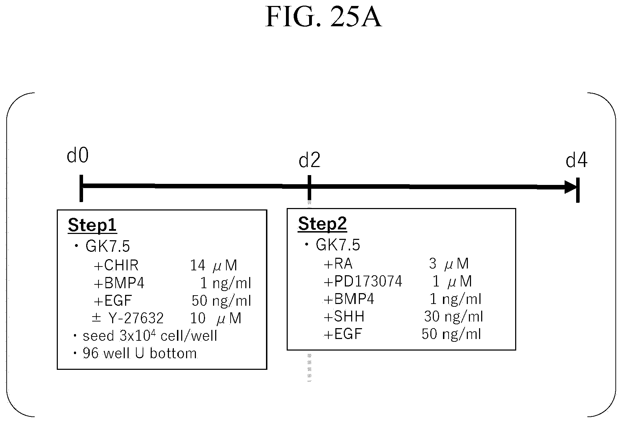

At the time of induction, the ES cells were cultured on mouse embryonic fibroblasts (MEF), which are feeder cells, treated with a proteolytic enzyme solution (CTK: collagenase, trypsin, and KSR), and then dissociated up to a single-cell level using TrypLEselect. Next, each of 30,000 cells was seeded in a low-adhesive 96-well U-bottom culture plate (hereinafter, step 1 was started by the same method unless otherwise specified). A TGOT No. 25 strain was used as a cell strain. The presence or absence of the ROCK inhibitor (Y-27632) in step 1 was examined. The detailed protocol is shown in FIG. 25A. In FIG. 25A, the base medium GK7.5 has a composition of GMEM, 7.5 v/v% KSR, 0.1 mM 2-ME, 0.1 mM non-essential amino acid (NEAA), 1 mM sodium pyruvate, 100 U/mL penicillin/streptomycin, and 2 mM L-glutamine. The induction was performed for a period in step 1 of 2 days and in step 2 of 2 days. In step 2, PD173074 was used instead of PD0325901 used in mice. PD173074 is an inhibitor of FGF, whereas PD0325901 is an MEK inhibitor downstream of the FGF signaling pathway.

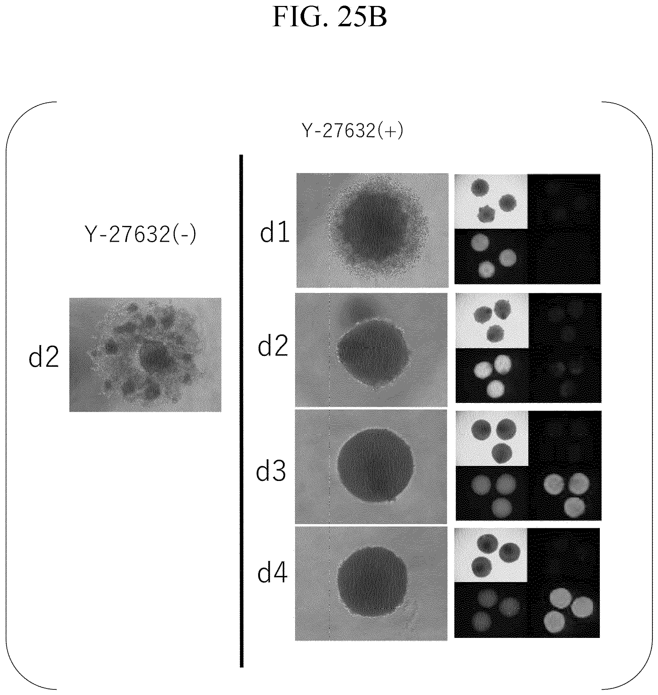

Induction was performed without using Y-27632 in step 1 as in the case of the mice, but the efficiency of forming cell aggregates (embryoid bodies) was significantly low even on day 2 of induction (see the photograph on the left side of FIG. 25B). Therefore, Y-27632 was thereafter added thereto to carry out an experiment. (See the photograph on the right side of FIG. 25B). Furthermore, Y-27632 was removed on day 1 unless otherwise specified.

As shown in the fluorescence photograph on the right side of FIG. 25B, it was confirmed that EGFP was expressed in step 1 (d1 and d2), tdTomato was expressed in step 2 (d3 and d4), and TBXT and OSR1 were expressed.

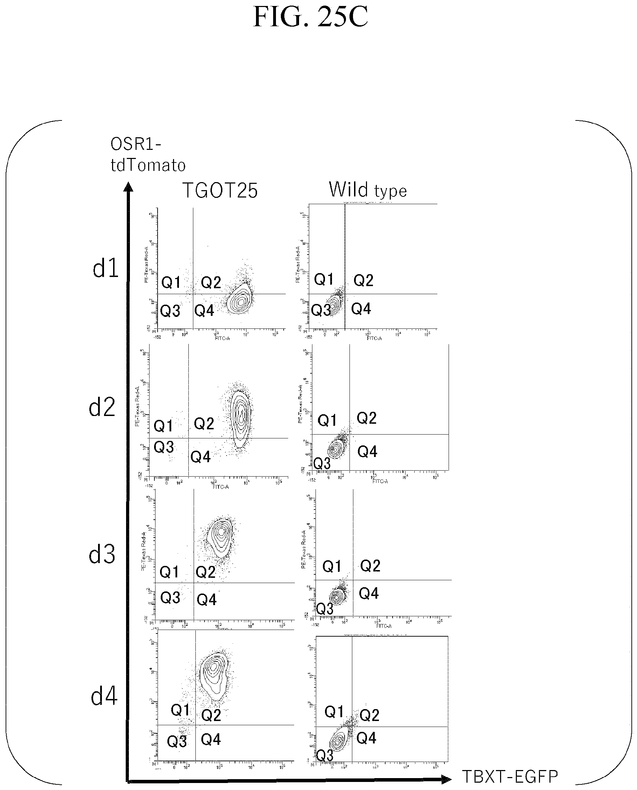

FIG. 25C is the result of FACS analysis, and it was found that these genes were clearly expressed even as compared with the Wild type analyzed as a control.

### 2. Optimization 1 of Concentration of CHIR99021 and Concentration of BMP4 in Step 1

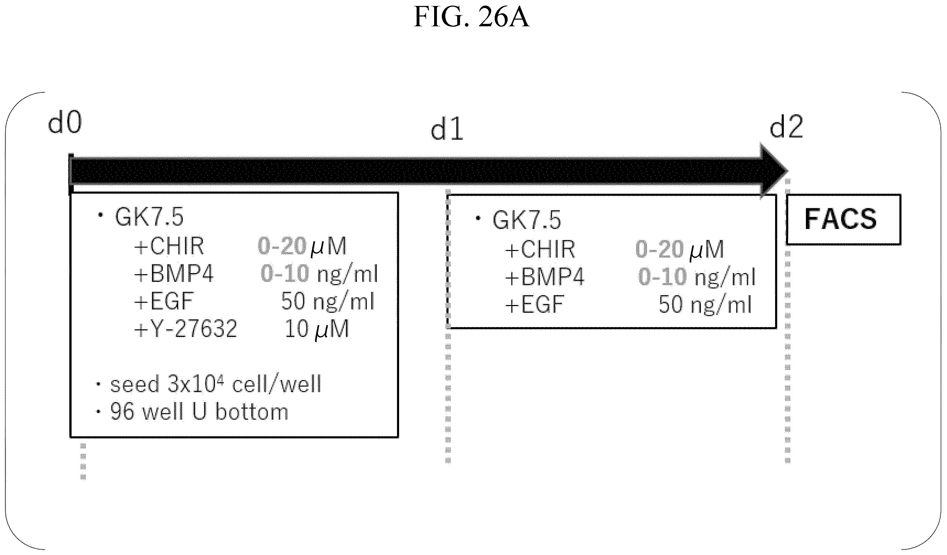

Subsequently, the period in step 1 was fixed for 2 days, and the concentration of CHIR99021 (hereinafter abbreviated as "CHIR") and the concentration of BMP4 required for TBXT expression and OSR1 expression were examined (see FIG. 26A). The conditions were changed to a concentration of CHIR of 0, 1, 3, 8, 14, and 20 µM, and a concentration of BMP4 of 0, 1, 3, and 10 ng/mL to perform the evaluation by FACS analysis on day 2 of induction. A TGOT No. 25 strain was used as a cell strain.

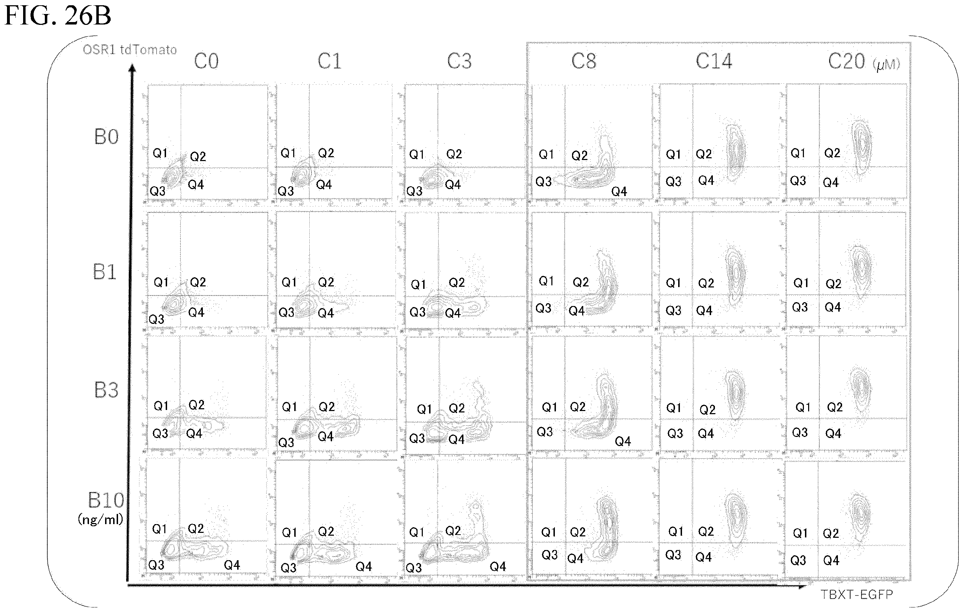

As a result, it was considered that TBXT can be expressed with high efficiency in a case where the concentration of CHIR99021 is 8 µM or more. (See FIG. 26B).

### 3. Optimization 2 of Concentration of CHIR99021 and Concentration of BMP4 in Step 1

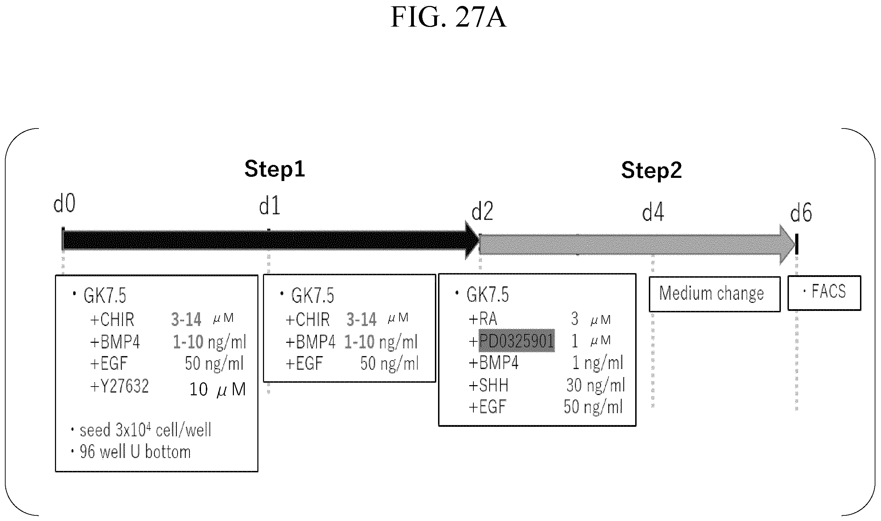

Based on the results in "2.", the conditions were changed to a concentration of CHIR99021 of 3, 8, and 14 µM, and a concentration of BMP4 of 1, 3, and 10 ng/mL to perform the induction for a period in step 1 of 2 days and in step 2 of 4 days (see FIG. 27A). Furthermore, in the subsequent experiments, PD0325901 was used in step 2 in the same manner as the mice. A TGOT No. 25 strain was used as a cell strain.

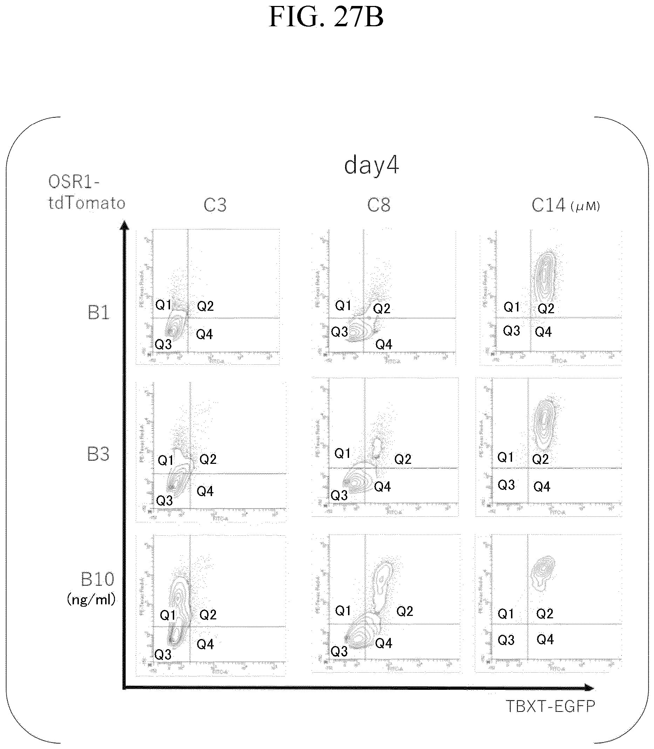

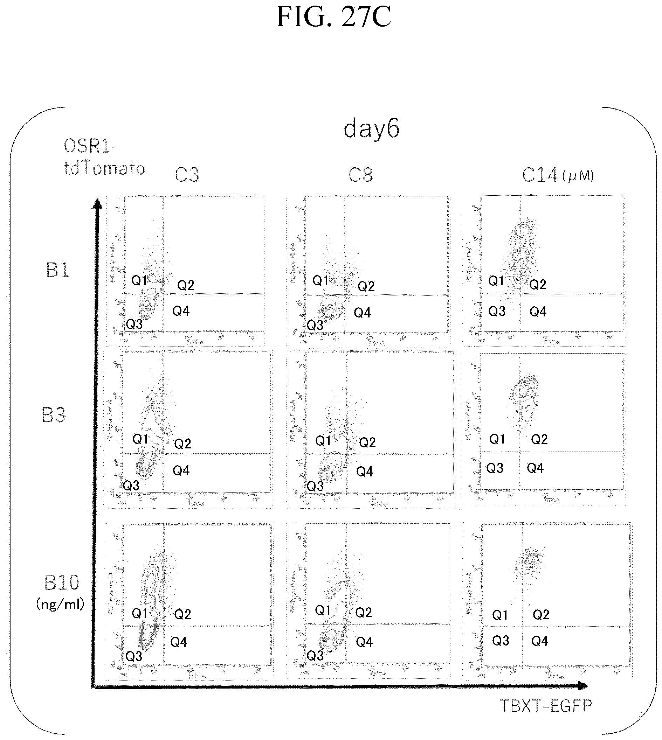

From the results in "2.", it was considered that TBXT can be expressed with high efficiency in a case where the concentration of CHIR99021 was 8 µM or more, but at a transition to step 2, OSR1 can be expressed with high efficiency by setting the concentration of CHIR99021 to 14 µM or more (see FIG. 27B and FIG. 27C). In addition, it was confirmed that the expression of OSR1 was enhanced by increasing the amount of BMP and the concentration of CHIR99021 was 14 µM or more (see FIG. 27B and FIG. 27C).

### 4. Optimization 3 of Concentration of CHIR99021 and Concentration of BMP4 in Step 1

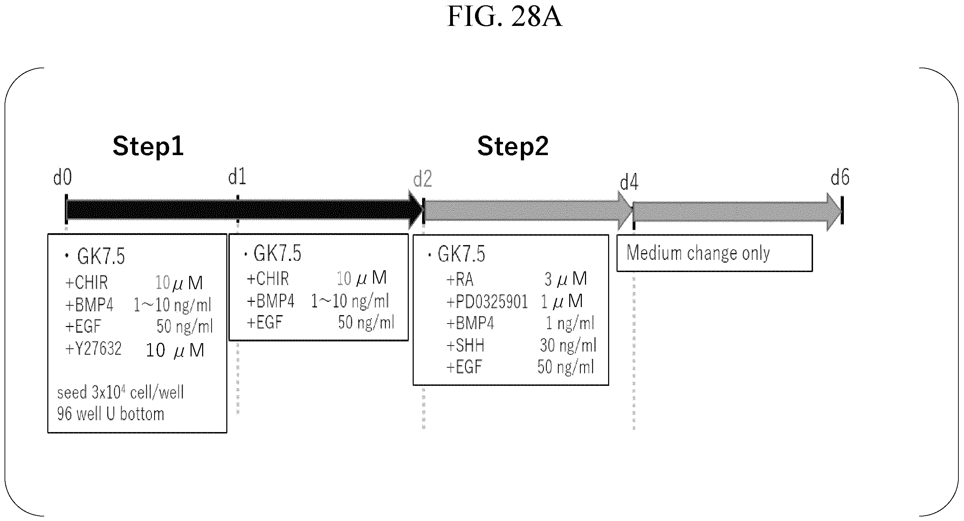

Based on the results in "3.", for the purpose of examining the lower limit of the optimum CHIR for the expression of OSR1, the conditions were changed to a concentration of CHIR fixed at 10 µM, and a concentration of BMP4 of 1, 3, and 10 ng/mL to perform the induction for a period in step 1 of 2 days and in step 2 of 4 days (see FIG. 28A). As a positive control, the conditions were also set where the concentration of BMP4 was 1 ng/mL and the concentration of CHIR was 14 µM. FACS analysis was performed on days 2, 4, and 6 of induction. A TGOT No. 25 strain was used as a cell strain.

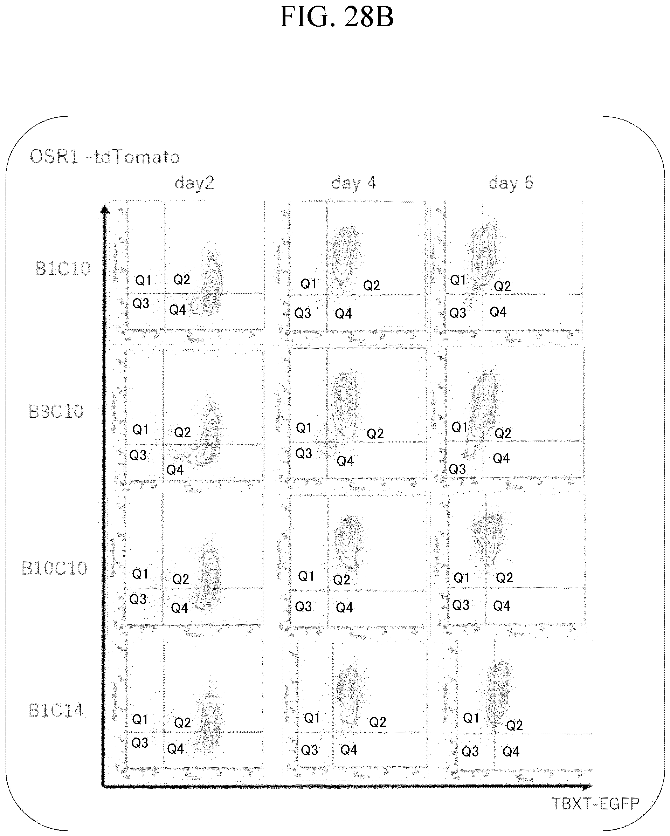

The expression of OSR1 was confirmed at a concentration of CHIR of 10 µM, regardless of the concentration of BMP4 (see FIG. 28B). In addition, the increase in the expression of OSR1 by the amount of BMP4 added was recognized even at a concentration of CHIR of 10 µM (see FIG. 28B).

From the above, it was considered that the optimum concentration of CHIR for induction of the intermediate mesoderm is 10 µM or more.

### 5. Examination 1 of Addition of bFGF and Activin A in Step 1

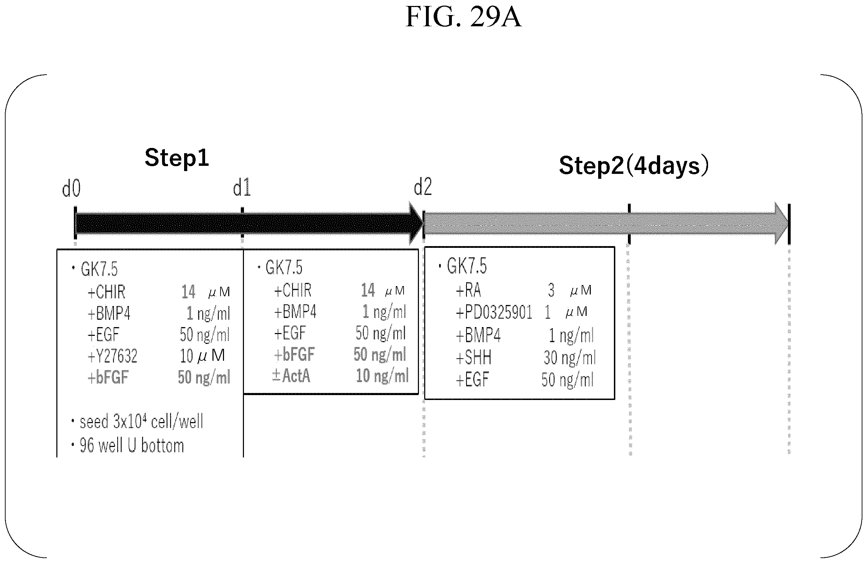

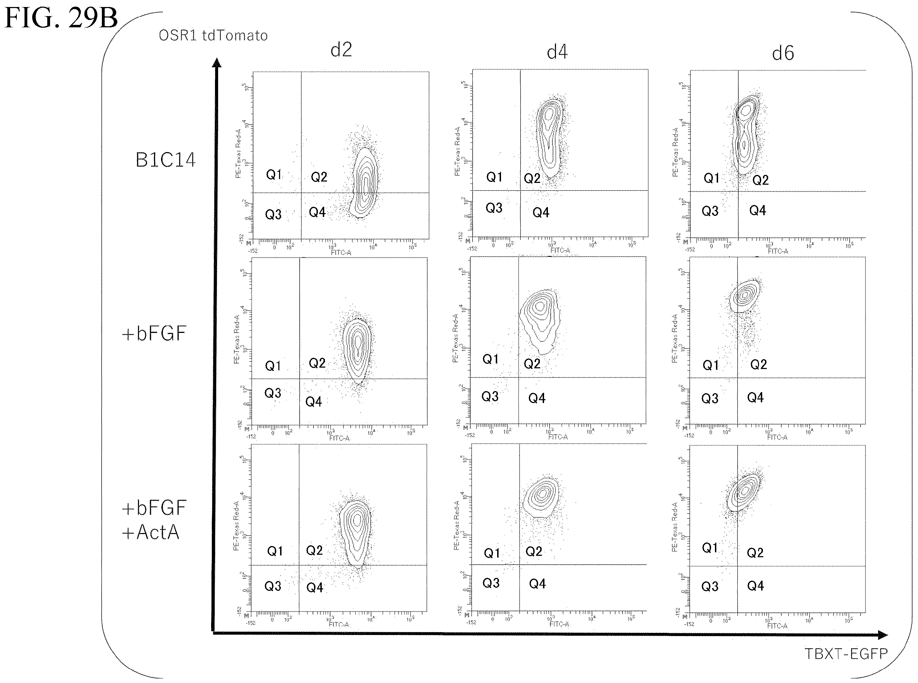

In the intermediate mesoderm induction of the previous research, there was a report that the intermediate mesoderm was induced by adding bFGF or Activin A, and the addition of these in step 1 was examined. The experiment was conducted under the conditions of addition of bFGF at 50 ng/mL through the entire period of step 1, addition of Activin A at 10 ng/mL only for one day from day 1 of induction, and no addition. A TGOT No. 25 strain was used as a cell strain. The results of FACS analysis on days 2, 4, and 6 of induction are shown in FIG. 29B.

As shown in FIG. 29B, the addition of any of bFGF and Activin A acted toward enhancing the expression of OSR1, and was considered to be useful for induction of the intermediate mesoderm.

### 6. Examination of Addition of BMS493 and Y-27632 in Step 1

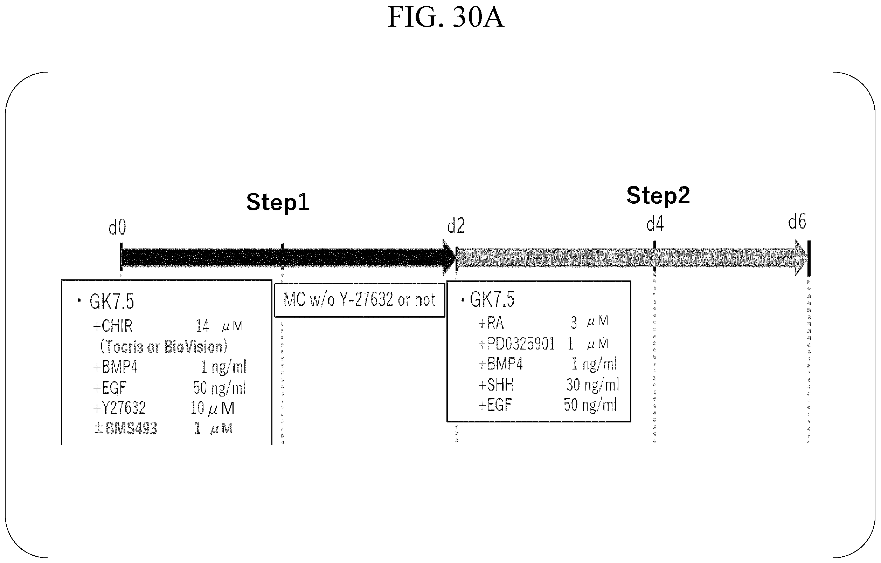

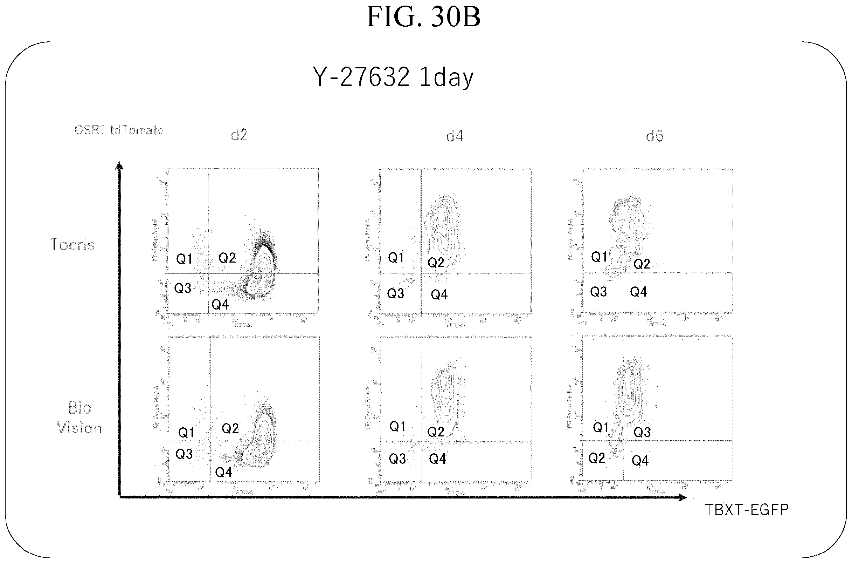

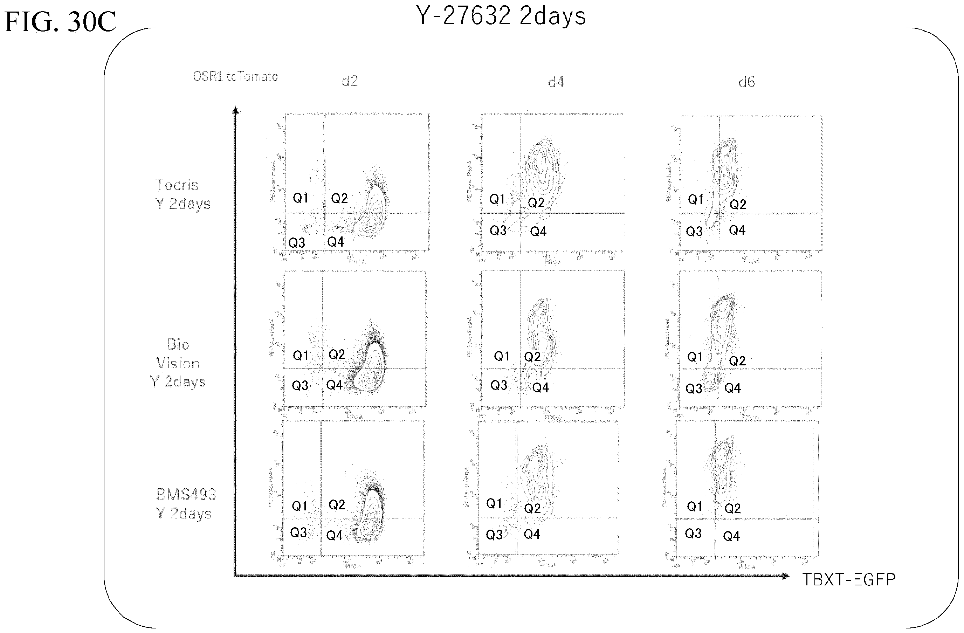

RA provides information on the anteriorization of the embryo and is responsible for regulation of the anterior-posterior direction of the embryo. Accordingly, it was examined whether a change was obtained by using BMS493, which is an inhibitor of RA, at 1 µM in step 1 for posteriorization of the embryo (see FIG. 30A). Furthermore, the effect of adding Y-27632 continuously for only 1 day or for 2 days was examined. A TGOT No. 25 strain was used as a cell strain. CHIR from two manufacturers (manufactured by Tocris, Inc. and BioVision, Inc.) was also tested. The results of FACS analysis on day 2, day 4, and day 6 of induction are shown in FIG. 30B and FIG. 30C.

As shown in FIG. 30B and FIG. 30C, since there was no significant difference in expression of marker genes for CHIR depending on the manufacturer, those from Tocris, Inc., were used as before. In regard to BMP493, the enhancement of OSR1 expression in step 2 was observed.

### 7. Examination of Addition of LDN in Step 1

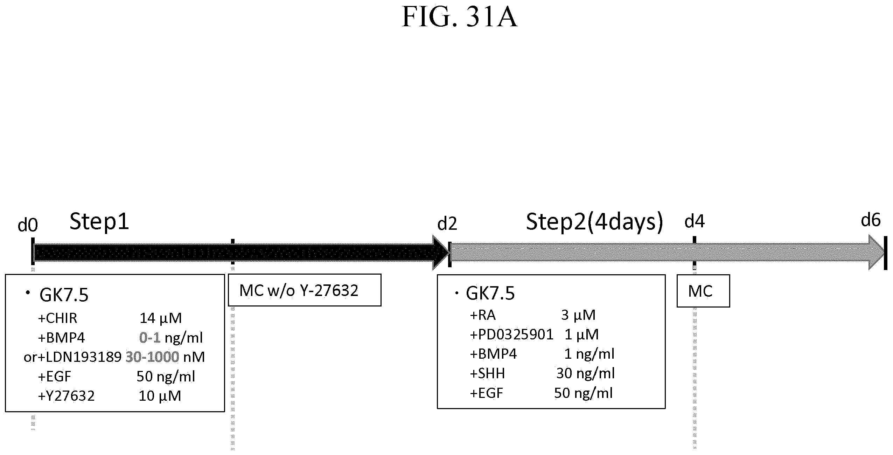

Since it is known that a BMP concentration defines a mesoderm fraction, OSR1 expression and FOXF1 expression were evaluated by using LDN193189, which is a BMP inhibitor, in step 1 (see FIG. 31A). Furthermore, FOXF1, which is a marker of the lateral plate mesoderm, was evaluated by qPCR. The concentration of BMP4 was changed to 1 ng/mL and 0 ng/mL, and the concentration of LDN 193189 was changed to 30 nM, 100 nM, and 1,000 nM. A TGOT No. 25 strain was used as a cell strain.

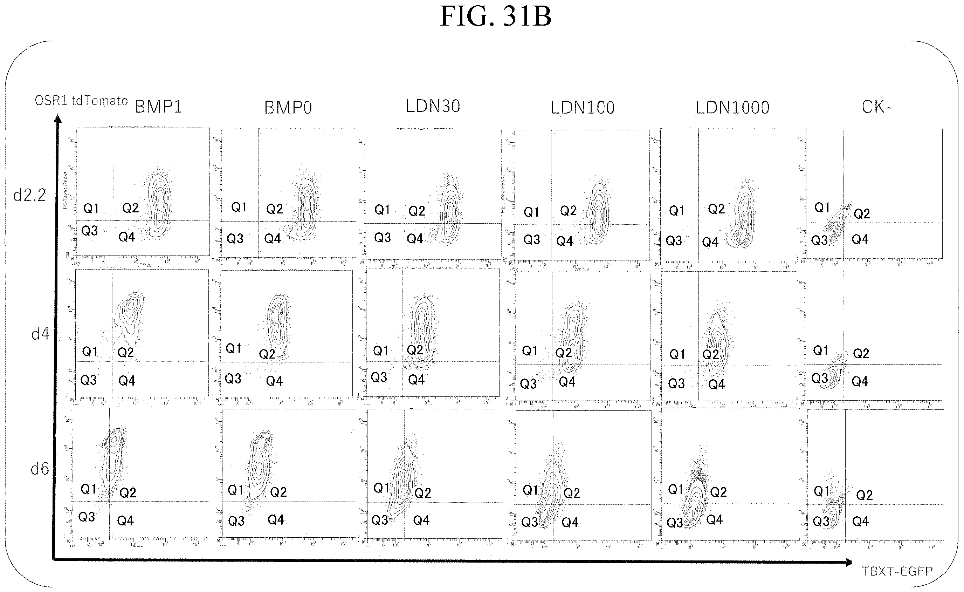

It was confirmed that in a case where the concentration of LDN was increased, the OSR1 expression at the end point of step 1 (day 2.2) decreased, and even in a case where the transition to step 2 (days 4 and 6) occurred, the increase in OSR1 expression became weaker (see FIG. 31B).

As expected, it was confirmed by qPCR that the expression of FOXF1, which is a marker of the lateral plate mesoderm, also almost disappeared without BMP4 and with the addition of LDN (see FIG. 31C). In addition, HOXD9 expressed in the gonad was expressed (see FIG. 31C) and the degree of posteriorization of the embryo was considered to be good.

From the results of FACS, it was shown that although almost all cells were OSR1-positive in a case where the concentration of BMP4 was 0 ng/mL or more (see FIG. 31B), the expression of PAX3, which is a marker of the paraxial mesoderm, was unexpectedly recognized (see FIG. 31C).

### 8. Induction of NR5A1-Positive Cells

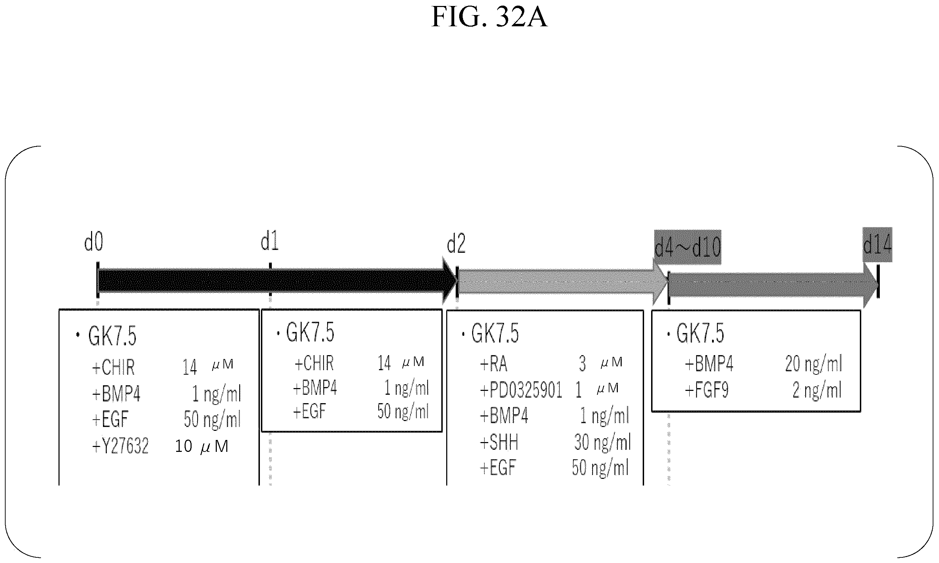

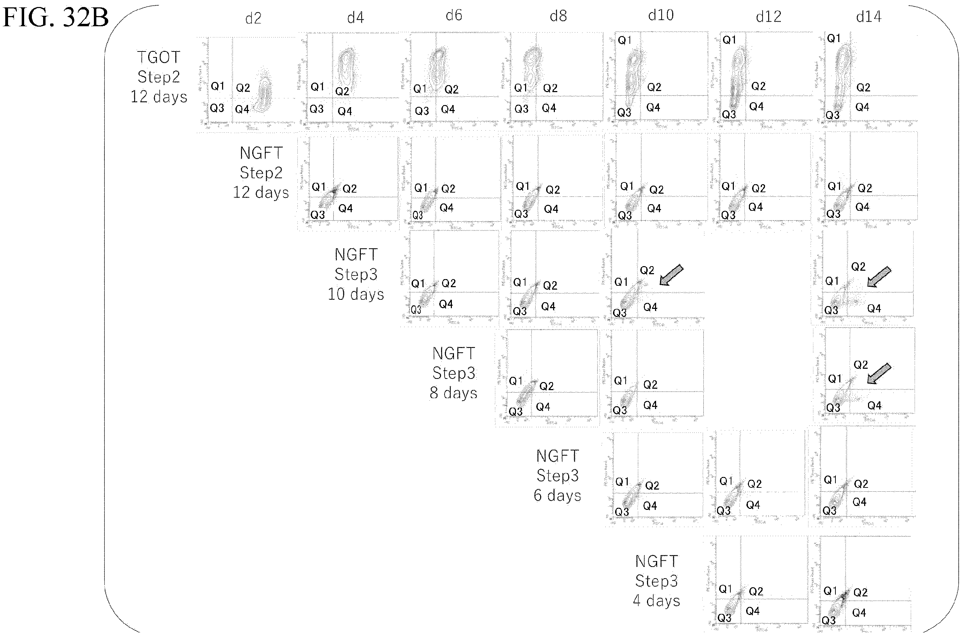

TGOT No. 25 strains and NGFT No. 69 strains (NR5A1-EGFP/FOXL2-tdTomato double reporter strains) were used as a cell strain to perform the induction for 14 days (see FIG. 32A). The period of step 1 was set to 2 days, the period of step 2 was set to 2 to 8 days, and the period of step 3 was set to the remaining period. Furthermore, in the case of TGOT No. 25 strains, step 2 was continued for 12 days. The results of FACS analysis are shown in FIG. 32B. In FIG. 32B, the results of flow cytometry for TGOT No. 25 strains every 2 days are shown in the uppermost row. In the FACS plot in FIG. 32B, the horizontal axis indicates the expression of EGFP (TBXT or NR5A1 (SF1)) and the vertical axis indicates the expression of tdTomato (OSR1 or FOXL2). The following in the second row show the results obtained in the NGFT No. 69 strain, and in the second row, the NGFT No. 69 strain was not transitioned to step 3 and step 2 was performed for 12 days.

As shown in FIG. 32B, the expression of NR5A1 was not recognized under the condition in which step 2 was performed for 12 days without transition to step 3 with the NGFT No. 69 strain in the second row. On the other hand, in a case where the period of step 3 was set to 10 days, cells (arrows) that expressed NR5A1 started to appear on day 10 of the induction and became clearly recognized on day 14. In a case where the period of step 2 was 6 days or longer (the period of step 3 was 6 days or less), NR5A1-positive cells were no longer found.

### 9. Induction of FOXL2-Positive Cells

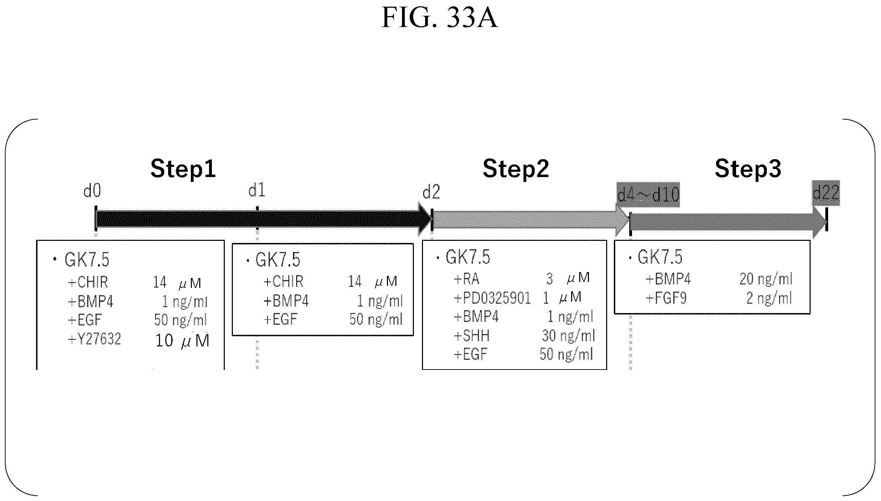

Since the expression of FOXL2 could not be confirmed from the results of "8.", the induction period was extended up to day 22 (see FIG. 33A). The period of step 1 was set to 2 days, the period of step 2 was set to 2 to 8 days, and the period of step 3 was set to the remaining period. An NGFT No. 69 strain was used as a cell strain.

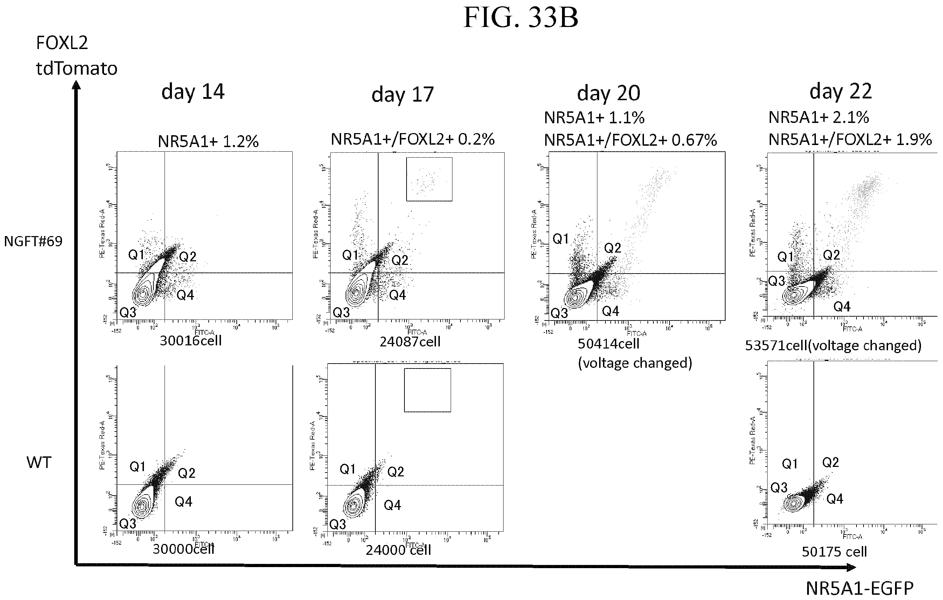

FACS analysis was performed on days 17, 20, and 22 under the condition in which the period of step 2 was 2 days, and thus, cells expressing FOXL2 were clearly recognized on day 17, indicating that the induction of somatic cells of the ovary was successful (see FIG. 33B).

### 10. Optimization 4 of Concentration of CHIR99021 and Concentration of BMP4 in Step 1

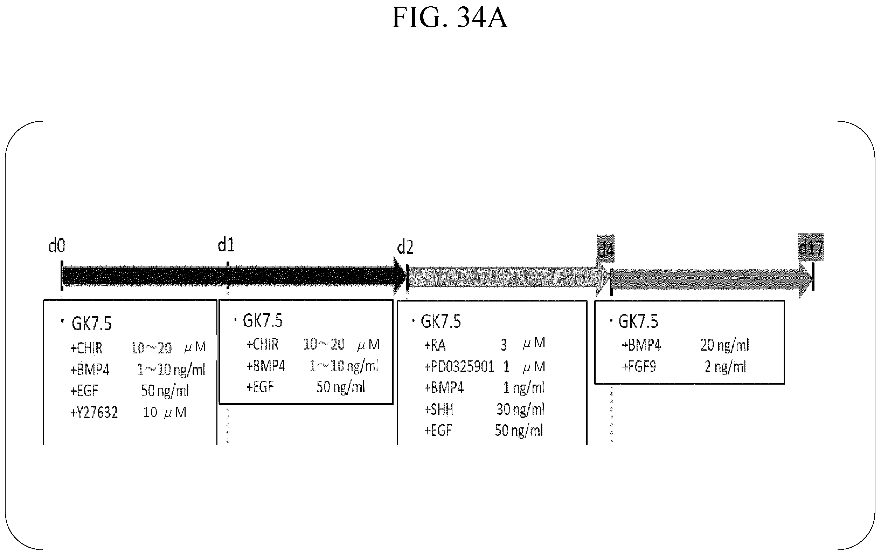

In order to evaluate the effect of the concentration of CHIR and the concentration of BMP4 in step 1 on the expression of NR5A1 and FOXL2, the periods of step 1 and step 2 were fixed to 2 days, the concentration of CHIR was set to 10, 14, and 20 µM, and the concentration of BMP4 was set to 1, 3, and 10 ng/mL to perform the culture until day 17 (see FIG. 34A). An NGFT No. 69 strain was used as a cell strain.

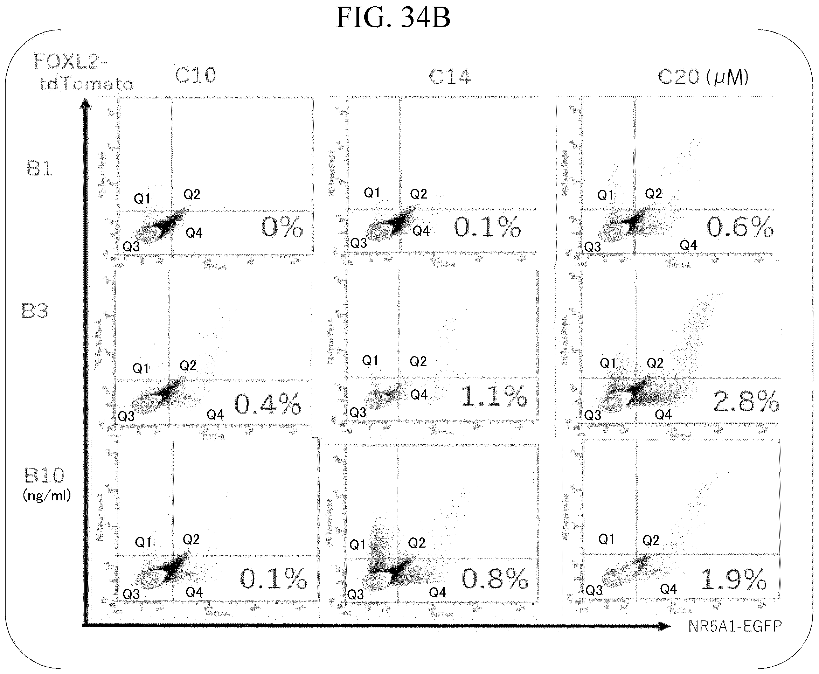

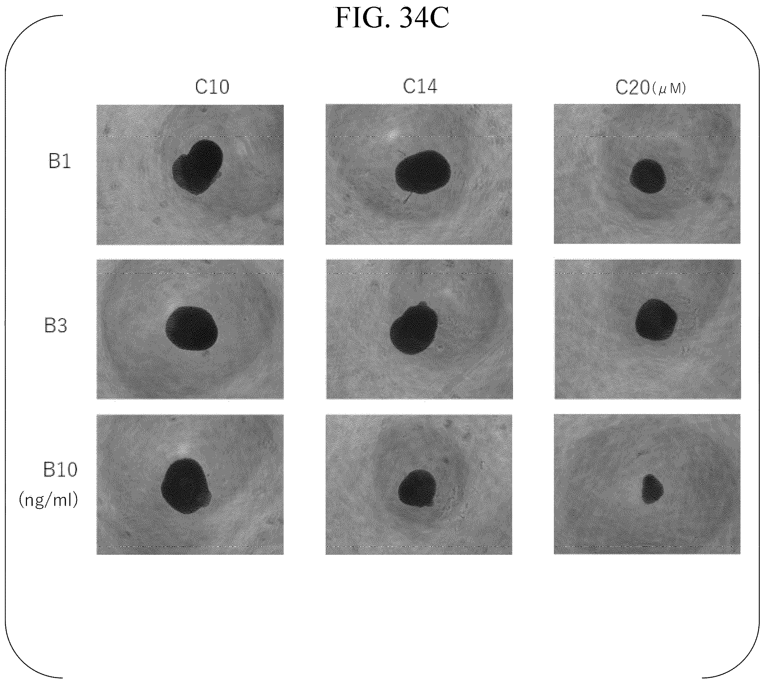

While a number of NR5A1-positive cells were observed under the condition where the concentration of CHIR was 20 µM, 20 µM was a considerably high concentration and the embryoid bodies tended to be significantly small. Thus, there was also a concern about cytotoxicity (see FIGS. 34B and 34C). Therefore, hereinafter, the condition in which the concentration of CHIR was 14 µM was examined. In addition, the same tendency was recognized in a case where the amount of BMP4 was excessively increased (see FIGS. 34B and 34C).

### 11. Optimization of Concentration of BMP4 and Concentration of LDN in Step 1

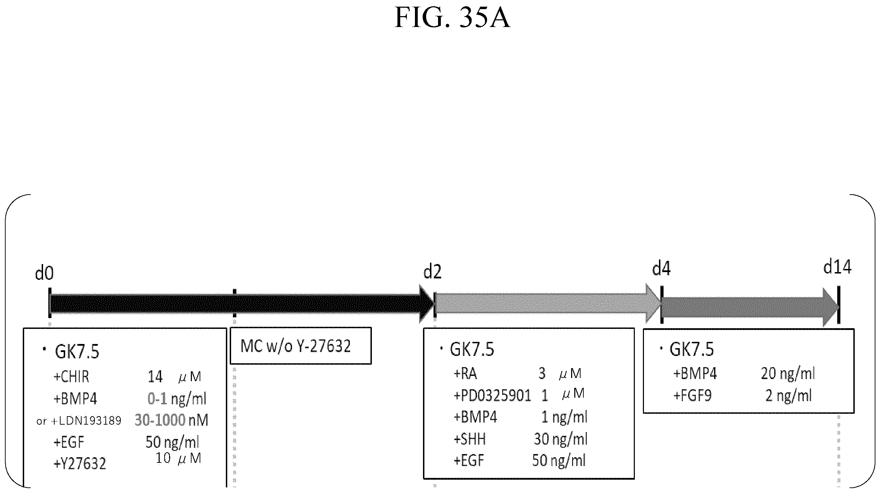

Since it is known that the expression levels of OSR1 and FOXF1 vary depending on the concentration of BMP, it was examined which condition contribute most to the appearance of NR5A1-positive cells in a case where LDN193189, which is a BMP inhibitor, was used in step 1 (see FIG. 35A). The concentrations of BMP were 1 ng/mL and 0 ng/mL, and the concentrations of LDN193189 were 30, 100, and 1,000 nM. An NGFT No. 69 strain was used as a cell strain.

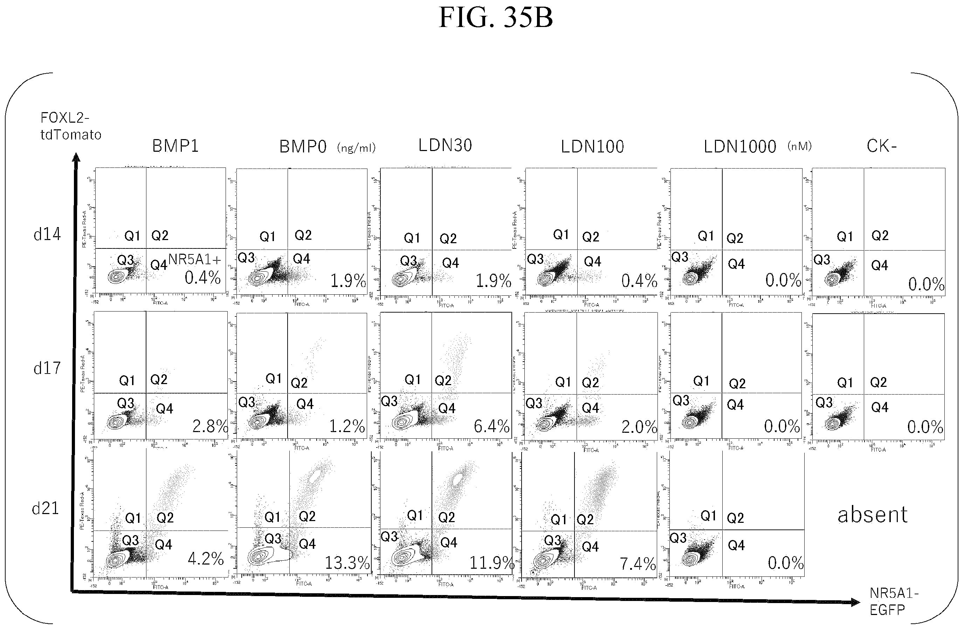

Although FACS analysis was performed on days 14, 17, and 21 of induction, almost no improvement in the proportion of NRSA1-positive cells by the addition of LDN was observed (see FIG. 35B).

### 12. Examination of Addition of BMP4, LDN, and BMS493 in Step 1

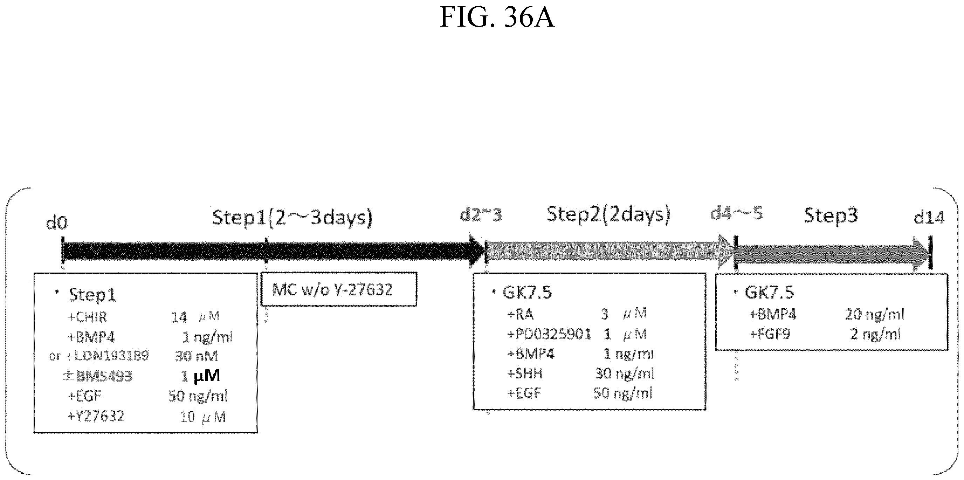

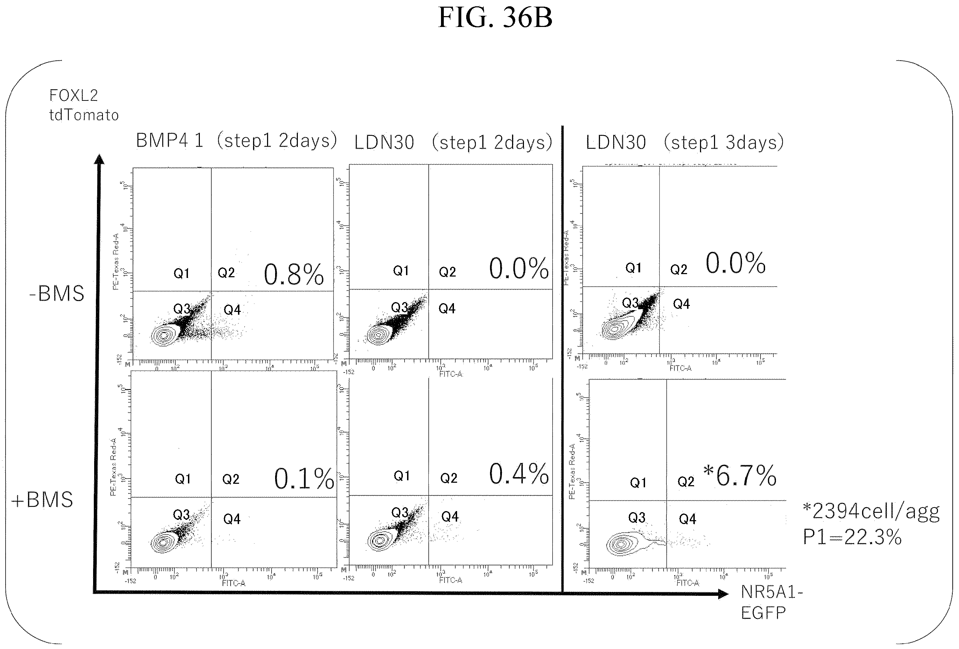

It was examined whether the addition of LDN and BMS493 in step 1 contributes to the induction of NR5A1-positive cells (see FIG. 36A). The period of step 1 was 2 days or 3 days (for a period of step 1 of 3 days, only LDN concentration of 30 nM), and under 6 conditions in which the concentration of BMP4 was 1 ng/mL and 0 ng/mL, BMS493 was added (1 µM), or no BMS493 was added, step 2 was fixed for 2 days, and FACS analysis was performed on day 14 of induction (see FIG. 36B). An NGFT No. 69 strain was used as a cell strain.

As shown in FIG. 36B, since NR5A1-positive cells were hardly induced, there was no particular advantage from the addition of LDN or BMS.

### 13. Examination 2 of Addition of bFGF and Activin A in Step 1

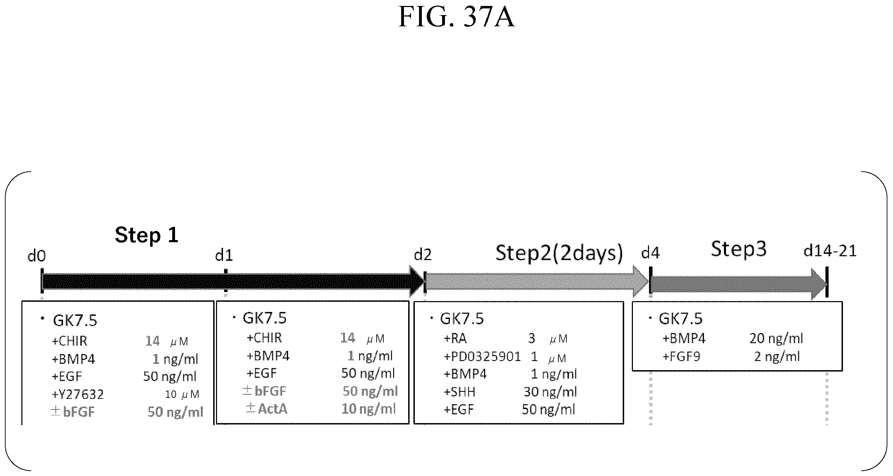

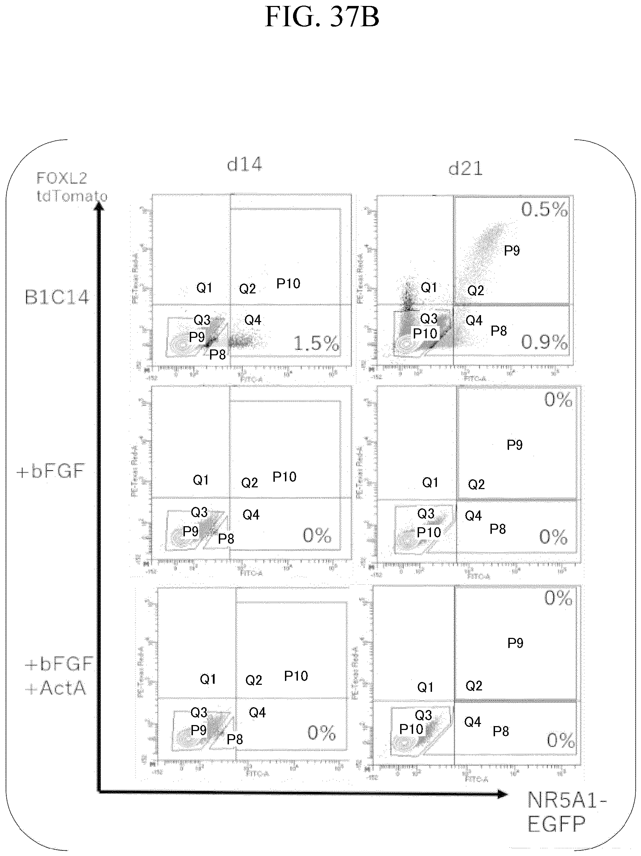

It was examined whether the induction of NR5A1-positive cells was improved by the addition of 50 ng/mL of bFGF and 10 ng/mL of Activin A (only from day 1) in step 1 (see FIG. 37A). The periods of step 1 and step 2 were both set to 2 days, and FACS analysis was performed on day 14 and day 21 of induction (refer to FIG. 37B). An NGFT No. 69 strain was used as a cell strain.

As shown in FIG. 36B, the improvement of the induction of NR5A1-positive cells was not recognized by the addition of bFGF and Activin A.

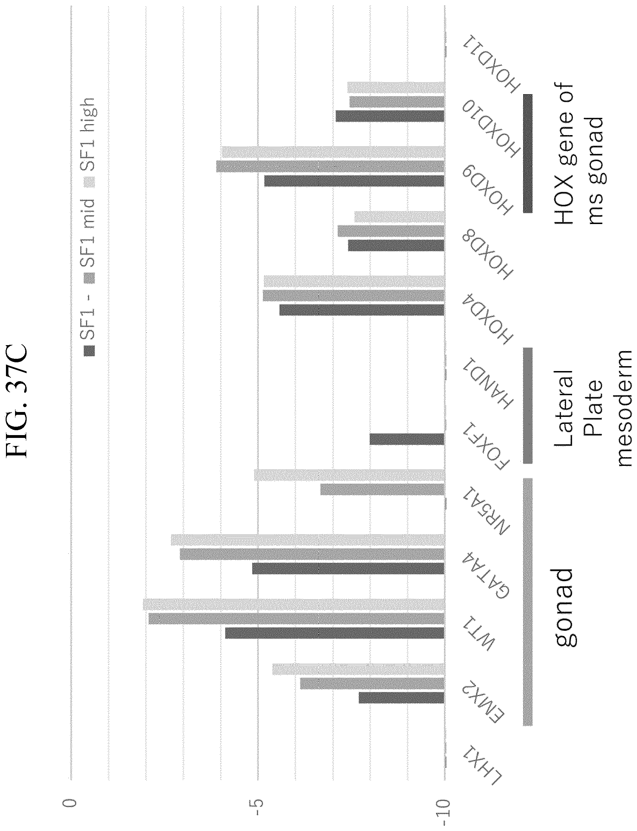

Moreover, in the FACS analysis, NR5A1-weakly positive cells and strong positive cells on day 14 of induction were sorted and qPCR was performed. The expression of WT1, GATA4, and NR5A1, which are markers of the gonad, and the expression of HOXD9 and HOXD10 expressed in the mouse ovary (see FIG. 37C) were recognized, and thus, this finding was not inconsistent with the progenitor cells of the gonadal somatic cells. FOXF1 and HAND1, which are markers of the lateral plate mesoderm, were found to be negative (see FIG. 37C).

### 14. Optimization of Concentration of BMP4 in Step 2 and Examination of Addition of LDN and FGF9

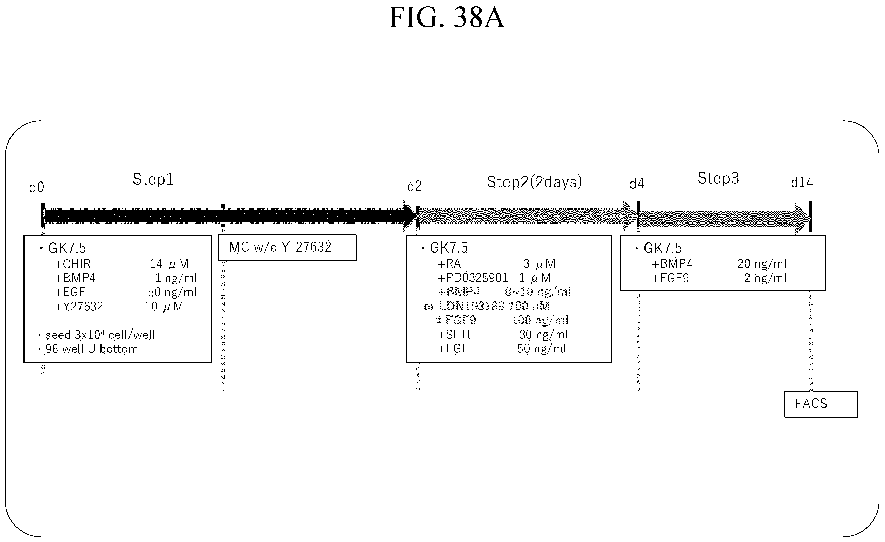

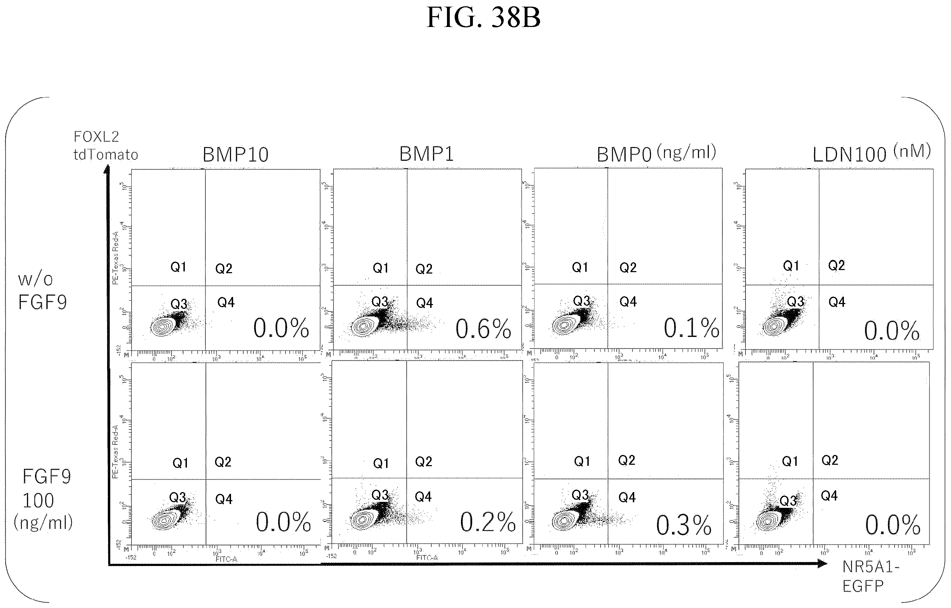

In the previous research, FGF9 and LDN were used for the induction of the intermediate mesoderm, and based on this, in step 2, the concentration of BMP4 was changed to 0, 1, and 10 ng/mL to examine whether the induction efficiency of NR5A1-positive cells (see FIG. 38A) was improved by the addition of LDN (100 nM) and the addition of FGF9 (100 ng/mL). An NGFT No. 69 strain was used as a cell strain. The induction was performed for a period in step 1 of 2 days and in step 2 of 2 days, and evaluated by FACS analysis on day 14 of induction (refer to FIG. 38B).

As shown in FIG. 38B, none of the conditions improved the induction efficiency of the NR5A1-positive cells.

### 15. Optimization of Number of Cells and Culture Plate in Step 1

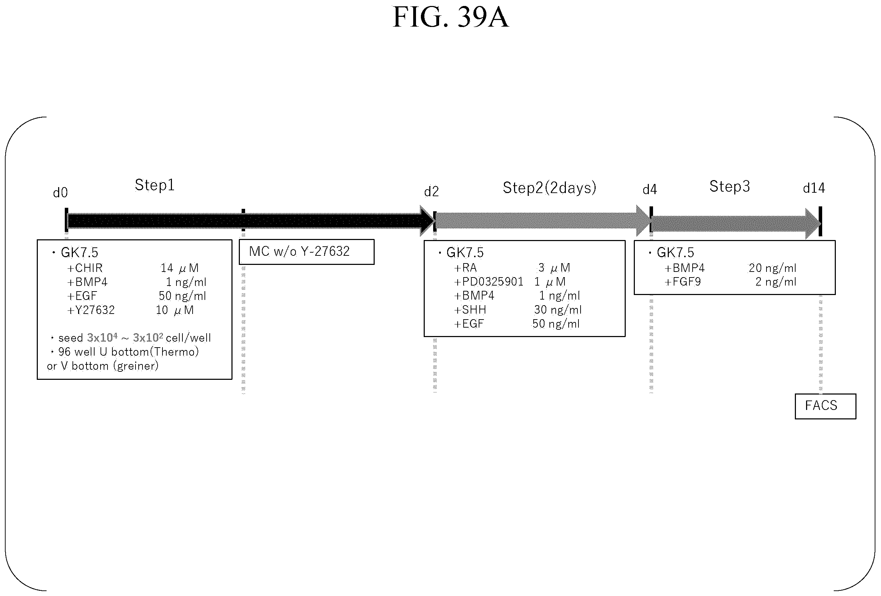

Next, it was examined whether the induction efficiency changed due to the number of cells and the culture plate (96-well V-bottom) (see FIG. 39A). Additionally, the number of cells to be seeded was also examined. The periods of both step 1 and step 2 were set to 2 days, and step 1 was performed at a concentration of CHIR of 14 µM and a concentration of BMP4 of 1 ng/mL. FACS analysis was performed on day 14 and day 21 of induction, and the states of the embryoid bodies were observed on day 14 of induction. An NGFT No. 69 strain was used as a cell strain. Furthermore, it was difficult to culture 300 cells.

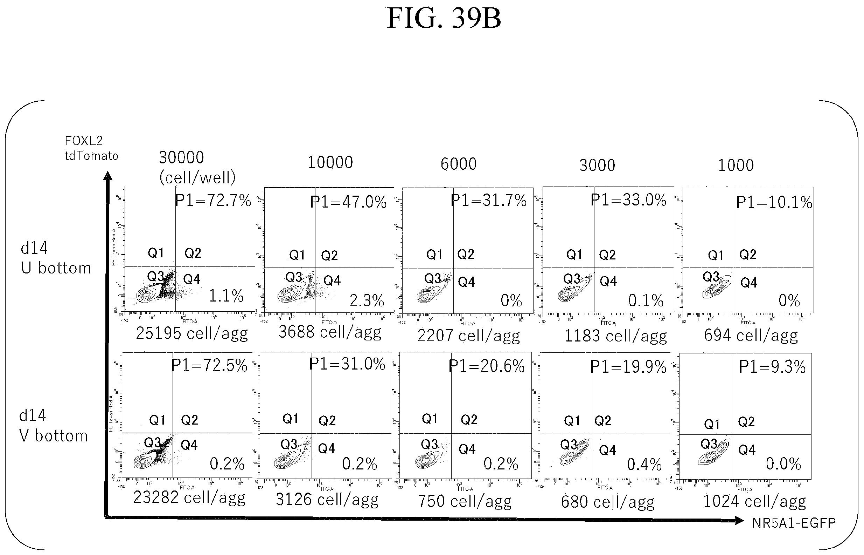

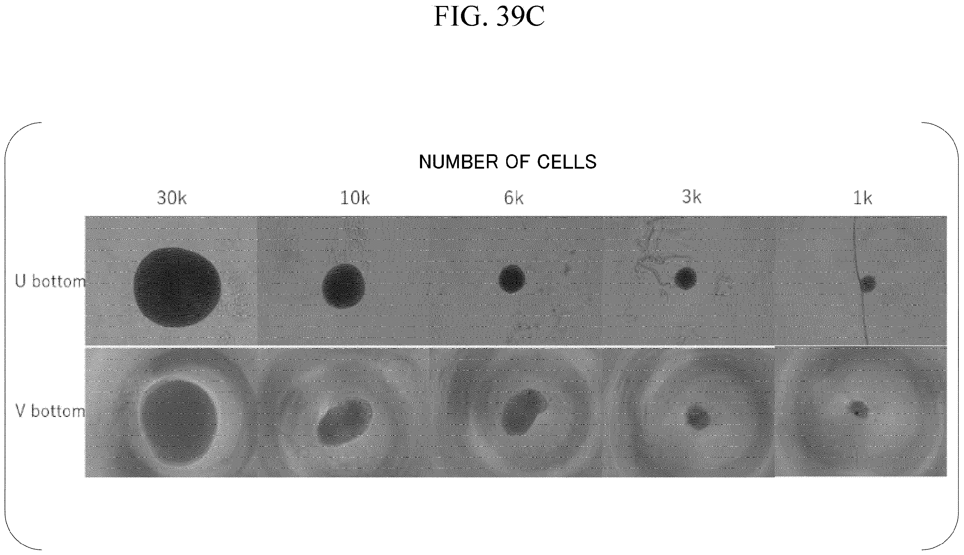

There was little induction as a whole, but no improvement was recognized by making the V-bottom (see FIG. 39B), and from the viewpoints of the number of living cells (the number of cells at a P1 gate) and the appearance of NR5A1-positive cells, 30,000 cells was considered appropriate for U-bottom (see FIG. 39B and FIG. 39C). In FIG. 39B, the number of cells at the P1 gate was calculated by "(Total number of cells) × (Proportion of P1)", and for example, the number of cells at the P1 gate in a case where the number of cells was 30,000 at the U bottom was calculated by 25,195 × 0.727.

### 16. Concentration of bFGF, Concentration of BMP4, and Culture Period of step 1, and Optimization of Culture Period of Step 1

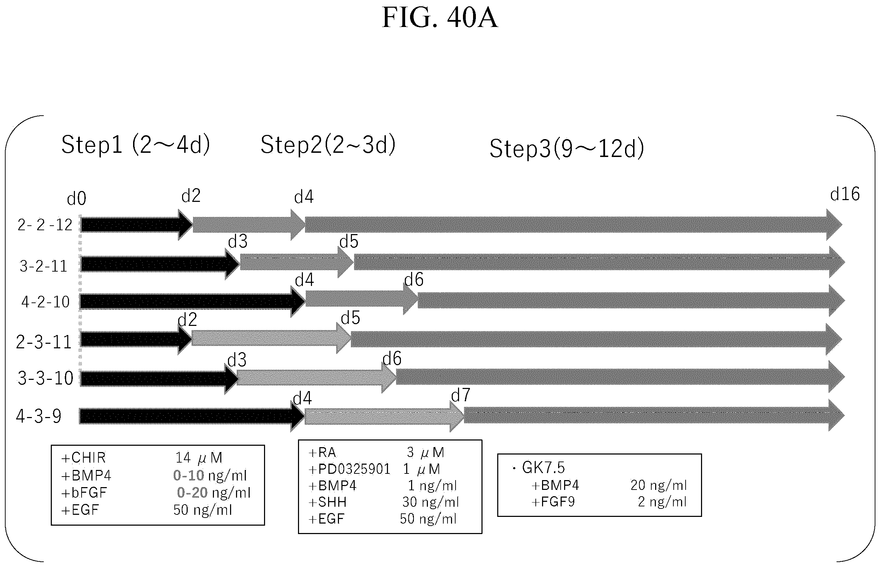

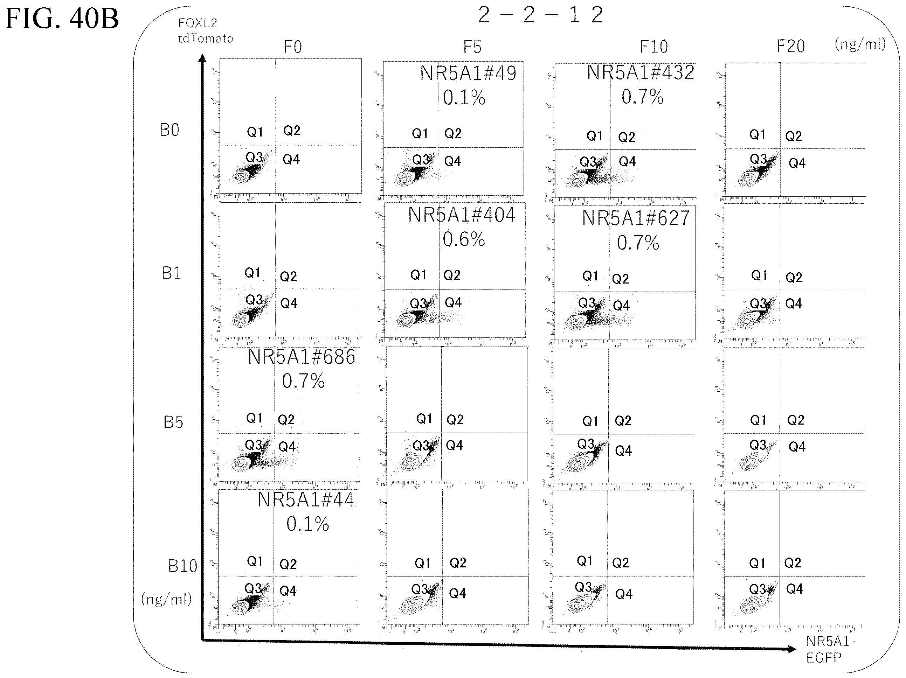

From the results so far, it was considered that in a case where the period of step 1 was 2 days and the period of step 2 was 2 days, the induction efficiency could not be expected to be further improved. Therefore, the conditions in which the period of step 1 was set to 2, 3, and 4 days, the concentration of BMP4 was set to 0, 1, 5, and 10 ng/mL, the concentration of bFGF was set to 0, 5, 10, and 20 ng/mL, and the periods of step 2 of 2 days and 3 days were examined (see FIG. 40A), and evaluation was performed by FACS analysis on day 16 of induction (see FIG. 40B to FIG. 40G). In FIG. 40B, B represents BMP4 and F represents bFGF. An NGFT No. 69 strain was used as a cell strain. Hereinafter, since the conditions are complex, the periods of step 1, step 2, and step 3 will be described as 2-2-10 (days). Furthermore, thereafter, Y-27632 was added at 10 µM on day 1 of step 1 unless otherwise specified.

As shown in FIG. 40B, in a case where the periods of step 1, step 2, and step 3 were 2-2-12 (days), the same NR5A1-positive cells as before were recognized. There was no significant change by the addition of bFGF.

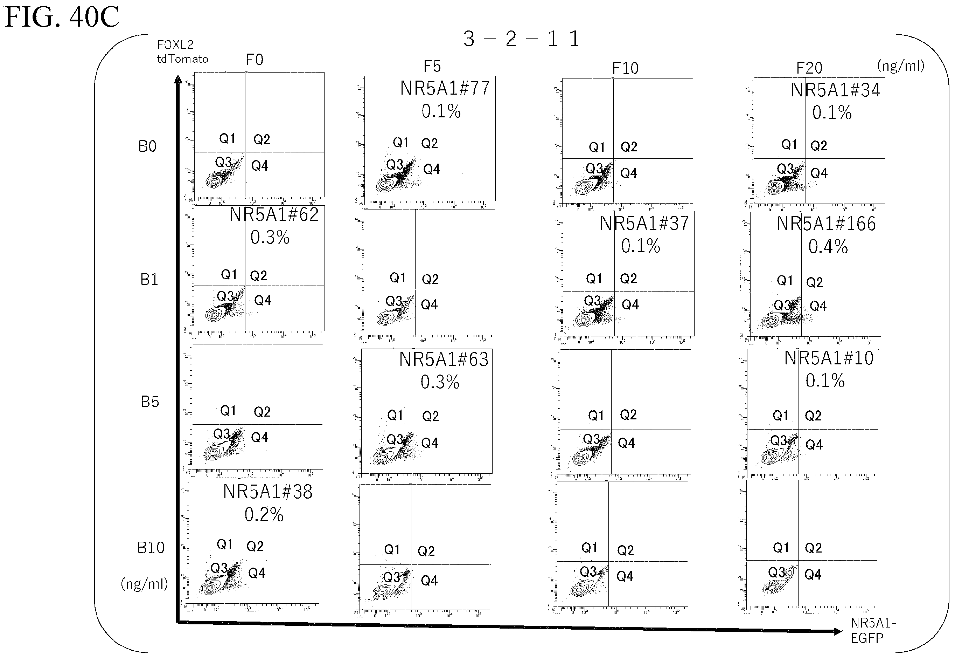

As shown in FIG. 40C, there was no significant change in a case where the periods of step 1, step 2, and step 3 were 3-2-11 (days).

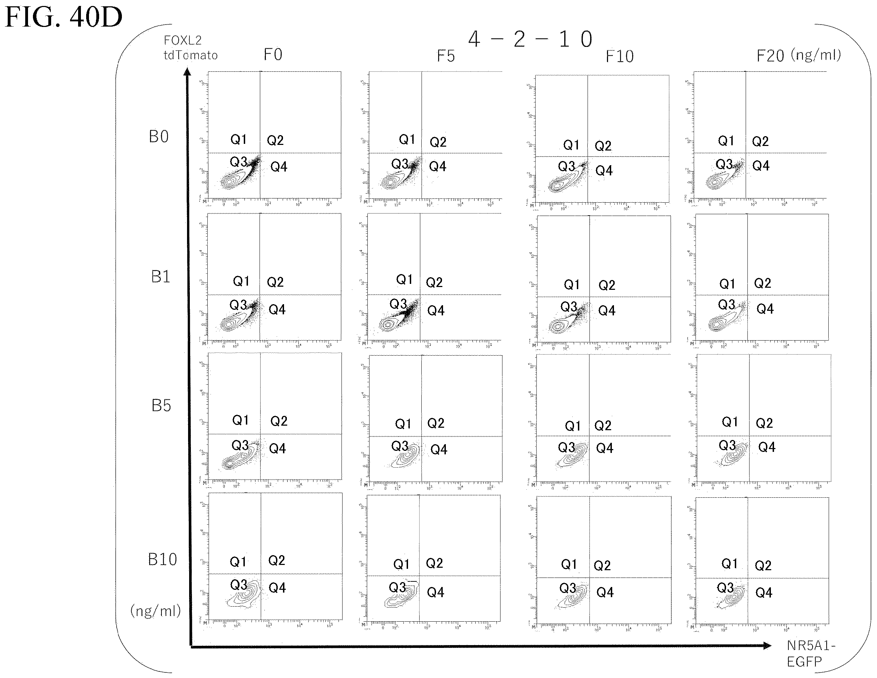

As shown in FIG. 40D, in a case where the periods of step 1, step 2, and step 3 were 4-2-10 (days), reduction in living cells was recognized in a case where the period of step 1 was 4 days or longer.

As shown in FIG. 40E, no significant change was observed in a case where the periods of step 1, step 2, and step 3 were 2-3-11 (days).

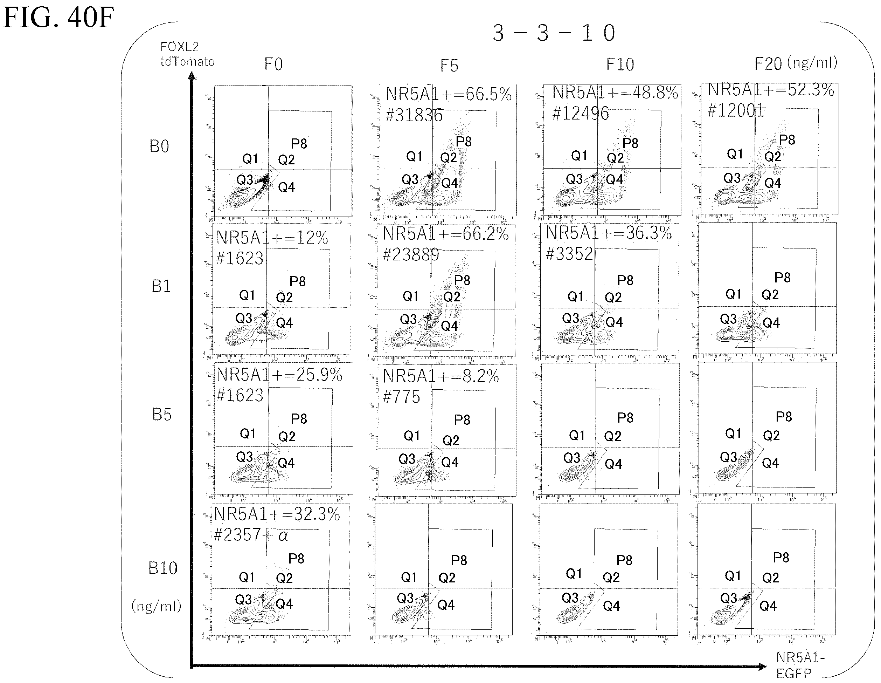

As shown in FIG. 40F, in a case where the periods of step 1, step 2, and step 3 were 3-3-10 (days), a remarkable improvement of the NRSA1-positive cell induction efficiency was recognized by the addition of bFGF in step 1. The number and the proportion of the positive cells were maximal at a concentration of BMP4 of 0 ng/mL and at the concentration of bFGF of 5 ng/mL.

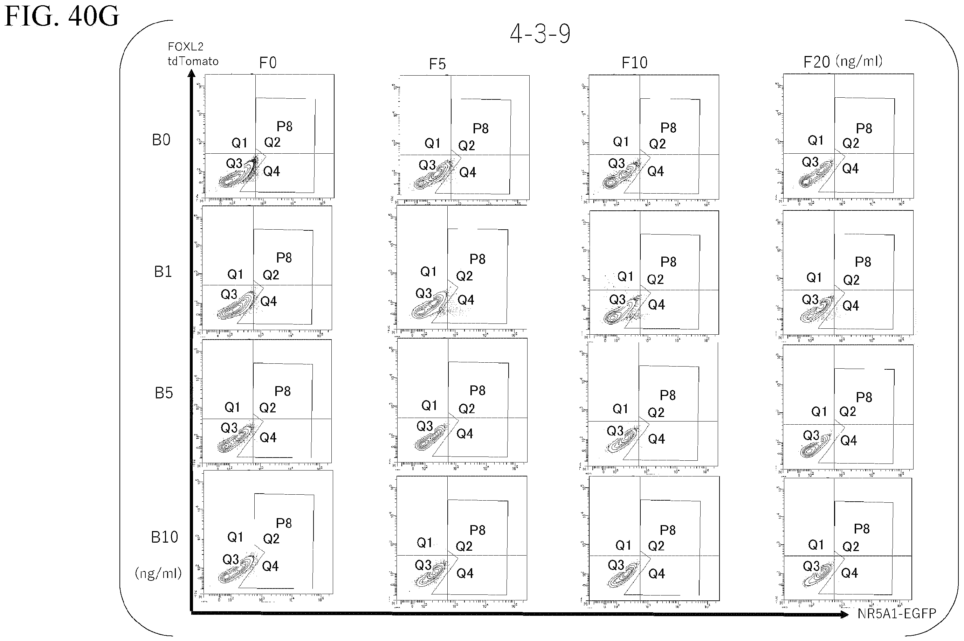

As shown in FIG. 40G, in a case where the periods of step 1, step 2, and step 3 were 4-3-9 (days), reduction of living cells was recognized in a case where the period of step 1 was set to 4 days.

From these results, it was considered that among the 96 culture conditions tested, only four patterns of culture conditions were very effective, and the culture condition in which the periods of step 1, step 2, and step 3 were 3-3-10 days, BMP4 was not added, and the concentration of bFGF was 5 ng/mL was very effective.

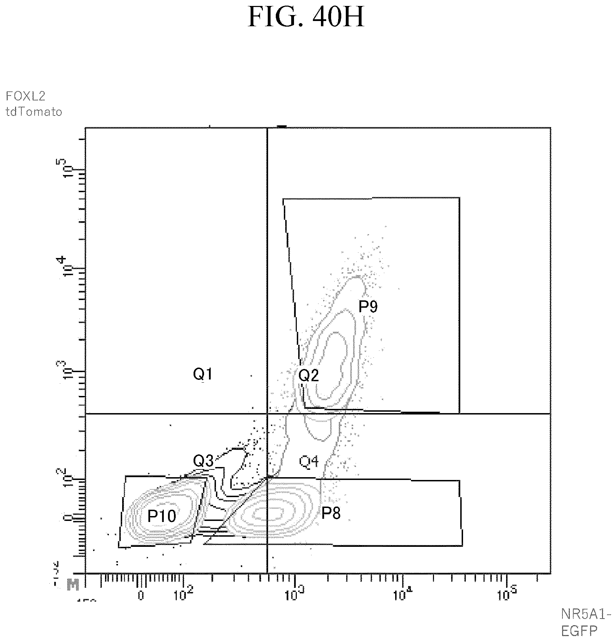

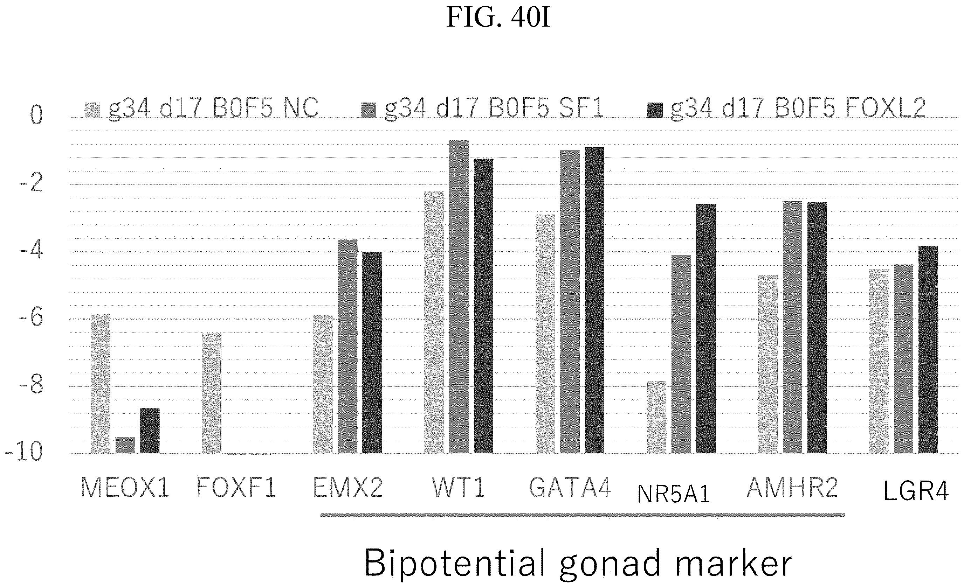

Under the conditions that the periods of step 1, step 2, and step 3 were 3-3-10 (days), BMP4 was not added, and the concentration of bFGF was 5 ng/mL, step 3 was added for another day (day 17 of induction), and NR5A1 -negative (P10 in FIG. 40H), NR5A1-positive and FOXL2-negative (P8 in FIG. 40H), and NRSA1-positive and FOXL2-positive (P9 in FIG. 40H) were sorted by FACS analysis, and qPCR was performed. The results are shown in FIGS. 40I to 40K. In FIG. 40I to FIG. 40K, "NC" represents NR5AI-negative cells, "SF1" represents NR5A1-positive and FOXL2-negative cells, and "FOXL2" represents NR5A1-positive and FOXL2-positive cells.

As shown in FIGS. 40I to 40K, NR5A1-positive and FOXL2-positive cells were FOXF1-negative and WT1-, GATA4-, NR5A1 -, and AMHR2-positive, and a gene expression pattern consistent with gonadal cells was recognized. The FOXL2-positive cells were RSPO1-, FOXL2-, WNT6-, and KITL-positive, and findings consistent with pregranulosa cells were recognized. The NR5A1-positive cells were WNT5a-positive, recognized to express PDGFRα and TCF21, and were gonadal somatic cells and progenitor cells thereof. However, at the time, it was not possible to determine whether the cells were differentiated into stromal cells or granulosa cells.

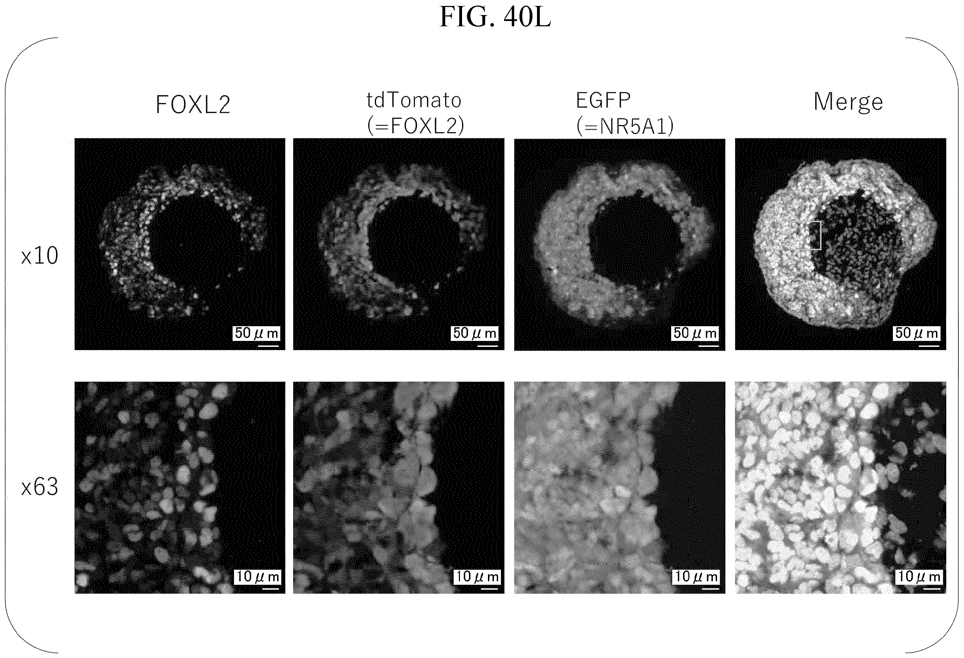

In addition, the embryoid bodies under the conditions of a concentration of BMP4 of 0 ng/mL and bFGF of 10 ng/mL were immunostained on day 17 of induction (see FIG. 40L, the scale bar is 50 µm for 10x and 10 µm for 63x).

As shown in FIG. 40L, it was confirmed that FOXL2 is reported by tdTomato, and it was confirmed that the marker of the pregranulosa cell was expressed at the protein level. In addition, it was also found that the gonadal somatic cells are generated mainly in the outer periphery.

### 17. Addition of Activin A in Step 1 and Examination of Period of Step 1

Since the condition in which bFGF was added in step 1 and BMP4 was not added was the best, an experiment on whether the addition of Activin A after day 1 of step 1 further improves the system was performed (see FIG. 41A). In addition, conditions for the period of step 1 were examined every half day from 2.5 to 3.5 days. An NGFT No. 69 strain was used as a cell strain. FACS analysis was performed on day 16 and day 28 of induction.

As shown in FIG. 41B, no improvement was observed due to the addition of Activin A, and it was considered that a period of 3 days was particularly favorable as the period of step 1. In addition, it was found that on day 28 of induction, the FOXL2-positive population and the FOXL2-negative population were clearly separated.

These FOXL2-positive cells were sorted to perform qPCR (see FIG. 41C). As a result, it was considered that while somatic cells of the ovary have a sex steroid synthesis ability, the transcription of a gene group required for steroid synthesis starts in the FOXL2-positive cell.

### 18. Optimization of Period of Step 2

Next, in step 1, the concentration of BMP4 was set to 0 ng/mL, the concentration of bFGF was set to 5 ng/mL, the period of step 1 was set to 3 days, and the period of step 2 was changed to 3, 4, 5, and 6 days to perform the induction until day 16 (see FIG. 42A), and evaluation was performed by FACS analysis (see FIG. 42B). An NGFT No. 69 strain was used as a cell strain.

As shown in FIG. 42B, the induction efficiency was not so good, but the extension of the period of step 2 did not improve the induction efficiency.

### 19. Optimization of PD032901 Concentration in Step 2

PD032901 is an MEK inhibitor. Thus, addition was performed at a concentration of PD0325901 of 3, 1, 0.3, and 0 µM, or at a concentration of FGF9 of 20 ng/mL (see FIG. 43A) to capture observation images of the embryoid bodies on day 6 of induction (see FIG. 43B) and to evaluate the induction efficiency by FACS analysis (see FIG. 43C) on day 16 of induction. An NGFT No. 69 strain was used as a cell strain.

As shown in FIG. 43B, the size at the end of step 2 was improved as the amount of PD0325901 was reduced and in a case where FGF9 was added.

In addition, as shown in FIG. 43C, the concentration of PD0325901 of 1 µM was considered to be optimum in the same manner as before.

### 20. Optimization of Concentration of BMP4 and Concentration of SHH in Step 2

Next, the concentration of BMP4 and concentration of SHH in step 2 were changed. The conditions were changed so that the concentrations of BMP4 were 0, 1, 5, and 10 ng/mL and the concentrations of SHH were 0, 30, 60, and 100 ng/mL (see FIG. 44A). An NGFT No. 69 strain was used as a cell strain.

In a case where the induction efficiency was evaluated by FACS analysis on day 16 of induction, a concentration of BMP4 of 1 ng/mL and a concentration of SHH of 100 ng/mL were the best (see FIG. 44B). In addition, the number of FOXL2-positive cells and NR5A I-positive cells increased, and the number of embryoid bodies also increased, depending on the concentration of SHH (see FIG. 44B).

### 21. Optimization of Concentration of RA and Concentration of SHH in Step 2

Next, the conditions of the concentration of RA and the concentration of SHH in step 2 were examined. In addition, it was examined whether SHH could be replaced with SAG, which is a small molecule. The conditions were changed so that the concentrations of RA were 0, 0.3, 1, 3, and 10 µM, the concentrations of SHH were 30, 100, and 200 ng/mL, or the SAG was at 100 nM (see FIG. 45A). The induction was performed with the number of cells of 23,333 cells/well. An NGFT No. 69 strain was used as a cell strain.

In a case where the induction efficiency was evaluated by FACS analysis on day 16 of induction, the induction efficiency was low, but in a case where the concentration of RA was 3 µM or more, the induction efficiency for NR5A1-positive cells was particularly excellent. In addition, a possibility was suggested that SHH can be replaced with SAG.

Based on the results above, at the time of this experiment, it was considered that in step 2, a concentration of RA of 3 µM, a concentration of PD032901 of 1 µM, a concentration of BMP4 of 1 ng/mL, and a concentration of EGF of 50 ng/mL were the best. In addition, sufficient induction could be performed even at a concentration of SHH of 30 ng/mL, but there was a tendency that the induction efficiency was improved as the amount of SHH increased.

### 22. Examination of MEF Removal in Gelatin-Coated Dish

In order to reduce the bringing-in of MEF as much as possible for culturing on a feeder, ES cells were seeded on a gelatin-coated dish, and the supernatant was recovered 30 minutes later to start induction in an attempt to reduce the bringing-in of MEF. An NGFT No. 69 strain was used as a cell strain. Induction was performed until day 16 from those induced in the usual manner and those treated with a gelatin-coated dish (see FIG. 46A), and evaluated by FACS analysis (see FIG. 46B).

As shown in FIG. 46B, an improvement of the induction efficiency by MEF removal was not recognized.

### 23. Examination of MEF Removal by Collagenase Type 4

In order to suppress the bringing-in of MEF as much as possible for culturing on a feeder, ES cells were recovered using a collagenase type 4 in an attempt to reduce the bringing-in of MEF. Those in which MEF removal was attempted by normal induction and those in which MEF removal was attempted by ES cell collection by collagenase type 4 were compared (see FIG. 47A). Since the embryoid bodies tended to be larger by MEF removal, the examination was performed by changing the conditions in which the number of cells per well was 30,000, 20,000, and 10,000. An NGFT No. 69 strain was used as a cell strain.

In a case where the induction efficiency was evaluated by FACS analysis on day 16 of induction, the induction efficiency was better by not performing the MEF removal treatment (see FIG. 47B).

### 24. Optimization of Concentration of BMP4 and Concentration of FGF9 in Step 3

As an examination on the conditions for step 3, the concentration of BMP4 and the concentration of FGF9 were examined. Induction was performed at a concentration of BMP4 of 0, 1, 20, and 50 ng/mL and a concentration of FGF9 of 0, 2, 10, and 20 ng/mL (see FIG. 48A), and evaluation was made by FACS analysis on day 16 (FIG. 48B) see). An NGFT No. 69 strain was used as a cell strain.

As shown in FIG. 48B, the conditions that most induced NR5A1-positive cells were a concentration of BMP4 of 1 ng/mL and a concentration of FGF9 of 10 ng/mL.

Since BMP4 induces GATA4 essential in gonadal progenitor cells, it was expected that a concentration of BMP4 of 50 ng/mL would result in better induction, but the results were completely opposite. FGF9 is a well-known factor in testicular action, and the result that it was effective at increasing the concentration of FGF9 was also an interesting finding.

Although the induction efficiency is not the best, the improvement of the dissociation efficiency by changing the cell dissociation reagent from trypsin to Accumax and increasing the size of the reaction system is also considered as one of factors to make the number of the obtained cells the largest in Examples so far.

### 25. Introduction of Four-Step Induction Method, and Verification of Necessity of BMP4, SHH, and FGF9 in Step 3

From the results so far, there is a possibility that SHH contributes to induction efficiency, and since it is considered that BMP4 contributes to the expression of GATA4, which is a marker of the genital ridge, and FGF9 is used for induction of the intermediate mesoderm in the kidney region, those cytokines were used after step 2 to verify the effects. An NGFT No. 69 strain was used as a cell strain. These cytokines were added from day 6 of induction to about day 10 of induction, on which the expression of NRSA1 was considered to be expressed (the period of step 3), and further, after day 10 of induction, the concentration of BMP4 and the concentration of FGF9 were verified (see FIG. 49A).

In a case where the induction efficiency was evaluated by FACS analysis on day 16 of induction, it was unnecessary to add BMP4 during the examination period, and rather, the induction efficiency was higher in a case where BMP4 was not added (see FIG. 49B and FIG. 49C). With regard to FGF9, the number of cells induced by the addition of 2 ng/mL increased slightly, but a constant tendency was not shown and the effect was slightly unclear (see FIG. 49B and FIG. 49C).

With regard to SHH, the induction efficiency was higher in a case where SHH was not added, under conditions other than the conditions of addition of SHH, a concentration of BMP4 of 1 ng/mL, and absence of FGF9, and in contrast to the verifications so far, it was considered that there is a possibility that SHH acts in an inhibitory manner (see FIG. 49B and FIG. 49C).

### 26. Verification of Necessity of SHH in Step 2 and Necessity of FGF9 in Step 4

Since a possibility was suggested that SHH acts in an inhibitory manner, the presence or absence of SHH in step 2 and the possibility of replacement with SAG, which is a small molecule, were verified. In addition, since the effect of FGF9 was unclear in "25.", it was verified what would occur in a case where PD0325901 was added in step 3. In addition, in order to confirm that BMP4 is not essential after step 3, a total of 20 conditions, that is, as shown in Fig. 50 A, 5 conditions in which the concentrations of SHH were 0 and 30 ng/mL, or the concentrations of SAG were 10, 30, and 100 ng/mL in step 2, and for these 5 conditions, after step 3, only FGF9 (concentration: 2 ng/mL) was added, BMP4 (concentration: 20 ng/mL) and FGF9 (concentration: 2 ng/mL) were added, PD0325901 (concentration: 1 µM) was added for 4 days, and then nothing was added, and PD0325901 (concentration: 1 µM) and BMP4 (concentration: 20 ng/mL) were added for 4 days, and then only BMP4 (concentration: 20 ng/mL) was added was performed (see FIG. 50A). An NGFT No. 69 strain was used as a cell strain.

In a case where the induction efficiency was evaluated by FACS analysis on day 16 of induction, it was found that SHH was unnecessary in step 2, and rather, the induction efficiency was higher in a case where SHH was not added (see FIG. 50B). With regard to FGF9 as well, it was found that induction efficiency was higher in a case where MEK downstream of FGF was inhibited with PD0325901 (see FIG. 50B).

### 27. Verification of Factors Useful in Step 3

It was verified whether or not there was a factor useful in a case of being used in step 3, other than PD0325901 among the cytokines of step 2. In step 3, a total of 16 patterns of addition or no addition of all of RA (concentration: 3 µM), PD0325901 (concentration: 1 µM), BMP4 (concentration: 1 ng/mL), and SHH (concentration: 30 ng/mL) to EGF were prepared, and as controls, patterns in which nothing was added after step 3, and BMP4 (concentration: 20 ng/mL) and FGF9 (concentration: 2 ng/mL) had been added in step 3 were prepared. An NGFT No. 69 strain was used as a cell strain. FACS plots on day 16 of induction in two controls, that is, a control (CK-) in which nothing was added after step 3 and a control in which after step 3, BMP4 (concentration: 20 ng/mL) and FGF9 (concentration: 2 ng/mL) with other conditions were used are shown in FIG. 51B.

As shown in FIG. 51B, the induction efficiency was extremely high in the pattern in which none of the cytokines were added after step 3.

It was verified what had occurred in a case where EGF (E) or one other cytokine was added in step 3, as compared with the control in which there was no cytokine (CK-) after step 3 (see FIG. 51C).

By the addition of BMP4 (B), the number of induced cells was large, but the induction efficiency was reduced and the effect was unclear (see FIG. 51C). In addition, as in the previous results, the addition of SHH (S) acted in a disadvantageous manner, and the addition of PD0325901 (P) decreased the number of cells but further increased the induction efficiency, and thus, a possibility was suggested that the addition of PD0325901 (P) is useful (see FIG. 51C). The addition of 3 µM of retinoic acid (R) significantly reduced the induction efficiency (see FIG. 51C).

The effect of the addition of each cytokine was verified in detail to see what would occur in a case of such addition (see FIG. 51D to FIG. 51G).

In a case where BMP4 was added to each condition, the number of induced cells decreased only in a case where BMP4 was added to EGF + RA (ER + B) (see FIG. 51D). A possibility was suggested that the addition of a small amount of BMP4 increases the number of induced cells.

In "25." above, since BMP4 (concentration: 1 ng/mL) was added until day 16 of induction, it was considered that there is a possibility that no addition of BMP4 after day 10 of induction is good.

In a case where SHH was added, the number of induced cells under all conditions decreased, and thus, it was suggested that SHH acts in an inhibitory manner (see FIG. 51E).

With regard to the addition of PD03, the tendency was not constant, and it was thought that the number of cells induced by the inhibition of MEK decreased but the induction efficiency itself was improved (see FIG. 51F).

The addition of RA consistently reduced the number of induced cells.

Based on the results above, it was considered to use PD0325901 and BMP4 (concentration: 1 ng/mL) as candidate factors in step 3.

### 28. Gene Expression of SF1- and FOXL2-Positive Cells Induced by Three-Step Method

From the results of "27.", it was found that the FOXL2-positive cells were efficiently induced even without adding any cytokine in step 3. RNAseq data in a case where this induction was performed was measured. On day 16 of induction, FOXL2-negative cells (P7 gate, abbreviated as "d16SF1+" hereinafter) and FOXL2-positive cells (P8 gate, and hereinafter abbreviated as "d16FOXL2+") that strongly express SF1 were sorted by FACS analysis (FIG. 52B), and subjected to RNAseq (see FIG. 52C). In the heat map in FIG. 52C, the left side (black) of "Color Key" indicates that no gene expression was observed and the right side (white) indicates that the gene expression was high.

All of the cells strongly expressed the marker gene of the genital ridge, and even in the SF1 single-positive cells, the expression of WNT6, which is a marker of the granulosa cell lineage, was recognized (see FIG. 52C).

In addition, the markers of the stromal cell, TCF21 and MAFB, decreased in the FOXL2-positive cells (see FIG. 52C). Also, since the expression of SOX11 and NR2F1, which are markers of somatic cells of the relatively immature genital ridge, is recognized (see FIG. 52C), it was considered that the process of differentiation from the genital ridge epithelium to granulosa cells was observed.

### 29. Verification of Addition Period of PD0325901 in Step 3

From the results so far, it was suggested that there is a possibility that the addition of PD0325901 decreases the number of induced cells, but there is also a possibility that the induction efficiency is improved.

It was verified what would occur in a case where the period of PD0325901 is shortened, using an NG-Puro strain as a cell strain. The verification was performed under the conditions of no addition of PD03, addition of PD03 for 48 hours, and addition of PD03 for 96 hours in step 3 (see FIG. 53A).

As a result, a possibility was suggested that the optimum addition period of PD03 in step 3 is 48 hours (see FIG. 53B).

### 30. Verification of Effect Given by PD0325901 in Step 3

In order to verify what kind of effect the addition of PD03 in step 3 has on the induction, verification was performed using WTGBNG No. 1 as a cell strain. In addition, in order to confirm that SHH was unnecessary for the induction system, a combination of addition or no addition of SHH in step 2 and step 3, a combination of addition or no addition of PD0325901 in step 3, was verified (see FIG. 54A).

FACS plots of the conditions in which PD03 was not added in step 3 are shown in FIG. 54B. In FIG. 54B, in the FACS plots in the left four columns, the horizontal axis indicates GATA4-mTagBFP2 and the vertical axis indicates WT1-tdTomato, in the order of d8, d10, d12, and d16 from the left. In the rightmost column, the horizontal axis indicates SF1-EGFP and the vertical axis indicates APC, in which the FACS plots for d16 are developed. The top row shows the addition (+) of SHH in step 2 and the addition (+) of SHH in step 3, the second row shows the addition (+) of SHH in step 2 and no addition (-) of SHH in step 3, the third row shows no addition (-) of SHH in step 2 and addition (+) of SHH in step 3, and the fourth row shows no addition (-) of SHH for the entire period.

As a result, SF1-positive cells were induced even without addition of SHH (see FIG. 54B). In addition, it was found that at the time point of d8, almost all cells were WT1-positive, and some cells had already started to express GATA4 (see FIG. 54B) and were differentiated into the genital ridge epithelium.

FACS plots of the conditions in which PD03 was added in step 3 are shown in FIG. 54C. The vertical axis, the horizontal axis, and the conditions in each row in FIG. 54C are the same as those in FIG. 54B.

In FIG. 54C, as compared to the FACS plots of FIG. 54B, the proportion of cells with a low expression of WT1 was reduced overall at the time point of d10, and almost all of the cells were GATA4-positive at d16. It should be noted that the number of the induced cells themselves was reduced, and the induction efficiency was the highest by addition of PD0325901 particularly under the conditions without addition of SHH.

Based on these results, there is a possibility that the yield of SF1-positive cells is reduced, but a possibility that no addition of SHH, and addition of PD0325901 in step 3 contribute to the stabilization of the induction system is considered.

Moreover, the fluorescence of the cell mass in a case where SHH was not added in step 2 and PD0325901 was added in step 3 was observed with a microscope (see FIG. 54D). As shown in FIG. 54D, it was confirmed that the expression of WT1 was generally recognized on day 8, the expression of GATA4 and SF1 gradually started from the outer peripheral portion of the cell mass, and the genital ridge was formed. This state was similar to the process in which the genital ridge epithelium was thickened and thus, the genital ridge was formed.

### 31. Optimization of Concentration of RA in Step 3

Since it was found that in a case where the concentration of RA of 3 µM was continued, the induction efficiency decreased significantly, thus, it was verified what occurred in a case where a small amount of RA was added in step 3, and conversely, it was verified what occurred in a case where the RA signal was inhibited. A WTGBNG No. 1 strain was used as a cell strain. In addition, the addition period of RA was set to 48 hours in accordance with PD03, the concentrations were 3, 1, 0.1, and 0 µM, or BMS493 (concentration: 1 µM), which is an inverse antagonist of RA signal, was used (see FIG. 55A). Evaluation was performed by FACS analysis on days 8, 10, 12, 14, and 16 from induction (see FIG. 55B and FIG. 55C).

In FACS plots in FIG. 55B, the vertical axis indicates the gene expression of WT1-tdTomato, the horizontal axis indicates the gene expression of GATA4-mTagBFP2, and each row shows the number of induction days. As shown in FIG. 55B, after step 3 was completed, almost all cells were positive for WT1, and it was thus considered that the intermediate mesoderm was efficiently induced under any conditions. In a case where the RA signal was inhibited by BMS493, the expression level of WT1 was slightly decreased. In addition, the expression of GATA4 was recognized in some cells on day 8 of induction, and it was thus suggested that the cells are gradually differentiated into the genital ridge epithelium. Under the condition in which RA was added, there was no significant difference between the expressions of WT1 and GATA4.

The FACS plots in FIG. 55C are developed with APC on the vertical axis and SF1-EGFP on the horizontal axis. Since SF1 was not expressed on day 8 of induction, data on days 10, 12, 14, and 16 of induction are presented. As shown in FIG. 55C, on day 16 of induction, SF1-positive cells were most efficiently induced in a case where RA was not added. In addition, in a case where BMS493 was added, although the induction efficiency of SF1-positive cells was poor, an interesting phenomenon in which the expression level of SF1-EGFP slightly increased was observed. There is also a report that WT1 acts suppressively on the expression of SF1, and a possibility was suggested that the addition of BMS493 can have this observed effect.

### 32. Confirmation of Earlier WT1 Expression

It was confirmed what kind of cells were induced at a stage after completion of step 2. The detailed protocol is shown in FIG. 56A. A WTNG No. 43 strain was used as a cell strain. Evaluation was performed by FACS analysis on days 6, 8, 10, and 14 of induction (see FIG. 56B).

So far, it has been found that almost all cells express OSR1, which is a marker for the intermediate mesoderm or the lateral plate mesoderm, at the end of step 2, and as shown in FIG. 56B, it was found that WT1-positive cells had already appeared on day 6 of induction.

### 33. Consideration of Reason Why WT1-Positive Cells Are Differentiated into Genital Ridge

Gene expression in WT1-positive cells was measured. The detailed protocol is shown in FIG. 57A. A WTNG No. 43 strain was used as a cell strain. WT1-positive cells on day 8 of induction, and WT1 single-positive cells and WT1 and SF1 co-positive cells on day 10 of induction were each sorted by FACS analysis (see FIG. 57B), and confirmation of the gene expression by qPCR was performed (see FIG. 57C).

In FIG. 57C, the bar graph shows, from the left side, the WT1-positive cells on day 8 of induction, the WT1 single-positive cells on day 10 of induction, and the WT1 and SF1 co-positive cells on day 10 of induction. The vertical axis of the graph shows dCT values for PPIA/GAPDH. As shown in FIG. 57C, it was found that the OSR1 expression had already been considerably reduced on day 8 of induction, and in the gene expression of any of cells, WT1 and GATA4, which are markers of the genital ridge, are well-expressed. On the other hand, WT1-positive and SF1-negative cells on days 8 to 10 of induction expressed BMP4, which promotes the expression of GATA4 (GATA4 is downstream of BMP4), and thus, a possibility was suggested of differentiation into the genital ridge. In addition, the gene expression of FOXF1 was also decreased in the SF1-positive cells, and a possibility was suggested that these cells were also affected by the decreased expression of BMP4.

### 34. Detection of Stromal Cells

The ovary consists of FOXL2-positive granulosa cells and PDGFRα-positive stromal cells. In the mouse ovarian somatic cell induction method of Yoshino, et al., SF1-positive cells are differentiated into a FOXL2-positive granulosa cell line, and SF1- and PDGFRα-positive stromal cells. In order to verify this fact also with the induction method for monkeys, FACS was periodically performed from around day 12 of induction, which is the time when FOXL2 started to be expressed, and monitoring of the expression of SF1, PDGFRα, and FOXL2 was performed. The detailed protocol is shown in FIG. 58A. NGFT No. 69 strain was used as a cell strain. FACS plots are shown in FIG. 58B. In FIG. 58B, the upper row shows the FACS plots on days 12, 14, 16, 18, and 22 of induction, with FOXL2-tdTomato on the vertical axis and SF1-EGFP on the horizontal axis. The lower left part shows the FACS plots on days 12 and 14 of induction, developed with PDGFRα-APC on the vertical axis and SF1-EGFP on the horizontal axis, and the lower right part shows the FACS plots of SF1-EGFP-positive cells on days 16, 18, and 22 of induction, developed by PDGFRα-APC on the vertical axis and FOXL2-tdTomato on the horizontal axis.

As shown in FIG. 58B, some of the cells with a relatively low value of SF1-EGFP expressed PDGFRα (arrow) on day 14 of induction, and these cells were FOXL2-negative from the expression level of SF1. Therefore, the cells were considered to be stromal cells. However, it was considered that there were few clear stromal cell-like signals in other FACS plots, and further examination of conditions was required for induction of stromal cells.

### 35. Verification of Necessity of EGF in Step 1 and Step 2

The usefulness of EGF in development of the genital ridge has been unclear, and the function of EGF and how it acts have not been well-known. Therefore, with regard to the further simplification of the induction system and cost reduction, the induction was performed under the conditions of no addition of EGF in step 1 and step 2. The detailed protocol is shown in FIG. 59A. NGFT No. 69 strain was used as a cell strain. FACS analysis was performed on day 16 of induction.

As a result, the effect of EGF was still unclear and was considered not essential for induction (see FIG. 59B).

### 36. Summary

Based on the results above, a method for efficiently and reproducibly inducing cells of a FOXL2-positive granulosa cell lineage from cynomolgus monkey ES cells was established (see FIG. 60A). In addition, the induction method can be used with a simple protocol using relatively inexpensive small molecules or the like, except for a small amount of bFGF and BMP4.

In the induction process, gastrulation found in the expression of TBXT, expression of OSR1 and WT1, which is differentiation into the intermediate mesoderm, expression of GATA4 and SF1, which is differentiation into the genital ridge epithelium, and expression of FOXL2, which is differentiation into the granulosa cell lineage, were successfully induced from ES cells corresponding to the epiblast immediately after implantation, so as to imitate an induction in vivo and induction method for mice (see FIG. 60B; a protocol diagram was created, as cited from Non-Patent Document 1). In addition, the appearance of cells considered to be progenitor cells of stromal cells that later play an important role in sex steroid production, although they were extremely small in number, was also recognized. It is considered that with this protocol, the same degree of induction of a granulosa cell line and a stromal cell line can be performed in the mouse induction system, and cells of a FOXL2-positive granulosa cell line can be selectively induced in the monkey system. A method for improving the induction efficiency of stromal cells is an issue for the future.

### [Industrial Applicability]

According to the production method and the method for inducing differentiation of the present embodiment, ovarian somatic cell-like cells at any developmental stage can be efficiently obtained from primate pluripotent stem cells.

## Claims

1. A method for producing ovarian somatic cell-like cells, the production method comprising:
a step 1 of culturing primate pluripotent stem cells in a medium including a GSK3 inhibitor and a ROCK inhibitor;
a step 2 of culturing the cells from the step 1 in a medium including BMP4, retinoic acid, and an MEK inhibitor; and
a step 3 of culturing the cells from the step 2 in a base medium to obtain ovarian somatic cell-like cells.

2. The production method according to Claim 1,
wherein the culture of the step 1 is performed for 3 days or longer.

3. The production method according to Claim 1 or 2,
wherein the culture of the step 2 is performed for 3 days or longer.

4. The production method according to any one of Claims 1 to 3,
wherein the culture of the step 3 is performed for 1 week or longer.

5. The production method according to any one of Claims 1 to 4,
wherein the medium in the step 1 further includes BMP4.

6. The production method according to any one of Claims 1 to 5,
wherein the step 1 includes
a step 1-1 of culturing primate pluripotent stem cells in a medium including a GSK3 inhibitor and a ROCK inhibitor, and
a step 1-2 of culturing the cells from the step 1-1 in a medium including a GSK3 inhibitor and Activin A.

7. The production method according to any one of Claims 1 to 6,
wherein the culture of the step 1 is a plane culture.

8. The production method according to Claim 7,
wherein a container coated with a cell scaffold material is used in the plane culture.

9. The production method according to Claim 7 or 8,
wherein the medium in the step 2 further includes a ROCK inhibitor.

10. The production method according to any one of Claims 1 to 9,
wherein the medium in the step 1 further includes bFGF.

11. The production method according to any one of Claims 1 to 10,
wherein the medium in the step 2 further includes a hedgehog signaling activator.

12. The production method according to any one of Claims 1 to 11,
wherein the step 3 includes
a step 3-1 of culturing the cells from the step 2 in a medium including an MEK inhibitor, and
a step 3-2 of culturing the cells from the step 3-1 in a base medium to obtain ovarian somatic cell-like cells.

13. The production method according to any one of Claims 1 to 12,
wherein the ovarian somatic cell-like cells are embryonic ovarian somatic cell-like cells.

14. The production method according to any one of Claims 1 to 13,
wherein the primate pluripotent stem cells are derived from cynomolgus monkey or human.

15. A method for inducing differentiation of primate pluripotent stem cells into ovarian somatic cell-like cells, the method comprising:
inducing differentiation of primate pluripotent stem cells into ovarian somatic cell-like cells using the production method according to any one of Claims 1 to 14.
